# EUROPEAN PATENT APPLICATION

(11) **EP 3 360 956 A1**
(43) Date of publication of application: **15.08.2018**
(21) Application number: 17155699.6
(22) Date of filing: 10.02.2017
(51) Int. Cl.: C12N 1/22, C12N 15/52, C12N 9/42, C12N 15/81, C12P 7/64

(54) **MUTANT YEAST STRAIN CAPABLE OF DEGRADING CELLULOSE**

(71) Applicant: Institut National De La Recherche Agronomique, 75007 Paris (FR); INSTITUT NATIONAL DES SCIENCES APPLIQUEES DE TOULOUSE, 31077 Toulouse Cedex 4 (FR); CENTRE NATIONAL DE LA RECHERCHE SCIENTIFIQUE, 75016 Paris (FR)
(72) Inventor: GUO, Zhongpeng, 31000 TOULOUSE (FR); MARTY, Alain, 31000 TOULOUSE (FR); O'DONOHUE, Michael, 31500 TOULOUSE (FR); BOZONNET, Sophie, 31500 TOULOUSE (FR); DUQUESNE, Sophie, 31300 TOULOUSE (FR); NICAUD, Jean-Marc, 78190 TRAPPES (FR)
(74) Representative: Gevers & Orès

(57) **Abstract**

The present invention relates to mutant yeast strains, such as *Yarrowia lipolytica* strains, capable of growing on cellulose as carbon source and means for obtaining such mutant strains.

SEQUENCE LISTING

<110>  INSTITUT NATIONAL DE LA RECHERCHE AGRONOMIQUE
       INSTITUT NATIONAL DES SCIENCES APPLIQUEES DE TOULOUSE
       CENTRE NATIONAL DE LA RECHERCHE SCIENTIFIQUE

<120>  MUTANT YEAST STRAIN CAPABLE OF DEGRADING CELLULOSE

<130>  F539-185/EP

<160>  213

<170>  PatentIn version 3.5

<210>  1
<211>  828
<212>  PRT
<213>  Yarrowia lipolytica

<400>  1

Ser Pro Asp Trp Tyr Pro Thr Pro Glu Ile Gly Ser Ile Thr Ser Asp
1               5                   10                  15

Trp Ala Asp Ala Leu Gly Glu Ser Met Asp Ile Leu Ala Gln Leu Thr
            20                  25                  30

Leu Pro Glu Lys Val Asn Ile Thr Thr Gly Thr Gly Trp Arg Gly Gly
        35                  40                  45

Gln Cys Val Gly Asn Thr Gly Ala Val Pro Arg Leu Gly Ile Lys Gly
    50                  55                  60

Leu Cys Leu Gln Asp Gly Pro Leu Gly Val Arg Phe Ala Asp Phe Val
65                  70                  75                  80

Asn Val Phe Pro Cys Gln Asn Ala Met Ala Ala Thr Phe Asp Arg Ile
                85                  90                  95

Leu Val His Gln Arg Gly Thr Ala Ile Gly Arg Gln Ser Arg Leu Lys
            100                 105                 110

Gly Val Asp Val His Leu Gly Pro Val Val Gly Pro Leu Gly Arg His
        115                 120                 125

Ala Thr Gly Gly Arg Asn Trp Glu Gly Phe Ser Pro Asp Pro Tyr Leu
    130                 135                 140

Ser Gly Lys Leu Ala Phe Glu Ala Ile Leu Gly Val Gln Glu Glu Gly
145                 150                 155                 160

Val Leu Ala Thr Ile Lys His Phe Ile Gly Asn Glu Gln Glu His Tyr

                165                 170                 175

Arg Arg Ala Glu Glu Trp Arg Asp Gly Phe Gly Phe Lys Asp Leu Lys
            180                 185                 190

Asp Ala Val Ser Ser Asn Ile Glu Asp Arg Ala Leu His Glu Leu Tyr
        195                 200                 205

Met Trp Pro Phe Ala Asp Ala Val Arg Ala Asn Val Gly Ser Val Met
    210                 215                 220

Cys Ser Tyr Asn Tyr Val Asn Gly Thr Gln Ala Cys Gln Asn Ser Asp
225                 230                 235                 240

Leu Leu Asn Gly Lys Leu Lys Ser Glu Leu Gly Phe Gln Gly Phe Val
                245                 250                 255

Met Ser Asp Trp Phe Ala Gln Gly Ser Gly Val Ser Asn Ala Leu Ala
            260                 265                 270

Gly Met Asp Met Ser Met Pro Gly Asn Asp Val Asp Glu Leu Glu Thr
        275                 280                 285

Val Phe Trp Gly Glu Gln Leu Thr Arg Met Val Ala Asn Gly Thr Leu
    290                 295                 300

Pro Glu Ala Arg Leu Asp Asp Met Val Leu Arg Ile Leu Thr Pro Leu
305                 310                 315                 320

Ile Tyr Phe Gly Ile Asp Asp Arg Thr Pro Asn Phe Ser Ser Phe Val
                325                 330                 335

Asp Thr Thr Val Gly Ser Pro His Pro Ala Ala Lys His Ser Lys Lys
            340                 345                 350

Val Lys Asp Val Ile Thr Asn Tyr His Leu Asp Val Arg Asp Gln Phe
        355                 360                 365

Ala Ala Asn Val Ala Leu Asp Ser Ala Arg Gly Ala Val Val Leu Leu
    370                 375                 380

Phe Asn Asp Gly Ile Leu Pro Leu Lys Asn Ile Ser Ala Ile Gly Val
385                 390                 395                 400

Phe Gly Val Gly Ser Arg Leu Gly Pro Asn Gly Ala Val Cys Gly Glu
                405                 410                 415

Asn Met Gln Cys Ser Asp Gly Ala Leu Ile Glu Gly Trp Gly Ser Gly
            420                 425                 430

Thr Ala Tyr Pro Thr Glu Tyr Glu Ser Pro Tyr Glu Ala Leu His Lys
        435                 440                 445

Lys Ala Ser Leu Leu Glu Val Ser Val Thr Gly Thr Thr Glu Ser Trp
    450                 455                 460

Asp Met Arg Leu Pro Leu Glu Leu Ala Gly Asp Thr Asp Val Asn Ile
465                 470                 475                 480

Val Tyr Val Leu Ala Asn Ser Gly Glu Ser Thr Ala Asn Val Asp Lys
                485                 490                 495

Asn Leu Gly Asp Arg Arg Asn Val Ser Leu Trp His Asn Gly Asp Glu
            500                 505                 510

Leu Ile Asn Thr Val Ala Ser Gln Gly Gln Thr Val Val Val Val Thr
        515                 520                 525

Thr Val Gly Gln Val Asp Met Thr Ala Trp Leu Asn His Pro Asn Ile
    530                 535                 540

Ser Ala Val Leu Leu Thr Ala Pro Ala Gly Asp Tyr Gly Gly Lys Ala
545                 550                 555                 560

Ile Ala Asp Val Leu Phe Gly Glu Val Asn Pro Ser Gly Lys Leu Pro
                565                 570                 575

Tyr Thr Ile Ala Ala Asn Thr Ser Asp Tyr Ile Pro Ile Val Thr Lys
            580                 585                 590

Ile Pro Arg Asp Gly Ala Pro Gln Ser Asp Phe Val Glu Gly Ile Tyr
        595                 600                 605

Leu Asp Tyr Lys Trp Tyr Asp Lys Phe Glu Arg Thr Pro Leu Tyr Glu
    610                 615                 620

Phe Gly Tyr Gly Leu Ser Tyr Thr Thr Tyr Ser Phe Ser Asn Leu His
625                 630                 635                 640

Leu Asp Val Lys Glu Ile Ser Glu Phe Leu Pro Pro Arg Pro Val Pro
                645                 650                 655

Val Gln Val Thr Lys Pro Lys Met Thr Asn Ile Asp Ile Glu Asp Leu
            660                 665                 670

Tyr Val Pro Asn Asp Phe Lys Met Ile Asp Gly Leu Val Tyr Pro Trp
        675                 680                 685

Ile Leu Asn Ala Ser Ala Pro Leu Ala Asp Ser Gln Thr Gln Phe Pro
    690                 695                 700

Phe Ala Asn Gly Ala Gly His Val Ser Asp Ala Ser Gly Gly Val Gly
705                 710                 715                 720

Gly His Pro Trp Leu Trp Ser Asn Ala Val Thr Val Thr His Asn Thr
                725                 730                 735

Thr Asn Cys Gly Asp Val Ala Gly Arg Val Val Ser Gln Leu Tyr Val
            740                 745                 750

Ala Phe Pro Glu Thr Leu Ile Asp Ser Pro Pro Val Gln Leu Arg Gly
        755                 760                 765

Phe Asp Lys Ser Lys Leu Leu Asn Pro Gly Glu Ser Gln Met Thr Glu
    770                 775                 780

Tyr Asn Leu Asn Trp Arg Asp Leu Ala Ile Trp Asp Val Glu Leu Gln
785                 790                 795                 800

Ser Trp Arg Val Gln Arg Gly Glu Tyr Ser Val Tyr Ile Gly His Ser
                805                 810                 815

Ser Arg Glu Phe Glu Leu Cys Glu Thr Phe Thr Leu
            820                 825

<210>  2
<211>  852
<212>  PRT
<213>  Yarrowia lipolytica

<400>  2

Asp Pro Phe Ser Asp Lys Asp Ala Tyr Lys His Ser Pro Pro Tyr Tyr
1               5                   10                  15

Pro Ala Pro Glu Ile Gly Arg Val Pro Thr Asp Leu Arg Trp Arg Ala
            20                  25                  30

Ala Leu Lys Val Ala Gln Gly Met Val Ala Asn Met Thr Leu Leu Glu
        35                  40                  45

Lys Val Asn Ile Thr Thr Gly Thr Gly Trp Glu Met Gly Pro Cys Val
    50                  55                  60

Gly Asn Thr Gly Thr Val Glu Arg Leu Gly Ile Lys Ser Leu Cys Leu
65                  70                  75                  80

Gln Asp Gly Pro Leu Gly Ile Arg Phe Ala Asp Leu Ile Thr Thr Phe
                85                  90                  95

Pro Ala Gly Ile Thr Ile Ala Ser Thr Phe Ser Arg Gln Leu Val Arg
            100                 105                 110

Glu Arg Gly Ala Ala Met Gly Arg Glu Asn Arg Arg Lys Gly Val Asp
        115                 120                 125

Ile Thr Leu Ser Pro Val Val Gly Pro Leu Gly Arg His Ala Asn Gly
    130                 135                 140

Gly Arg Ile Trp Glu Gly Phe Ser Ala Asp Pro Tyr Leu Ala Gly Lys
145                 150                 155                 160

Leu Ala Ala Glu Ala Val Thr Gly Ile Gln Gly Gln Asn Val Met Ala
                165                 170                 175

Val Val Lys His Met Val Gly Asn Glu Gln Glu His Phe Arg Gln Leu
            180                 185                 190

Gly Glu Trp Gln Gly Phe Gly Phe Lys Asp Leu Lys Gln Pro Leu Ser
        195                 200                 205

Ser Asn Ile Asp Asp Arg Thr Leu Asn Glu Ala Tyr Leu Trp Pro Phe
    210                 215                 220

Ala Asp Ala Val Arg Ala Asn Val Gly Ser Val Met Cys Ser Tyr Gln
225                 230                 235                 240

Gln Ile Asn Gly Ser Gln Gly Cys Gln Asn Ala His Ile Leu Asn Gly
                245                 250                 255

Lys Leu Lys Glu Glu Met Gly Phe Gln Gly Phe Val Met Ser Asp Trp
            260                 265                 270

Leu Ala Gln Arg Ser Gly Val Ala Ser Val Leu Ala Gly Leu Asp Met
        275                 280                 285

Ser Met Pro Gly Asp Gly Leu Val Trp Ala Asp Gly Val Pro Leu Met
    290                 295                 300

Gly Tyr Glu Leu Thr Arg Ser Val Leu Asn Gly Thr Ile Asp Glu Ser
305                 310                 315                 320

Arg Val Asp Asp Met Val Thr Arg Ile Leu Thr Pro Ile Leu Tyr Leu
                325                 330                 335

Ser Ile Thr Pro Thr Asp Pro Asn Phe Ser Ser Trp Thr Asn Asp Thr
            340                 345                 350

Thr Ser Tyr Lys Tyr Tyr Gly Ala Lys Ala Gly Gly Asn Val Thr Val
        355                 360                 365

Asn Arg His Ile Asp Val Arg Asp Gln Tyr Thr Thr Lys Ala Ala Leu
    370                 375                 380

Asp Gly Ala Asn Ala Ala Leu Val Leu Leu Lys Asn Glu Lys Lys Thr
385                 390                 395                 400

Leu Pro Leu Asn Pro Thr Asn Ile Gly Asn Leu Asn Ile Phe Gly Ile
                405                 410                 415

Gly Ser Lys Thr Gly Pro Leu Gly Ala Val Cys Gly Glu Asn Met Gln
            420                 425                 430

Cys Ser Asp Gly Ala Leu Ile Glu Gly Trp Gly Ser Gly Ser Val Tyr
        435                 440                 445

Pro Thr Asp Tyr Gln Ser Pro Tyr Asp Ala Ile Lys Glu Arg Ala Ser
    450                 455                 460

Lys Asp Asn Ile Thr Ile Gly Gly Thr Thr Gln Ser Trp Gly Asn Leu
465                 470                 475                 480

Ser Asn Val Glu Ile Leu Ser Ala Ala Ala Asp Ala Ser Val Val Phe
                485                 490                 495

Val Leu Ser Asp Ser Gly Glu Ser Thr Gly Ile Val Asp Gly Asn Ile
            500                 505                 510

Gly Asp Arg Asn Asn Leu Thr Leu Trp His Asn Gly Asp Glu Val Val
        515                 520                 525

Lys Ala Val Ala Ser Lys Asn Pro Asn Thr Ile Val Val Val Thr Thr
    530                 535                 540

Val Gly Pro Val Asn Leu Glu Lys Trp Ile Asp Asn Pro Asn Val Thr
545                 550                 555                 560

Ala Val Leu Leu Thr Gly Pro Ala Gly Asp Phe Gly Gly Arg Ala Ala

                565                 570                 575

Ala Ser Ile Leu Phe Gly Asp Ile Ala Pro Ser Gly Lys Leu Pro Phe
            580                 585                 590

Thr Ile Ala Lys Asn Asp Thr Asp Tyr Ile Pro Leu Thr Thr Lys Ile
        595                 600                 605

Pro Glu Asp Gly Leu Pro Gln Asp Tyr Phe Thr Glu Gly Thr Leu Leu
    610                 615                 620

Asp Tyr Lys Arg Phe Asp Glu Asn Gln Val Thr Pro Arg Phe Glu Phe
625                 630                 635                 640

Gly Tyr Gly Leu Ser Tyr Ser Asn Ile Thr Val Glu Asn Leu Glu Ala
                645                 650                 655

Arg Tyr Ala Phe Pro Ser Ile Pro Glu Phe Leu Pro Thr Pro Phe Ala
            660                 665                 670

Pro Ser Asn Pro Asn Lys Pro Lys Asn Ala Phe Thr Pro His Ala Asn
        675                 680                 685

Glu Ser Val Phe Pro Ser Asp Ile Asp Pro Leu Asn Lys Tyr Val Tyr
    690                 695                 700

Pro Tyr Leu Asn Asp Thr Ser Glu Ile Phe Ser Asn Glu Thr His Tyr
705                 710                 715                 720

Pro Tyr Pro Glu Gly Tyr Ser Ser Glu Gln Ser Asn Ser Thr Asn Ile
                725                 730                 735

Asn Gly Gly Ala Val Gly Gly Asn Pro Ala Leu Trp Leu Ser Ala Val
            740                 745                 750

Tyr Ile Val His Ser Val Ser Asn Tyr Gly Pro Tyr Asp Thr Gly Val
        755                 760                 765

Val Thr Gln Met Tyr Ile Ala Phe Pro Gln Asp Asn Asp Asp Leu Lys
    770                 775                 780

Thr Ala Pro Arg Gln Leu Arg Gly Phe Glu Arg Ser Glu Leu Lys Val
785                 790                 795                 800

Gly Glu Arg Gln Gly Ile Leu Tyr Asp Val Gln Trp Arg Asp Leu Ala
                805                 810                 815

Val Trp Asp Val Lys Leu Gln Ser Trp Arg Val Gln Arg Gly Glu Tyr
        820                 825                 830

Lys Val Tyr Val Gly His Ser Ser Arg Asp Phe Val Leu Thr Thr Ser
    835                 840                 845

Phe Thr Leu Lys
    850

<210>  3
<211>  459
<212>  PRT
<213>  Hypocrea jecorina

<400>  3

Met Ala Pro Ser Val Thr Leu Pro Leu Thr Thr Ala Ile Leu Ala Ile
1               5                   10                  15

Ala Arg Leu Val Ala Ala Gln Gln Pro Gly Thr Ser Thr Pro Glu Val
            20                  25                  30

His Pro Lys Leu Thr Thr Tyr Lys Cys Thr Lys Ser Gly Gly Cys Val
        35                  40                  45

Ala Gln Asp Thr Ser Val Val Leu Asp Trp Asn Tyr Arg Trp Met His
    50                  55                  60

Asp Ala Asn Tyr Asn Ser Cys Thr Val Asn Gly Gly Val Asn Thr Thr
65                  70                  75                  80

Leu Cys Pro Asp Glu Ala Thr Cys Gly Lys Asn Cys Phe Ile Glu Gly
                85                  90                  95

Val Asp Tyr Ala Ala Ser Gly Val Thr Thr Ser Gly Ser Ser Leu Thr
            100                 105                 110

Met Asn Gln Tyr Met Pro Ser Ser Ser Gly Gly Tyr Ser Ser Val Ser
        115                 120                 125

Pro Arg Leu Tyr Leu Leu Asp Ser Asp Gly Glu Tyr Val Met Leu Lys
    130                 135                 140

Leu Asn Gly Gln Glu Leu Ser Phe Asp Val Asp Leu Ser Ala Leu Pro
145                 150                 155                 160

Cys Gly Glu Asn Gly Ser Leu Tyr Leu Ser Gln Met Asp Glu Asn Gly
                165                 170                 175

Gly Ala Asn Gln Tyr Asn Thr Ala Gly Ala Asn Tyr Gly Ser Gly Tyr
            180                 185                 190

Cys Asp Ala Gln Cys Pro Val Gln Thr Trp Arg Asn Gly Thr Leu Asn
        195                 200                 205

Thr Ser His Gln Gly Phe Cys Cys Asn Glu Met Asp Ile Leu Glu Gly
    210                 215                 220

Asn Ser Arg Ala Asn Ala Leu Thr Pro His Ser Cys Thr Ala Thr Ala
225                 230                 235                 240

Cys Asp Ser Ala Gly Cys Gly Phe Asn Pro Tyr Gly Ser Gly Tyr Lys
                245                 250                 255

Ser Tyr Tyr Gly Pro Gly Asp Thr Val Asp Thr Ser Lys Thr Phe Thr
            260                 265                 270

Ile Ile Thr Gln Phe Asn Thr Asp Asn Gly Ser Pro Ser Gly Asn Leu
        275                 280                 285

Val Ser Ile Thr Arg Lys Tyr Gln Gln Asn Gly Val Asp Ile Pro Ser
    290                 295                 300

Ala Gln Pro Gly Gly Asp Thr Ile Ser Ser Cys Pro Ser Ala Ser Ala
305                 310                 315                 320

Tyr Gly Gly Leu Ala Thr Met Gly Lys Ala Leu Ser Ser Gly Met Val
                325                 330                 335

Leu Val Phe Ser Ile Trp Asn Asp Asn Ser Gln Tyr Met Asn Trp Leu
            340                 345                 350

Asp Ser Gly Asn Ala Gly Pro Cys Ser Ser Thr Glu Gly Asn Pro Ser
        355                 360                 365

Asn Ile Leu Ala Asn Asn Pro Asn Thr His Val Val Phe Ser Asn Ile
    370                 375                 380

Arg Trp Gly Asp Ile Gly Ser Thr Thr Asn Ser Thr Ala Pro Pro Pro
385                 390                 395                 400

Pro Pro Ala Ser Ser Thr Thr Phe Ser Thr Thr Arg Arg Ser Ser Thr
                405                 410                 415

Thr Ser Ser Ser Pro Ser Cys Thr Gln Thr His Trp Gly Gln Cys Gly
            420                 425                 430

Gly Ile Gly Tyr Ser Gly Cys Lys Thr Cys Thr Ser Gly Thr Thr Cys
        435                 440                 445

Gln Tyr Ser Asn Asp Tyr Tyr Ser Gln Cys Leu
    450                 455

<210>  4
<211>  418
<212>  PRT
<213>  Hypocrea jecorina

<400>  4

Met Asn Lys Ser Val Ala Pro Leu Leu Leu Ala Ala Ser Ile Leu Tyr
1               5                   10                  15

Gly Gly Ala Val Ala Gln Gln Thr Val Trp Gly Gln Cys Gly Gly Ile
            20                  25                  30

Gly Trp Ser Gly Pro Thr Asn Cys Ala Pro Gly Ser Ala Cys Ser Thr
        35                  40                  45

Leu Asn Pro Tyr Tyr Ala Gln Cys Ile Pro Gly Ala Thr Thr Ile Thr
    50                  55                  60

Thr Ser Thr Arg Pro Pro Ser Gly Pro Thr Thr Thr Thr Arg Ala Thr
65                  70                  75                  80

Ser Thr Ser Ser Ser Thr Pro Pro Thr Ser Ser Gly Val Arg Phe Ala
                85                  90                  95

Gly Val Asn Ile Ala Gly Phe Asp Phe Gly Cys Thr Thr Asp Gly Thr
            100                 105                 110

Cys Val Thr Ser Lys Val Tyr Pro Pro Leu Lys Asn Phe Thr Gly Ser
        115                 120                 125

Asn Asn Tyr Pro Asp Gly Ile Gly Gln Met Gln His Phe Val Asn Glu
    130                 135                 140

Asp Gly Met Thr Ile Phe Arg Leu Pro Val Gly Trp Gln Tyr Leu Val
145                 150                 155                 160

Asn Asn Asn Leu Gly Gly Asn Leu Asp Ser Thr Ser Ile Ser Lys Tyr
                165                 170                 175

Asp Gln Leu Val Gln Gly Cys Leu Ser Leu Gly Ala Tyr Cys Ile Val
            180                 185                 190

Asp Ile His Asn Tyr Ala Arg Trp Asn Gly Gly Ile Ile Gly Gln Gly
        195                 200                 205

Gly Pro Thr Asn Ala Gln Phe Thr Ser Leu Trp Ser Gln Leu Ala Ser
    210                 215                 220

Lys Tyr Ala Ser Gln Ser Arg Val Trp Phe Gly Ile Met Asn Glu Pro
225                 230                 235                 240

His Asp Val Asn Ile Asn Thr Trp Ala Ala Thr Val Gln Glu Val Val
                245                 250                 255

Thr Ala Ile Arg Asn Ala Gly Ala Thr Ser Gln Phe Ile Ser Leu Pro
            260                 265                 270

Gly Asn Asp Trp Gln Ser Ala Gly Ala Phe Ile Ser Asp Gly Ser Ala
        275                 280                 285

Ala Ala Leu Ser Gln Val Thr Asn Pro Asp Gly Ser Thr Thr Asn Leu
    290                 295                 300

Ile Phe Asp Val His Lys Tyr Leu Asp Ser Asp Asn Ser Gly Thr His
305                 310                 315                 320

Ala Glu Cys Thr Thr Asn Asn Ile Asp Gly Ala Phe Ser Pro Leu Ala
                325                 330                 335

Thr Trp Leu Arg Gln Asn Asn Arg Gln Ala Ile Leu Thr Glu Thr Gly
            340                 345                 350

Gly Gly Asn Val Gln Ser Cys Ile Gln Asp Met Cys Gln Gln Ile Gln
        355                 360                 365

Tyr Leu Asn Gln Asn Ser Asp Val Tyr Leu Gly Tyr Val Gly Trp Gly
    370                 375                 380

Ala Gly Ser Phe Asp Ser Thr Tyr Val Leu Thr Glu Thr Pro Thr Ser
385                 390                 395                 400

Ser Gly Asn Ser Trp Thr Asp Thr Ser Leu Val Ser Ser Cys Leu Ala
                405                 410                 415

Arg Lys

<210>  5
<211>  521

<212>  PRT
<213>  Neurospora crassa

<400>  5

Met Leu Ala Lys Phe Ala Ala Leu Ala Ala Leu Val Ala Ser Ala Asn
1               5                   10                  15

Ala Gln Ala Val Cys Ser Leu Thr Ala Glu Thr His Pro Ser Leu Asn
            20                  25                  30

Trp Ser Lys Cys Thr Ser Ser Gly Cys Thr Asn Val Ala Gly Ser Ile
        35                  40                  45

Thr Val Asp Ala Asn Trp Arg Trp Thr His Ile Thr Ser Gly Ser Thr
    50                  55                  60

Asn Cys Tyr Ser Gly Asn Glu Trp Asp Thr Ser Leu Cys Ser Thr Asn
65                  70                  75                  80

Thr Asp Cys Ala Thr Lys Cys Cys Val Asp Gly Ala Glu Tyr Ser Ser
                85                  90                  95

Thr Tyr Gly Ile Gln Thr Ser Gly Asn Ser Leu Ser Leu Gln Phe Val
            100                 105                 110

Thr Lys Gly Ser Tyr Ser Thr Asn Ile Gly Ser Arg Thr Tyr Leu Met
        115                 120                 125

Asn Gly Ala Asp Ala Tyr Gln Gly Phe Glu Leu Leu Gly Asn Glu Phe
    130                 135                 140

Thr Phe Asp Val Asp Val Ser Gly Thr Gly Cys Gly Leu Asn Gly Ala
145                 150                 155                 160

Leu Tyr Phe Val Ser Met Asp Leu Asp Gly Gly Lys Ala Lys Tyr Thr
                165                 170                 175

Asn Asn Lys Ala Gly Ala Lys Tyr Gly Thr Gly Tyr Cys Asp Ala Gln
            180                 185                 190

Cys Pro Arg Asp Leu Lys Tyr Ile Asn Gly Ile Ala Asn Val Glu Gly
        195                 200                 205

Trp Thr Pro Ser Thr Asn Asp Ala Asn Ala Gly Ile Gly Asp His Gly
    210                 215                 220

Thr Cys Cys Ser Glu Met Asp Ile Trp Glu Ala Asn Lys Val Ser Thr
225                 230                 235                 240

Ala Phe Thr Pro His Pro Cys Thr Thr Ile Glu Gln His Met Cys Glu
                245                 250                 255

Gly Asp Ser Cys Gly Gly Thr Tyr Ser Asp Asp Arg Tyr Gly Gly Thr
            260                 265                 270

Cys Asp Ala Asp Gly Cys Asp Phe Asn Ser Tyr Arg Met Gly Asn Thr
        275                 280                 285

Thr Phe Tyr Gly Glu Gly Lys Thr Val Asp Thr Ser Ser Lys Phe Thr
    290                 295                 300

Val Val Thr Gln Phe Ile Lys Asp Ser Ala Gly Asp Leu Ala Glu Ile
305                 310                 315                 320

Lys Arg Phe Tyr Val Gln Asn Gly Lys Val Ile Glu Asn Ser Gln Ser
                325                 330                 335

Asn Val Asp Gly Val Ser Gly Asn Ser Ile Thr Gln Ser Phe Cys Asn
            340                 345                 350

Ala Gln Lys Thr Ala Phe Gly Asp Ile Asp Asp Phe Asn Lys Lys Gly
        355                 360                 365

Gly Leu Lys Gln Met Gly Lys Ala Leu Ala Lys Pro Met Val Leu Val
    370                 375                 380

Met Ser Ile Trp Asp Asp His Ala Ala Asn Met Leu Trp Leu Asp Ser
385                 390                 395                 400

Thr Tyr Pro Val Glu Gly Gly Pro Gly Ala Tyr Arg Gly Glu Cys Pro
                405                 410                 415

Thr Thr Ser Gly Val Pro Ala Glu Val Glu Ala Asn Ala Pro Asn Ser
            420                 425                 430

Lys Val Ile Phe Ser Asn Ile Lys Phe Gly Pro Ile Gly Ser Thr Phe
        435                 440                 445

Ser Gly Gly Ser Ser Gly Thr Pro Pro Ser Asn Pro Ser Ser Ser Val
    450                 455                 460

Lys Pro Val Thr Ser Thr Ala Lys Pro Ser Ser Thr Ser Thr Ala Ser
465                 470                 475                 480

Asn Pro Ser Gly Thr Gly Ala Ala His Trp Ala Gln Cys Gly Gly Ile

485                 490                 495

Gly Phe Ser Gly Pro Thr Thr Cys Gln Ser Pro Tyr Thr Cys Gln Lys
            500                 505                 510

Ile Asn Asp Tyr Tyr Ser Gln Cys Val
        515                 520

<210>  6
<211>  471
<212>  PRT
<213>  Hypocrea jecorina

<400>  6

Met Ile Val Gly Ile Leu Thr Thr Leu Ala Thr Leu Ala Thr Leu Ala
1               5                   10                  15

Ala Ser Val Pro Leu Glu Glu Arg Gln Ala Cys Ser Ser Val Trp Gly
            20                  25                  30

Gln Cys Gly Gly Gln Asn Trp Ser Gly Pro Thr Cys Cys Ala Ser Gly
        35                  40                  45

Ser Thr Cys Val Tyr Ser Asn Asp Tyr Tyr Ser Gln Cys Leu Pro Gly
    50                  55                  60

Ala Ala Ser Ser Ser Ser Ser Thr Arg Ala Ala Ser Thr Thr Ser Arg
65                  70                  75                  80

Val Ser Pro Thr Thr Ser Arg Ser Ser Ser Ala Thr Pro Pro Pro Gly
                85                  90                  95

Ser Thr Thr Thr Arg Val Pro Pro Val Gly Ser Gly Thr Ala Thr Tyr
            100                 105                 110

Ser Gly Asn Pro Phe Val Gly Val Thr Pro Trp Ala Asn Ala Tyr Tyr
        115                 120                 125

Ala Ser Glu Val Ser Ser Leu Ala Ile Pro Ser Leu Thr Gly Ala Met
    130                 135                 140

Ala Thr Ala Ala Ala Ala Val Ala Lys Val Pro Ser Phe Met Trp Leu
145                 150                 155                 160

Asp Thr Leu Asp Lys Thr Pro Leu Met Glu Gln Thr Leu Ala Asp Ile
                165                 170                 175

Arg Thr Ala Asn Lys Asn Gly Gly Asn Tyr Ala Gly Gln Phe Val Val

            180                 185                 190

Tyr Asp Leu Pro Asp Arg Asp Cys Ala Ala Leu Ala Ser Asn Gly Glu
        195                 200                 205

Tyr Ser Ile Ala Asp Gly Gly Val Ala Lys Tyr Lys Asn Tyr Ile Asp
    210                 215                 220

Thr Ile Arg Gln Ile Val Val Glu Tyr Ser Asp Ile Arg Thr Leu Leu
225                 230                 235                 240

Val Ile Glu Pro Asp Ser Leu Ala Asn Leu Val Thr Asn Leu Gly Thr
                245                 250                 255

Pro Lys Cys Ala Asn Ala Gln Ser Ala Tyr Leu Glu Cys Ile Asn Tyr
            260                 265                 270

Ala Val Thr Gln Leu Asn Leu Pro Asn Val Ala Met Tyr Leu Asp Ala
        275                 280                 285

Gly His Ala Gly Trp Leu Gly Trp Pro Ala Asn Gln Asp Pro Ala Ala
    290                 295                 300

Gln Leu Phe Ala Asn Val Tyr Lys Asn Ala Ser Ser Pro Arg Ala Leu
305                 310                 315                 320

Arg Gly Leu Ala Thr Asn Val Ala Asn Tyr Asn Gly Trp Asn Ile Thr
                325                 330                 335

Ser Pro Pro Ser Tyr Thr Gln Gly Asn Ala Val Tyr Asn Glu Lys Leu
            340                 345                 350

Tyr Ile His Ala Ile Gly Pro Leu Leu Ala Asn His Gly Trp Ser Asn
        355                 360                 365

Ala Phe Phe Ile Thr Asp Gln Gly Arg Ser Gly Lys Gln Pro Thr Gly
    370                 375                 380

Gln Gln Gln Trp Gly Asp Trp Cys Asn Val Ile Gly Thr Gly Phe Gly
385                 390                 395                 400

Ile Arg Pro Ser Ala Asn Thr Gly Asp Ser Leu Leu Asp Ser Phe Val
                405                 410                 415

Trp Val Lys Pro Gly Gly Glu Cys Asp Gly Thr Ser Asp Ser Ser Ala
            420                 425                 430

Pro Arg Phe Asp Ser His Cys Ala Leu Pro Asp Ala Leu Gln Pro Ala
        435                 440                 445

Pro Gln Ala Gly Ala Trp Phe Gln Ala Tyr Phe Val Gln Leu Leu Thr
    450                 455                 460

Asn Ala Asn Pro Ser Phe Leu
465                 470

<210>  7
<211>  344
<212>  PRT
<213>  Hypocrea jecorina

<400>  7

Met Ile Gln Lys Leu Ser Asn Leu Leu Val Thr Ala Leu Ala Val Ala
1               5                   10                  15

Thr Gly Val Val Gly His Gly His Ile Asn Asp Ile Val Ile Asn Gly
            20                  25                  30

Val Trp Tyr Gln Ala Tyr Asp Pro Thr Thr Phe Pro Tyr Glu Ser Asn
        35                  40                  45

Pro Pro Ile Val Val Gly Trp Thr Ala Ala Asp Leu Asp Asn Gly Phe
    50                  55                  60

Val Ser Pro Asp Ala Tyr Gln Asn Pro Asp Ile Ile Cys His Lys Asn
65                  70                  75                  80

Ala Thr Asn Ala Lys Gly His Ala Ser Val Lys Ala Gly Asp Thr Ile
                85                  90                  95

Leu Phe Gln Trp Val Pro Val Pro Trp Pro His Pro Gly Pro Ile Val
            100                 105                 110

Asp Tyr Leu Ala Asn Cys Asn Gly Asp Cys Glu Thr Val Asp Lys Thr
        115                 120                 125

Thr Leu Glu Phe Phe Lys Ile Asp Gly Val Gly Leu Leu Ser Gly Gly
    130                 135                 140

Asp Pro Gly Thr Trp Ala Ser Asp Val Leu Ile Ser Asn Asn Asn Thr
145                 150                 155                 160

Trp Val Val Lys Ile Pro Asp Asn Leu Ala Pro Gly Asn Tyr Val Leu
                165                 170                 175

Arg His Glu Ile Ile Ala Leu His Ser Ala Gly Gln Ala Asn Gly Ala
            180                 185                 190

Gln Asn Tyr Pro Gln Cys Phe Asn Ile Ala Val Ser Gly Ser Gly Ser
        195                 200                 205

Leu Gln Pro Ser Gly Val Leu Gly Thr Asp Leu Tyr His Ala Thr Asp
    210                 215                 220

Pro Gly Val Leu Ile Asn Ile Tyr Thr Ser Pro Leu Asn Tyr Ile Ile
225                 230                 235                 240

Pro Gly Pro Thr Val Val Ser Gly Leu Pro Thr Ser Val Ala Gln Gly
                245                 250                 255

Ser Ser Ala Ala Thr Ala Thr Ala Ser Ala Thr Val Pro Gly Gly Gly
            260                 265                 270

Ser Gly Pro Thr Ser Arg Thr Thr Thr Thr Ala Arg Thr Thr Gln Ala
        275                 280                 285

Ser Ser Arg Pro Ser Ser Thr Pro Pro Ala Thr Thr Ser Ala Pro Ala
    290                 295                 300

Gly Gly Pro Thr Gln Thr Leu Tyr Gly Gln Cys Gly Gly Ser Gly Tyr
305                 310                 315                 320

Ser Gly Pro Thr Arg Cys Ala Pro Pro Ala Thr Cys Ser Thr Leu Asn
                325                 330                 335

Pro Tyr Tyr Ala Gln Cys Leu Asn
            340

<210> 8
<211> 2535
<212> DNA
<213> Yarrowia lipolytica

<220>
<221> sig_peptide
<222> (1)..(48)

<400> 8
atgatcttct ctctgcaact actactgacg acggcactag cggcctcttc gcctgactgg 60

taccccacac ccgaaatcgg ttcaattacc agtgattggg ccgatgctct tggtgagtcc 120

atggacatcc tggcccaatt gactcttccc gaaaaggtca acatcaccac cggtaccgga 180

tggagaggtg ggcagtgtgt tggaaacaca ggggctgttc ctcgtctcgg aatcaagggc 240

ctgtgtctcc aagacggtcc tctgggcgtc cgttttgccg actttgtcaa tgtcttccct 300

tgccagaacg caatggccgc cacctttgat cgtatcctag ttcaccaacg aggaaccgct 360

attggacgtc agtctagact caagggagtc gatgttcatc tcggaccagt ggtgggacct 420

cttggacgac acgctaccgg cggaagaaac tgggaaggtt tctcccccga cccatatctg 480

tctggaaagc tcgccttcga agcaattctt ggagtccagg aagagggagt tcttgcaacc 540

atcaagcact tcattggaaa cgaacaagaa cattaccggc gagccgaaga gtggagagac 600

ggctttggat ttaaagacct gaaggacgcc gtctcttcca acattgaaga cagagctcta 660

catgagttgt acatgtggcc gtttgccgat gctgttaggg ctaatgtcgg ctcagtcatg 720

tgctcctaca actacgtgaa cggaacccag gcttgccaga acagtgactt gctcaacgga 780

aagctcaagt ccgagctcgg tttccagggc tttgtcatgt ccgactggtt tgctcaggga 840

agcggagtgt ctaacgctct ggctggaatg gacatgagta tgcctggaaa tgacgttgat 900

gagttagaaa ctgtcttctg gggagaacag ctgacccgaa tggttgccaa cggtaccctt 960

ccagaagccc gtctcgatga tatggttctg cgaattctga cccctctcat ctactttggg 1020

atcgacgatc gaacacccaa cttctcctcc tttgtcgaca ctacagtggg aagtccccac 1080

cccgctgcta agcactccaa gaaagtcaag gatgttatca ccaactacca tctcgatgtg 1140

cgagaccagt ttgcagccaa tgttgctctt gatagtgctc gaggagctgt tgttctgctt 1200

ttcaatgacg gtattcttcc cctgaagaac atttccgcta ttggagtatt tggagtcggc 1260

tctagacttg gccccaatgg agctgtttgt ggggaaaaca tgcagtgctc agacggagct 1320

cttatcgaag gatggggaag tggaactgct taccctacgg agtacgaaag cccttacgaa 1380

gctctccaca aaaaggcttc tctacttgaa gtgtcagtga caggaaccac cgagtcatgg 1440

gatatgagac ttcctcttga gctggctgga gacactgacg tgaatattgt ttatgtattg 1500

gcaaattcag gagagtctac tgccaacgtt gataagaacc ttggggatcg ccgaaatgtc 1560

agtctatggc acaatggcga tgaacttatt aatacagttg ccagtcaggg acagactgtc 1620

gttgttgtca ctacggttgg acaagttgac atgaccgctt ggctcaacca ccccaacatc 1680

agtgccgttc ttctgactgc tcctgctggt gattacggag gaaaagccat cgctgatgtg 1740

ttatttggag aggttaatcc ctcaggaaag ctgccttata ctatcgcagc aaatacttct 1800

gattatattc ctattgtcac caagatccct cgagatggag ctccccagtc cgactttgtg 1860

gagggaatct atcttgacta caagtggtac gacaagtttg aaaggactcc cctctacgaa 1920

tttggttacg gtctgtcata caccacctac tccttcagca atctgcatct tgatgttaag 1980

gagattagtg agttccttcc tccccggcct gttcctgtac aggttactaa acccaagatg 2040

accaatatcg acattgagga cctttatgtc cccaatgact ttaaaatgat tgacggtctt 2100

gtttaccctt ggattctcaa cgccagtgcg cccctcgcgg actctcagac tcagttcccc 2160

tttgcaaatg gagctggaca tgtcagtgac gcttctggag gagtgggtgg tcacccctgg 2220

ctttggtcta acgctgttac tgtcactcac aacaccacca actgcggtga tgttgctgga 2280

cgagtagttt ctcagctgta cgttgccttc cctgaaaccc ttatcgactc tcctccggtg 2340

cagcttcgag gattcgacaa gtccaagctc ctgaaccctg gtgagtctca gatgaccgag 2400

tacaacctca actggcgaga tctggccatt tgggacgtgg aactgcagag ctggagagtg 2460

cagcgaggcg agtattccgt gtacattggt cattccagcc gagaattcga gctatgtgag 2520

actttcactt tgtag 2535

<210> 9
<211> 2610
<212> DNA
<213> Yarrowia lipolytica

<220>
<221> sig_peptide
<222> (1)..(51)

<400> 9
atgctcgcat tcgtcctact gctgacgatg ctgctcgcag cagcgcttgc tgacccgttc 60

tcagataaag acgcttacaa acacagccct ccatactacc ctgctccgga gattggcaga 120

gttcccaccg acctgcgatg gagagctgcc ttgaaggtgg cccagggtat ggtcgctaac 180

atgacactgc ttgagaaggt gaacatcacc accggtacgg gctgggagat gggtccttgt 240

gttggaaaca ctggtaccgt cgaacgactg ggtataaaat cgctgtgcct tcaagacggc 300

cctcttggga ttcgatttgc tgacctcatt accacattcc ctgctggtat cactattgcc 360

tctaccttct ctcgacagct ggttagagag cgaggtgctg ctatgggacg ggagaataga 420

cgcaagggag tggatatcac tctcagccct gtggttggac cactgggaag acatgctaac 480

ggaggtcgaa tctgggaggg cttctctgct gacccctacc ttgctggaaa gctcgccgcc 540

gaggccgtga caggtataca gggccagaac gtcatggctg tggtaaagca tatggttgga 600

aacgagcagg aacattttcg acaacttggc gagtggcagg gattcggatt caaggatctg 660

aagcagcccc tctcttcaaa catcgacgac cgaactctta acgaagcgta cctttggccc 720

tttgctgatg ctgttcgagc caatgttgga tctgtcatgt gttcctatca gcagatcaat 780

ggctctcagg gttgtcaaaa cgcccacatt ctgaacggta agctcaagga ggaaatgggt 840

ttccagggct ttgtcatgtc cgactggctt gcccagcgaa gtggcgtggc gtctgttctt 900

gctggtctcg atatgagcat gcctggagac ggtcttgtct gggcggacgg tgttccgctc 960

atgggatacg agttgaccag gagtgtgctg aatggaacca ttgatgaaag ccgagtggac 1020

gacatggtta cccgaatcct tacccccata ctgtatctct caattactcc gaccgacccc 1080

aacttcagct cttggaccaa cgacactact agctacaagt actacggagc caaggctggt 1140

ggaaacgtca ctgttaaccg acatattgat gtgagagacc agtacactac caaggctgct 1200

cttgatggag caaacgctgc gcttgttctt ctcaagaatg agaagaagac tcttcctctg 1260

aaccctacca atattggaaa cctcaacatt ttcggtattg gttctaaaac cggcccactt 1320

ggagctgtct gtggagaaaa tatgcagtgt agtgatggcg cccttattga gggatggggg 1380

tccggttccg tctaccctac cgattatcaa tctccttacg acgccattaa ggagagagcc 1440

tccaaggaca acatcaccat cggaggcact acgcaatctt ggggtaacct gtcgaacgtt 1500

gagatccttt cagctgctgc cgacgccagt gtcgtctttg ttctttccga ctccggtgag 1560

agtactggta ttgttgacgg caacattggg gatcgaaaca acttgacgct gtggcacaat 1620

ggagacgagg ttgtcaaggc tgtggcatct aagaacccca acactatcgt tgttgttacc 1680

actgtcggcc ctgtgaacct cgaaaagtgg atcgacaacc caaacgtcac tgccgtgctt 1740

ctcactggac ccgctggtga ctttggagga agagctgcag cctctattct cttcggcgac 1800

atcgcccctt caggaaaact ccctttcact attgccaaga atgacaccga ctacattcct 1860

cttactacta agatccctga agacggcctt cctcaagact atttcactga gggtactctt 1920

ttggactaca aacggttcga cgagaaccag gtgactccta ggtttgaatt tggctacggt 1980

ctgtcttact ctaacattac ggtggagaat ctcgaagccc ggtatgcttt ccctagcatt 2040

cctgagttct tgcccactcc ctttgcccct tcgaacccca acaagcctaa gaacgcattt 2100

actcctcacg ccaatgagtc cgtcttcccc agtgacattg atcctttgaa caagtacgtc 2160

tatccatacc tgaacgatac ctcggagatc ttctctaacg agacccatta tccctatcct 2220

gaggggtact ccagtgagca gtccaacagt accaacatta acggcggggc tgtcggaggc 2280

aaccctgctc tgtggctctc tgcagtctac attgtccaca gcgtgtctaa ctatggtccc 2340

tatgatactg gagtggtcac ccagatgtac attgccttcc ctcaggataa cgacgatctt 2400

aaaaccgctc ctagacagct tcgaggattc gaacggtccg agctcaaggt gggagaacgg 2460

cagggaattc tatacgatgt tcaatggcga gatctcgcgg tctgggatgt caaacttcag 2520

agctggcggg tccaacgagg agagtacaag gtttacgtag gccacagttc gcgagacttt 2580

gttctgacca ccagcttcac tctcaagtag 2610

<210> 10
<211> 1314
<212> DNA
<213> Hypocrea jecorina

<400> 10
cagcaaccgg gtaccagcac ccccgaggtc catcccaagt tgacaaccta caagtgtaca 60

aagtccgggg ggtgcgtggc ccaggacacc tcggtggtcc ttgactggaa ctaccgctgg 120

atgcacgacg caaactacaa ctcgtgcacc gtcaacggcg gcgtcaacac cacgctctgc 180

cctgacgagg cgacctgtgg caagaactgc ttcatcgagg gcgtcgacta cgccgcctcg 240

ggcgtcacga cctcgggcag cagcctcacc atgaaccagt acatgcccag cagctctggc 300

ggctacagca gcgtctctcc tcggctgtat ctcctggact ctgacggtga gtacgtgatg 360

ctgaagctca acggccagga gctgagcttc gacgtcgacc tctctgctct gccgtgtgga 420

gagaacggct cgctctacct gtctcagatg gacgagaacg ggggcgccaa ccagtataac 480

acggccggtg ccaactacgg gagcggctac tgcgatgctc agtgccccgt ccagacatgg 540

aggaacggca ccctcaacac tagccaccag ggcttctgct gcaacgagat ggatatcctg 600

gagggcaact cgagggcgaa tgccttgacc cctcactctt gcacggccac ggcctgcgac 660

tctgccggtt gcggcttcaa cccctatggc agcggctaca aaagctacta cggccccgga 720

gataccgttg acacctccaa gaccttcacc atcatcaccc agttcaacac ggacaacggc 780

tcgccctcgg gcaaccttgt gagcatcacc cgcaagtacc agcaaaacgg cgtcgacatc 840

cccagcgccc agcccggcgg cgacaccatc tcgtcctgcc cgtccgcctc agcctacggc 900

ggcctcgcca ccatgggcaa ggccctgagc agcggcatgg tgctcgtgtt cagcatttgg 960

aacgacaaca gccagtacat gaactggctc gacagcggca acgccggccc ctgcagcagc 1020

accgagggca acccatccaa catcctggcc aacaacccca acacgcacgt cgtcttctcc 1080

aacatccgct ggggagacat tgggtctact acgaactcga ctgcgccccc gcccccgcct 1140

gcgtccagca cgacgttttc gactacacgg aggagctcga cgacttcgag cagcccgagc 1200

tgcacgcaga ctcactgggg gcagtgcggt ggcattgggt acagcgggtg caagacgtgc 1260

acgtcgggca ctacgtgcca gtatagcaac gactactact cgcaatgcct ttag 1314

<210> 11
<211> 1197
<212> DNA
<213> Hypocrea jecorina

<400> 11
gcacagcaga ctgtctgggg ccagtgtgga ggtattggtt ggagcggacc tacgaattgt 60

gctcctggct cagcttgttc gaccctcaat ccttattatg cgcaatgtat tccgggagcc 120

actactatca ccacttcgac ccggccacca tccggtccaa ccaccaccac cagggctacc 180

tcaacaagct catcaactcc acccacgagc tctggggtcc gatttgccgg cgttaacatc 240

gcgggttttg actttggctg taccacagat ggcacttgcg ttacctcgaa ggtttatcct 300

ccgttgaaga acttcaccgg ctcaaacaac taccccgatg gcatcggcca gatgcagcac 360

ttcgtcaacg aggacgggat gactattttc cgcttacctg tcggatggca gtacctcgtc 420

aacaacaatt tgggcggcaa tcttgattcc acgagcattt ccaagtatga tcagcttgtt 480

caggggtgcc tgtctctggg cgcatactgc atcgtcgaca tccacaatta tgctcgatgg 540

aacggtggga tcattggtca gggcggccct actaatgctc aattcacgag cctttggtcg 600

cagttggcat caaagtacgc atctcagtcg agggtgtggt tcggcatcat gaatgagccc 660

cacgacgtga acatcaacac ctgggctgcc acggtccaag aggttgtaac cgcaatccgc 720

aacgctggtg ctacgtcgca attcatctct ttgcctggaa atgattggca atctgctggg 780

gctttcatat ccgatggcag tgcagccgcc ctgtctcaag tcacgaaccc ggatgggtca 840

acaacgaatc tgatttttga cgtgcacaaa tacttggact cagacaactc cggtactcac 900

gccgaatgta ctacaaataa cattgacggc gccttttctc cgcttgccac ttggctccga 960

cagaacaatc gccaggctat cctgacagaa accggtggtg gcaacgttca gtcctgcata 1020

caagacatgt gccagcaaat ccaatatctc aaccagaact cagatgtcta tcttggctat 1080

gttggttggg gtgccggatc atttgatagc acgtatgtcc tgacggaaac accgactggc 1140

agtggtaact catggacgga cacatccttg gtcagctcgt gtctcgcaag aaagtag 1197

<210> 12
<211> 1515
<212> DNA
<213> Neurospora crassa

<400> 12
caggctgttt gctctctcac tgccgagacc catccctccc tcaactggtc caagtgcact 60

tcctccggat gcacgaacgt cgccggatcc atcactgttg atgccaactg gcgctggact 120

cacattactt ctggtagcac caactgctac agcggcaacg agtgggacac ctccctctgc 180

agcaccaaca ccgattgcgc gaccaagtgc tgcgttgatg gtgctgagta ctcttctacc 240

tatggtattc agaccagcgg caactcgctc agccttcagt tcgtcaccaa gggctcgtac 300

tcgaccaaca ttggttcccg tacttacctt atgaacggtg ccgatgccta ccagggcttc 360

gagctccttg gcaacgagtt caccttcgat gtcgatgtgt ccggcaccgg ctgcggtctt 420

aacggcgccc tctacttcgt ctccatggat cttgatggtg gcaaggccaa gtacaccaac 480

aacaaggctg gtgccaagta cggtaccggt tactgcgacg ctcagtgccc ccgtgatctc 540

aagtacatca acggtatcgc caacgttgag ggctggaccc cctccaccaa cgatgctaac 600

gctggtattg gtgaccacgg tacttgctgc tctgagatgg atatctggga agcgaacaaa 660

gtctctacag cgttcacccc gcacccctgc accaccatcg aacagcacat gtgcgagggt 720

gactcctgcg gtggtaccta ttccgacgac cgctatggcg gtacttgcga tgccgatggt 780

tgtgacttca acagctaccg catgggcaac accaccttct acggtgaggg caagactgtc 840

gataccagct ccaagttcac cgttgtcacc cagttcatca aggactccgc tggcgatctt 900

gctgagatca agcgcttcta cgtccagaac ggaaaagtca ttgagaactc tcagtccaac 960

gttgatggag tttctggcaa ctccatcacc cagtctttct gcaatgctca gaagactgct 1020

ttcggcgata tcgatgactt caacaagaag ggtggcctga agcaaatggg caaggccctt 1080

gccaagccca tggtcctcgt catgtccatc tgggacgacc atgccgccaa catgctctgg 1140

ctcgactcca cctaccctgt cgagggtggc cccggtgctt accgtggcga gtgccctacc 1200

acctcgggtg tccccgctga ggtcgaggcc aatgctccca actccaaggt catcttctcc 1260

aacatcaagt tcggccccat cggctctacc tttagcggcg gctcttccgg cacccctcct 1320

tccaaccctt cgagctccgt caagcccgtc acctccactg ctaagccttc ttccacctct 1380

actgcctcca accccagcgg taccggtgct gctcactggg ctcagtgcgg tggtattggc 1440

ttctctggcc ccaccacttg ccagagccct tacacttgcc aaaagatcaa cgactattac 1500

tcccagtgcg tgtaa 1515

<210> 13
<211> 1362
<212> DNA
<213> Hypocrea jecorina

<400> 13
gtgcctctag aggagcggca agcttgctca agcgtctggg gccaatgtgg tggccagaat 60

tggtcgggtc cgacttgctg tgcttccgga agcacatgcg tctactccaa cgactattac 120

tcccagtgtc ttcccggcgc tgcaagctca agctcgtcca cgcgcgccgc gtcgacgact 180

tctcgagtat cccccacaac atcccggtcg agctccgcga cgcctccacc tggttctact 240

actaccagag tacctccagt cggatcggga accgctacgt attcaggcaa cccttttgtt 300

ggggtcactc cttgggccaa tgcatattac gcctctgaag ttagcagcct cgctattcct 360

agcttgactg gagccatggc cactgctgca gcagctgtcg caaaggttcc ctcttttatg 420

tggctagata ctcttgacaa gacccctctc atggagcaaa ccttggccga catccgcacc 480

gccaacaaga atggcggtaa ctatgccgga cagtttgtgg tgtatgactt gccggatcgc 540

gattgcgctg cccttgcctc gaatggcgaa tactctattg ccgatggtgg cgtcgccaaa 600

tataagaact atatcgacac cattcgtcaa attgtcgtgg aatattccga tatccggacc 660

ctcctggtta ttgagcctga ctctcttgcc aacctggtga ccaacctcgg tactccaaag 720

tgtgccaatg ctcagtcagc ctaccttgag tgcatcaact acgccgtcac acagctgaac 780

cttccaaatg ttgcgatgta tttggacgct ggccatgcag gatggcttgg ctggccggca 840

aaccaagacc cggccgctca gctatttgca aatgtttaca agaatgcatc gtctccgaga 900

gctcttcgcg gattggcaac caatgtcgcc aactacaacg ggtggaacat taccagcccc 960

ccatcgtaca cgcaaggcaa cgctgtctac aacgagaagc tgtacatcca cgctattgga 1020

cctcttcttg ccaatcacgg ctggtccaac gccttcttca tcactgatca aggtcgatcg 1080

ggaaagcagc ctaccggaca gcaacagtgg ggagactggt gcaatgtgat cggcaccgga 1140

tttggtattc gcccatccgc aaacactggg gactcgttgc tggattcgtt tgtctgggtc 1200

aagccaggcg gcgagtgtga cggcaccagc gacagcagtg cgccacgatt tgactcccac 1260

tgtgcgctcc cagatgcctt gcaaccggcg cctcaagctg gtgcttggtt ccaagcctac 1320

tttgtgcagc ttctcacaaa cgcaaaccca tcgttcctgt aa 1362

<210> 14
<211> 972
<212> DNA
<213> Hypocrea jecorina

<400> 14
catggacata ttaatgacat tgtcatcaac ggggtgtggt atcaggccta tgatcctaca 60

acgtttccat acgagtcaaa cccccccata gtagtgggct ggacggctgc cgaccttgac 120

aacggcttcg tttcacccga cgcataccaa aaccctgaca tcatctgcca caagaatgct 180

acgaatgcca aggggcacgc gtctgtcaag gccggagaca ctattctctt ccagtgggtg 240

ccagttccat ggccgcaccc tggtcccatt gtcgactacc tggccaactg caatggtgac 300

tgcgagaccg ttgacaagac gacgcttgag ttcttcaaga tcgatggcgt tggtctcctc 360

agcggcgggg atccgggcac ctgggcctca gacgtgctga tctccaacaa caacacctgg 420

gtcgtcaaga tccccgacaa tcttgcgcca ggcaattacg tgctccgcca cgagatcatc 480

gcgttacaca gcgccgggca ggcaaacggc gctcagaact acccccagtg cttcaacatt 540

gccgtctcag gctcgggttc tctgcagccc agcggcgttc tagggaccga cctctatcac 600

gcgacggacc ctggtgttct catcaacatc tacaccagcc cgctcaacta catcatccct 660

ggacctaccg tggtatcagg cctgccaacg agtgttgccc aggggagctc cgccgcgacg 720

gccaccgcca gcgccactgt tcctggaggc ggtagcggcc cgaccagcag aaccacgaca 780

acggcgagga cgacgcaggc ctcaagcagg cccagctcta cgcctcccgc aaccacgtcg 840

gcacctgctg gcggcccaac ccagactctg tacggccagt gtggtggcag cggttacagc 900

gggcctactc gatgcgcgcc gccagccact tgctctacct tgaaccccta ctacgcccag 960

tgccttaact ag 972

<210> 15
<211> 831
<212> PRT
<213> Yarrowia galli

<400> 15

Ala Tyr Ser Pro Asp Trp Tyr Pro Thr Pro Glu Ile Gly Thr Ile Asn
1               5                   10                  15

Asn Asp Trp Ala Asp Ala Leu Glu Gln Ser Met Asp Ile Leu Ser Gln
            20                  25                  30

Leu Thr Leu Pro Glu Lys Val Asn Ile Thr Thr Gly Thr Gly Trp Met
        35                  40                  45

Gly Gly Gln Cys Val Gly Asn Thr Gly Gly Val Pro Arg Leu Gly Ile
    50                  55                  60

Lys Gly Leu Cys Leu Gln Asp Gly Pro Leu Gly Val Arg Phe Ala Asp
65                  70                  75                  80

Phe Val Asn Val Phe Pro Cys Gln Asn Ala Met Ala Ala Thr Phe Asp
                85                  90                  95

Arg Ile Leu Val His Gln Arg Gly Thr Ala Ile Gly His Gln Ser Lys
            100                 105                 110

Leu Lys Gly Val Asp Val His Leu Gly Pro Val Val Gly Pro Ile Gly
        115                 120                 125

Arg His Ala Thr Gly Gly Arg Asn Trp Glu Gly Phe Ser Pro Asp Pro
    130                 135                 140

Tyr Leu Ser Gly Lys Leu Ala Tyr Glu Ala Ile Gln Gly Ile Gln Glu
145                 150                 155                 160

Glu Asn Val Leu Ala Thr Ile Lys His Phe Ile Gly Asn Glu Gln Asp
                165                 170                 175

His Tyr Arg Arg Ala Gln Glu Trp Arg Asp Gly Phe Asn Phe Thr Gly
            180                 185                 190

Leu Lys Leu Pro Val Ser Ser Asn Asn Ile Asp Asp Arg Ala Leu His
        195                 200                 205

Glu Leu Tyr Met Trp Pro Phe Ala Asp Ala Val Lys Ala Gly Val Gly
    210                 215                 220

Ser Val Met Cys Ala Tyr Asn Asn Val Asn Gly Thr Gln Ala Cys Gln
225                 230                 235                 240

Asn Ser Asp Leu Leu Asn Gly Lys Leu Lys Ser Glu Leu Gly Phe Gln
                245                 250                 255

Gly Phe Val Met Ser Asp Trp Phe Ala Gln Gly Asn Gly Val Ala Asn
            260                 265                 270

Ala Leu Ala Gly Met Asp Met Ser Met Pro Gly Thr Asp Val Asp Glu
        275                 280                 285

Phe Glu Thr Val Phe Trp Gly Glu Gln Leu Thr Arg Met Val Ala Asn
    290                 295                 300

Gly Thr Leu Pro Glu Ser Arg Leu Asp Asp Met Val Leu Arg Ile Leu
305                 310                 315                 320

Thr Pro Leu Met Tyr Phe Gly Ile Asp Asp Arg Glu Pro Asn Phe Ala
                325                 330                 335

Ser Phe Val Asp Thr Thr Val Gly Ser Pro Tyr Pro Ala Ala Lys Asn
            340                 345                 350

Pro Lys Gln Val Gln Asp Lys Ile Val Asn Tyr His Leu Asp Val Arg
        355                 360                 365

Asn Gln Phe Ser Ala Asn Val Ala Leu Glu Ser Ala Arg Gly Ala Ile
    370                 375                 380

Val Leu Leu Phe Asn Ser Gly Val Leu Pro Leu Lys Asp Lys Ser Asn
385                 390                 395                 400

Ile Gly Val Phe Gly Val Gly Ser Val Ile Gly Pro Asn Gly Ala Ile
                405                 410                 415

Cys Glu Asn Met Gln Cys Ser Asp Gly Ala Leu Ile Glu Gly Trp Gly
            420                 425                 430

Ser Gly Thr Ala Tyr Pro Thr Glu Tyr Glu Ser Pro Tyr Glu Ala Leu
        435                 440                 445

His Lys Lys Ala Ser Leu Leu Asp Val His Val Thr Gly Thr Ser Glu
    450                 455                 460

Ser Trp Asp Met Ser Leu Pro Ile Glu Ile Ala Gly Ser Thr Asp Ile
465                 470                 475                 480

Asn Ile Val Tyr Val Leu Ala Ala Ser Gly Glu Ser Thr Ala Ser Val
                485                 490                 495

Asp Gly Asn Ile Gly Asp Arg Asn Asn Val Ser Leu Trp His Asn Gly
            500                 505                 510

Asp Glu Leu Ile Asn Ala Val Ala Asp Gln Gly Glu Thr Val Val Val
        515                 520                 525

Val Thr Thr Val Gly Gln Val Asp Met Ser Glu Trp Leu Phe His Pro
    530                 535                 540

Asn Val Ser Ala Val Leu Leu Thr Ala Pro Ala Gly Asp Tyr Gly Gly
545                 550                 555                 560

Lys Ala Met Ala Asp Val Leu Phe Gly Glu Val Asn Pro Ser Gly Lys
                565                 570                 575

Leu Pro Tyr Thr Leu Ala Asp Asp Ile Ser Glu Tyr Ile Pro Ile Val
            580                 585                 590

Thr Glu Ile Pro Ala Asp Gly Ala Pro Gln Ser Asp Phe Val Glu Gly
        595                 600                 605

Ile Tyr Leu Asp Tyr Lys Trp Tyr Asp Lys Phe Glu Lys Thr Pro Leu
    610                 615                 620

Tyr Glu Phe Gly Tyr Gly Leu Ser Tyr Thr Asn Tyr Thr Phe Thr Asp
625                 630                 635                 640

Leu Ser Leu Asp Ile Lys Glu Ile Asp Glu Phe Leu Pro Ala Arg Pro
                645                 650                 655

Asp Pro Val Val Val Thr Lys Pro Glu Asp Phe Pro Ile Asp Leu Ala
            660                 665                 670

Asp Leu Tyr Val Pro Asp Gly Phe Glu Met Ile Asp Gly Leu Val Tyr
        675                 680                 685

Pro Trp Ile Gln Asn Val Ser Val Pro Leu Ser Asp Ser Glu Ala Gln
    690                 695                 700

Phe Pro Phe Ala Asp Gly Ala Gly His Ile Ser Asp Ala Ser Gly Gly
705                 710                 715                 720

Val Gly Gly His Pro Trp Leu Trp Ser Val Ala Ala Thr Ile Thr His
                725                 730                 735

Asn Thr Thr Asn Cys Gly Asp Val Ala Gly Arg Ala Val Ser Gln Leu
            740                 745                 750

Tyr Ile Ala Phe Pro Glu Thr Cys Val Asp Val Pro Leu Val Gln Leu
        755                 760                 765

Arg Gly Phe Asp Lys Ser Gly Met Leu Asn Pro Gly Glu Ser Gln Glu

770                 775                 780

Thr Val Tyr Asn Leu His Trp Arg Asp Leu Ala Ile Trp Asp Val Glu
785                 790                 795                 800

Leu Gln Ser Trp Arg Val Gln Arg Gly Glu Tyr Ala Val Phe Ile Gly
                805                 810                 815

His Ser Ser Arg Asp Phe Glu Leu Phe Asp Ser Phe Thr Leu Glu
            820                 825                 830

<210>  16
<211>  854
<212>  PRT
<213>  Artificial Sequence

<220>
<223>  BGL2 consensus sequence

<220>
<221>  VARIANT
<222>  (1)..(1)
<223>  Replace = Phe

<220>
<221>  VARIANT
<222>  (5)..(5)
<223>  Replace = Ala

<220>
<221>  VARIANT
<222>  (7)..(7)
<223>  Replace = Arg

<220>
<221>  VARIANT
<222>  (8)..(8)
<223>  Replace = Ala

<220>
<221>  VARIANT
<222>  (9)..(9)
<223>  Replace = Ser

<220>
<221>  VARIANT
<222>  (25)..(25)
<223>  Replace = Ile

<220>
<221>  VARIANT
<222>  (26)..(26)
<223>  Replace = Leu

<220>
<221>  VARIANT
<222>  (29)..(29)
<223>  Replace = Arg

<220>
<221>  VARIANT
<222>  (32)..(32)
<223>  Replace = Ala

<220>
<221>  VARIANT
<222>  (33)..(33)
<223>  Replace = Thr

<220>
<221>  VARIANT
<222>  (35)..(35)
<223>  Replace = Met

<220>
<221>  VARIANT
<222>  (36)..(36)
<223>  Replace = Glu or Asn

<220>
<221>  VARIANT
<222>  (37)..(37)
<223>  Replace = Ala

<220>
<221>  VARIANT
<222>  (40)..(40)
<223>  Replace = Gly, Asn or Ser

<220>
<221>  VARIANT
<222>  (41)..(41)
<223>  Replace = Leu

<220>
<221>  VARIANT
<222>  (48)..(48)
<223>  Replace = Leu or Tyr

<220>
<221>  VARIANT
<222>  (55)..(55)
<223>  Replace = Ser

<220>
<221>  VARIANT
<222>  (69)..(69)
<223>  Replace = Ser

<220>
<221>  VARIANT
<222>  (70)..(70)
<223>  Replace = Pro

<220>
<221>  VARIANT
<222>  (79)..(79)
<223>  Replace = Ile

<220>
<221>  VARIANT

<222>  (88)..(88)
<223>  Replace = Val

<220>
<221>  VARIANT
<222>  (90)..(90)
<223>  Replace = Leu

<220>
<221>  VARIANT
<222>  (91)..(91)
<223>  Replace = Thr

<220>
<221>  VARIANT
<222>  (93)..(93)
<223>  Replace = Lys

<220>
<221>  VARIANT
<222>  (96)..(96)
<223>  Replace = Ala

<220>
<221>  VARIANT
<222>  (103)..(103)
<223>  Replace = Met

<220>
<221>  VARIANT
<222>  (109)..(109)
<223>  Replace = Arg

<220>
<221>  VARIANT
<222>  (110)..(110)
<223>  Replace = Lys

<220>
<221>  VARIANT
<222>  (113)..(113)
<223>  Replace = Gln

<220>
<221>  VARIANT
<222>  (117)..(117)
<223>  Replace = Ala

<220>
<221>  VARIANT
<222>  (142)..(142)
<223>  Replace = Asn

<220>
<221>  VARIANT
<222>  (145)..(145)
<223>  Replace = Gly

<220>
<221>  VARIANT
<222>  (165)..(165)
<223>  Replace = Gln

<220>
<221>  VARIANT
<222>  (168)..(168)
<223>  Replace = Glu

<220>
<221>  VARIANT
<222>  (172)..(172)
<223>  Replace = Gly

<220>
<221>  VARIANT
<222>  (182)..(182)
<223>  Replace = Met or Leu

<220>
<221>  VARIANT
<222>  (199)..(199)
<223>  Replace = Tyr

<220>
<221>  VARIANT
<222>  (201)..(201)
<223>  Replace = Tyr

<220>
<221>  VARIANT
<222>  (217)..(217)
<223>  Replace = Met

<220>
<221>  VARIANT
<222>  (229)..(229)
<223>  Replace = Ile

<220>
<221>  VARIANT
<222>  (245)..(245)
<223>  Replace = Ser

<220>
<221>  VARIANT
<222>  (252)..(252)
<223>  Replace = Ala

<220>
<221>  VARIANT
<222>  (253)..(253)
<223>  Replace = Phe or Ala

<220>
<221>  VARIANT
<222>  (254)..(254)
<223>  Replace = Leu

<220>
<221>  VARIANT
<222>  (259)..(259)
<223>  Replace = Ile

<220>
<221>  VARIANT
<222>  (263)..(263)

<223>  Replace = Phe

<220>
<221>  VARIANT
<222>  (269)..(269)
<223>  Replace = Ile

<220>
<221>  VARIANT
<222>  (299)..(299)
<223>  Replace = Gly

<220>
<221>  VARIANT
<222>  (300)..(300)
<223>  Replace = Leu

<220>
<221>  VARIANT
<222>  (302)..(302)
<223>  Replace = Ile

<220>
<221>  VARIANT
<222>  (311)..(311)
<223>  Replace = Arg

<220>
<221>  VARIANT
<222>  (312)..(312)
<223>  Replace = Met

<220>
<221>  VARIANT
<222>  (318)..(318)
<223>  Replace = Val

<220>
<221>  VARIANT
<222>  (319)..(319)
<223>  Replace = Glu

<220>
<221>  VARIANT
<222>  (320)..(320)
<223>  Replace = Glu or Gln

<220>
<221>  VARIANT
<222>  (321)..(321)
<223>  Replace = Ser

<220>
<221>  VARIANT
<222>  (334)..(334)
<223>  Replace = Ile

<220>
<221>  VARIANT
<222>  (335)..(335)
<223>  Replace = Leu

<220>

<221>  VARIANT
<222>  (339)..(339)
<223>  Replace = Leu

<220>
<221>  VARIANT
<222>  (342)..(342)
<223>  Replace = Thr or Arg

<220>
<221>  VARIANT
<222>  (343)..(343)
<223>  Replace = Asp or Thr

<220>
<221>  VARIANT
<222>  (352)..(352)
<223>  Replace = Glu

<220>
<221>  VARIANT
<222>  (354)..(354)
<223>  Replace = Tyr

<220>
<221>  VARIANT
<222>  (355)..(355)
<223>  Replace = Gly

<220>
<221>  VARIANT
<222>  (356)..(356)
<223>  Replace = Leu

<220>
<221>  VARIANT
<222>  (357)..(357)
<223>  Replace = Glu or Gln

<220>
<221>  VARIANT
<222>  (358)..(358)
<223>  Replace = Tyr or Phe

<220>
<221>  VARIANT
<222>  (359)..(359)
<223>  Replace = Pro

<220>
<221>  VARIANT
<222>  (361)..(361)
<223>  Replace = Asn

<220>
<221>  VARIANT
<222>  (362)..(362)
<223>  Replace = Asp

<220>
<221>  VARIANT
<222>  (363)..(363)
<223>  Replace = Gly or Arg

<220>
<221>  VARIANT
<222>  (365)..(365)
<223>  Replace = Arg or Pro

<220>
<221>  VARIANT
<222>  (367)..(367)
<223>  Replace = Ile

<220>
<221>  VARIANT
<222>  (369)..(369)
<223>  Replace = Ile

<220>
<221>  VARIANT
<222>  (371)..(371)
<223>  Replace = Gln

<220>
<221>  VARIANT
<222>  (373)..(373)
<223>  Replace = Val

<220>
<221>  VARIANT
<222>  (374)..(374)
<223>  Replace = Glu

<220>
<221>  VARIANT
<222>  (378)..(378)
<223>  Replace = Gln, Ala or Ser

<220>
<221>  VARIANT
<222>  (379)..(379)
<223>  Replace = Tyr

<220>
<221>  VARIANT
<222>  (381)..(381)
<223>  Replace = Ile

<220>
<221>  VARIANT
<222>  (382)..(382)
<223>  Replace = Gln

<220>
<221>  VARIANT
<222>  (385)..(385)
<223>  Replace = Gln

<220>
<221>  VARIANT
<222>  (389)..(389)
<223>  Replace = Thr

<220>
<221>  VARIANT

<222>  (398)..(398)
<223>  Replace = Glu

<220>
<221>  VARIANT
<222>  (399)..(399)
<223>  Replace = Gln

<220>
<221>  VARIANT
<222>  (400)..(400)
<223>  Replace = Lys or Ser

<220>
<221>  VARIANT
<222>  (401)..(401)
<223>  Replace = Thr

<220>
<221>  VARIANT
<222>  (405)..(405)
<223>  Replace = Asn or Asp

<220>
<221>  VARIANT
<222>  (406)..(406)
<223>  Replace = nothing

<220>
<221>  VARIANT
<222>  (407)..(407)
<223>  Replace = Ala or nothing

<220>
<221>  VARIANT
<222>  (408)..(408)
<223>  Replace = Lys

<220>
<221>  VARIANT
<222>  (409)..(409)
<223>  Replace = Leu or Val

<220>
<221>  VARIANT
<222>  (410)..(410)
<223>  Replace = Thr

<220>
<221>  VARIANT
<222>  (411)..(411)
<223>  Replace = Thr or Lys

<220>
<221>  VARIANT
<222>  (412)..(412)
<223>  Replace = Ile

<220>
<221>  VARIANT
<222>  (417)..(417)
<223>  Replace = Val

<220>
<221>  VARIANT
<222>  (420)..(420)
<223>  Replace = Lys or Thr

<220>
<221>  VARIANT
<222>  (423)..(423)
<223>  Replace = Asn

<220>
<221>  VARIANT
<222>  (425)..(425)
<223>  Replace = Ser

<220>
<221>  VARIANT
<222>  (426)..(426)
<223>  Replace = nothing

<220>
<221>  VARIANT
<222>  (427)..(427)
<223>  Replace = nothing

<220>
<221>  VARIANT
<222>  (430)..(430)
<223>  Replace = Gln

<220>
<221>  VARIANT
<222>  (433)..(433)
<223>  Replace = Glu

<220>
<221>  VARIANT
<222>  (436)..(436)
<223>  Replace = Ser

<220>
<221>  VARIANT
<222>  (441)..(441)
<223>  Replace = Gln or Leu

<220>
<221>  VARIANT
<222>  (451)..(451)
<223>  Replace = Ser

<220>
<221>  VARIANT
<222>  (454)..(454)
<223>  Replace = Leu

<220>
<221>  VARIANT
<222>  (458)..(458)
<223>  Replace = Asp or Asn

<220>
<221>  VARIANT
<222>  (465)..(465)

<223>  Replace = Ser or Glu

<220>
<221>  VARIANT
<222>  (466)..(466)
<223>  Replace = Lys

<220>
<221>  VARIANT
<222>  (467)..(467)
<223>  Replace = Asn

<220>
<221>  VARIANT
<222>  (468)..(468)
<223>  Replace = Lys

<220>
<221>  VARIANT
<222>  (470)..(470)
<223>  Replace = Gln

<220>
<221>  VARIANT
<222>  (471)..(471)
<223>  Replace = Ile

<220>
<221>  VARIANT
<222>  (476)..(476)
<223>  Replace = His or Glu

<220>
<221>  VARIANT
<222>  (482)..(482)
<223>  Replace = Thr

<220>
<221>  VARIANT
<222>  (483)..(483)
<223>  Replace = Ile or Arg

<220>
<221>  VARIANT
<222>  (486)..(486)
<223>  Replace = Lys or Gln

<220>
<221>  VARIANT
<222>  (488)..(488)
<223>  Replace = Ala

<220>
<221>  VARIANT
<222>  (490)..(490)
<223>  Replace = Glu

<220>
<221>  VARIANT
<222>  (491)..(491)
<223>  Replace = Ser

<220>

<221>  VARIANT
<222>  (495)..(495)
<223>  Replace = Ile

<220>
<221>  VARIANT
<222>  (508)..(508)
<223>  Replace = Pro

<220>
<221>  VARIANT
<222>  (513)..(513)
<223>  Replace = Tyr

<220>
<221>  VARIANT
<222>  (527)..(527)
<223>  Replace = Glu or Gln

<220>
<221>  VARIANT
<222>  (530)..(530)
<223>  Replace = Arg

<220>
<221>  VARIANT
<222>  (531)..(531)
<223>  Replace = Thr

<220>
<221>  VARIANT
<222>  (535)..(535)
<223>  Replace = Lys or Gln

<220>
<221>  VARIANT
<222>  (536)..(536)
<223>  Replace = Asn

<220>
<221>  VARIANT
<222>  (537)..(537)
<223>  Replace = Ala

<220>
<221>  VARIANT
<222>  (556)..(556)
<223>  Replace = Asp

<220>
<221>  VARIANT
<222>  (557)..(557)
<223>  Replace = His

<220>
<221>  VARIANT
<222>  (575)..(575)
<223>  Replace = Ser or Lys

<220>
<221>  VARIANT
<222>  (580)..(580)
<223>  Replace = Val

<220>
<221>  VARIANT
<222>  (585)..(585)
<223>  Replace = Val

<220>
<221>  VARIANT
<222>  (597)..(597)
<223>  Replace = Arg

<220>
<221>  VARIANT
<222>  (599)..(599)
<223>  Replace = Asn

<220>
<221>  VARIANT
<222>  (603)..(603)
<223>  Replace = Val

<220>
<221>  VARIANT
<222>  (606)..(606)
<223>  Replace = Val

<220>
<221>  VARIANT
<222>  (609)..(609)
<223>  Replace = Ile

<220>
<221>  VARIANT
<222>  (611)..(611)
<223>  Replace = Glu or Ser

<220>
<221>  VARIANT
<222>  (618)..(618)
<223>  Replace = Phe

<220>
<221>  VARIANT
<222>  (632)..(632)
<223>  Replace = Ala

<220>
<221>  VARIANT
<222>  (633)..(633)
<223>  Replace = Leu

<220>
<221>  VARIANT
<222>  (634)..(634)
<223>  Replace = Gln

<220>
<221>  VARIANT
<222>  (635)..(635)
<223>  Replace = Lys

<220>
<221>  VARIANT

<222>  (652)..(652)
<223>  Replace = Val or Leu

<220>
<221>  VARIANT
<222>  (653)..(653)
<223>  Replace = Gly

<220>
<221>  VARIANT
<222>  (656)..(656)
<223>  Replace = Asp or Gln

<220>
<221>  VARIANT
<222>  (657)..(657)
<223>  Replace = Val

<220>
<221>  VARIANT
<222>  (658)..(658)
<223>  Replace = Leu or Lys

<220>
<221>  VARIANT
<222>  (659)..(659)
<223>  Replace = Tyr

<220>
<221>  VARIANT
<222>  (662)..(662)
<223>  Replace = Asn

<220>
<221>  VARIANT
<222>  (663)..(663)
<223>  Replace = Ser

<220>
<221>  VARIANT
<222>  (665)..(665)
<223>  Replace = Ser

<220>
<221>  VARIANT
<222>  (670)..(670)
<223>  Replace = Ala

<220>
<221>  VARIANT
<222>  (672)..(672)
<223>  Replace = Ser

<220>
<221>  VARIANT
<222>  (673)..(673)
<223>  Replace = Pro

<220>
<221>  VARIANT
<222>  (675)..(675)
<223>  Replace = Ser

<220>
<221>  VARIANT
<222>  (676)..(676)
<223>  Replace = Ile or Asp

<220>
<221>  VARIANT
<222>  (678)..(678)
<223>  Replace = Asn

<220>
<221>  VARIANT
<222>  (681)..(681)
<223>  Replace = Lys or Ala

<220>
<221>  VARIANT
<222>  (682)..(682)
<223>  Replace = Asn

<220>
<221>  VARIANT
<222>  (683)..(683)
<223>  Replace = Ala or Arg

<220>
<221>  VARIANT
<222>  (685)..(685)
<223>  Replace = Pro

<220>
<221>  VARIANT
<222>  (686)..(686)
<223>  Replace = Thr

<220>
<221>  VARIANT
<222>  (687)..(687)
<223>  Replace = Hir, Ser or Glu

<220>
<221>  VARIANT
<222>  (688)..(688)
<223>  Replace = Leu

<220>
<221>  VARIANT
<222>  (690)..(690)
<223>  Replace = Glu or Gly

<220>
<221>  VARIANT
<222>  (692)..(692)
<223>  Replace = Ile

<220>
<221>  VARIANT
<222>  (695)..(695)
<223>  Replace = Ser

<220>
<221>  VARIANT
<222>  (696)..(696)

<223>  Replace = Glu

<220>
<221>  VARIANT
<222>  (697)..(697)
<223>  Replace = Phe

<220>
<221>  VARIANT
<222>  (698)..(698)
<223>  Replace = Asp or Lys

<220>
<221>  VARIANT
<222>  (699)..(699)
<223>  Replace = Gln

<220>
<221>  VARIANT
<222>  (700)..(700)
<223>  Replace = Ile

<220>
<221>  VARIANT
<222>  (701)..(701)
<223>  Replace = Asn, Lys or Pro

<220>
<221>  VARIANT
<222>  (702)..(702)
<223>  Replace = Asn

<220>
<221>  VARIANT
<222>  (706)..(706)
<223>  Replace = Thr

<220>
<221>  VARIANT
<222>  (710)..(710)
<223>  Replace = Arg or Ser

<220>
<221>  VARIANT
<222>  (713)..(713)
<223>  Replace = Asp

<220>
<221>  VARIANT
<222>  (714)..(714)
<223>  Replace = Leu

<220>
<221>  VARIANT
<222>  (715)..(715)
<223>  Replace = Ala or Asn

<220>
<221>  VARIANT
<222>  (716)..(716)
<223>  Replace = nothing

<220>

<221>  VARIANT
<222>  (719)..(719)
<223>  Replace = Ile

<220>
<221>  VARIANT
<222>  (720)..(720)
<223>  Replace = Ser

<220>
<221>  VARIANT
<222>  (721)..(721)
<223>  Replace = His or Arg

<220>
<221>  VARIANT
<222>  (728)..(728)
<223>  Replace = Phe

<220>
<221>  VARIANT
<222>  (730)..(730)
<223>  Replace = Ser or Val

<220>
<221>  VARIANT
<222>  (731)..(731)
<223>  Replace = Glu or Val

<220>
<221>  VARIANT
<222>  (735)..(735)
<223>  Replace = Ser or Gly

<220>
<221>  VARIANT
<222>  (752)..(752)
<223>  Replace = Pro or Thr

<220>
<221>  VARIANT
<222>  (753)..(753)
<223>  Replace = Ala

<220>
<221>  VARIANT
<222>  (754)..(754)
<223>  Replace = Leu

<220>
<221>  VARIANT
<222>  (756)..(756)
<223>  Replace = Val

<220>
<221>  VARIANT
<222>  (757)..(757)
<223>  Replace = Ala

<220>
<221>  VARIANT
<222>  (760)..(760)
<223>  Replace = Ile

<220>
<221>  VARIANT
<222>  (763)..(763)
<223>  Replace = His

<220>
<221>  VARIANT
<222>  (770)..(770)
<223>  Replace = Ala

<220>
<221>  VARIANT
<222>  (774)..(774)
<223>  Replace = Leu

<220>
<221>  VARIANT
<222>  (777)..(777)
<223>  Replace = Ser

<220>
<221>  VARIANT
<222>  (783)..(783)
<223>  Replace = Glu

<220>
<221>  VARIANT
<222>  (784)..(784)
<223>  Replace = Gly

<220>
<221>  VARIANT
<222>  (786)..(786)
<223>  Replace = Arg or Gln

<220>
<221>  VARIANT
<222>  (801)..(801)
<223>  Replace = Pro

<220>
<221>  VARIANT
<222>  (804)..(804)
<223>  Replace = Asp

<220>
<221>  VARIANT
<222>  (805)..(805)
<223>  Replace = Gln

<220>
<221>  VARIANT
<222>  (806)..(806)
<223>  Replace = Gln or Arg

<220>
<221>  VARIANT
<222>  (808)..(808)
<223>  Replace = Val

<220>
<221>  VARIANT

<222>  (809)..(809)
<223>  Replace = Gln

<220>
<221>  VARIANT
<222>  (811)..(811)
<223>  Replace = Asp

<220>
<221>  VARIANT
<222>  (812)..(812)
<223>  Replace = Ile

<220>
<221>  VARIANT
<222>  (823)..(823)
<223>  Replace = Ser

<220>
<221>  VARIANT
<222>  (824)..(824)
<223>  Replace = Ile

<220>
<221>  VARIANT
<222>  (833)..(833)
<223>  Replace = Lys or Asp

<220>
<221>  VARIANT
<222>  (835)..(835)
<223>  Replace = Thr or Glu

<220>
<221>  VARIANT
<222>  (836)..(836)
<223>  Replace = Ile

<220>
<221>  VARIANT
<222>  (837)..(837)
<223>  Replace = Phe

<220>
<221>  VARIANT
<222>  (838)..(838)
<223>  Replace = Ile

<220>
<221>  VARIANT
<222>  (845)..(845)
<223>  Replace = Leu

<220>
<221>  VARIANT
<222>  (848)..(848)
<223>  Replace = Ser or Ala

<220>
<221>  VARIANT
<222>  (850)..(850)
<223>  Replace = Thr or Lys

<220>
<221>  VARIANT
<222>  (854)..(854)
<223>  Replace = Lys or Asn

<400>  16

Ala Asp Pro Phe Ser Asp Lys Asp Ala Tyr Lys His Ser Pro Pro Tyr
1               5                   10                  15

Tyr Pro Ala Pro Glu Ile Gly Arg Val Pro Thr Asp Leu Arg Trp Arg
            20                  25                  30

Ala Ala Leu Lys Val Ala Gln Asp Met Val Ala Asn Met Thr Leu Ile
        35                  40                  45

Glu Lys Val Asn Ile Thr Thr Gly Thr Gly Trp Glu Met Gly Pro Cys
    50                  55                  60

Val Gly Asn Thr Gly Thr Val Glu Arg Leu Gly Ile Lys Ser Leu Cys
65                  70                  75                  80

Leu Gln Asp Gly Pro Leu Gly Ile Arg Phe Ala Asp Leu Ile Thr Thr
                85                  90                  95

Phe Pro Ala Gly Ile Thr Ile Ala Ser Thr Phe Ser Lys Gln Leu Val
            100                 105                 110

Arg Glu Arg Gly Glu Ala Met Gly Arg Glu Asn Arg Arg Lys Gly Val
        115                 120                 125

Asp Ile Thr Leu Ser Pro Val Val Gly Pro Leu Gly Arg His Ala Asn
    130                 135                 140

Ala Gly Arg Ile Trp Glu Gly Phe Ser Ala Asp Pro Tyr Leu Ala Gly
145                 150                 155                 160

Lys Leu Ala Ala Glu Ala Val Thr Gly Ile Gln Ser Gln Asn Val Met
                165                 170                 175

Ala Val Val Lys His Ile Val Gly Asn Glu Gln Glu His Phe Arg Gln
            180                 185                 190

Leu Gly Glu Trp Gln Gly Phe Gly Phe Lys Asp Leu Lys Gln Pro Leu
        195                 200                 205

Ser Ser Asn Ile Asp Asp Arg Thr Leu Asn Glu Ala Tyr Leu Trp Pro
    210                 215                 220

Phe Ala Asp Ala Val Arg Ala Asn Val Gly Ser Val Met Cys Ser Tyr
225                 230                 235                 240

Gln Gln Ile Asn Gly Ser Gln Gly Cys Gln Asn Ser His Ile Leu Asn
                245                 250                 255

Gly Lys Leu Lys Glu Glu Met Gly Phe Gln Gly Phe Val Met Ser Asp
            260                 265                 270

Trp Leu Ala Gln Arg Ser Gly Val Ala Ser Val Leu Ala Gly Leu Asp
        275                 280                 285

Met Ser Met Pro Gly Asp Gly Leu Val Trp Ala Asp Gly Val Pro Leu
    290                 295                 300

Met Gly Tyr Glu Leu Thr Lys Ser Val Leu Asn Gly Thr Ile Asp Ile
305                 310                 315                 320

Asn Arg Val Asp Asp Met Val Thr Arg Ile Leu Thr Pro Leu Met Tyr
                325                 330                 335

Leu Ser Ile Thr Pro Gly Asn Pro Asn Phe Ser Ser Trp Thr Asn Asp
            340                 345                 350

Thr Thr Ser Tyr Lys Ile Tyr Gly Ala Lys Ala Gly Gly Asn Val Thr
        355                 360                 365

Val Asn Arg His Ile Asp Val Arg Asp Pro Trp Thr Thr Lys Ala Ala
    370                 375                 380

Leu Asp Gly Ala Asn Ala Ala Leu Val Leu Leu Lys Asn Val Lys Asn
385                 390                 395                 400

Ala Leu Pro Leu Lys Pro Thr Asn Ile Gly Asn Leu Asn Ile Phe Gly
                405                 410                 415

Ile Gly Ser Ala Thr Gly Pro Leu Gly Ala Val Cys Gly Glu Asn Met
            420                 425                 430

Gln Cys Ser Asp Gly Ala Leu Ile Glu Gly Trp Gly Ser Gly Ser Val
        435                 440                 445

Tyr Pro Thr Asp Tyr Gln Ser Pro Tyr Glu Ala Ile Lys Glu Arg Ala
    450                 455                 460

Ala Gln Asp Asn Ile Thr Val Gly Gly Thr Thr Gln Ser Trp Gly Asn
465                 470                 475                 480

Leu Ser Asn Val Glu Ile Leu Ser Ala Ala Ala Asp Ala Ser Val Val
                485                 490                 495

Phe Val Leu Ser Asp Ser Gly Glu Ser Thr Gly Ile Val Asp Gly Asn
            500                 505                 510

Ile Gly Asp Arg Asn Asn Leu Thr Leu Trp His Asn Gly Asp Ala Val
        515                 520                 525

Val Lys Ala Val Ala Ser Arg Asn Pro Asn Thr Ile Val Val Val Thr
    530                 535                 540

Thr Val Gly Pro Val Asn Leu Glu Lys Trp Ile Asn Asn Pro Asn Val
545                 550                 555                 560

Thr Ala Val Leu Leu Thr Gly Pro Ala Gly Asp Phe Gly Gly Arg Ala
                565                 570                 575

Ala Ala Ser Ile Leu Phe Gly Asp Ile Ala Pro Ser Gly Lys Leu Pro
            580                 585                 590

Phe Thr Ile Ala Lys Asn Asp Thr Asp Tyr Ile Pro Leu Thr Thr Lys
        595                 600                 605

Val Pro Lys Asp Gly Leu Pro Gln Asp Tyr Phe Thr Glu Gly Thr Leu
    610                 615                 620

Leu Asp Tyr Lys Arg Phe Asp Glu Asn Lys Val Thr Pro Arg Phe Glu
625                 630                 635                 640

Phe Gly Tyr Gly Leu Ser Tyr Ser Asn Ile Thr Met Glu Asn Leu Glu
                645                 650                 655

Ala Arg Phe Ala Phe Pro Asn Ile Pro Glu Phe Leu Pro Thr Pro Phe
            660                 665                 670

Ala Pro Leu Asn Pro His Lys Pro Asn Asp Val Phe Thr Pro Asn Ala
        675                 680                 685

Asn Gln Ser Val Phe Pro Asp Asp Ile His Pro Leu His Lys Tyr Val
    690                 695                 700

Tyr Pro Tyr Leu Asn Asp Thr Ser Glu Ile Phe Thr Ser Asn Glu Thr
705                 710                 715                 720

Asn Tyr Pro Tyr Pro Glu Gly Tyr Ser Gln Lys Gln Ser Asn Thr Thr
                725                 730                 735

Asn Ile Asn Gly Gly Ala Val Gly Gly Asn Pro Ala Leu Trp Leu Ser
            740                 745                 750

Ser Val Tyr Ile Val His Ser Val Ser Asn Tyr Gly Pro Tyr Asp Thr
        755                 760                 765

Gly Val Val Thr Gln Met Tyr Ile Ala Phe Pro Gln Asp Asn Asp Asp
    770                 775                 780

Leu Lys Thr Ala Pro Arg Gln Leu Arg Gly Phe Glu Arg Ser Glu Leu
785                 790                 795                 800

Lys Val Gly Glu Arg His Gly Ile Leu Tyr Asn Val Gln Trp Arg Asp
                805                 810                 815

Leu Ala Val Trp Asp Val Lys Leu Gln Ser Trp Arg Val Gln Arg Gly
            820                 825                 830

Glu Tyr Lys Val Tyr Val Gly His Ser Ser Arg Asp Phe Val Leu Thr
        835                 840                 845

Thr Ser Phe Thr Leu Gln
    850

<210>  17
<211>  854
<212>  PRT
<213>  Yarrowia galli

<400>  17

Ala Asp Pro Phe Ser Asp Lys Asp Ala Tyr Lys His Ser Pro Pro Tyr
1               5                   10                  15

Tyr Pro Ala Pro Glu Ile Gly Arg Val Pro Thr Asp Leu Arg Trp Arg
            20                  25                  30

Ala Ala Leu Lys Val Ala Gln Asp Met Val Ala Asn Met Thr Leu Ile
        35                  40                  45

Glu Lys Val Asn Ile Thr Thr Gly Thr Gly Trp Glu Met Gly Pro Cys
    50                  55                  60

Val Gly Asn Thr Gly Thr Val Glu Arg Leu Gly Ile Lys Ser Leu Cys
65                  70                  75                  80

Leu Gln Asp Gly Pro Leu Gly Ile Arg Phe Ala Asp Leu Ile Thr Thr
                85                  90                  95

Phe Pro Ala Gly Ile Thr Ile Ala Ser Thr Phe Ser Lys Gln Leu Val
        100                 105                 110

Arg Glu Arg Gly Glu Ala Met Gly Arg Glu Asn Arg Arg Lys Gly Val
        115                 120                 125

Asp Ile Thr Leu Ser Pro Val Val Gly Pro Leu Gly Arg His Ala Asn
    130                 135                 140

Ala Gly Arg Ile Trp Glu Gly Phe Ser Ala Asp Pro Tyr Leu Ala Gly
145                 150                 155                 160

Lys Leu Ala Ala Glu Ala Val Thr Gly Ile Gln Ser Gln Asn Val Met
                165                 170                 175

Ala Val Val Lys His Ile Val Gly Asn Glu Gln Glu His Phe Arg Gln
        180                 185                 190

Leu Gly Glu Trp Gln Gly Phe Gly Phe Lys Asp Leu Lys Gln Pro Leu
        195                 200                 205

Ser Ser Asn Ile Asp Asp Arg Thr Leu Asn Glu Ala Tyr Leu Trp Pro
    210                 215                 220

Phe Ala Asp Ala Val Arg Ala Asn Val Gly Ser Val Met Cys Ser Tyr
225                 230                 235                 240

Gln Gln Ile Asn Gly Ser Gln Gly Cys Gln Asn Ser His Ile Leu Asn
                245                 250                 255

Gly Lys Leu Lys Glu Glu Met Gly Phe Gln Gly Phe Val Met Ser Asp
        260                 265                 270

Trp Leu Ala Gln Arg Ser Gly Val Ala Ser Val Leu Ala Gly Leu Asp
        275                 280                 285

Met Ser Met Pro Gly Asp Gly Leu Val Trp Ala Asp Gly Val Pro Leu
    290                 295                 300

Met Gly Tyr Glu Leu Thr Lys Ser Val Leu Asn Gly Thr Ile Asp Ile
305                 310                 315                 320

Asn Arg Val Asp Asp Met Val Thr Arg Ile Leu Thr Pro Leu Met Tyr
                325                 330                 335

Leu Ser Ile Thr Pro Gly Asn Pro Asn Phe Ser Ser Trp Thr Asn Asp

340                 345                 350

Thr Thr Ser Tyr Lys Ile Tyr Gly Ala Lys Ala Gly Gly Asn Val Thr
        355                 360                 365

Val Asn Arg His Ile Asp Val Arg Asp Pro Trp Thr Thr Lys Ala Ala
    370                 375                 380

Leu Asp Gly Ala Asn Ala Ala Leu Val Leu Leu Lys Asn Val Lys Asn
385                 390                 395                 400

Ala Leu Pro Leu Lys Pro Thr Asn Ile Gly Asn Leu Asn Ile Phe Gly
                405                 410                 415

Ile Gly Ser Ala Thr Gly Pro Leu Gly Ala Val Cys Gly Glu Asn Met
            420                 425                 430

Gln Cys Ser Asp Gly Ala Leu Ile Glu Gly Trp Gly Ser Gly Ser Val
        435                 440                 445

Tyr Pro Thr Asp Tyr Gln Ser Pro Tyr Glu Ala Ile Lys Glu Arg Ala
    450                 455                 460

Ala Gln Asp Asn Ile Thr Val Gly Gly Thr Thr Gln Ser Trp Gly Asn
465                 470                 475                 480

Leu Ser Asn Val Glu Ile Leu Ser Ala Ala Ala Asp Ala Ser Val Val
                485                 490                 495

Phe Val Leu Ser Asp Ser Gly Glu Ser Thr Gly Ile Val Asp Gly Asn
            500                 505                 510

Ile Gly Asp Arg Asn Asn Leu Thr Leu Trp His Asn Gly Asp Ala Val
        515                 520                 525

Val Lys Ala Val Ala Ser Arg Asn Pro Asn Thr Ile Val Val Val Thr
    530                 535                 540

Thr Val Gly Pro Val Asn Leu Glu Lys Trp Ile Asn Asn Pro Asn Val
545                 550                 555                 560

Thr Ala Val Leu Leu Thr Gly Pro Ala Gly Asp Phe Gly Gly Arg Ala
                565                 570                 575

Ala Ala Ser Ile Leu Phe Gly Asp Ile Ala Pro Ser Gly Lys Leu Pro
            580                 585                 590

Phe Thr Ile Ala Lys Asn Asp Thr Asp Tyr Ile Pro Leu Thr Thr Lys
        595                 600                 605

Val Pro Lys Asp Gly Leu Pro Gln Asp Tyr Phe Thr Glu Gly Thr Leu
    610                 615                 620

Leu Asp Tyr Lys Arg Phe Asp Glu Asn Lys Val Thr Pro Arg Phe Glu
625                 630                 635                 640

Phe Gly Tyr Gly Leu Ser Tyr Ser Asn Ile Thr Met Glu Asn Leu Glu
                645                 650                 655

Ala Arg Phe Ala Phe Pro Asn Ile Pro Glu Phe Leu Pro Thr Pro Phe
            660                 665                 670

Ala Pro Leu Asn Pro His Lys Pro Asn Asp Val Phe Thr Pro Asn Ala
        675                 680                 685

Asn Gln Ser Val Phe Pro Asp Asp Ile His Pro Leu His Lys Tyr Val
    690                 695                 700

Tyr Pro Tyr Leu Asn Asp Thr Ser Glu Ile Phe Thr Ser Asn Glu Thr
705                 710                 715                 720

Asn Tyr Pro Tyr Pro Glu Gly Tyr Ser Gln Lys Gln Ser Asn Thr Thr
                725                 730                 735

Asn Ile Asn Gly Gly Ala Val Gly Gly Asn Pro Ala Leu Trp Leu Ser
            740                 745                 750

Ser Val Tyr Ile Val His Ser Val Ser Asn Tyr Gly Pro Tyr Asp Thr
        755                 760                 765

Gly Val Val Thr Gln Met Tyr Ile Ala Phe Pro Gln Asp Asn Asp Asp
    770                 775                 780

Leu Lys Thr Ala Pro Arg Gln Leu Arg Gly Phe Glu Arg Ser Glu Leu
785                 790                 795                 800

Lys Val Gly Glu Arg His Gly Ile Leu Tyr Asn Val Gln Trp Arg Asp
                805                 810                 815

Leu Ala Val Trp Asp Val Lys Leu Gln Ser Trp Arg Val Gln Arg Gly
            820                 825                 830

Glu Tyr Lys Val Tyr Val Gly His Ser Ser Arg Asp Phe Val Leu Thr
        835                 840                 845

Thr Ser Phe Thr Leu Gln
    850

<210>  18
<211>  850
<212>  PRT
<213>  Yarrowia yakushimensis

<400>  18

Asp Pro Phe Ser Asp Lys Ala Ala Tyr Lys His Ser Pro Pro Tyr Tyr
1               5                   10                  15

Pro Ala Pro Glu Ile Gly Arg Ile Pro Thr Asp Leu Arg Trp Ala Ala
            20                  25                  30

Ala Leu Glu Val Ala Gln Asn Leu Val Ala Asn Met Thr Leu Ile Glu
        35                  40                  45

Lys Val Asn Ile Thr Thr Gly Thr Gly Trp Glu Met Gly Pro Cys Val
    50                  55                  60

Gly Asn Thr Ser Pro Val Glu Arg Leu Gly Ile Lys Ser Leu Cys Leu
65                  70                  75                  80

Gln Asp Gly Pro Leu Gly Val Arg Leu Thr Asp Leu Ile Thr Ala Phe
                85                  90                  95

Pro Ala Gly Ile Thr Met Ala Ser Thr Phe Ser Arg Gln Leu Val Arg
            100                 105                 110

Glu Arg Gly Ala Ala Met Gly Arg Glu Asn Arg Arg Lys Gly Val Asp
        115                 120                 125

Ile Thr Leu Ser Pro Val Val Gly Pro Leu Gly Arg Asn Ala Asn Ala
    130                 135                 140

Gly Arg Ile Trp Glu Gly Phe Ser Ala Asp Pro Tyr Leu Ala Gly Lys
145                 150                 155                 160

Leu Ala Ala Glu Ala Val Thr Gly Ile Gln Ser Gln Asn Val Met Ala
                165                 170                 175

Val Val Lys His Leu Val Gly Asn Glu Gln Glu His Phe Arg Gln Leu
            180                 185                 190

Gly Glu Trp Gln Gly Tyr Gly Tyr Lys Asp Leu Lys Gln Pro Leu Ser
        195                 200                 205

Ser Asn Ile Asp Asp Arg Thr Leu Asn Glu Ala Tyr Leu Trp Pro Phe
    210                 215                 220

Ala Asp Ala Ile Arg Ala Asn Val Gly Ser Val Met Cys Ser Tyr Gln
225                 230                 235                 240

Gln Ile Asn Ser Ser Gln Gly Cys Gln Asn Ser Phe Ile Leu Asn Gly
                245                 250                 255

Lys Ile Lys Glu Glu Met Gly Phe Gln Gly Phe Ile Met Ser Asp Trp
            260                 265                 270

Leu Ala Gln Arg Ser Gly Val Ala Ser Val Leu Ala Gly Leu Asp Met
        275                 280                 285

Ser Met Pro Gly Asp Gly Leu Val Trp Gly Leu Gly Ile Pro Leu Met
    290                 295                 300

Gly Tyr Glu Leu Thr Arg Ser Val Leu Asn Gly Thr Ile Asp Glu Ser
305                 310                 315                 320

Arg Val Asp Asp Met Val Thr Arg Ile Leu Thr Pro Ile Leu Tyr Leu
                325                 330                 335

Ser Leu Thr Pro Arg Thr Pro Asn Phe Ser Ser Trp Thr Asn Asp Thr
            340                 345                 350

Tyr Gly Tyr Glu Tyr Tyr Gly Asn Asp Gly Gly Arg Asn Ile Thr Ile
        355                 360                 365

Asn Arg His Val Asp Val Arg Asp Ala Tyr Thr Ile Gln Ala Ala Gln
    370                 375                 380

Asp Gly Ala Asn Ala Ala Leu Val Leu Leu Lys Asn Glu Lys Asn Ala
385                 390                 395                 400

Leu Pro Leu Asp Pro Ala Lys Leu Gly Thr Ile Asn Ile Phe Gly Ile
                405                 410                 415

Gly Ser Lys Thr Gly Asn Leu Ser Cys Gly Gln Asn Met Glu Cys Ser
            420                 425                 430

Asn Gly Ala Leu Ile Gln Gly Trp Gly Ser Gly Ser Val Tyr Pro Thr
        435                 440                 445

Asp Tyr Leu Ser Pro Tyr Asn Ala Ile Lys Glu Arg Ala Ala Gln Asn
    450                 455                 460

Asn Ile Thr Val Gly Gly Thr Thr His Ser Trp Gly Asn Leu Ser Ile
465                 470                 475                 480

Val Glu Lys Leu Ser Ala Ala Ala Asp Ala Ser Val Val Phe Val Leu
                485                 490                 495

Ser Asp Ser Gly Glu Ser Thr Gly Pro Val Asp Gly Asn Tyr Gly Asp
            500                 505                 510

Arg Asn Asn Leu Thr Leu Trp His Asn Gly Asp Gln Val Val Lys Ala
        515                 520                 525

Val Ala Ser Lys Asn Pro Asn Thr Ile Val Val Val Thr Thr Val Gly
    530                 535                 540

Pro Val Asn Leu Glu Lys Trp Ile Asp His Pro Asn Val Thr Ala Val
545                 550                 555                 560

Leu Leu Thr Gly Pro Ala Gly Asp Phe Gly Gly Ser Ala Ala Ala Ser
                565                 570                 575

Ile Leu Phe Gly Asp Ile Ala Pro Ser Gly Lys Leu Pro Phe Thr Ile
            580                 585                 590

Ala Lys Asn Asn Thr Asp Tyr Ile Pro Leu Val Thr Lys Val Pro Glu
        595                 600                 605

Asp Gly Leu Pro Gln Asp Tyr Phe Thr Glu Gly Thr Leu Leu Asp Tyr
    610                 615                 620

Lys Arg Phe Asp Glu Asn Lys Val Thr Pro Arg Phe Glu Phe Gly Tyr
625                 630                 635                 640

Gly Leu Ser Tyr Ser Asn Ile Thr Val Glu Asn Leu Asp Val Leu Phe
                645                 650                 655

Ala Phe Pro Asn Ile Ser Glu Phe Leu Pro Ala Pro Ser Ala Pro Leu
            660                 665                 670

Ile Pro His Lys Pro Lys Asn Arg Phe Thr Pro Ser Leu Asn Glu Ser
        675                 680                 685

Ile Phe Pro Ser Gly Phe Lys Gln Leu Lys Asn Tyr Val Tyr Thr Tyr
    690                 695                 700

Leu Asn Arg Thr Ser Asp Ile Ala Ser Asn Ile Thr His Tyr Pro Tyr

705                 710                 715                 720

Pro Glu Gly Tyr Ser Ser Val Gln Ser Asn Thr Thr Asn Ile Asn Gly
                725                 730                 735

Gly Ala Val Gly Gly Asn Pro Ala Leu Trp Leu Pro Ala Leu Tyr Val
            740                 745                 750

Val His Ser Val Ser Asn Tyr Gly Pro Tyr Asp Thr Gly Ala Val Thr
        755                 760                 765

Gln Leu Tyr Ile Ser Phe Pro Gln Asp Asn Glu Asp Leu Arg Thr Ala
    770                 775                 780

Pro Arg Gln Leu Arg Gly Phe Glu Arg Ser Glu Leu Lys Val Gly Asp
785                 790                 795                 800

Gln Gln Gly Val Leu Tyr Asp Ile Gln Trp Arg Asp Leu Ala Val Trp
                805                 810                 815

Asp Val Lys Leu Gln Ser Trp Arg Val Gln Arg Gly Lys Tyr Thr Val
            820                 825                 830

Tyr Ile Gly His Ser Ser Arg Asp Leu Val Leu Ser Thr Thr Phe Thr
        835                 840                 845

Leu Lys
    850

<210>  19
<211>  850
<212>  PRT
<213>  Yarrowia alimentaria

<400>  19

Asp Pro Phe Ala Asp Arg Asp Ser Tyr Lys His Ser Pro Pro Tyr Tyr
1               5                   10                  15

Pro Ala Pro Glu Ile Gly Arg Val Leu Thr Asp Arg Arg Trp Arg Thr
            20                  25                  30

Ala Met Asn Ala Ala Gln Ser Leu Val Ala Asn Met Thr Leu Tyr Glu
        35                  40                  45

Lys Val Asn Ile Thr Ser Gly Thr Gly Trp Glu Met Gly Pro Cys Val
    50                  55                  60

Gly Asn Thr Gly Thr Val Glu Arg Leu Gly Ile Lys Ser Ile Cys Leu

65                  70                  75                  80

Gln Asp Gly Pro Leu Gly Ile Arg Phe Ala Asp Lys Ile Thr Ala Phe
                85                  90                  95

Pro Ala Gly Ile Thr Ile Ala Ser Thr Phe Ser Arg Lys Leu Val Gln
            100                 105                 110

Glu Arg Gly Ala Ala Met Gly Arg Glu Asn Arg Arg Lys Gly Val Asp
        115                 120                 125

Ile Thr Leu Ser Pro Val Val Gly Pro Leu Gly Arg His Ala Asn Gly
    130                 135                 140

Gly Arg Ile Trp Glu Gly Phe Ser Ala Asp Pro Tyr Leu Ala Gly Lys
145                 150                 155                 160

Leu Ala Ala Gln Ala Val Glu Gly Ile Gln Ser Gln Asn Val Met Ala
                165                 170                 175

Val Val Lys His Met Val Gly Asn Glu Gln Glu His Phe Arg Gln Leu
            180                 185                 190

Gly Glu Trp Gln Gly Phe Gly Phe Lys Asp Leu Lys Gln Pro Leu Ser
        195                 200                 205

Ser Asn Ile Asp Asp Arg Thr Met Asn Glu Ala Tyr Leu Trp Pro Phe
    210                 215                 220

Ala Asp Ala Val Arg Ala Asn Val Gly Ser Val Met Cys Ser Tyr Gln
225                 230                 235                 240

Gln Ile Asn Gly Ser Gln Gly Cys Gln Asn Ser Ala Leu Leu Asn Gly
                245                 250                 255

Lys Leu Lys Glu Glu Phe Gly Phe Gln Gly Phe Val Met Ser Asp Trp
            260                 265                 270

Leu Ala Gln Arg Ser Gly Val Ala Ser Val Leu Ala Gly Leu Asp Met
        275                 280                 285

Ser Met Pro Gly Asp Gly Leu Val Trp Ala Asp Gly Ile Pro Leu Met
    290                 295                 300

Gly Tyr Glu Leu Thr Lys Met Val Leu Asn Gly Thr Val Glu Gln Ser
305                 310                 315                 320

Arg Val Asp Asp Met Val Thr Arg Ile Leu Thr Pro Leu Leu Tyr Leu
                325                 330                 335

Ser Ile Thr Pro Gly Asn Pro Asn Phe Ser Ser Trp Thr Asn Glu Thr
            340                 345                 350

Thr Gly Leu Gln Phe Pro Gly Ala Asp Arg Gly Pro Asn Ile Thr Val
        355                 360                 365

Asn Gln His Val Glu Val Arg Asp Ser Tyr Thr Thr Lys Ala Ala Leu
    370                 375                 380

Asp Gly Ala Thr Ala Ala Leu Val Leu Leu Lys Asn Val Gln Ser Ala
385                 390                 395                 400

Leu Pro Leu Lys Asn Val Thr Lys Leu Asn Ile Phe Gly Val Gly Ser
                405                 410                 415

Thr Thr Gly Pro Leu Gly Ala Val Cys Gly Glu Asn Met Gln Cys Ser
            420                 425                 430

Asp Gly Ala Leu Ile Leu Gly Trp Gly Ser Gly Ser Val Tyr Pro Ser
        435                 440                 445

Asp Tyr Gln Ser Pro Tyr Glu Ala Ile Lys Glu Arg Ala Glu Gln Asp
    450                 455                 460

Lys Ile Gln Val Gly Gly Thr Thr Glu Ser Trp Gly Asn Leu Thr Arg
465                 470                 475                 480

Val Glu Gln Leu Ala Ala Glu Ser Asp Ala Ser Ile Val Phe Val Leu
                485                 490                 495

Ser Asp Ser Gly Glu Ser Thr Gly Ile Val Asp Gly Asn Ile Gly Asp
            500                 505                 510

Arg Asn Asn Leu Thr Leu Trp His Asn Gly Asp Glu Val Val Arg Thr
        515                 520                 525

Val Ala Ser Gln Ser Ala Asn Thr Ile Val Val Val Thr Thr Val Gly
    530                 535                 540

Pro Val Asn Leu Glu Lys Trp Ile Asn His Pro Asn Val Thr Ala Val
545                 550                 555                 560

Leu Leu Thr Gly Pro Ala Gly Asp Phe Gly Gly Lys Ala Ala Ala Ser
                565                 570                 575

Val Leu Phe Gly Asp Val Ala Pro Ser Gly Lys Leu Pro Phe Thr Ile
            580                 585                 590

Ala Arg Asn Asp Thr Asp Tyr Val Pro Leu Thr Thr Lys Val Pro Ser
        595                 600                 605

Asp Gly Leu Pro Gln Asp Phe Phe Thr Glu Gly Thr Leu Leu Asp Tyr
    610                 615                 620

Lys Arg Phe Asp Ala Leu Lys Lys Thr Pro Arg Phe Glu Phe Gly Tyr
625                 630                 635                 640

Gly Leu Ser Tyr Ser Asn Ile Thr Leu Gly Asn Leu Gln Ala Lys Phe
                645                 650                 655

Ala Phe Asn Asn Ile Pro Glu Phe Leu Pro Thr Pro Phe Pro Pro Leu
            660                 665                 670

Asp Pro His Lys Pro Ala Asn Val Phe Pro Thr Glu Leu Asn Gly Ser
        675                 680                 685

Val Phe Pro Ser Asp Ile Asp Pro Ile Pro Lys Tyr Val Tyr Pro Tyr
    690                 695                 700

Leu Asn Ser Thr Ser Glu Leu Asn Ser Asn Ile Ser Arg Tyr Pro Tyr
705                 710                 715                 720

Pro Glu Gly Phe Ser Val Val Gln Ser Asn Gly Thr Asn Ile Asn Gly
                725                 730                 735

Gly Ala Val Gly Gly Asn Pro Ala Leu Trp Leu Thr Ala Leu Tyr Val
            740                 745                 750

Ala His Ser Ile Ser Asn His Gly Pro Tyr Asp Thr Gly Val Val Thr
        755                 760                 765

Gln Leu Tyr Ile Ala Phe Pro Gln Asp Asn Glu Gly Leu Gln Thr Ala
    770                 775                 780

Pro Arg Gln Leu Arg Gly Phe Glu Arg Ser Glu Leu Pro Val Gly Glu
785                 790                 795                 800

Gln Arg Gly Val Gln Tyr Asp Ile Gln Trp Arg Asp Leu Ala Val Trp
                805                 810                 815

Asp Val Ser Ile Gln Ser Trp Arg Val Gln Arg Gly Asp Tyr Glu Ile
            820                 825                 830

Phe Val Gly His Ser Ser Arg Asp Leu Val Leu Ala Thr Lys Phe Thr
        835                 840                 845

Leu Asn
    850

<210>  20
<211>  844
<212>  PRT
<213>  Yarrowia lipolytica

<400>  20

Met Ile Phe Ser Leu Gln Leu Leu Leu Thr Thr Ala Leu Ala Ala Ser
1               5                   10                  15

Ser Pro Asp Trp Tyr Pro Thr Pro Glu Ile Gly Ser Ile Thr Ser Asp
            20                  25                  30

Trp Ala Asp Ala Leu Gly Glu Ser Met Asp Ile Leu Ala Gln Leu Thr
        35                  40                  45

Leu Pro Glu Lys Val Asn Ile Thr Thr Gly Thr Gly Trp Arg Gly Gly
    50                  55                  60

Gln Cys Val Gly Asn Thr Gly Ala Val Pro Arg Leu Gly Ile Lys Gly
65                  70                  75                  80

Leu Cys Leu Gln Asp Gly Pro Leu Gly Val Arg Phe Ala Asp Phe Val
                85                  90                  95

Asn Val Phe Pro Cys Gln Asn Ala Met Ala Ala Thr Phe Asp Arg Ile
            100                 105                 110

Leu Val His Gln Arg Gly Thr Ala Ile Gly Arg Gln Ser Arg Leu Lys
        115                 120                 125

Gly Val Asp Val His Leu Gly Pro Val Val Gly Pro Leu Gly Arg His
    130                 135                 140

Ala Thr Gly Gly Arg Asn Trp Glu Gly Phe Ser Pro Asp Pro Tyr Leu
145                 150                 155                 160

Ser Gly Lys Leu Ala Phe Glu Ala Ile Leu Gly Val Gln Glu Glu Gly
                165                 170                 175

Val Leu Ala Thr Ile Lys His Phe Ile Gly Asn Glu Gln Glu His Tyr
            180                 185                 190

Arg Arg Ala Glu Glu Trp Arg Asp Gly Phe Gly Phe Lys Asp Leu Lys
        195                 200                 205

Asp Ala Val Ser Ser Asn Ile Glu Asp Arg Ala Leu His Glu Leu Tyr
    210                 215                 220

Met Trp Pro Phe Ala Asp Ala Val Arg Ala Asn Val Gly Ser Val Met
225                 230                 235                 240

Cys Ser Tyr Asn Tyr Val Asn Gly Thr Gln Ala Cys Gln Asn Ser Asp
                245                 250                 255

Leu Leu Asn Gly Lys Leu Lys Ser Glu Leu Gly Phe Gln Gly Phe Val
            260                 265                 270

Met Ser Asp Trp Phe Ala Gln Gly Ser Gly Val Ser Asn Ala Leu Ala
        275                 280                 285

Gly Met Asp Met Ser Met Pro Gly Asn Asp Val Asp Glu Leu Glu Thr
    290                 295                 300

Val Phe Trp Gly Glu Gln Leu Thr Arg Met Val Ala Asn Gly Thr Leu
305                 310                 315                 320

Pro Glu Ala Arg Leu Asp Asp Met Val Leu Arg Ile Leu Thr Pro Leu
                325                 330                 335

Ile Tyr Phe Gly Ile Asp Asp Arg Thr Pro Asn Phe Ser Ser Phe Val
            340                 345                 350

Asp Thr Thr Val Gly Ser Pro His Pro Ala Ala Lys His Ser Lys Lys
        355                 360                 365

Val Lys Asp Val Ile Thr Asn Tyr His Leu Asp Val Arg Asp Gln Phe
    370                 375                 380

Ala Ala Asn Val Ala Leu Asp Ser Ala Arg Gly Ala Val Val Leu Leu
385                 390                 395                 400

Phe Asn Asp Gly Ile Leu Pro Leu Lys Asn Ile Ser Ala Ile Gly Val
                405                 410                 415

Phe Gly Val Gly Ser Arg Leu Gly Pro Asn Gly Ala Val Cys Gly Glu
            420                 425                 430

Asn Met Gln Cys Ser Asp Gly Ala Leu Ile Glu Gly Trp Gly Ser Gly

435                 440                 445

Thr Ala Tyr Pro Thr Glu Tyr Glu Ser Pro Tyr Glu Ala Leu His Lys
    450                 455                 460

Lys Ala Ser Leu Leu Glu Val Ser Val Thr Gly Thr Thr Glu Ser Trp
465                 470                 475                 480

Asp Met Arg Leu Pro Leu Glu Leu Ala Gly Asp Thr Asp Val Asn Ile
                485                 490                 495

Val Tyr Val Leu Ala Asn Ser Gly Glu Ser Thr Ala Asn Val Asp Lys
            500                 505                 510

Asn Leu Gly Asp Arg Arg Asn Val Ser Leu Trp His Asn Gly Asp Glu
        515                 520                 525

Leu Ile Asn Thr Val Ala Ser Gln Gly Gln Thr Val Val Val Val Thr
    530                 535                 540

Thr Val Gly Gln Val Asp Met Thr Ala Trp Leu Asn His Pro Asn Ile
545                 550                 555                 560

Ser Ala Val Leu Leu Thr Ala Pro Ala Gly Asp Tyr Gly Gly Lys Ala
                565                 570                 575

Ile Ala Asp Val Leu Phe Gly Glu Val Asn Pro Ser Gly Lys Leu Pro
            580                 585                 590

Tyr Thr Ile Ala Ala Asn Thr Ser Asp Tyr Ile Pro Ile Val Thr Lys
        595                 600                 605

Ile Pro Arg Asp Gly Ala Pro Gln Ser Asp Phe Val Glu Gly Ile Tyr
    610                 615                 620

Leu Asp Tyr Lys Trp Tyr Asp Lys Phe Glu Arg Thr Pro Leu Tyr Glu
625                 630                 635                 640

Phe Gly Tyr Gly Leu Ser Tyr Thr Thr Tyr Ser Phe Ser Asn Leu His
                645                 650                 655

Leu Asp Val Lys Glu Ile Ser Glu Phe Leu Pro Pro Arg Pro Val Pro
            660                 665                 670

Val Gln Val Thr Lys Pro Lys Met Thr Asn Ile Asp Ile Glu Asp Leu
        675                 680                 685

Tyr Val Pro Asn Asp Phe Lys Met Ile Asp Gly Leu Val Tyr Pro Trp
    690                 695                 700

Ile Leu Asn Ala Ser Ala Pro Leu Ala Asp Ser Gln Thr Gln Phe Pro
705                 710                 715                 720

Phe Ala Asn Gly Ala Gly His Val Ser Asp Ala Ser Gly Gly Val Gly
                725                 730                 735

Gly His Pro Trp Leu Trp Ser Asn Ala Val Thr Val Thr His Asn Thr
            740                 745                 750

Thr Asn Cys Gly Asp Val Ala Gly Arg Val Val Ser Gln Leu Tyr Val
        755                 760                 765

Ala Phe Pro Glu Thr Leu Ile Asp Ser Pro Pro Val Gln Leu Arg Gly
    770                 775                 780

Phe Asp Lys Ser Lys Leu Leu Asn Pro Gly Glu Ser Gln Met Thr Glu
785                 790                 795                 800

Tyr Asn Leu Asn Trp Arg Asp Leu Ala Ile Trp Asp Val Glu Leu Gln
                805                 810                 815

Ser Trp Arg Val Gln Arg Gly Glu Tyr Ser Val Tyr Ile Gly His Ser
            820                 825                 830

Ser Arg Glu Phe Glu Leu Cys Glu Thr Phe Thr Leu
        835                 840

<210>  21
<211>  847
<212>  PRT
<213>  Yarrowia galli

<220>
<221>  SIGNAL
<222>  (1)..(16)

<400>  21

Met Ile Phe Ser Leu Gln Leu Leu Leu Thr Thr Val Pro Val Leu Ala
1               5                   10                  15

Ala Tyr Ser Pro Asp Trp Tyr Pro Thr Pro Glu Ile Gly Thr Ile Asn
            20                  25                  30

Asn Asp Trp Ala Asp Ala Leu Glu Gln Ser Met Asp Ile Leu Ser Gln
        35                  40                  45

Leu Thr Leu Pro Glu Lys Val Asn Ile Thr Thr Gly Thr Gly Trp Met
    50                  55                  60

Gly Gly Gln Cys Val Gly Asn Thr Gly Gly Val Pro Arg Leu Gly Ile
65                  70                  75                  80

Lys Gly Leu Cys Leu Gln Asp Gly Pro Leu Gly Val Arg Phe Ala Asp
                85                  90                  95

Phe Val Asn Val Phe Pro Cys Gln Asn Ala Met Ala Ala Thr Phe Asp
            100                 105                 110

Arg Ile Leu Val His Gln Arg Gly Thr Ala Ile Gly His Gln Ser Lys
        115                 120                 125

Leu Lys Gly Val Asp Val His Leu Gly Pro Val Val Gly Pro Ile Gly
    130                 135                 140

Arg His Ala Thr Gly Gly Arg Asn Trp Glu Gly Phe Ser Pro Asp Pro
145                 150                 155                 160

Tyr Leu Ser Gly Lys Leu Ala Tyr Glu Ala Ile Gln Gly Ile Gln Glu
                165                 170                 175

Glu Asn Val Leu Ala Thr Ile Lys His Phe Ile Gly Asn Glu Gln Asp
            180                 185                 190

His Tyr Arg Arg Ala Gln Glu Trp Arg Asp Gly Phe Asn Phe Thr Gly
        195                 200                 205

Leu Lys Leu Pro Val Ser Ser Asn Asn Ile Asp Asp Arg Ala Leu His
    210                 215                 220

Glu Leu Tyr Met Trp Pro Phe Ala Asp Ala Val Lys Ala Gly Val Gly
225                 230                 235                 240

Ser Val Met Cys Ala Tyr Asn Asn Val Asn Gly Thr Gln Ala Cys Gln
                245                 250                 255

Asn Ser Asp Leu Leu Asn Gly Lys Leu Lys Ser Glu Leu Gly Phe Gln
            260                 265                 270

Gly Phe Val Met Ser Asp Trp Phe Ala Gln Gly Asn Gly Val Ala Asn
        275                 280                 285

Ala Leu Ala Gly Met Asp Met Ser Met Pro Gly Thr Asp Val Asp Glu
    290                 295                 300

Phe Glu Thr Val Phe Trp Gly Glu Gln Leu Thr Arg Met Val Ala Asn
305                 310                 315                 320

Gly Thr Leu Pro Glu Ser Arg Leu Asp Asp Met Val Leu Arg Ile Leu
                325                 330                 335

Thr Pro Leu Met Tyr Phe Gly Ile Asp Asp Arg Glu Pro Asn Phe Ala
            340                 345                 350

Ser Phe Val Asp Thr Thr Val Gly Ser Pro Tyr Pro Ala Ala Lys Asn
        355                 360                 365

Pro Lys Gln Val Gln Asp Lys Ile Val Asn Tyr His Leu Asp Val Arg
    370                 375                 380

Asn Gln Phe Ser Ala Asn Val Ala Leu Glu Ser Ala Arg Gly Ala Ile
385                 390                 395                 400

Val Leu Leu Phe Asn Ser Gly Val Leu Pro Leu Lys Asp Lys Ser Asn
                405                 410                 415

Ile Gly Val Phe Gly Val Gly Ser Val Ile Gly Pro Asn Gly Ala Ile
            420                 425                 430

Cys Glu Asn Met Gln Cys Ser Asp Gly Ala Leu Ile Glu Gly Trp Gly
        435                 440                 445

Ser Gly Thr Ala Tyr Pro Thr Glu Tyr Glu Ser Pro Tyr Glu Ala Leu
    450                 455                 460

His Lys Lys Ala Ser Leu Leu Asp Val His Val Thr Gly Thr Ser Glu
465                 470                 475                 480

Ser Trp Asp Met Ser Leu Pro Ile Glu Ile Ala Gly Ser Thr Asp Ile
                485                 490                 495

Asn Ile Val Tyr Val Leu Ala Ala Ser Gly Glu Ser Thr Ala Ser Val
            500                 505                 510

Asp Gly Asn Ile Gly Asp Arg Asn Asn Val Ser Leu Trp His Asn Gly
        515                 520                 525

Asp Glu Leu Ile Asn Ala Val Ala Asp Gln Gly Glu Thr Val Val Val
    530                 535                 540

Val Thr Thr Val Gly Gln Val Asp Met Ser Glu Trp Leu Phe His Pro

545                 550                 555                 560

Asn Val Ser Ala Val Leu Leu Thr Ala Pro Ala Gly Asp Tyr Gly Gly
                565                 570                 575

Lys Ala Met Ala Asp Val Leu Phe Gly Glu Val Asn Pro Ser Gly Lys
            580                 585                 590

Leu Pro Tyr Thr Leu Ala Asp Asp Ile Ser Glu Tyr Ile Pro Ile Val
        595                 600                 605

Thr Glu Ile Pro Ala Asp Gly Ala Pro Gln Ser Asp Phe Val Glu Gly
    610                 615                 620

Ile Tyr Leu Asp Tyr Lys Trp Tyr Asp Lys Phe Glu Lys Thr Pro Leu
625                 630                 635                 640

Tyr Glu Phe Gly Tyr Gly Leu Ser Tyr Thr Asn Tyr Thr Phe Thr Asp
                645                 650                 655

Leu Ser Leu Asp Ile Lys Glu Ile Asp Glu Phe Leu Pro Ala Arg Pro
            660                 665                 670

Asp Pro Val Val Val Thr Lys Pro Glu Asp Phe Pro Ile Asp Leu Ala
        675                 680                 685

Asp Leu Tyr Val Pro Asp Gly Phe Glu Met Ile Asp Gly Leu Val Tyr
    690                 695                 700

Pro Trp Ile Gln Asn Val Ser Val Pro Leu Ser Asp Ser Glu Ala Gln
705                 710                 715                 720

Phe Pro Phe Ala Asp Gly Ala Gly His Ile Ser Asp Ala Ser Gly Gly
                725                 730                 735

Val Gly Gly His Pro Trp Leu Trp Ser Val Ala Ala Thr Ile Thr His
            740                 745                 750

Asn Thr Thr Asn Cys Gly Asp Val Ala Gly Arg Ala Val Ser Gln Leu
        755                 760                 765

Tyr Ile Ala Phe Pro Glu Thr Cys Val Asp Val Pro Leu Val Gln Leu
    770                 775                 780

Arg Gly Phe Asp Lys Ser Gly Met Leu Asn Pro Gly Glu Ser Gln Glu
785                 790                 795                 800

Thr Val Tyr Asn Leu His Trp Arg Asp Leu Ala Ile Trp Asp Val Glu
                805                 810                 815

Leu Gln Ser Trp Arg Val Gln Arg Gly Glu Tyr Ala Val Phe Ile Gly
            820                 825                 830

His Ser Ser Arg Asp Phe Glu Leu Phe Asp Ser Phe Thr Leu Glu
        835                 840                 845

<210>  22
<211>  869
<212>  PRT
<213>  Yarrowia lipolytica

<400>  22

Met Leu Ala Phe Val Leu Leu Leu Thr Met Leu Leu Ala Ala Ala Leu
1               5                   10                  15

Ala Asp Pro Phe Ser Asp Lys Asp Ala Tyr Lys His Ser Pro Pro Tyr
            20                  25                  30

Tyr Pro Ala Pro Glu Ile Gly Arg Val Pro Thr Asp Leu Arg Trp Arg
        35                  40                  45

Ala Ala Leu Lys Val Ala Gln Gly Met Val Ala Asn Met Thr Leu Leu
    50                  55                  60

Glu Lys Val Asn Ile Thr Thr Gly Thr Gly Trp Glu Met Gly Pro Cys
65                  70                  75                  80

Val Gly Asn Thr Gly Thr Val Glu Arg Leu Gly Ile Lys Ser Leu Cys
                85                  90                  95

Leu Gln Asp Gly Pro Leu Gly Ile Arg Phe Ala Asp Leu Ile Thr Thr
            100                 105                 110

Phe Pro Ala Gly Ile Thr Ile Ala Ser Thr Phe Ser Arg Gln Leu Val
        115                 120                 125

Arg Glu Arg Gly Ala Ala Met Gly Arg Glu Asn Arg Arg Lys Gly Val
    130                 135                 140

Asp Ile Thr Leu Ser Pro Val Val Gly Pro Leu Gly Arg His Ala Asn
145                 150                 155                 160

Gly Gly Arg Ile Trp Glu Gly Phe Ser Ala Asp Pro Tyr Leu Ala Gly
                165                 170                 175

Lys Leu Ala Ala Glu Ala Val Thr Gly Ile Gln Gly Gln Asn Val Met
            180                 185                 190

Ala Val Val Lys His Met Val Gly Asn Glu Gln Glu His Phe Arg Gln
        195                 200                 205

Leu Gly Glu Trp Gln Gly Phe Gly Phe Lys Asp Leu Lys Gln Pro Leu
    210                 215                 220

Ser Ser Asn Ile Asp Asp Arg Thr Leu Asn Glu Ala Tyr Leu Trp Pro
225                 230                 235                 240

Phe Ala Asp Ala Val Arg Ala Asn Val Gly Ser Val Met Cys Ser Tyr
                245                 250                 255

Gln Gln Ile Asn Gly Ser Gln Gly Cys Gln Asn Ala His Ile Leu Asn
            260                 265                 270

Gly Lys Leu Lys Glu Glu Met Gly Phe Gln Gly Phe Val Met Ser Asp
        275                 280                 285

Trp Leu Ala Gln Arg Ser Gly Val Ala Ser Val Leu Ala Gly Leu Asp
    290                 295                 300

Met Ser Met Pro Gly Asp Gly Leu Val Trp Ala Asp Gly Val Pro Leu
305                 310                 315                 320

Met Gly Tyr Glu Leu Thr Arg Ser Val Leu Asn Gly Thr Ile Asp Glu
                325                 330                 335

Ser Arg Val Asp Asp Met Val Thr Arg Ile Leu Thr Pro Ile Leu Tyr
            340                 345                 350

Leu Ser Ile Thr Pro Thr Asp Pro Asn Phe Ser Ser Trp Thr Asn Asp
        355                 360                 365

Thr Thr Ser Tyr Lys Tyr Tyr Gly Ala Lys Ala Gly Gly Asn Val Thr
    370                 375                 380

Val Asn Arg His Ile Asp Val Arg Asp Gln Tyr Thr Thr Lys Ala Ala
385                 390                 395                 400

Leu Asp Gly Ala Asn Ala Ala Leu Val Leu Leu Lys Asn Glu Lys Lys
                405                 410                 415

Thr Leu Pro Leu Asn Pro Thr Asn Ile Gly Asn Leu Asn Ile Phe Gly
            420                 425                 430

Ile Gly Ser Lys Thr Gly Pro Leu Gly Ala Val Cys Gly Glu Asn Met
        435                 440                 445

Gln Cys Ser Asp Gly Ala Leu Ile Glu Gly Trp Gly Ser Gly Ser Val
    450                 455                 460

Tyr Pro Thr Asp Tyr Gln Ser Pro Tyr Asp Ala Ile Lys Glu Arg Ala
465                 470                 475                 480

Ser Lys Asp Asn Ile Thr Ile Gly Gly Thr Thr Gln Ser Trp Gly Asn
                485                 490                 495

Leu Ser Asn Val Glu Ile Leu Ser Ala Ala Ala Asp Ala Ser Val Val
            500                 505                 510

Phe Val Leu Ser Asp Ser Gly Glu Ser Thr Gly Ile Val Asp Gly Asn
        515                 520                 525

Ile Gly Asp Arg Asn Asn Leu Thr Leu Trp His Asn Gly Asp Glu Val
    530                 535                 540

Val Lys Ala Val Ala Ser Lys Asn Pro Asn Thr Ile Val Val Val Thr
545                 550                 555                 560

Thr Val Gly Pro Val Asn Leu Glu Lys Trp Ile Asp Asn Pro Asn Val
                565                 570                 575

Thr Ala Val Leu Leu Thr Gly Pro Ala Gly Asp Phe Gly Gly Arg Ala
            580                 585                 590

Ala Ala Ser Ile Leu Phe Gly Asp Ile Ala Pro Ser Gly Lys Leu Pro
        595                 600                 605

Phe Thr Ile Ala Lys Asn Asp Thr Asp Tyr Ile Pro Leu Thr Thr Lys
    610                 615                 620

Ile Pro Glu Asp Gly Leu Pro Gln Asp Tyr Phe Thr Glu Gly Thr Leu
625                 630                 635                 640

Leu Asp Tyr Lys Arg Phe Asp Glu Asn Gln Val Thr Pro Arg Phe Glu
                645                 650                 655

Phe Gly Tyr Gly Leu Ser Tyr Ser Asn Ile Thr Val Glu Asn Leu Glu
            660                 665                 670

Ala Arg Tyr Ala Phe Pro Ser Ile Pro Glu Phe Leu Pro Thr Pro Phe
        675                 680                 685

Ala Pro Ser Asn Pro Asn Lys Pro Lys Asn Ala Phe Thr Pro His Ala
    690                 695                 700

Asn Glu Ser Val Phe Pro Ser Asp Ile Asp Pro Leu Asn Lys Tyr Val
705                 710                 715                 720

Tyr Pro Tyr Leu Asn Asp Thr Ser Glu Ile Phe Ser Asn Glu Thr His
                725                 730                 735

Tyr Pro Tyr Pro Glu Gly Tyr Ser Ser Glu Gln Ser Asn Ser Thr Asn
            740                 745                 750

Ile Asn Gly Gly Ala Val Gly Gly Asn Pro Ala Leu Trp Leu Ser Ala
        755                 760                 765

Val Tyr Ile Val His Ser Val Ser Asn Tyr Gly Pro Tyr Asp Thr Gly
    770                 775                 780

Val Val Thr Gln Met Tyr Ile Ala Phe Pro Gln Asp Asn Asp Asp Leu
785                 790                 795                 800

Lys Thr Ala Pro Arg Gln Leu Arg Gly Phe Glu Arg Ser Glu Leu Lys
                805                 810                 815

Val Gly Glu Arg Gln Gly Ile Leu Tyr Asp Val Gln Trp Arg Asp Leu
            820                 825                 830

Ala Val Trp Asp Val Lys Leu Gln Ser Trp Arg Val Gln Arg Gly Glu
        835                 840                 845

Tyr Lys Val Tyr Val Gly His Ser Ser Arg Asp Phe Val Leu Thr Thr
    850                 855                 860

Ser Phe Thr Leu Lys
865

<210>  23
<211>  874
<212>  PRT
<213>  Yarrowia galli

<220>
<221>  SIGNAL
<222>  (1)..(20)

<400>  23

Met Leu Ala Phe Val Leu Leu Leu Thr Thr Leu Gln Ala Leu Leu Thr

1               5                   10                  15

Ala Val Leu Ala Ala Asp Pro Phe Ser Asp Lys Asp Ala Tyr Lys His
            20                  25                  30

Ser Pro Pro Tyr Tyr Pro Ala Pro Glu Ile Gly Arg Val Pro Thr Asp
        35                  40                  45

Leu Arg Trp Arg Ala Ala Leu Lys Val Ala Gln Asp Met Val Ala Asn
    50                  55                  60

Met Thr Leu Ile Glu Lys Val Asn Ile Thr Thr Gly Thr Gly Trp Glu
65                  70                  75                  80

Met Gly Pro Cys Val Gly Asn Thr Gly Thr Val Glu Arg Leu Gly Ile
                85                  90                  95

Lys Ser Leu Cys Leu Gln Asp Gly Pro Leu Gly Ile Arg Phe Ala Asp
            100                 105                 110

Leu Ile Thr Thr Phe Pro Ala Gly Ile Thr Ile Ala Ser Thr Phe Ser
        115                 120                 125

Lys Gln Leu Val Arg Glu Arg Gly Glu Ala Met Gly Arg Glu Asn Arg
    130                 135                 140

Arg Lys Gly Val Asp Ile Thr Leu Ser Pro Val Val Gly Pro Leu Gly
145                 150                 155                 160

Arg His Ala Asn Ala Gly Arg Ile Trp Glu Gly Phe Ser Ala Asp Pro
                165                 170                 175

Tyr Leu Ala Gly Lys Leu Ala Ala Glu Ala Val Thr Gly Ile Gln Ser
            180                 185                 190

Gln Asn Val Met Ala Val Val Lys His Ile Val Gly Asn Glu Gln Glu
        195                 200                 205

His Phe Arg Gln Leu Gly Glu Trp Gln Gly Phe Gly Phe Lys Asp Leu
    210                 215                 220

Lys Gln Pro Leu Ser Ser Asn Ile Asp Asp Arg Thr Leu Asn Glu Ala
225                 230                 235                 240

Tyr Leu Trp Pro Phe Ala Asp Ala Val Arg Ala Asn Val Gly Ser Val
                245                 250                 255

Met Cys Ser Tyr Gln Gln Ile Asn Gly Ser Gln Gly Cys Gln Asn Ser
            260                 265                 270

His Ile Leu Asn Gly Lys Leu Lys Glu Glu Met Gly Phe Gln Gly Phe
        275                 280                 285

Val Met Ser Asp Trp Leu Ala Gln Arg Ser Gly Val Ala Ser Val Leu
    290                 295                 300

Ala Gly Leu Asp Met Ser Met Pro Gly Asp Gly Leu Val Trp Ala Asp
305                 310                 315                 320

Gly Val Pro Leu Met Gly Tyr Glu Leu Thr Lys Ser Val Leu Asn Gly
                325                 330                 335

Thr Ile Asp Ile Asn Arg Val Asp Asp Met Val Thr Arg Ile Leu Thr
            340                 345                 350

Pro Leu Met Tyr Leu Ser Ile Thr Pro Gly Asn Pro Asn Phe Ser Ser
        355                 360                 365

Trp Thr Asn Asp Thr Thr Ser Tyr Lys Ile Tyr Gly Ala Lys Ala Gly
    370                 375                 380

Gly Asn Val Thr Val Asn Arg His Ile Asp Val Arg Asp Pro Trp Thr
385                 390                 395                 400

Thr Lys Ala Ala Leu Asp Gly Ala Asn Ala Ala Leu Val Leu Leu Lys
                405                 410                 415

Asn Val Lys Asn Ala Leu Pro Leu Lys Pro Thr Asn Ile Gly Asn Leu
            420                 425                 430

Asn Ile Phe Gly Ile Gly Ser Ala Thr Gly Pro Leu Gly Ala Val Cys
        435                 440                 445

Gly Glu Asn Met Gln Cys Ser Asp Gly Ala Leu Ile Glu Gly Trp Gly
    450                 455                 460

Ser Gly Ser Val Tyr Pro Thr Asp Tyr Gln Ser Pro Tyr Glu Ala Ile
465                 470                 475                 480

Lys Glu Arg Ala Ala Gln Asp Asn Ile Thr Val Gly Gly Thr Thr Gln
                485                 490                 495

Ser Trp Gly Asn Leu Ser Asn Val Glu Ile Leu Ser Ala Ala Ala Asp
            500                 505                 510

Ala Ser Val Val Phe Val Leu Ser Asp Ser Gly Glu Ser Thr Gly Ile
        515                 520                 525

Val Asp Gly Asn Ile Gly Asp Arg Asn Asn Leu Thr Leu Trp His Asn
    530                 535                 540

Gly Asp Ala Val Val Lys Ala Val Ala Ser Arg Asn Pro Asn Thr Ile
545                 550                 555                 560

Val Val Val Thr Thr Val Gly Pro Val Asn Leu Glu Lys Trp Ile Asn
                565                 570                 575

Asn Pro Asn Val Thr Ala Val Leu Leu Thr Gly Pro Ala Gly Asp Phe
            580                 585                 590

Gly Gly Arg Ala Ala Ala Ser Ile Leu Phe Gly Asp Ile Ala Pro Ser
        595                 600                 605

Gly Lys Leu Pro Phe Thr Ile Ala Lys Asn Asp Thr Asp Tyr Ile Pro
    610                 615                 620

Leu Thr Thr Lys Val Pro Lys Asp Gly Leu Pro Gln Asp Tyr Phe Thr
625                 630                 635                 640

Glu Gly Thr Leu Leu Asp Tyr Lys Arg Phe Asp Glu Asn Lys Val Thr
                645                 650                 655

Pro Arg Phe Glu Phe Gly Tyr Gly Leu Ser Tyr Ser Asn Ile Thr Met
            660                 665                 670

Glu Asn Leu Glu Ala Arg Phe Ala Phe Pro Asn Ile Pro Glu Phe Leu
        675                 680                 685

Pro Thr Pro Phe Ala Pro Leu Asn Pro His Lys Pro Asn Asp Val Phe
    690                 695                 700

Thr Pro Asn Ala Asn Gln Ser Val Phe Pro Asp Asp Ile His Pro Leu
705                 710                 715                 720

His Lys Tyr Val Tyr Pro Tyr Leu Asn Asp Thr Ser Glu Ile Phe Thr
                725                 730                 735

Ser Asn Glu Thr Asn Tyr Pro Tyr Pro Glu Gly Tyr Ser Gln Lys Gln
            740                 745                 750

Ser Asn Thr Thr Asn Ile Asn Gly Gly Ala Val Gly Gly Asn Pro Ala
        755                 760                 765

Leu Trp Leu Ser Ser Val Tyr Ile Val His Ser Val Ser Asn Tyr Gly
    770                 775                 780

Pro Tyr Asp Thr Gly Val Val Thr Gln Met Tyr Ile Ala Phe Pro Gln
785                 790                 795                 800

Asp Asn Asp Asp Leu Lys Thr Ala Pro Arg Gln Leu Arg Gly Phe Glu
                805                 810                 815

Arg Ser Glu Leu Lys Val Gly Glu Arg His Gly Ile Leu Tyr Asn Val
            820                 825                 830

Gln Trp Arg Asp Leu Ala Val Trp Asp Val Lys Leu Gln Ser Trp Arg
        835                 840                 845

Val Gln Arg Gly Glu Tyr Lys Val Tyr Val Gly His Ser Ser Arg Asp
    850                 855                 860

Phe Val Leu Thr Thr Ser Phe Thr Leu Gln
865                 870

<210>  24
<211>  867
<212>  PRT
<213>  Yarrowia yakushimensis

<220>
<221>  SIGNAL
<222>  (1)..(17)

<400>  24

Met Leu Ala Phe Val Leu Leu Leu Thr Ser Leu Leu Thr Ala Val Leu
1               5                   10                  15

Ala Asp Pro Phe Ser Asp Lys Ala Ala Tyr Lys His Ser Pro Pro Tyr
            20                  25                  30

Tyr Pro Ala Pro Glu Ile Gly Arg Ile Pro Thr Asp Leu Arg Trp Ala
        35                  40                  45

Ala Ala Leu Glu Val Ala Gln Asn Leu Val Ala Asn Met Thr Leu Ile
    50                  55                  60

Glu Lys Val Asn Ile Thr Thr Gly Thr Gly Trp Glu Met Gly Pro Cys
65                  70                  75                  80

Val Gly Asn Thr Ser Pro Val Glu Arg Leu Gly Ile Lys Ser Leu Cys

                85                  90                  95

Leu Gln Asp Gly Pro Leu Gly Val Arg Leu Thr Asp Leu Ile Thr Ala
            100                 105                 110

Phe Pro Ala Gly Ile Thr Met Ala Ser Thr Phe Ser Arg Gln Leu Val
        115                 120                 125

Arg Glu Arg Gly Ala Ala Met Gly Arg Glu Asn Arg Arg Lys Gly Val
    130                 135                 140

Asp Ile Thr Leu Ser Pro Val Val Gly Pro Leu Gly Arg Asn Ala Asn
145                 150                 155                 160

Ala Gly Arg Ile Trp Glu Gly Phe Ser Ala Asp Pro Tyr Leu Ala Gly
                165                 170                 175

Lys Leu Ala Ala Glu Ala Val Thr Gly Ile Gln Ser Gln Asn Val Met
            180                 185                 190

Ala Val Val Lys His Leu Val Gly Asn Glu Gln Glu His Phe Arg Gln
        195                 200                 205

Leu Gly Glu Trp Gln Gly Tyr Gly Tyr Lys Asp Leu Lys Gln Pro Leu
    210                 215                 220

Ser Ser Asn Ile Asp Asp Arg Thr Leu Asn Glu Ala Tyr Leu Trp Pro
225                 230                 235                 240

Phe Ala Asp Ala Ile Arg Ala Asn Val Gly Ser Val Met Cys Ser Tyr
                245                 250                 255

Gln Gln Ile Asn Ser Ser Gln Gly Cys Gln Asn Ser Phe Ile Leu Asn
            260                 265                 270

Gly Lys Ile Lys Glu Glu Met Gly Phe Gln Gly Phe Ile Met Ser Asp
        275                 280                 285

Trp Leu Ala Gln Arg Ser Gly Val Ala Ser Val Leu Ala Gly Leu Asp
    290                 295                 300

Met Ser Met Pro Gly Asp Gly Leu Val Trp Gly Leu Gly Ile Pro Leu
305                 310                 315                 320

Met Gly Tyr Glu Leu Thr Arg Ser Val Leu Asn Gly Thr Ile Asp Glu
                325                 330                 335

Ser Arg Val Asp Asp Met Val Thr Arg Ile Leu Thr Pro Ile Leu Tyr
        340                 345                 350

Leu Ser Leu Thr Pro Arg Thr Pro Asn Phe Ser Ser Trp Thr Asn Asp
    355                 360                 365

Thr Tyr Gly Tyr Glu Tyr Tyr Gly Asn Asp Gly Gly Arg Asn Ile Thr
    370                 375                 380

Ile Asn Arg His Val Asp Val Arg Asp Ala Tyr Thr Ile Gln Ala Ala
385                 390                 395                 400

Gln Asp Gly Ala Asn Ala Ala Leu Val Leu Leu Lys Asn Glu Lys Asn
                405                 410                 415

Ala Leu Pro Leu Asp Pro Ala Lys Leu Gly Thr Ile Asn Ile Phe Gly
            420                 425                 430

Ile Gly Ser Lys Thr Gly Asn Leu Ser Cys Gly Gln Asn Met Glu Cys
        435                 440                 445

Ser Asn Gly Ala Leu Ile Gln Gly Trp Gly Ser Gly Ser Val Tyr Pro
    450                 455                 460

Thr Asp Tyr Leu Ser Pro Tyr Asn Ala Ile Lys Glu Arg Ala Ala Gln
465                 470                 475                 480

Asn Asn Ile Thr Val Gly Gly Thr Thr His Ser Trp Gly Asn Leu Ser
                485                 490                 495

Ile Val Glu Lys Leu Ser Ala Ala Ala Asp Ala Ser Val Val Phe Val
            500                 505                 510

Leu Ser Asp Ser Gly Glu Ser Thr Gly Pro Val Asp Gly Asn Tyr Gly
        515                 520                 525

Asp Arg Asn Asn Leu Thr Leu Trp His Asn Gly Asp Gln Val Val Lys
    530                 535                 540

Ala Val Ala Ser Lys Asn Pro Asn Thr Ile Val Val Val Thr Thr Val
545                 550                 555                 560

Gly Pro Val Asn Leu Glu Lys Trp Ile Asp His Pro Asn Val Thr Ala
                565                 570                 575

Val Leu Leu Thr Gly Pro Ala Gly Asp Phe Gly Gly Ser Ala Ala Ala
            580                 585                 590

Ser Ile Leu Phe Gly Asp Ile Ala Pro Ser Gly Lys Leu Pro Phe Thr
        595                 600                 605

Ile Ala Lys Asn Asn Thr Asp Tyr Ile Pro Leu Val Thr Lys Val Pro
    610                 615                 620

Glu Asp Gly Leu Pro Gln Asp Tyr Phe Thr Glu Gly Thr Leu Leu Asp
625                 630                 635                 640

Tyr Lys Arg Phe Asp Glu Asn Lys Val Thr Pro Arg Phe Glu Phe Gly
                645                 650                 655

Tyr Gly Leu Ser Tyr Ser Asn Ile Thr Val Glu Asn Leu Asp Val Leu
            660                 665                 670

Phe Ala Phe Pro Asn Ile Ser Glu Phe Leu Pro Ala Pro Ser Ala Pro
        675                 680                 685

Leu Ile Pro His Lys Pro Lys Asn Arg Phe Thr Pro Ser Leu Asn Glu
    690                 695                 700

Ser Ile Phe Pro Ser Gly Phe Lys Gln Leu Lys Asn Tyr Val Tyr Thr
705                 710                 715                 720

Tyr Leu Asn Arg Thr Ser Asp Ile Ala Ser Asn Ile Thr His Tyr Pro
                725                 730                 735

Tyr Pro Glu Gly Tyr Ser Ser Val Gln Ser Asn Thr Thr Asn Ile Asn
            740                 745                 750

Gly Gly Ala Val Gly Gly Asn Pro Ala Leu Trp Leu Pro Ala Leu Tyr
        755                 760                 765

Val Val His Ser Val Ser Asn Tyr Gly Pro Tyr Asp Thr Gly Ala Val
    770                 775                 780

Thr Gln Leu Tyr Ile Ser Phe Pro Gln Asp Asn Glu Asp Leu Arg Thr
785                 790                 795                 800

Ala Pro Arg Gln Leu Arg Gly Phe Glu Arg Ser Glu Leu Lys Val Gly
                805                 810                 815

Asp Gln Gln Gly Val Leu Tyr Asp Ile Gln Trp Arg Asp Leu Ala Val
            820                 825                 830

Trp Asp Val Lys Leu Gln Ser Trp Arg Val Gln Arg Gly Lys Tyr Thr
        835                 840                 845

Val Tyr Ile Gly His Ser Ser Arg Asp Leu Val Leu Ser Thr Thr Phe
    850                 855                 860

Thr Leu Lys
865

<210>  25
<211>  866
<212>  PRT
<213>  Yarrowia alimentaria

<220>
<221>  SIGNAL
<222>  (1)..(16)

<400>  25
   Met Phe Ser Leu Leu Leu Leu Leu Thr Leu Leu Ser Thr Ala Leu Ala
   1               5                   10                  15
   Asp Pro Phe Ala Asp Arg Asp Ser Tyr Lys His Ser Pro Pro Tyr Tyr
               20                  25                  30
   Pro Ala Pro Glu Ile Gly Arg Val Leu Thr Asp Arg Arg Trp Arg Thr
           35                  40                  45
   Ala Met Asn Ala Ala Gln Ser Leu Val Ala Asn Met Thr Leu Tyr Glu
       50                  55                  60
   Lys Val Asn Ile Thr Ser Gly Thr Gly Trp Glu Met Gly Pro Cys Val
   65                  70                  75                  80
   Gly Asn Thr Gly Thr Val Glu Arg Leu Gly Ile Lys Ser Ile Cys Leu
                   85                  90                  95
   Gln Asp Gly Pro Leu Gly Ile Arg Phe Ala Asp Lys Ile Thr Ala Phe
               100                 105                 110
   Pro Ala Gly Ile Thr Ile Ala Ser Thr Phe Ser Arg Lys Leu Val Gln
           115                 120                 125
   Glu Arg Gly Ala Ala Met Gly Arg Glu Asn Arg Arg Lys Gly Val Asp
       130                 135                 140
   Ile Thr Leu Ser Pro Val Val Gly Pro Leu Gly Arg His Ala Asn Gly
   145                 150                 155                 160
   Gly Arg Ile Trp Glu Gly Phe Ser Ala Asp Pro Tyr Leu Ala Gly Lys
   165 170 175
   Leu Ala Ala Gln Ala Val Glu Gly Ile Gln Ser Gln Asn Val Met Ala
   180 185 190
   Val Val Lys His Met Val Gly Asn Glu Gln Glu His Phe Arg Gln Leu
   195 200 205
   Gly Glu Trp Gln Gly Phe Gly Phe Lys Asp Leu Lys Gln Pro Leu Ser
   210 215 220
   Ser Asn Ile Asp Asp Arg Thr Met Asn Glu Ala Tyr Leu Trp Pro Phe
   225 230 235 240
   Ala Asp Ala Val Arg Ala Asn Val Gly Ser Val Met Cys Ser Tyr Gln
   245 250 255
   Gln Ile Asn Gly Ser Gln Gly Cys Gln Asn Ser Ala Leu Leu Asn Gly
   260 265 270
   Lys Leu Lys Glu Glu Phe Gly Phe Gln Gly Phe Val Met Ser Asp Trp
   275 280 285
   Leu Ala Gln Arg Ser Gly Val Ala Ser Val Leu Ala Gly Leu Asp Met
   290 295 300
   Ser Met Pro Gly Asp Gly Leu Val Trp Ala Asp Gly Ile Pro Leu Met
   305 310 315 320
   Gly Tyr Glu Leu Thr Lys Met Val Leu Asn Gly Thr Val Glu Gln Ser
   325 330 335
   Arg Val Asp Asp Met Val Thr Arg Ile Leu Thr Pro Leu Leu Tyr Leu
   340 345 350
   Ser Ile Thr Pro Gly Asn Pro Asn Phe Ser Ser Trp Thr Asn Glu Thr
   355 360 365
   Thr Gly Leu Gln Phe Pro Gly Ala Asp Arg Gly Pro Asn Ile Thr Val
   370 375 380
   Asn Gln His Val Glu Val Arg Asp Ser Tyr Thr Thr Lys Ala Ala Leu
   385 390 395 400
   Asp Gly Ala Thr Ala Ala Leu Val Leu Leu Lys Asn Val Gln Ser Ala
   405 410 415
   Leu Pro Leu Lys Asn Val Thr Lys Leu Asn Ile Phe Gly Val Gly Ser
               420                 425                 430
   Thr Thr Gly Pro Leu Gly Ala Val Cys Gly Glu Asn Met Gln Cys Ser
           435                 440                 445
   Asp Gly Ala Leu Ile Leu Gly Trp Gly Ser Gly Ser Val Tyr Pro Ser
       450                 455                 460
   Asp Tyr Gln Ser Pro Tyr Glu Ala Ile Lys Glu Arg Ala Glu Gln Asp
   465                 470                 475                 480
   Lys Ile Gln Val Gly Gly Thr Thr Glu Ser Trp Gly Asn Leu Thr Arg
                   485                 490                 495
   Val Glu Gln Leu Ala Ala Glu Ser Asp Ala Ser Ile Val Phe Val Leu
               500                 505                 510
   Ser Asp Ser Gly Glu Ser Thr Gly Ile Val Asp Gly Asn Ile Gly Asp
           515                 520                 525
   Arg Asn Asn Leu Thr Leu Trp His Asn Gly Asp Glu Val Val Arg Thr
       530                 535                 540
   Val Ala Ser Gln Ser Ala Asn Thr Ile Val Val Val Thr Thr Val Gly
   545                 550                 555                 560
   Pro Val Asn Leu Glu Lys Trp Ile Asn His Pro Asn Val Thr Ala Val
                   565                 570                 575
   Leu Leu Thr Gly Pro Ala Gly Asp Phe Gly Gly Lys Ala Ala Ala Ser
               580                 585                 590
   Val Leu Phe Gly Asp Val Ala Pro Ser Gly Lys Leu Pro Phe Thr Ile
           595                 600                 605
   Ala Arg Asn Asp Thr Asp Tyr Val Pro Leu Thr Thr Lys Val Pro Ser
       610                 615                 620
   Asp Gly Leu Pro Gln Asp Phe Phe Thr Glu Gly Thr Leu Leu Asp Tyr
   625                 630                 635                 640
   Lys Arg Phe Asp Ala Leu Lys Lys Thr Pro Arg Phe Glu Phe Gly Tyr
                   645                 650                 655
   Gly Leu Ser Tyr Ser Asn Ile Thr Leu Gly Asn Leu Gln Ala Lys Phe
               660                 665                 670
   Ala Phe Asn Asn Ile Pro Glu Phe Leu Pro Thr Pro Phe Pro Pro Leu
   675 680 685
   Asp Pro His Lys Pro Ala Asn Val Phe Pro Thr Glu Leu Asn Gly Ser
   690 695 700
   Val Phe Pro Ser Asp Ile Asp Pro Ile Pro Lys Tyr Val Tyr Pro Tyr
   705 710 715 720
   Leu Asn Ser Thr Ser Glu Leu Asn Ser Asn Ile Ser Arg Tyr Pro Tyr
   725 730 735
   Pro Glu Gly Phe Ser Val Val Gln Ser Asn Gly Thr Asn Ile Asn Gly
   740 745 750
   Gly Ala Val Gly Gly Asn Pro Ala Leu Trp Leu Thr Ala Leu Tyr Val
   755 760 765
   Ala His Ser Ile Ser Asn His Gly Pro Tyr Asp Thr Gly Val Val Thr
   770 775 780
   Gln Leu Tyr Ile Ala Phe Pro Gln Asp Asn Glu Gly Leu Gln Thr Ala
   785 790 795 800
   Pro Arg Gln Leu Arg Gly Phe Glu Arg Ser Glu Leu Pro Val Gly Glu
   805 810 815
   Gln Arg Gly Val Gln Tyr Asp Ile Gln Trp Arg Asp Leu Ala Val Trp
   820 825 830
   Asp Val Ser Ile Gln Ser Trp Arg Val Gln Arg Gly Asp Tyr Glu Ile
   835 840 845
   Phe Val Gly His Ser Ser Arg Asp Leu Val Leu Ala Thr Lys Phe Thr
   850 855 860
   Leu Asn
   865

<210>  26
<211>  16
<212>  PRT
<213>  Yarrowia lipolytica

<400>  26

Met Ile Phe Ser Leu Gln Leu Leu Leu Thr Thr Ala Leu Ala Ala Ser
1               5                   10                  15

<210>  27
<211>  16
<212>  PRT
<213>  Yarrowia galli

<400>  27

Met Ile Phe Ser Leu Gln Leu Leu Leu Thr Thr Val Pro Val Leu Ala
1               5                   10                  15


<210>  28
<211>  17
<212>  PRT
<213>  Yarrowia lipolytica

<400>  28

Met Leu Ala Phe Val Leu Leu Leu Thr Met Leu Leu Ala Ala Ala Leu
1               5                   10                  15


Ala


<210>  29
<211>  20
<212>  PRT
<213>  Yarrowia galli

<400>  29

Met Leu Ala Phe Val Leu Leu Leu Thr Thr Leu Gln Ala Leu Leu Thr
1               5                   10                  15


Ala Val Leu Ala
            20


<210>  30
<211>  17
<212>  PRT
<213>  Yarrowia yakushimensis

<400>  30

Met Leu Ala Phe Val Leu Leu Leu Thr Ser Leu Leu Thr Ala Val Leu
1               5                   10                  15


Ala


<210>  31
<211>  16
<212>  PRT
<213>  Yarrowia alimentaria

<400>  31

Met Phe Ser Leu Leu Leu Leu Leu Thr Leu Leu Ser Thr Ala Leu Ala
1               5                   10                  15


<210>  32
<211>  11
<212>  PRT
<213>  Artificial Sequence

<220>
<223>  EG1 First consensus domain sequence


<220>
<221>  VARIANT
<222>  (2)..(2)
<223>  Replace = Asn

<220>
<221>  VARIANT
<222>  (4)..(4)
<223>  Replace = Lys

<220>
<221>  VARIANT
<222>  (7)..(7)
<223>  Replace = Thr

<400>  32

Leu Leu Gly Gln Glu Leu Ser Phe Asp Val Asp
1               5                   10


<210>  33
<211>  10
<212>  PRT
<213>  Artificial Sequence

<220>
<223>  EG1 Second consensus domain sequence


<220>
<221>  VARIANT
<222>  (8)..(8)
<223>  Ala

<400>  33

Leu Pro Cys Gly Glu Asn Gly Ser Leu Tyr
1               5                   10


<210>  34
<211>  9
<212>  PRT
<213>  Artificial Sequence

<220>
<223>  EG1 Third consensus domain sequence

<400>  34

Cys Cys Asn Glu Met Asp Ile Leu Glu
1               5


<210>  35
<211>  459
<212>  PRT
<213>  Hypocrea rufa

<400>  35

Met Ala Pro Ser Val Thr Leu Pro Leu Thr Thr Ala Ile Leu Ala Ile
1               5                   10                  15

Ala Arg Leu Val Ala Ala Gln Gln Pro Gly Thr Ser Thr Pro Glu Val
            20                  25                  30

His Pro Lys Leu Thr Thr Tyr Lys Cys Thr Lys Ser Gly Gly Cys Val
        35                  40                  45

Ala Gln Asp Thr Ser Val Val Leu Asp Trp Asn Tyr Arg Trp Met His
    50                  55                  60

Asp Ala Asn Tyr Asn Ser Cys Thr Val Asn Gly Gly Val Asn Thr Thr
65                  70                  75                  80

Leu Cys Pro Asp Glu Ala Thr Cys Gly Lys Asn Cys Phe Ile Glu Gly
                85                  90                  95

Val Asp Tyr Ala Ala Ser Gly Val Thr Thr Ser Gly Ser Ser Leu Thr
            100                 105                 110

Met Asn Gln Tyr Met Pro Ser Ser Ser Gly Gly Tyr Ser Ser Val Ser
        115                 120                 125

Pro Arg Leu Tyr Leu Leu Asp Ser Asp Gly Glu Tyr Val Met Leu Lys
    130                 135                 140

Leu Asn Gly Gln Glu Leu Ser Phe Asp Val Asp Leu Ser Ala Leu Pro
145                 150                 155                 160

Cys Gly Glu Asn Gly Ser Leu Tyr Leu Ser Gln Met Asp Glu Asn Gly
                165                 170                 175

Gly Ala Asn Gln Tyr Asn Thr Ala Gly Ala Asn Tyr Gly Ser Gly Tyr
            180                 185                 190

Cys Asp Ala Gln Cys Pro Val Gln Thr Trp Arg Asn Gly Thr Leu Asn
        195                 200                 205

Thr Ser His Gln Gly Phe Cys Cys Asn Glu Met Asp Ile Leu Glu Gly
    210                 215                 220

Asn Ser Arg Ala Asn Ala Leu Thr Pro His Ser Cys Thr Ala Thr Ala
225                 230                 235                 240

Cys Asp Ser Ala Gly Cys Gly Phe Asn Pro Tyr Gly Ser Gly Tyr Lys
                245                 250                 255

Ser Tyr Tyr Gly Pro Gly Asp Thr Val Asp Thr Ser Lys Thr Phe Thr
            260                 265                 270

Ile Ile Thr Gln Phe Asn Thr Asp Asn Gly Ser Pro Ser Gly Asn Leu
        275                 280                 285

Val Gly Ile Thr Arg Lys Tyr Gln Gln Asn Gly Val Asp Ile Pro Ser
    290                 295                 300

Ala Gln Pro Gly Gly Asp Thr Ile Ser Ser Cys Pro Ser Ala Ser Ala
305                 310                 315                 320

Tyr Gly Gly Leu Ala Thr Met Gly Lys Ala Leu Ser Ser Gly Met Val
                325                 330                 335

Leu Val Phe Ser Ile Trp Asn Asp Asn Ser Gln Tyr Met Asn Trp Leu
            340                 345                 350

Asp Ser Gly Asn Ala Gly Pro Cys Ser Ser Thr Glu Gly Asn Pro Ser
        355                 360                 365

Asn Ile Leu Ala Asn Asn Pro Asn Thr His Val Val Phe Ser Asn Ile
    370                 375                 380

Arg Trp Gly Asp Ile Gly Ser Thr Thr Asn Ser Thr Ala Pro Pro Pro
385                 390                 395                 400

Pro Pro Ala Ser Ser Thr Thr Phe Ser Thr Thr Arg Arg Ser Ser Thr
                405                 410                 415

Thr Ser Ser Ser Pro Ser Cys Thr Gln Thr His Trp Gly Gln Cys Gly
            420                 425                 430

Gly Ile Gly Tyr Ser Gly Cys Lys Thr Cys Thr Ser Gly Thr Thr Cys
        435                 440                 445

Gln Tyr Ser Asn Asp Tyr Tyr Ser Gln Cys Leu

450                 455

<210>  36
<211>  463
<212>  PRT
<213>  Trichoderma longibrachiatum

<400>  36

Met Ala Pro Ser Ala Thr Leu Pro Leu Thr Thr Ala Ile Leu Ala Ile
1               5                   10                  15

Gly Arg Leu Val Ala Ala Gln Gln Pro Gly Thr Ser Thr Pro Glu Val
            20                  25                  30

His Pro Lys Leu Thr Thr Tyr Lys Cys Thr Thr Ser Gly Gly Cys Val
        35                  40                  45

Ala Gln Asp Thr Ser Val Val Leu Asp Trp Asn Tyr Arg Trp Met His
    50                  55                  60

Asp Ala Asn Tyr Asn Ser Cys Thr Val Asn Gly Gly Val Asn Thr Thr
65                  70                  75                  80

Leu Cys Pro Asp Glu Ala Thr Cys Gly Lys Asn Cys Tyr Ile Glu Gly
                85                  90                  95

Val Asp Tyr Ala Ala Ser Gly Val Thr Ala Ser Gly Ser Thr Leu Thr
            100                 105                 110

Leu Asn Gln Tyr Met Pro Ser Ser Ser Gly Gly Tyr Ser Ser Val Ser
        115                 120                 125

Pro Arg Leu Tyr Leu Leu Gly Pro Asp Gly Glu Tyr Val Met Leu Lys
    130                 135                 140

Leu Asn Gly Gln Glu Leu Ser Phe Asp Val Asp Leu Ser Ala Leu Pro
145                 150                 155                 160

Cys Gly Glu Asn Gly Ser Leu Tyr Leu Ser Gln Met Asp Glu Asn Gly
                165                 170                 175

Gly Ala Asn Gln Tyr Asn Thr Ala Gly Ala Asn Tyr Gly Ser Gly Tyr
            180                 185                 190

Cys Asp Ala Gln Cys Pro Val Gln Thr Trp Arg Asn Gly Thr Leu Asn
        195                 200                 205

Thr Ser Gly Gln Gly Phe Cys Cys Asn Glu Met Asp Ile Leu Glu Gly

    210                 215                 220

Asn Ser Arg Ala Asn Ala Leu Thr Pro His Ser Cys Thr Ala Thr Ala
225                 230                 235                 240

Cys Asp Ser Ala Gly Cys Gly Phe Asn Pro Tyr Gly Ser Gly Tyr Pro
                245                 250                 255

Asn Tyr Phe Gly Pro Gly Asp Thr Val Asp Thr Ser Lys Thr Phe Thr
            260                 265                 270

Ile Ile Thr Gln Phe Asn Thr Asp Asn Gly Ser Pro Ser Gly Asn Leu
        275                 280                 285

Val Ser Ile Thr Arg Lys Tyr Arg Gln Asn Gly Val Asp Ile Pro Ser
    290                 295                 300

Ala Lys Pro Gly Gly Asp Thr Ile Ser Ser Cys Pro Ser Ala Ser Ala
305                 310                 315                 320

Tyr Gly Gly Leu Ala Thr Met Gly Lys Ala Leu Ser Ser Gly Met Val
                325                 330                 335

Leu Val Phe Ser Ile Trp Asn Asp Asn Ser Gln Tyr Met Asn Trp Leu
            340                 345                 350

Asp Ser Gly Arg Ala Gly Pro Cys Ser Ser Thr Glu Gly Asn Pro Ser
        355                 360                 365

Asn Ile Leu Ala Asn Asn Pro Gly Thr His Val Val Tyr Ser Asn Ile
    370                 375                 380

Arg Trp Gly Asp Ile Gly Ser Thr Thr Asn Ser Thr Gly Gly Asn Pro
385                 390                 395                 400

Pro Pro Pro Pro Pro Pro Ala Ser Ser Thr Thr Phe Ser Thr Thr Arg
                405                 410                 415

Arg Ser Ser Thr Thr Ser Ser Ser Pro Ser Cys Thr Gln Thr His Trp
            420                 425                 430

Gly Gln Cys Gly Gly Ile Gly Tyr Thr Gly Cys Lys Thr Cys Thr Ser
        435                 440                 445

Gly Thr Thr Cys Gln Tyr Gly Asn Asp Tyr Tyr Ser Gln Cys Leu
    450                 455                 460

<210>  37
<211>  461
<212>  PRT
<213>  Trichoderma orientale

<400>  37

Met Ala Pro Ser Val Thr Leu Pro Leu Thr Thr Ala Met Leu Ala Leu
1               5                   10                  15

Val Arg Leu Val Ala Ala Gln Gln Pro Gly Thr Ser Thr Pro Glu Val
            20                  25                  30

His Pro Lys Leu Thr Thr Tyr Lys Cys Thr Thr Ser Gly Gly Cys Val
        35                  40                  45

Ala Gln Asp Thr Ser Val Val Leu Asp Trp Asn Tyr Arg Trp Met His
    50                  55                  60

Asp Ala Asn Tyr Asn Ser Cys Thr Val Asn Gly Gly Val Asn Thr Thr
65                  70                  75                  80

Leu Cys Pro Asp Glu Ala Thr Cys Gly Lys Asn Cys Phe Ile Glu Gly
                85                  90                  95

Val Asp Tyr Ala Ala Ser Gly Val Thr Ala Ser Gly Ser Thr Leu Thr
            100                 105                 110

Leu Asn Gln Tyr Met Pro Ser Ser Ser Gly Gly Tyr Ser Ser Val Ser
        115                 120                 125

Pro Arg Leu Tyr Leu Leu Gly Pro Asp Gly Glu Tyr Val Met Leu Lys
    130                 135                 140

Leu Asn Gly Gln Glu Leu Ser Phe Asp Val Asp Leu Ser Ala Leu Pro
145                 150                 155                 160

Cys Gly Glu Asn Gly Ser Leu Tyr Leu Ser Gln Met Asp Glu Asn Gly
                165                 170                 175

Gly Ala Asn Gln Tyr Asn Thr Ala Gly Ala Asn Tyr Gly Ser Gly Tyr
            180                 185                 190

Cys Asp Ala Gln Cys Pro Val Gln Thr Trp Arg Asn Gly Thr Leu Asn
        195                 200                 205

Thr Ser Gly Gln Gly Phe Cys Cys Asn Glu Met Asp Ile Leu Glu Gly
    210                 215                 220

Asn Ser Arg Ala Asn Ala Leu Thr Pro His Ser Cys Thr Ala Thr Ala
225                 230                 235                 240

Cys Asp Ser Ala Gly Cys Gly Phe Asn Pro Tyr Gly Ser Gly Tyr Pro
                245                 250                 255

Asn Tyr Phe Gly Pro Gly Asp Thr Val Asp Thr Ser Lys Pro Phe Thr
            260                 265                 270

Ile Ile Thr Gln Phe Asn Thr Asp Asn Gly Ser Pro Ser Gly Asn Leu
        275                 280                 285

Val Ser Ile Thr Arg Lys Tyr Arg Gln Asn Gly Val Asp Ile Pro Ser
    290                 295                 300

Ala Lys Pro Gly Gly Asp Thr Ile Ser Ser Cys Pro Ser Ala Ser Ala
305                 310                 315                 320

Tyr Gly Gly Leu Ala Thr Met Gly Lys Ala Leu Ser Ser Gly Met Val
                325                 330                 335

Leu Ile Phe Ser Ile Trp Asn Asp Asn Ser Gln Tyr Met Asn Trp Leu
            340                 345                 350

Asp Ser Gly Ser Ala Gly Pro Cys Ser Ser Thr Glu Gly Asn Pro Ser
        355                 360                 365

Asn Ile Leu Ala Asn Asn Pro Gly Thr His Val Val Tyr Ser Asn Ile
    370                 375                 380

Arg Trp Gly Asp Ile Gly Ser Thr Thr Asn Ser Thr Gly Gly Asn Pro
385                 390                 395                 400

Pro Pro Pro Pro Ala Ser Ser Thr Thr Phe Ser Thr Thr Arg Arg Ser
                405                 410                 415

Ser Thr Thr Ser Ser Ser Pro Ser Cys Thr Gln Thr His Trp Gly Gln
            420                 425                 430

Cys Gly Gly Ile Gly Tyr Thr Gly Cys Lys Thr Cys Thr Ser Gly Thr
        435                 440                 445

Thr Cys Gln Tyr Gly Asn Asp Tyr Tyr Ser Gln Cys Leu
    450                 455                 460

<210>  38
<211>  461
<212>  PRT

<213>  Trichoderma pseudokoningii

<400>  38

Met Ala Pro Ser Ala Thr Leu Pro Leu Thr Thr Ala Met Leu Ala Leu
1               5                   10                  15

Val Arg Leu Val Ala Ala Gln Gln Pro Gly Thr Ser Thr Pro Glu Val
            20                  25                  30

His Pro Lys Leu Thr Thr Tyr Lys Cys Thr Thr Ser Gly Gly Cys Val
        35                  40                  45

Ala Gln Asp Thr Ser Val Val Leu Asp Trp Asn Tyr Arg Trp Met His
    50                  55                  60

Asp Ala Asn Tyr Asn Ser Cys Thr Val Asn Gly Gly Val Asn Thr Thr
65                  70                  75                  80

Leu Cys Pro Asp Glu Ala Thr Cys Gly Lys Asn Cys Phe Ile Glu Gly
                85                  90                  95

Val Asp Tyr Ala Ala Ser Gly Val Thr Ala Ser Gly Ser Thr Leu Thr
            100                 105                 110

Leu Asn Gln Tyr Met Pro Ser Ser Ser Gly Gly Tyr Ser Ser Val Ser
        115                 120                 125

Pro Arg Leu Tyr Leu Leu Gly Pro Asp Gly Glu Tyr Val Met Leu Lys
    130                 135                 140

Leu Asn Gly Gln Glu Leu Ser Phe Asp Val Asp Leu Ser Ala Leu Pro
145                 150                 155                 160

Cys Gly Glu Asn Gly Ser Leu Tyr Leu Ser Gln Met Asp Glu Asn Gly
                165                 170                 175

Gly Ala Asn Gln Tyr Asn Thr Ala Gly Ala Asn Tyr Gly Ser Gly Tyr
            180                 185                 190

Cys Asp Ala Gln Cys Pro Val Gln Thr Trp Arg Asn Gly Thr Leu Asn
        195                 200                 205

Thr Ser Gly Gln Gly Phe Cys Cys Asn Glu Met Asp Ile Leu Glu Gly
    210                 215                 220

Asn Ser Arg Ala Asn Ala Leu Thr Pro His Ser Cys Thr Ala Thr Ala
225                 230                 235                 240

Cys Asp Ser Ala Gly Cys Gly Phe Asn Pro Tyr Gly Ser Gly Tyr Pro
                245                 250                 255

Asn Tyr Phe Gly Pro Gly Asp Thr Val Asp Thr Ser Lys Pro Phe Thr
            260                 265                 270

Ile Ile Thr Gln Phe Asn Thr Asp Asn Gly Ser Pro Ser Gly Asn Leu
        275                 280                 285

Val Ser Ile Thr Arg Lys Tyr Arg Gln Asn Gly Val Asp Ile Pro Ser
    290                 295                 300

Ala Lys Pro Gly Gly Asp Thr Ile Ser Ser Cys Pro Ser Ala Ser Ala
305                 310                 315                 320

Tyr Gly Gly Leu Ala Thr Met Gly Lys Ala Leu Ser Ser Gly Met Val
                325                 330                 335

Leu Ile Phe Ser Ile Trp Asn Asp Asn Ser Gln Tyr Met Asn Trp Leu
            340                 345                 350

Asp Ser Gly Ser Ala Gly Pro Cys Ser Ser Thr Glu Gly Asn Pro Ser
        355                 360                 365

Asn Ile Leu Ala Asn Asn Pro Gly Thr His Val Val Tyr Ser Asn Ile
    370                 375                 380

Arg Trp Gly Asp Ile Gly Ser Thr Thr Asn Ser Thr Gly Gly Asn Pro
385                 390                 395                 400

Pro Pro Pro Pro Ala Ser Ser Thr Thr Phe Ser Thr Thr Arg Arg Ser
                405                 410                 415

Ser Thr Thr Ser Ser Ser Pro Ser Cys Thr Gln Thr His Trp Gly Gln
            420                 425                 430

Cys Gly Gly Ile Gly Tyr Thr Gly Cys Lys Thr Cys Thr Ser Gly Thr
        435                 440                 445

Thr Cys Gln Tyr Gly Asn Asp Tyr Tyr Ser Gln Cys Leu
    450                 455                 460

<210>  39
<211>  472
<212>  PRT
<213>  Hypocrea atroviridis

<400>  39

Met Ala Pro Ser Gly Thr Leu Pro Leu Ala Ala Ala Ile Leu Ala Leu
1               5                   10                  15

Ala Gly Ile Val Thr Ala Gln Gln Pro Gly Thr Ser Thr Pro Glu Val
            20                  25                  30

His Pro Lys Leu Thr Thr Tyr Gln Cys Thr Thr Ser Gly Gly Cys Val
        35                  40                  45

Ala Gln Asp Thr Ser Val Val Leu Asp Trp Asn Tyr Arg Trp Met His
    50                  55                  60

Asp Ala Asn Tyr Asn Ser Cys Thr Val Asn Gly Gly Val Asn Thr Thr
65                  70                  75                  80

Ile Cys Pro Asp Glu Ala Thr Cys Gly Thr Asn Cys Phe Ile Glu Gly
                85                  90                  95

Val Asn Tyr Thr Ala Ser Gly Val Thr Thr Ser Gly Ser Ser Leu Thr
            100                 105                 110

Met Asn Gln Tyr Met Pro Ala Thr Thr Gly Gly Tyr Ser Ser Val Ser
        115                 120                 125

Pro Arg Leu Tyr Leu Leu Gly Ala Asp Gly Asn Tyr Val Leu Leu Gln
    130                 135                 140

Leu Asn Gly Lys Glu Leu Ser Phe Asp Val Asp Leu Ser Ala Leu Pro
145                 150                 155                 160

Cys Gly Glu Asn Gly Ser Leu Tyr Leu Ser Gln Met Ala Asp Asn Gly
                165                 170                 175

Gly Ala Asn Gln Tyr Asn Thr Ala Gly Ala Asn Tyr Gly Ser Gly Tyr
            180                 185                 190

Cys Asp Ala Gln Cys Pro Val Gln Thr Trp Lys Asn Gly Thr Leu Asn
        195                 200                 205

Thr Asn His Ser Gly Tyr Cys Cys Asn Glu Met Asp Ile Leu Glu Gly
    210                 215                 220

Asn Ser Glu Ala Asn Ala Leu Thr Pro His Ser Cys Thr Ala Thr Ala
225                 230                 235                 240

Cys Asp Ser Ser Gly Cys Gly Leu Asn Pro Tyr Ala Ser Gly Phe His
                245                 250                 255

Ser Tyr Tyr Gly Pro Gly Leu Thr Val Asp Thr Ser Lys Pro Phe Thr
            260                 265                 270

Ile Thr Thr Gln Phe Asn Thr Asp Asn Gly Ser Pro Ser Gly Asn Leu
        275                 280                 285

Val Ser Ile Thr Arg Lys Tyr Ile Gln Asn Gly Val Ser Ile Pro Ser
    290                 295                 300

Ala Gln Ser Gly Gly Asp Ile Ile Ser Ser Cys Pro Ser Ala Ser Ala
305                 310                 315                 320

Tyr Gly Gly Leu Thr Thr Met Gly Lys Ala Leu Thr Ser Gly Met Val
                325                 330                 335

Leu Ile Phe Ser Ile Trp Asn Asp Ser Gly Gly Tyr Met Asn Trp Leu
            340                 345                 350

Asp Ser Gly Ser Ser Gly Pro Cys Ser Ser Thr Glu Gly Asn Pro Ser
        355                 360                 365

Thr Ile Leu Ala Asn Asn Pro Gly Thr His Val Thr Phe Ser Asn Ile
    370                 375                 380

Lys Trp Gly Asp Ile Gly Ser Thr Thr Ser Gly Gly Ser Ser Pro Pro
385                 390                 395                 400

Pro Pro Pro Ala Ser Ser Thr Thr Leu Arg Thr Ser Thr Thr Thr Ser
                405                 410                 415

Lys Thr Ser Thr Ala Pro Pro Ser Cys Thr Gln Thr His Trp Gly Gln
            420                 425                 430

Cys Gly Gly Asn Gly Tyr Thr Gly Cys Lys Thr Cys Ala Ser Gly Thr
        435                 440                 445

Thr Cys Gln Tyr Ser Asn Asp Cys Glu Tyr Ser Met Asn Ile Ser Lys
    450                 455                 460

Arg Arg Phe Arg Ile Leu Ile Met
465                 470

<210>  40
<211>  470
<212>  PRT
<213>  Trichoderma gamsii

<400> 40

Met Ala Pro Pro Ser Thr Leu Pro Leu Ala Ala Ala Val Leu Ala Leu
1               5                   10                  15

Ala Gly Ile Val Thr Ala Gln Gln Pro Gly Thr Ser Thr Pro Glu Val
            20                  25                  30

His Pro Lys Leu Thr Thr Tyr Gln Cys Thr Thr Ser Gly Gly Cys Val
        35                  40                  45

Ala Gln Asp Thr Ser Val Val Leu Asp Trp Asn Tyr Arg Trp Met His
    50                  55                  60

Asp Val Asn Tyr Asn Ser Cys Thr Val Asn Gly Gly Val Asn Ser Thr
65                  70                  75                  80

Ile Cys Pro Asn Glu Ala Thr Cys Gly Thr Asn Cys Phe Ile Glu Gly
                85                  90                  95

Val Asn Tyr Thr Ala Ser Gly Val Thr Thr Ser Gly Ser Ser Leu Thr
            100                 105                 110

Leu Asn Gln Tyr Met Pro Ala Thr Thr Gly Gly Tyr Ser Ser Val Ser
        115                 120                 125

Pro Arg Leu Tyr Leu Leu Asp Ala Asp Gly Asn Tyr Val Leu Leu Gln
    130                 135                 140

Leu Asn Gly Lys Glu Leu Ser Phe Asp Val Asp Ile Ser Ala Leu Pro
145                 150                 155                 160

Cys Gly Glu Asn Gly Ser Leu Tyr Leu Ser Gln Met Ala Ala Asn Gly
                165                 170                 175

Gly Ala Asn Gln Tyr Asn Thr Ala Gly Ala Asn Tyr Gly Ser Gly Tyr
            180                 185                 190

Cys Asp Ala Gln Cys Pro Val Gln Ala Trp Lys Asn Gly Thr Leu Asn
        195                 200                 205

Thr Asn Asn Ser Gly Tyr Cys Cys Asn Glu Met Asp Ile Leu Glu Gly
    210                 215                 220

Asn Ser Glu Ala Asn Ala Leu Thr Pro His Ser Cys Thr Ala Thr Ala
225                 230                 235                 240

Cys Asp Ser Ser Gly Cys Gly Leu Asn Pro Tyr Ala Ser Gly Phe His

                245                 250                 255

Ser Tyr Tyr Gly Pro Gly Leu Thr Val Asp Thr Ser Lys Pro Phe Thr
            260                 265                 270

Ile Ile Thr Gln Phe Asn Thr Asp Thr Gly Ser Pro Ser Gly Asn Leu
        275                 280                 285

Val Ser Ile Thr Arg Lys Tyr Ile Gln Asn Gly Val Ser Ile Pro Ser
    290                 295                 300

Ala Gln Ser Gly Gly Asp Ile Ile Ser Ser Cys Pro Ser Ala Ser Asn
305                 310                 315                 320

Tyr Gly Gly Leu Asn Thr Ile Gly Lys Ala Leu Thr Ser Gly Met Val
                325                 330                 335

Leu Ile Phe Ser Ile Trp Asn Asp Asn Ser Gly Phe Met Asn Trp Leu
            340                 345                 350

Asp Ser Gly Ser Ser Gly Pro Cys Ser Ser Thr Glu Gly Asn Pro Ala
        355                 360                 365

Thr Ile Leu Ala Asn Asn Pro Asn Thr His Ile Thr Tyr Ser Asn Ile
    370                 375                 380

Lys Trp Gly Asp Ile Gly Ser Thr Thr Ser Asn Gly Ser Ser Pro Pro
385                 390                 395                 400

Pro Val Ser Ser Ser Ser Ser Ser Ser Lys Pro Pro Ala Ser Ser Thr
                405                 410                 415

Thr Leu Arg Thr Ser Thr Thr Thr Ser Lys Thr Ser Thr Ala Pro Pro
            420                 425                 430

Ser Cys Thr Gln Thr His Trp Gly Gln Cys Gly Gly Asn Gly Tyr Thr
        435                 440                 445

Gly Cys Lys Thr Cys Ala Ser Gly Thr Thr Cys Gln Tyr Ser Asn Asp
    450                 455                 460

Tyr Tyr Ser Gln Cys Leu
465                 470

<210> 41
<211> 471
<212> PRT
<213> Trichoderma harzianum

<400>  41

Met Ala Leu Ser Gly Pro Phe Pro Leu Ala Thr Ala Val Leu Val Ala
1               5                   10                  15

Cys Ile Ala Gln Leu Val Ala Gly Gln Gln Pro Gly Thr Ser Thr Pro
            20                  25                  30

Glu Val His Pro Lys Leu Thr Thr Tyr Lys Cys Thr Lys Ser Gly Gly
        35                  40                  45

Cys Val Ala Gln Asp Thr Ser Val Val Leu Asp Trp Asn Tyr Arg Trp
    50                  55                  60

Met His Asp Lys Asn Phe Asn Ser Cys Thr Val Asn Gly Gly Val Asn
65                  70                  75                  80

Thr Thr Leu Cys Pro Asp Glu Ala Thr Cys Gly Ala Asn Cys Phe Ile
                85                  90                  95

Glu Gly Ala Asp Tyr Ala Ala Ser Gly Val Thr Val Ser Gly Asn Ser
            100                 105                 110

Leu Thr Leu Asn Gln Tyr Met Pro Ser Ser Thr Gly Gly Tyr Ser Ser
        115                 120                 125

Val Ser Pro Arg Leu Tyr Leu Leu Gly Ser Asp Gly Asp Tyr Glu Leu
    130                 135                 140

Leu Gln Leu Asn Gly Gln Glu Leu Ser Phe Asp Val Asp Leu Ser Thr
145                 150                 155                 160

Leu Pro Cys Gly Glu Asn Gly Ala Leu Tyr Leu Ser Glu Met Ala Ala
                165                 170                 175

Asn Gly Gly Ala Asn Gln Tyr Asn Thr Ala Gly Ala Asn Tyr Gly Ser
            180                 185                 190

Gly Tyr Cys Asp Ala Gln Cys Pro Val Gln Thr Trp Lys Asn Gly Thr
        195                 200                 205

Leu Asn Thr Asn Asn Ser Gly Tyr Cys Cys Asn Glu Met Asp Ile Leu
    210                 215                 220

Glu Ala Asn Ser Arg Ala Asn Ala Phe Thr Pro His Ser Cys Thr Ala
225                 230                 235                 240

Thr Ala Cys Asp Ala Ser Gly Cys Gly Phe Asn Pro Tyr Ala Ser Gly
                245                 250                 255

Phe Gln Arg Tyr Trp Gly Pro Gly Phe Thr Leu Asp Thr Ser Lys Val
            260                 265                 270

Phe Thr Ile Ile Thr Gln Phe Asn Thr Asp Asn Gly Leu Pro Ser Gly
        275                 280                 285

Asn Leu Val Ser Ile Thr Arg Lys Tyr Arg Gln Asn Gly Val Asp Val
    290                 295                 300

Pro Ser Ala Gln Ser Gly Gly Asp Thr Ile Ser Ser Cys Ser Ser Gly
305                 310                 315                 320

Ser Ala Tyr Gly Gly Leu Thr Thr Met Gly Lys Ala Leu Ala Asn Gly
                325                 330                 335

Met Val Leu Val Phe Ser Ile Trp Asn Asp Asn Gly Gly Asn Met Asn
            340                 345                 350

Trp Leu Asp Ser Gly Asn Ala Gly Pro Cys Ser Ser Thr Glu Gly Asn
        355                 360                 365

Pro Ser Thr Ile Val Ala Asn Asn Pro Gly Thr His Val Ile Phe Ser
    370                 375                 380

Asn Ile Arg Trp Gly Asp Ile Gly Ser Thr Thr Gly Ser Thr Gly Gly
385                 390                 395                 400

Asn Pro Pro Pro Ser Thr Ser Arg Thr Ser Thr Thr Pro Pro Pro Ser
                405                 410                 415

Thr Thr Leu Arg Thr Ser Thr Ser Ser Arg Thr Thr Thr Ser Ser Ala
            420                 425                 430

Ala Gly Cys Thr Gln Thr His Trp Gly Gln Cys Gly Gly Asn Gly Tyr
        435                 440                 445

Thr Gly Cys Lys Ile Cys Thr Ala Gly Thr Thr Cys Gln Tyr Ser Asn
    450                 455                 460

Asp Phe Tyr Ser Gln Cys Leu
465                 470

<210>  42
<211>  464
<212>  PRT

<213>  Thielavia terrestris

<400>  42

Met Gly Gln Lys Thr Leu His Gly Phe Ala Ala Thr Ala Leu Ala Val
1               5                   10                  15

Leu Pro Phe Val Lys Ala Gln Gln Pro Gly Asn Phe Thr Pro Glu Val
            20                  25                  30

His Pro Gln Leu Pro Thr Trp Lys Cys Thr Thr Ala Gly Gly Cys Val
        35                  40                  45

Gln Gln Asp Thr Ser Val Val Leu Asp Trp Asn Tyr Arg Trp Ile His
    50                  55                  60

Asn Ala Asp Gly Thr Ala Ser Cys Thr Thr Ser Ser Gly Val Asp His
65                  70                  75                  80

Thr Leu Cys Pro Asp Glu Ala Thr Cys Ala Lys Asn Cys Phe Val Glu
                85                  90                  95

Gly Val Asn Tyr Thr Ser Ser Gly Val Thr Thr Ser Gly Ser Ser Leu
            100                 105                 110

Thr Met Arg Gln Tyr Phe Lys Gly Ser Asn Gly Gln Thr Asn Ser Val
        115                 120                 125

Ser Pro Arg Leu Tyr Leu Leu Gly Ser Asp Gly Asn Tyr Val Met Leu
    130                 135                 140

Lys Leu Leu Gly Gln Glu Leu Ser Phe Asp Val Asp Leu Ser Thr Leu
145                 150                 155                 160

Pro Cys Gly Glu Asn Gly Ala Leu Tyr Leu Ser Glu Met Asp Ala Thr
                165                 170                 175

Gly Gly Arg Asn Gln Tyr Asn Thr Gly Gly Ala Asn Tyr Gly Ser Gly
            180                 185                 190

Tyr Cys Asp Ala Gln Cys Pro Val Gln Thr Trp Met Asn Gly Thr Leu
        195                 200                 205

Asn Thr Asn Gly Gln Gly Tyr Cys Cys Asn Glu Met Asp Ile Leu Glu
    210                 215                 220

Ala Asn Ser Arg Ala Asn Ala Met Thr Pro His Pro Cys Ala Asn Gly
225                 230                 235                 240

Ser Cys Asp Lys Ser Gly Cys Gly Leu Asn Pro Tyr Ala Glu Gly Tyr
                245                 250                 255

Lys Ser Tyr Tyr Gly Pro Gly Leu Thr Val Asp Thr Ser Lys Pro Phe
            260                 265                 270

Thr Ile Ile Thr Arg Phe Ile Thr Asp Asp Gly Thr Thr Ser Gly Thr
        275                 280                 285

Leu Asn Gln Ile Gln Arg Ile Tyr Val Gln Asn Gly Lys Thr Val Ala
    290                 295                 300

Ser Ala Ala Ser Gly Gly Asp Ile Ile Thr Ala Ser Gly Cys Thr Ser
305                 310                 315                 320

Ala Gln Ala Phe Gly Gly Leu Ala Asn Met Gly Ala Ala Leu Gly Arg
                325                 330                 335

Gly Met Val Leu Thr Phe Ser Ile Trp Asn Asp Ala Gly Gly Tyr Met
            340                 345                 350

Asn Trp Leu Asp Ser Gly Asn Asn Gly Pro Cys Ser Ser Thr Glu Gly
        355                 360                 365

Asn Pro Ser Asn Ile Leu Ala Asn Tyr Pro Asp Thr His Val Val Phe
    370                 375                 380

Ser Asn Ile Arg Trp Gly Asp Ile Gly Ser Thr Val Gln Val Ser Gly
385                 390                 395                 400

Gly Gly Asn Gly Gly Ser Thr Thr Thr Thr Ser Thr Thr Thr Leu Arg
                405                 410                 415

Thr Ser Thr Thr Thr Thr Thr Thr Ala Pro Thr Ala Thr Ala Thr His
            420                 425                 430

Trp Gly Gln Cys Gly Gly Ile Gly Trp Thr Gly Pro Thr Val Cys Glu
        435                 440                 445

Ser Pro Tyr Ala Cys Lys Glu Leu Asn Pro Trp Tyr Tyr Gln Cys Leu
    450                 455                 460

<210>  43
<211>  499
<212>  PRT
<213>  Dactylellina haptotyla

<400>  43

Met Thr Ala Arg Tyr Ala Phe Lys Phe Ser Leu Ala Thr Leu Val Phe
1               5                   10                  15

Ser Arg Leu Ala Ser Ala Gln Val Pro Gly Thr Gly Thr Pro Glu Val
            20                  25                  30

His Pro Lys Leu Pro Thr Tyr Lys Cys Thr Thr Ser Gly Gly Cys Val
        35                  40                  45

Met Gln Asp Thr Ser Val Val Leu Asp Trp Asn Tyr Arg Tyr Ile His
    50                  55                  60

Ser Gly Ser Thr Ser Cys Thr Thr Ser Ser Gly Val Asn Thr Ala Leu
65                  70                  75                  80

Cys Pro Asn Gln Ala Thr Cys Ser Ala Asn Cys Gln Ile Glu Gly Val
                85                  90                  95

Asp Tyr Ala Ala Ala Gly Val Thr Thr Ser Gly Asn Ala Val Thr Met
            100                 105                 110

Tyr Gln Tyr Lys Thr Ala Asn Gly Lys Thr Ser Ser Val Ser Pro Arg
        115                 120                 125

Ile Tyr Leu Leu Gly Pro Asp Gly Asn Tyr Val Met Met Lys Leu Asn
    130                 135                 140

Gly Gln Glu Leu Ser Phe Asp Val Asp Leu Ser Thr Leu Pro Cys Gly
145                 150                 155                 160

Glu Asn Gly Ala Leu Tyr Leu Ser Glu Met Ser Ala Thr Gly Gly Arg
                165                 170                 175

Asn Thr Asn Asn Pro Gly Gly Ala Asn Tyr Gly Ser Gly Tyr Cys Asp
            180                 185                 190

Ala Gln Cys Pro Tyr Glu Thr Trp Arg Asn Gly Thr Ile Asp Ser Ala
        195                 200                 205

Gly Pro Phe Cys Cys Asn Glu Met Asp Ile Leu Glu Ala Asn Ser Arg
    210                 215                 220

Ala Asn Ala Phe Thr Pro His Pro Cys Thr Thr Asn Asp Cys Asp Lys
225                 230                 235                 240

Gly Gly Cys Gly Phe Asn Pro Tyr Ala Ser Gly Tyr His Asn Tyr Trp
                245                 250                 255

Ala Pro Gly Gly Thr Leu Asp Thr Ser Lys Pro Phe Thr Val Ile Thr
            260                 265                 270

Gln Phe Val Thr Ser Asp Gly Ser Gln Ser Gly Ser Leu Thr Thr Ile
        275                 280                 285

Thr Arg Lys Tyr Ile Gln Asn Gly Arg Gln Ile Ala Ser Ala Val Ser
    290                 295                 300

Ser Gly Asp Ser Val Tyr Ala Asn Asn Cys Gln Ser Gly Ala Pro Tyr
305                 310                 315                 320

Gly Gly Leu Asn Thr Met Gly Gln Ala Leu Ala Arg Gly Met Val Leu
                325                 330                 335

Ile Phe Ser Ile Trp Asn Asp Ala Gly Gln Asn Met Asn Trp Leu Asp
            340                 345                 350

Ser Gly Ser Ser Gly Pro Cys Ser Ser Thr Glu Gly Ala Pro Ser Asn
        355                 360                 365

Ile Leu Ala Asn Asn Pro Asn Thr His Val Ile Phe Ser Asn Ile Arg
    370                 375                 380

Trp Gly Asp Ile Gly Ser Thr Thr Gly Gly Ile Ile Val Ser Ser Ser
385                 390                 395                 400

Ser Ser Thr Thr Arg Thr Thr Thr Thr Ser Ser Arg Thr Thr Thr Thr
                405                 410                 415

Ser Ser Arg Thr Thr Thr Thr Ser Ser Arg Thr Thr Thr Thr Thr Thr
            420                 425                 430

Thr Ser Ser Arg Thr Thr Thr Thr Ser Ser Arg Thr Thr Thr Pro Ser
        435                 440                 445

Thr Thr Thr Thr Thr Thr Thr Arg Thr Thr Thr Ala Asn Gly Ala Gln
    450                 455                 460

Gln Thr His Trp Gly Gln Cys Gly Gly Ile Gly Trp Thr Gly Pro Thr
465                 470                 475                 480

Ala Cys Ser Asp Gly Phe Ser Cys Ser Val Leu Asn Pro Tyr Tyr Ser
                485                 490                 495

Gln Cys Leu

<210>  44
<211>  464
<212>  PRT
<213>  Myceliophthora thermophila

<400>  44

Met Gly Gln Lys Thr Leu Gln Gly Leu Val Ala Ala Ala Ala Leu Ala
1               5                   10                  15

Ala Ser Val Ala Asn Ala Gln Gln Pro Gly Thr Phe Thr Pro Glu Val
            20                  25                  30

His Pro Thr Leu Pro Thr Trp Lys Cys Thr Thr Ser Gly Gly Cys Val
        35                  40                  45

Gln Gln Asp Thr Ser Val Val Leu Asp Trp Asn Tyr Arg Trp Phe His
    50                  55                  60

Thr Glu Asp Gly Ser Lys Ser Cys Ile Thr Ser Ser Gly Val Asp Arg
65                  70                  75                  80

Thr Leu Cys Pro Asp Glu Ala Thr Cys Ala Lys Asn Cys Phe Val Glu
                85                  90                  95

Gly Val Asn Tyr Thr Ser Ser Gly Val Glu Thr Ser Gly Ser Ser Leu
            100                 105                 110

Thr Leu Arg Gln Phe Phe Lys Gly Ser Asp Gly Ala Ile Asn Ser Val
        115                 120                 125

Ser Pro Arg Val Tyr Leu Leu Gly Gly Asp Gly Asn Tyr Val Val Leu
    130                 135                 140

Lys Leu Leu Gly Gln Glu Leu Ser Phe Asp Val Asp Val Ser Ser Leu
145                 150                 155                 160

Pro Cys Gly Glu Asn Ala Ala Leu Tyr Leu Ser Glu Met Asp Ala Thr
                165                 170                 175

Gly Gly Arg Asn Glu Tyr Asn Thr Gly Gly Ala Glu Tyr Gly Ser Gly
            180                 185                 190

Tyr Cys Asp Ala Gln Cys Pro Val Gln Asn Trp Asn Asn Gly Thr Leu
        195                 200                 205

Asn Thr Gly Arg Val Gly Ser Cys Cys Asn Glu Met Asp Ile Leu Glu

210                 215                 220

Ala Asn Ser Lys Ala Glu Ala Phe Thr Pro His Pro Cys Ile Gly Asn
225                 230                 235                 240

Ser Cys Asp Lys Ser Gly Cys Gly Phe Asn Ala Tyr Ala Arg Gly Tyr
                245                 250                 255

His Asn Tyr Trp Ala Pro Gly Gly Thr Leu Asp Thr Ser Arg Pro Phe
            260                 265                 270

Thr Met Ile Thr Arg Phe Val Thr Asp Asp Gly Thr Thr Ser Gly Lys
        275                 280                 285

Leu Ala Arg Ile Glu Arg Val Tyr Val Gln Asp Gly Lys Lys Val Pro
    290                 295                 300

Ser Ala Ala Pro Gly Gly Asp Val Ile Thr Ala Asp Gly Cys Thr Ser
305                 310                 315                 320

Ala Gln Pro Tyr Gly Gly Leu Ser Gly Met Gly Asp Ala Leu Gly Arg
                325                 330                 335

Gly Met Val Leu Ala Leu Ser Ile Trp Asn Asp Ala Ser Gly Tyr Met
            340                 345                 350

Asn Trp Leu Asp Ala Gly Ser Asn Gly Pro Cys Ser Asp Thr Glu Gly
        355                 360                 365

Asn Pro Ser Asn Ile Leu Ala Asn His Pro Asp Ala His Val Val Leu
    370                 375                 380

Ser Asn Ile Arg Trp Gly Asp Ile Gly Ser Thr Val Asp Thr Gly Asp
385                 390                 395                 400

Gly Asp Asn Asn Gly Gly Gly Pro Asn Pro Ser Ser Thr Thr Thr Ala
                405                 410                 415

Thr Ala Thr Thr Thr Ser Ser Gly Pro Ala Glu Pro Thr Gln Thr His
            420                 425                 430

Tyr Gly Gln Cys Gly Gly Lys Gly Trp Thr Gly Pro Thr Arg Cys Glu
        435                 440                 445

Thr Pro Tyr Thr Cys Lys Tyr Gln Asn Asp Trp Tyr Ser Gln Cys Leu
    450                 455                 460

<210>  45
<211>  460
<212>  PRT
<213>  Aspergillus udagawae

<400>  45

Met Asp Ser Lys Arg Gly Ile Val Ala Ala Val Leu Ala Leu Leu Pro
1               5                   10                  15

Leu Val Ser Ala Gln Gln Pro Ala Ser Ser Ser Ala Gly Asn Pro Lys
            20                  25                  30

Leu Thr Thr Tyr Lys Cys Thr Thr Ala Gly Gly Cys Val Ala Gln Asp
        35                  40                  45

Thr Ser Val Val Leu Asp Trp Ala Tyr His Trp Ile His Thr Val Asn
    50                  55                  60

Gly Tyr Thr Ser Cys Thr Thr Ser Ser Gly Val Asn Ser Thr Leu Cys
65                  70                  75                  80

Pro Asp Gln Ala Thr Cys Ala Lys Asn Cys Val Ile Glu Pro Ala Asn
                85                  90                  95

Tyr Thr Ser Ala Gly Val Ala Thr Ser Gly Asp Thr Leu Thr Met Ser
            100                 105                 110

Gln Tyr Val Leu Ser Asn Gly Val Tyr Thr Asn Ala Ser Pro Arg Leu
        115                 120                 125

Tyr Leu Leu Gly Pro Asp Lys Asn Tyr Val Met Phe Lys Leu Leu Gly
    130                 135                 140

Gln Glu Leu Ser Phe Asp Val Asp Leu Ser Thr Leu Pro Cys Gly Glu
145                 150                 155                 160

Asn Gly Ala Leu Tyr Phe Ser Glu Met Ser Ala Thr Gly Gly Arg Asn
                165                 170                 175

Glu Tyr Asn Thr Gly Gly Ala Glu Tyr Gly Ser Gly Tyr Cys Asp Ala
            180                 185                 190

Gln Cys Pro Val Ile Thr Trp Lys Asn Gly Thr Leu Asn Thr Ser Gly
        195                 200                 205

Ala Ser Tyr Cys Cys Asn Glu Met Asp Ile Leu Glu Ala Asn Ser Arg
    210                 215                 220

Ala Asn Ser Tyr Thr Pro His Pro Cys Ser Ala Thr Asp Cys Asp Lys
225                 230                 235                 240

Gly Gly Cys Gly Phe Asn Pro Tyr Ser Leu Gly Gln Lys Ser Tyr Trp
                245                 250                 255

Gly Pro Gly Gly Thr Val Asp Thr Ser Lys Pro Phe Thr Ile Thr Thr
            260                 265                 270

Gln Phe Val Thr Asn Asp Gly Thr Thr Thr Gly Thr Leu Thr Glu Ile
        275                 280                 285

Arg Arg Gln Tyr Ile Gln Asn Gly Lys Val Ile Ala Asn Ala Val Ser
    290                 295                 300

Ser Ser Gly Val Ser Ser Ile Thr Glu Asp Trp Cys Thr Ser Val Asp
305                 310                 315                 320

Gly Ser Ala Ala Thr Phe Gly Gly Leu Thr Arg Met Gly Gln Ala Leu
                325                 330                 335

Gly Arg Gly Met Val Leu Ala Phe Ser Ile Trp Asn Asp Ala Ser Gly
            340                 345                 350

Tyr Met Asn Trp Leu Asp Ser Gly Asn Ser Gly Pro Cys Ser Ser Thr
        355                 360                 365

Glu Gly Asn Pro Asp Leu Ile Lys Ala Gln Asn Pro Thr Thr His Val
    370                 375                 380

Val Phe Ser Asn Ile Arg Trp Gly Asp Ile Gly Ser Thr Phe Lys Gly
385                 390                 395                 400

Ser Gly Gly Ser Ser Thr Thr Thr Thr Thr Thr Thr Ser Thr Lys Thr
                405                 410                 415

Thr Thr Ser Thr Ala Pro Gly Pro Thr Gln Thr His Tyr Gly Gln Cys
            420                 425                 430

Gly Gly Gln Gly Trp Thr Gly Pro Lys Val Cys Ala Ser Pro Tyr Thr
        435                 440                 445

Cys Lys Phe Gln Asn Gln Trp Tyr Ser Gln Cys Leu
    450                 455                 460

<210>  46
<211>  460
<212>  PRT

<213>  Neosartorya fumigata

<400>  46

Met Asp Ser Lys Arg Gly Val Val Ala Ala Val Leu Ala Leu Leu Pro
1               5                   10                  15

Leu Val Ser Ala Gln Gln Pro Ala Ala Ser Ser Ala Gly Asn Pro Lys
            20                  25                  30

Leu Thr Thr Tyr Lys Cys Thr Thr Ala Gly Gly Cys Val Ala Gln Asp
        35                  40                  45

Thr Ser Val Val Leu Asp Trp Gly Tyr His Trp Ile His Thr Val Asp
    50                  55                  60

Gly Tyr Thr Ser Cys Thr Thr Ser Ser Gly Val Asp Ser Thr Leu Cys
65                  70                  75                  80

Pro Asp Ala Ala Thr Cys Ala Lys Asn Cys Val Ile Glu Pro Ala Asn
                85                  90                  95

Tyr Thr Ser Ala Gly Val Thr Thr Ser Gly Asp Ser Leu Thr Met Tyr
            100                 105                 110

Gln Tyr Val Gln Ser Asn Gly Val Tyr Thr Asn Ala Ser Pro Arg Leu
        115                 120                 125

Tyr Leu Leu Gly Pro Asp Lys Asn Tyr Val Met Leu Lys Leu Leu Gly
    130                 135                 140

Gln Glu Leu Thr Phe Asp Val Asp Leu Ser Thr Leu Pro Cys Gly Glu
145                 150                 155                 160

Asn Gly Ala Leu Tyr Leu Ser Glu Met Ser Ala Thr Gly Gly Arg Asn
                165                 170                 175

Glu Tyr Asn Thr Gly Gly Ala Glu Tyr Gly Ser Gly Tyr Cys Asp Ala
            180                 185                 190

Gln Cys Pro Val Ile Ala Trp Lys Asn Gly Thr Leu Asn Thr Ser Gly
        195                 200                 205

Ala Ser Tyr Cys Cys Asn Glu Met Asp Ile Leu Glu Ala Asn Ser Arg
    210                 215                 220

Ala Asn Ser Tyr Thr Pro His Pro Cys Ser Ala Thr Asp Cys Asp Lys
225                 230                 235                 240

Gly Gly Cys Gly Phe Asn Pro Tyr Ala Leu Gly Gln Lys Ser Tyr Trp
                245                 250                 255

Gly Pro Gly Gly Thr Val Asp Thr Ser Lys Pro Phe Thr Ile Thr Thr
            260                 265                 270

Gln Phe Ile Thr Asp Asp Gly Thr Thr Thr Gly Thr Leu Ser Glu Ile
        275                 280                 285

Arg Arg Gln Tyr Met Gln Asn Gly Lys Val Ile Ala Asn Ala Val Ser
    290                 295                 300

Ser Thr Gly Val Asn Ser Ile Thr Glu Asp Trp Cys Thr Ser Val Asp
305                 310                 315                 320

Gly Ser Ala Ala Thr Phe Gly Gly Leu Thr Thr Met Gly Lys Ala Leu
                325                 330                 335

Gly Arg Gly Met Val Leu Ile Phe Ser Ile Trp Asn Asp Ala Ser Gly
            340                 345                 350

Phe Met Asn Trp Leu Asp Ser Gly Asn Ala Gly Pro Cys Ser Ser Thr
        355                 360                 365

Glu Gly Asn Pro Asp Leu Ile Lys Ala Gln Asn Pro Thr Thr His Val
    370                 375                 380

Val Phe Ser Asn Ile Arg Trp Gly Asp Ile Gly Ser Thr Phe Lys Gly
385                 390                 395                 400

Ser Asp Gly Ser Val Thr Thr Thr Thr Ser Thr Thr Ser Thr Lys Thr
                405                 410                 415

Thr Thr Ser Thr Ala Pro Gly Pro Thr Gln Thr His Tyr Gly Gln Cys
            420                 425                 430

Gly Gly Gln Gly Trp Thr Gly Pro Thr Ala Cys Ala Ser Pro Tyr Thr
        435                 440                 445

Cys Gln Val Leu Asn Pro Trp Tyr Ser Gln Cys Leu
    450                 455                 460

<210>  47
<211>  469
<212>  PRT
<213>  Neosartorya fischeri

<400>  47

Met Asp Ser Lys Arg Gly Ile Val Ala Ala Val Leu Ala Leu Leu Ser
1               5                   10                  15

Val Val Ser Ala Gln Gln Pro Ala Thr Ser Ser Ala Gly Asn Pro Lys
            20                  25                  30

Leu Thr Thr Tyr Lys Cys Thr Thr Ala Gly Gly Cys Val Ala Gln Asp
        35                  40                  45

Thr Ser Val Val Leu Asp Trp Gly Ser His Trp Ile His Thr Val Asn
    50                  55                  60

Gly Tyr Thr Ser Cys Thr Thr Ser Ser Gly Val Asp Ser Thr Leu Cys
65                  70                  75                  80

Pro Asp Glu Ala Thr Cys Ala Lys Asn Cys Val Ile Glu Pro Ala Asn
                85                  90                  95

Tyr Thr Ser Ala Gly Val Ala Thr Ser Gly Asp Ser Leu Thr Met Tyr
            100                 105                 110

Gln Tyr Val Gln Asn Asn Gly Val Tyr Thr Asn Ala Ser Pro Arg Leu
        115                 120                 125

Tyr Leu Leu Gly Pro Asp Lys Asp Tyr Val Met Leu Lys Leu Leu Gly
    130                 135                 140

Gln Glu Leu Ser Phe Asp Val Asp Leu Ser Thr Leu Pro Cys Gly Glu
145                 150                 155                 160

Asn Gly Ala Leu Tyr Leu Ser Glu Met Ser Ala Ser Gly Gly Arg Asn
                165                 170                 175

Glu Tyr Asn Thr Gly Gly Ala Glu Tyr Gly Ser Gly Tyr Cys Asp Ala
            180                 185                 190

Gln Cys Pro Val Ile Ala Trp Lys Asn Gly Thr Leu Asn Thr Ser Gly
        195                 200                 205

Ala Ser Tyr Cys Cys Asn Glu Met Asp Ile Leu Glu Ala Asn Ser Arg
    210                 215                 220

Ala Asn Ser Tyr Thr Pro His Pro Cys Ser Ala Thr Asp Cys Asp Lys
225                 230                 235                 240

Gly Gly Cys Gly Phe Asn Pro Tyr Ala Leu Gly Gln Lys Ser Tyr Trp
                245                 250                 255

Gly Pro Gly Gly Thr Val Asp Thr Ser Lys Pro Phe Thr Ile Thr Thr
            260                 265                 270

Gln Phe Ile Thr Asn Asp Gly Thr Thr Thr Gly Thr Leu Ser Glu Ile
        275                 280                 285

Arg Arg Gln Tyr Ile Gln Asn Gly Lys Val Ile Ala Asn Ala Val Ser
    290                 295                 300

Ser Ala Gly Val Ser Ser Ile Thr Glu Asp Trp Cys Thr Ser Val Asp
305                 310                 315                 320

Ser Ser Ala Ala Thr Phe Gly Gly Leu Thr Thr Met Gly Lys Ala Leu
                325                 330                 335

Gly Arg Gly Met Val Leu Ile Phe Ser Ile Trp Asn Asp Ala Ser Gly
            340                 345                 350

Phe Met Asn Trp Leu Asp Ser Gly Asn Ser Gly Pro Cys Ser Ser Thr
        355                 360                 365

Glu Gly Asn Pro Asp Leu Ile Lys Ala Gln Asn Pro Thr Thr His Val
    370                 375                 380

Val Phe Ser Asn Ile Arg Trp Gly Asp Ile Gly Ser Thr Phe Lys Gly
385                 390                 395                 400

Ser Asp Asp Ser Gly Thr Thr Thr Thr Lys Thr Thr Thr Thr Lys Thr
                405                 410                 415

Ser Thr Ser Thr Thr Ser Thr Lys Thr Thr Thr Ser Thr Ala Pro Gly
            420                 425                 430

Ala Thr Gln Thr His Tyr Gly Gln Cys Gly Gly Gln Gly Trp Thr Gly
        435                 440                 445

Pro Thr Ala Cys Ala Ser Pro Tyr Thr Cys Lys Ala Gln Asn Gln Trp
    450                 455                 460

Tyr Ser Gln Cys Leu
465

<210>  48
<211>  469
<212>  PRT
<213>  Aspergillus fischeri

<400> 48

Met Asp Ser Lys Arg Gly Val Val Ala Ala Val Leu Ala Leu Leu Pro
1               5                   10                  15

Leu Val Ser Ala Gln Gln Pro Ala Thr Ser Ser Ala Gly Asn Pro Lys
            20                  25                  30

Leu Thr Thr Tyr Lys Cys Thr Thr Ala Gly Gly Cys Val Ala Gln Asp
        35                  40                  45

Thr Ser Val Val Leu Asp Trp Gly Ser His Trp Ile His Thr Val Asn
    50                  55                  60

Gly Tyr Thr Ser Cys Thr Thr Ser Ser Gly Val Asp Ser Thr Leu Cys
65                  70                  75                  80

Pro Asp Glu Ala Thr Cys Ala Lys Asn Cys Val Ile Glu Pro Ala Asn
                85                  90                  95

Tyr Thr Ser Ala Gly Val Ala Thr Ser Gly Asp Ser Leu Thr Met Tyr
            100                 105                 110

Gln Tyr Val Gln Asn Asn Gly Val Tyr Thr Asn Ala Ser Pro Arg Leu
        115                 120                 125

Tyr Leu Leu Gly Pro Asp Lys Asp Tyr Val Met Leu Lys Leu Leu Gly
    130                 135                 140

Gln Glu Leu Ser Phe Asp Val Asp Leu Ser Thr Leu Pro Cys Gly Glu
145                 150                 155                 160

Asn Gly Ala Leu Tyr Leu Ser Glu Met Ser Ala Ser Gly Gly Arg Asn
                165                 170                 175

Glu Tyr Asn Thr Gly Gly Ala Glu Tyr Gly Ser Gly Tyr Cys Asp Ala
            180                 185                 190

Gln Cys Pro Val Ile Ala Trp Lys Asn Gly Thr Leu Asn Thr Ser Gly
        195                 200                 205

Ala Ser Tyr Cys Cys Asn Glu Met Asp Ile Leu Glu Ala Asn Ser Arg
    210                 215                 220

Ala Asn Ser Tyr Thr Pro His Pro Cys Ser Ala Thr Asp Cys Asp Lys
225                 230                 235                 240

Gly Gly Cys Gly Phe Asn Pro Tyr Ala Leu Gly Gln Lys Ser Tyr Trp

245                 250                 255

Gly Pro Gly Gly Thr Val Asp Thr Ser Lys Pro Phe Thr Ile Thr Thr
            260                 265                 270

Gln Phe Ile Thr Asn Asp Gly Thr Thr Thr Gly Thr Leu Ser Glu Ile
        275                 280                 285

Arg Arg Gln Tyr Ile Gln Asn Gly Lys Val Ile Ala Asn Ala Val Ser
    290                 295                 300

Ser Ala Gly Val Ser Ser Ile Thr Glu Asp Trp Cys Thr Ser Val Asp
305                 310                 315                 320

Ser Ser Ala Ala Ile Phe Gly Gly Leu Thr Thr Met Gly Lys Ala Leu
                325                 330                 335

Gly Arg Gly Met Val Leu Ile Phe Ser Ile Trp Asn Asp Ala Ser Gly
            340                 345                 350

Phe Met Asn Trp Leu Asp Ser Gly Asn Ser Gly Pro Cys Ser Ser Thr
        355                 360                 365

Glu Gly Asn Pro Asp Leu Ile Lys Ala Gln Asn Pro Thr Thr His Val
    370                 375                 380

Val Phe Ser Asn Ile Arg Trp Gly Asp Ile Gly Ser Thr Phe Lys Gly
385                 390                 395                 400

Ser Asp Asp Ser Gly Thr Thr Thr Thr Lys Thr Thr Thr Thr Lys Thr
                405                 410                 415

Ser Thr Ser Thr Thr Ser Thr Lys Thr Thr Thr Ser Thr Ala Pro Gly
            420                 425                 430

Ala Thr Gln Thr His Tyr Gly Gln Cys Gly Gly Gln Gly Trp Thr Gly
        435                 440                 445

Pro Thr Ala Cys Ala Ser Pro Tyr Thr Cys Lys Ala Gln Asn Gln Trp
    450                 455                 460

Tyr Ser Gln Cys Leu
465

<210>  49
<211>  482
<212>  PRT
<213>  Penicillium brasilianum

<400>  49

Met Pro Ala Thr Arg Val Ser Ile Val Ala Ala Ile Leu Ala Leu Leu
1               5                   10                  15

Pro Leu Asn Gln Ala Gln Gln Pro Ala Thr Thr Ala Pro Gly Asn Pro
            20                  25                  30

Ala Leu Thr Thr Trp Lys Cys Ser Ser Thr Gly Gly Cys Val Ile Gln
        35                  40                  45

Asp Thr Ser Val Val Leu Asp Trp Gly Tyr His Trp Ile His Ala Ala
    50                  55                  60

Gly Gly Tyr Glu Ser Cys Thr Thr Ser Ser Gly Val Asn Lys Thr Leu
65                  70                  75                  80

Cys Pro Asp Glu Ala Thr Cys Ala Lys Asn Cys Val Ile Glu Ala Ala
                85                  90                  95

Asn Tyr Thr Ser Ala Gly Val Ser Val Ser Gly Asp Ser Leu Thr Met
            100                 105                 110

His Gln Tyr Val Glu Ser Asn Gly Val Tyr Ser Asn Ala Ser Pro Arg
        115                 120                 125

Leu Tyr Leu Leu Gly Glu Asp Gly Asn Tyr Val Met Leu Gln Leu Leu
    130                 135                 140

Gly Gly Glu Leu Ser Phe Asp Val Asp Met Ser Ser Leu Pro Cys Gly
145                 150                 155                 160

Glu Asn Gly Ala Leu Tyr Leu Ser Glu Met Ser Ala Thr Gly Gly Arg
                165                 170                 175

Asn Glu Tyr Asn Thr Gly Gly Ala Glu Tyr Gly Ser Gly Tyr Cys Asp
            180                 185                 190

Ala Gln Cys Pro Val Gln Thr Trp Lys Asn Gly Thr Leu Asn Ser Gly
        195                 200                 205

Gly Gln Gly Tyr Cys Cys Asn Glu Met Asp Ile Leu Glu Ala Asn Ser
    210                 215                 220

Met Ala Asn Ala Tyr Thr Pro His Pro Cys Ser Ser Thr Asp Cys Asp
225                 230                 235                 240

Lys Gly Gly Cys Gly Phe Asn Pro Tyr Ala Gln Gly Ile Thr Asn Tyr
                245                 250                 255

Trp Gly Pro Gly Asp Thr Val Asp Thr Ser Lys Pro Phe Thr Ile Thr
            260                 265                 270

Thr Gln Phe Leu Thr Asp Asp Gly Thr Lys Thr Gly Thr Leu Thr Glu
        275                 280                 285

Ile Arg Arg Gln Tyr Ile Gln Asn Gly Lys Ile Ile Ala Asn Ala Lys
    290                 295                 300

Ser Ser Ala Gly Leu Asp Ser Ile Thr Gln Ser Trp Cys Glu Ser Ser
305                 310                 315                 320

Asp Gly Ser Ala Ala Thr Phe Gly Gly Leu Thr Thr Met Gly Gln Ala
                325                 330                 335

Leu Gly Arg Gly Met Val Leu Ile Phe Ser Ile Trp Asn Asp Gly Ser
            340                 345                 350

Gln Tyr Met Asn Trp Leu Asp Ser Gly Ser Asn Gly Pro Cys Ser Ser
        355                 360                 365

Ala Ala Gly Asn Pro Ala Thr Ile Leu Ala Gln Asn Ala Thr Thr His
    370                 375                 380

Val Val Phe Ser Asn Ile Arg Tyr Gly Asp Ile Asp Ser Thr Tyr Thr
385                 390                 395                 400

Asn Pro Glu Gly Ser Gly Ser Gly Thr Thr Thr Ser Ser Ser Thr Ser
                405                 410                 415

Leu Thr Thr Ser Thr Ala Thr Lys Thr Thr Thr Thr Lys Ala Thr Thr
            420                 425                 430

Lys Ala Thr Thr Thr Thr Thr Thr Thr Thr Ala Ile Gly Ala Ile Gln
        435                 440                 445

Thr His Trp Gly Gln Cys Gly Gly Gln Gly Trp Thr Gly Pro Thr Ala
    450                 455                 460

Cys Val Ser Gly Thr Thr Cys Lys Val Gln Asn Pro Tyr Tyr Ser Gln
465                 470                 475                 480

Cys Leu

<210>  50
<211>  460
<212>  PRT
<213>  Aspergillus fumigatus

<400>  50

Met Asp Ser Lys Arg Gly Val Val Ala Ala Val Leu Ala Leu Leu Pro
1               5                   10                  15

Leu Val Ser Ala Gln Gln Pro Ala Ala Ser Ser Ala Gly Asn Pro Lys
            20                  25                  30

Leu Thr Thr Tyr Lys Cys Thr Thr Ala Gly Gly Cys Val Ala Gln Asp
        35                  40                  45

Thr Ser Val Val Leu Asp Trp Gly Tyr His Trp Ile His Thr Val Asp
    50                  55                  60

Gly Tyr Thr Ser Cys Thr Thr Ser Ser Gly Val Asp Ser Thr Leu Cys
65                  70                  75                  80

Pro Asp Ala Ala Thr Cys Ala Lys Asn Cys Val Ile Glu Pro Ala Asn
                85                  90                  95

Tyr Thr Ser Ala Gly Val Thr Thr Ser Gly Asp Ser Leu Thr Met Tyr
            100                 105                 110

Gln Tyr Val Gln Ser Asn Gly Val Tyr Thr Asn Ala Ser Pro Arg Leu
        115                 120                 125

Tyr Leu Leu Gly Pro Asp Lys Asn Tyr Val Met Leu Lys Leu Leu Gly
    130                 135                 140

Gln Glu Leu Thr Phe Asp Val Asp Leu Ser Thr Leu Pro Cys Gly Glu
145                 150                 155                 160

Asn Gly Ala Leu Tyr Leu Ser Glu Met Ser Ala Thr Gly Gly Arg Asn
                165                 170                 175

Glu Tyr Asn Thr Gly Gly Ala Glu Tyr Gly Ser Gly Tyr Cys Asp Ala
            180                 185                 190

Gln Cys Pro Val Ile Ala Trp Lys Asn Gly Thr Leu Asn Thr Ser Gly
        195                 200                 205

Ala Ser Tyr Cys Cys Asn Glu Met Asp Ile Leu Glu Ala Asn Ser Arg
    210                 215                 220

Ala Asn Ser Tyr Thr Pro His Pro Cys Ser Ala Thr Asp Cys Asp Lys
225                 230                 235                 240

Gly Gly Cys Gly Phe Asn Pro Tyr Ala Leu Gly Gln Lys Ser Tyr Trp
                245                 250                 255

Gly Pro Gly Gly Thr Val Asp Thr Ser Lys Pro Phe Thr Ile Thr Thr
            260                 265                 270

Gln Phe Ile Thr Asn Asp Gly Thr Thr Thr Gly Thr Leu Ser Glu Ile
        275                 280                 285

Arg Arg Gln Tyr Ile Gln Asn Gly Lys Val Ile Ala Asn Ala Val Ser
    290                 295                 300

Ser Thr Gly Val Asn Ser Ile Thr Glu Asp Trp Cys Thr Ser Val Asp
305                 310                 315                 320

Gly Ser Ala Ala Thr Phe Gly Gly Leu Thr Thr Met Gly Lys Ala Leu
                325                 330                 335

Gly Arg Gly Met Val Leu Ile Phe Ser Ile Trp Asn Asp Ala Ser Gly
            340                 345                 350

Phe Met Asn Trp Leu Asp Ser Gly Asn Ala Gly Pro Cys Ser Ser Thr
        355                 360                 365

Glu Gly Asn Pro Asp Leu Ile Lys Ala Gln Asn Pro Thr Thr His Val
    370                 375                 380

Val Phe Ser Asn Ile Arg Trp Gly Asp Ile Gly Ser Thr Phe Lys Gly
385                 390                 395                 400

Ser Asp Gly Ser Val Thr Thr Thr Thr Ser Thr Thr Ser Thr Lys Thr
                405                 410                 415

Thr Thr Ser Thr Ala Pro Gly Pro Thr Gln Thr His Tyr Gly Gln Cys
            420                 425                 430

Gly Gly Gln Gly Trp Thr Gly Pro Thr Ala Cys Ala Ser Pro Tyr Thr
        435                 440                 445

Cys Gln Val Leu Asn Pro Trp Tyr Ser Gln Cys Leu
    450                 455                 460

<210>  51
<211>  471

<212>  PRT
<213>  Aspergillus lentulus

<400>  51

Met Asp Ser Lys Arg Ser Ile Val Ala Ala Val Leu Ala Leu Leu Pro
1               5                   10                  15

Leu Ile Ser Ala Gln Gln Pro Ala Thr Ser Ser Ala Gly Asn Pro Lys
            20                  25                  30

Leu Thr Thr Tyr Lys Cys Thr Thr Ala Gly Gly Cys Val Ala Gln Asp
        35                  40                  45

Thr Ser Val Val Leu Asp Trp Gly Tyr His Trp Ile His Thr Val Asn
    50                  55                  60

Gly Tyr Thr Ser Cys Thr Thr Ser Ser Gly Val Asn Ser Thr Leu Cys
65                  70                  75                  80

Pro Asp Glu Ala Thr Cys Ala Lys Asn Cys Val Ile Glu Pro Ala Asn
                85                  90                  95

Tyr Thr Ser Ala Gly Val Ile Thr Ser Gly Asp Ser Leu Thr Met Tyr
            100                 105                 110

Gln Tyr Val Gln Ser Asn Gly Val Tyr Thr Asn Ala Ser Pro Arg Leu
        115                 120                 125

Tyr Leu Leu Gly Pro Asp Lys Asn Tyr Val Met Leu Lys Leu Leu Gly
    130                 135                 140

Gln Glu Leu Ser Phe Asp Val Asp Leu Ser Thr Leu Pro Cys Gly Glu
145                 150                 155                 160

Asn Gly Ala Leu Tyr Leu Ser Glu Met Ser Ala Thr Gly Gly Arg Asn
                165                 170                 175

Glu Tyr Asn Thr Gly Gly Ala Glu Tyr Gly Ser Gly Tyr Cys Asp Ala
            180                 185                 190

Gln Cys Pro Val Ile Thr Trp Lys Asn Gly Thr Leu Asn Thr Ser Gly
        195                 200                 205

Ala Ser Tyr Cys Cys Asn Glu Met Asp Ile Leu Glu Ala Asn Ser Arg
    210                 215                 220

Ala Asn Ser Tyr Thr Pro His Pro Cys Ser Ala Thr Asp Cys Asp Lys
225                 230                 235                 240

Gly Gly Cys Gly Phe Asn Pro Tyr Ala Leu Gly Gln Gln Ser Tyr Trp
                245                 250                 255

Ser Pro Gly Gly Thr Val Asp Thr Ser Lys Pro Phe Thr Ile Thr Thr
            260                 265                 270

Gln Phe Val Thr Asn Asp Gly Thr Thr Thr Gly Ile Leu Ser Glu Ile
        275                 280                 285

Arg Arg Gln Tyr Ile Gln Asn Gly Lys Val Ile Ala Asn Ala Val Ser
    290                 295                 300

Ser Ala Gly Val Ser Ser Ile Thr Glu Asp Trp Cys Thr Ser Val Asp
305                 310                 315                 320

Gly Ser Ala Ala Thr Phe Gly Gly Leu Thr Thr Met Gly Lys Ala Leu
                325                 330                 335

Gly Arg Gly Met Val Leu Ile Phe Ser Ile Trp Asn Asp Ala Gly Gly
            340                 345                 350

Phe Met Asn Trp Leu Asp Ser Gly Ser Ser Gly Pro Cys Ser Ser Thr
        355                 360                 365

Glu Gly Asn Pro Asp Leu Ile Lys Ala Gln Asn Pro Thr Thr His Val
    370                 375                 380

Val Phe Ser Asn Ile Arg Trp Gly Asp Ile Gly Ser Thr Phe Lys Gly
385                 390                 395                 400

Ser Gly Gly Ser Gly Thr Thr Thr Thr Lys Thr Thr Thr Thr Thr Thr
                405                 410                 415

Thr Ser Lys Thr Ser Thr Thr Ser Thr Lys Thr Thr Thr Ser Thr Ala
            420                 425                 430

Pro Gly Ala Thr Gln Thr His Tyr Gly Gln Cys Gly Gly Gln Gly Trp
        435                 440                 445

Thr Gly Pro Thr Ala Cys Ala Ser Pro Tyr Thr Cys Lys Ala Gln Asn
    450                 455                 460

Gln Trp Tyr Ser Gln Cys Leu
465                 470

<210> 52

<211>  483
<212>  PRT
<213>  Rosellinia necatrix

<400>  52

Met Ala Pro Ser Phe Arg Ser Val Leu Ala Ala Thr Ala Leu Leu Ala
1               5                   10                  15

Ser Cys Gln Phe Val Asn Ala Gln Asn Pro Asp Glu Gln Pro Asp Arg
            20                  25                  30

Lys Ser Asn Pro His Ser Gly Ser Lys Gly Cys Gly His Ala Leu Lys
        35                  40                  45

Thr Cys Ile Phe Tyr Gln Cys Thr Lys Ala Gly Gly Cys Ala Ala Lys
    50                  55                  60

Glu Arg Ser Val Val Ile Asp Trp Asn Tyr His Trp Phe His Thr Ala
65                  70                  75                  80

Asp Trp Leu Ser Cys Thr Thr Ser Ser Gly Val Asn Ala Thr Leu Cys
                85                  90                  95

Pro Asn Gln Ala Thr Cys Ala Gln Asn Cys Phe Val Gln Gly Ala Ser
            100                 105                 110

Asn Tyr Thr Asn Asn Gly Val Ser Thr Ser Gly Asp Thr Leu Thr Met
        115                 120                 125

Tyr Gln Tyr Thr Ile Asn Glu Asp Gly Glu Ala Gln Asn Ala Ser Pro
    130                 135                 140

Arg Leu Tyr Leu Leu Gly Asp Asp Gly Asn Tyr Glu Met Leu Lys Leu
145                 150                 155                 160

Leu Gly Gln Glu Leu Thr Phe Thr Val Asp Leu Ser Thr Leu Pro Cys
                165                 170                 175

Gly Glu Asn Gly Ala Leu Tyr Leu Gly Glu Met Asp Lys Thr Gly Gly
            180                 185                 190

Arg Asn Gln Tyr Asn Thr Gly Gly Ala Asn Tyr Gly Ser Gly Tyr Cys
        195                 200                 205

Asp Ala Gln Cys Pro Val Gln Thr Trp Lys Asn Gly Thr Leu Asn Thr
    210                 215                 220

Asp Gly Ser Gly Tyr Cys Cys Asn Glu Met Asp Ile Leu Glu Ala Asn

225                 230                 235                 240

Ser Arg Ala His Ser Phe Thr Pro His Pro Cys Ser Asp Thr Asp Cys
                245                 250                 255

Asp Arg Gly Gly Cys Gly Leu Asn Pro Tyr Ala Lys Gly Tyr Thr Asn
            260                 265                 270

Tyr Tyr Gly Pro Gly Gly Thr Val Asp Thr Ser Lys Pro Phe Thr Val
        275                 280                 285

Ile Thr Gln Phe Asn Thr Asn Asp Gly Thr Thr Thr Gly Thr Leu Thr
    290                 295                 300

Ser Ile Thr Arg Arg Tyr Met Gln Asn Gly Lys Leu Ile Pro Thr Ala
305                 310                 315                 320

Asp Pro Lys Gly Asp Leu Ile Thr Thr Glu Ser Cys Asn Ala Ala Asp
                325                 330                 335

Gly Ser Ala Ala Gln Phe Gly Ser Leu Thr Thr Met Gly Lys Ala Leu
            340                 345                 350

Gly Arg Gly Met Val Leu Thr Phe Ala Ile Trp Asn Asp Ala Gly Gln
        355                 360                 365

Phe Met Asn Trp Leu Asp Ala Gly Ala Asn Gly Pro Cys Ser Glu Thr
    370                 375                 380

Glu Gly Asn Pro Ala Leu Ile Ala Gln Asn Asn Pro Lys Thr His Val
385                 390                 395                 400

Ile Phe Ser Asn Ile Arg Trp Gly Asp Ile Gly Ser Thr Phe Glu Asp
                405                 410                 415

Ser Gly Ser Gly Thr Thr Thr Thr Thr Ala Ser Pro Thr Lys Thr Thr
            420                 425                 430

Thr Thr Lys Thr Ser Ala Thr Thr Thr Thr Thr Ala Ser Gly Pro Ala
        435                 440                 445

Gln Thr His Trp Gly Gln Cys Gly Gly Ile Gly Trp Thr Gly Pro Lys
    450                 455                 460

Thr Cys Ala Ser Pro Tyr Gln Cys Thr Lys Ala Asn Asp Tyr Tyr Tyr
465                 470                 475                 480

Gln Cys Leu

<210> 53
<211> 9
<212> PRT
<213> Artificial Sequence

<220>
<223> EG2 First consensus domain sequence

<220>
<221> VARIANT
<222> (3)..(3)
<223> Replace = Val

<220>
<221> VARIANT
<222> (4)..(4)
<223> Replace = Val

<400> 53

Phe Gly Ile Met Asn Glu Pro His Asp
1               5

<210> 54
<211> 10
<212> PRT
<213> Artificial Sequence

<220>
<223> EG2 Second consensus domain sequence

<220>
<221> VARIANT
<222> (7)..(7)
<223> Thr

<220>
<221> VARIANT
<222> (8)..(8)
<223> Ala

<400> 54

Glu Thr Gly Gly Gly Asn Val Gln Ser Cys
1               5                   10

<210> 55
<211> 418
<212> PRT
<213> Hypocrea rufa

<400> 55

Met Asn Lys Ser Val Ala Pro Leu Leu Leu Ala Ala Ser Ile Leu Tyr
1               5                   10                  15

Gly Gly Ala Ala Ala Gln Gln Thr Val Trp Gly Gln Cys Gly Gly Ile
        20                  25                  30

Gly Trp Ser Gly Pro Thr Asn Cys Ala Pro Gly Ser Ala Cys Ser Thr
    35                  40                  45

Leu Asn Pro Tyr Tyr Ala Gln Cys Ile Pro Gly Ala Thr Thr Ile Thr
50                  55                  60

Thr Ser Thr Arg Pro Pro Ser Gly Pro Thr Thr Thr Thr Arg Ala Thr
65                  70                  75                  80

Ser Thr Ser Ser Ser Thr Pro Pro Thr Ser Ser Gly Val Arg Phe Ala
                85                  90                  95

Gly Val Asn Ile Ala Gly Phe Asp Phe Gly Cys Thr Thr Asp Gly Thr
            100                 105                 110

Cys Val Thr Ser Lys Val Tyr Pro Pro Leu Lys Asn Phe Thr Gly Ser
        115                 120                 125

Asn Asn Tyr Pro Asp Gly Ile Gly Gln Met Gln His Phe Val Asn Asp
    130                 135                 140

Asp Gly Met Thr Ile Phe Arg Leu Pro Val Gly Trp Gln Tyr Leu Val
145                 150                 155                 160

Asn Asn Asn Leu Gly Gly Asn Leu Asp Ser Thr Ser Ile Ser Lys Tyr
                165                 170                 175

Asp Gln Leu Val Gln Gly Cys Leu Ser Leu Gly Ala Tyr Cys Ile Val
            180                 185                 190

Asp Ile His Asn Tyr Ala Arg Trp Asn Gly Gly Ile Ile Gly Gln Gly
        195                 200                 205

Gly Pro Thr Asn Ala Gln Phe Thr Ser Leu Trp Ser Gln Leu Ala Ser
    210                 215                 220

Lys Tyr Ala Ser Gln Ser Arg Val Trp Phe Gly Ile Met Asn Glu Pro
225                 230                 235                 240

His Asp Val Asn Ile Asn Thr Trp Ala Ala Thr Val Gln Glu Val Val
                245                 250                 255

Thr Ala Ile Arg Asn Ala Gly Ala Thr Ser Gln Phe Ile Ser Leu Pro

260                 265                 270

Gly Asn Asp Trp Gln Ser Ala Gly Ala Phe Ile Ser Asp Gly Ser Ala
        275                 280                 285

Ala Ala Leu Ser Gln Val Thr Asn Pro Asp Gly Ser Thr Thr Asn Leu
    290                 295                 300

Ile Phe Asp Val His Lys Tyr Leu Asp Ser Asp Asn Ser Gly Thr His
305                 310                 315                 320

Ala Glu Cys Thr Thr Asn Asn Ile Asp Gly Ala Phe Ser Pro Leu Ala
                325                 330                 335

Thr Trp Leu Arg Gln Asn Asn Arg Gln Ala Ile Leu Thr Glu Thr Gly
            340                 345                 350

Gly Gly Asn Val Gln Ser Cys Ile Gln Asp Met Cys Gln Gln Ile Gln
        355                 360                 365

Tyr Leu Asn Gln Asn Ser Asp Val Tyr Leu Gly Tyr Val Gly Trp Gly
    370                 375                 380

Ala Gly Ser Phe Asp Ser Thr Tyr Val Leu Thr Glu Thr Pro Thr Gly
385                 390                 395                 400

Ser Gly Asn Ser Trp Thr Asp Thr Ser Leu Val Ser Ser Cys Leu Ala
                405                 410                 415

Arg Lys

<210>  56
<211>  418
<212>  PRT
<213>  Hypocrea atroviridis

<400>  56

Met Asn Lys Ser Val Ala Pro Leu Leu Leu Ala Ala Ser Thr Leu Val
1               5                   10                  15

Gly Gly Val Ala Ala Gln Gln Thr Val Trp Gly Gln Cys Gly Gly Ile
            20                  25                  30

Gly Trp Ser Gly Pro Thr Asn Cys Ala Pro Gly Ser Ala Cys Ser Thr
        35                  40                  45

Leu Asn Ser Tyr Tyr Ala Gln Cys Ile Pro Gly Ala Thr Ser Met Thr

50                  55                  60

Thr Ser Thr Arg Pro Pro Ser Gly Pro Thr Thr Thr Thr Arg Ala Thr
65                  70                  75                  80

Ser Thr Thr Ser Ser Thr Pro Pro Thr Ser Ser Gly Asp Arg Phe Val
                85                  90                  95

Gly Val Asn Val Ala Gly Phe Asp Phe Gly Cys Ser Thr Asp Gly Thr
            100                 105                 110

Cys Val Thr Ser Lys Val Tyr Pro Pro Leu Lys Asn Phe Thr Gly Ser
        115                 120                 125

Asn Asn Tyr Pro Asp Gly Ile Gly Gln Met Gln His Phe Val Asn Asp
    130                 135                 140

Asp Gly Met Thr Ile Phe Arg Leu Pro Val Gly Trp Gln Tyr Leu Val
145                 150                 155                 160

Asn Asn Asn Leu Gly Gly Pro Leu Asp Pro Thr Ser Ile Ser Lys Tyr
                165                 170                 175

Asp Gln Leu Val Gln Gly Cys Leu Ser Leu Gly Val Tyr Cys Ile Val
            180                 185                 190

Asp Ile His Asn Tyr Ala Arg Trp Asn Gly Gly Ile Ile Gly Gln Gly
        195                 200                 205

Gly Pro Thr Asn Ala Gln Phe Thr Ser Leu Trp Ser Gln Leu Ala Ser
    210                 215                 220

Lys Tyr Ala Ser Gln Ser Arg Val Trp Phe Gly Ile Met Asn Glu Pro
225                 230                 235                 240

His Asp Val Asn Ile Asn Thr Trp Ala Ala Thr Val Gln Glu Val Val
                245                 250                 255

Thr Ala Ile Arg Asn Ala Gly Ala Thr Ser Gln Tyr Ile Ser Leu Pro
            260                 265                 270

Gly Asn Asp Trp Gln Ser Ala Ala Ala Phe Ile Ser Asp Gly Ser Ala
        275                 280                 285

Ala Ala Leu Ser Gln Val Thr Asn Pro Asp Gly Ser Thr Thr Asn Leu
    290                 295                 300

Ile Phe Asp Val His Lys Tyr Leu Asp Ser Asp Asn Ser Gly Thr His
305                 310                 315                 320

Ala Glu Cys Thr Thr Asn Asn Ile Asp Gly Ala Phe Ala Pro Leu Ala
                325                 330                 335

Thr Trp Leu Arg Gln Asn Asn Arg Gln Ala Ile Leu Thr Glu Thr Gly
            340                 345                 350

Gly Gly Asn Val Gln Ser Cys Ile Gln Tyr Leu Cys Gln Gln Val Gln
        355                 360                 365

Tyr Leu Asn Gln Asn Ser Asp Val Tyr Leu Gly Tyr Val Gly Trp Gly
    370                 375                 380

Ala Gly Ser Phe Asp Asn Thr Tyr Val Leu Thr Glu Thr Pro Thr Gly
385                 390                 395                 400

Ser Gly Asn Ser Trp Thr Asp Thr Ser Leu Val Ser Ser Cys Leu Ala
                405                 410                 415

Arg Lys

<210>  57
<211>  418
<212>  PRT
<213>  Trichoderma orientale

<400>  57

Met Asn Lys Ser Val Ala Pro Leu Leu Leu Ala Ala Ser Thr Leu Val
1               5                   10                  15

Gly Gly Val Ala Ala Gln Gln Thr Val Trp Gly Gln Cys Gly Gly Ile
            20                  25                  30

Gly Trp Ser Gly Pro Thr Asn Cys Ala Pro Gly Ser Ala Cys Ser Thr
        35                  40                  45

Leu Asn Ser Tyr Tyr Ala Gln Cys Ile Pro Gly Ala Thr Ser Met Thr
    50                  55                  60

Thr Ser Thr Arg Pro Pro Ser Gly Pro Thr Thr Thr Thr Arg Ala Thr
65                  70                  75                  80

Ser Thr Thr Ser Ser Thr Pro Pro Thr Ser Ser Gly Asp Arg Phe Val
                85                  90                  95

Gly Val Asn Val Ala Gly Phe Asp Phe Gly Cys Ser Thr Asp Gly Thr
        100                 105                 110

Cys Val Thr Ser Lys Val Tyr Pro Pro Leu Lys Asn Phe Thr Gly Ser
    115                 120                 125

Asn Asn Tyr Pro Asp Gly Ile Gly Gln Met Gln His Phe Val Asn Asp
    130                 135                 140

Asp Gly Met Thr Ile Phe Arg Leu Pro Val Gly Trp Gln Tyr Leu Val
145                 150                 155                 160

Asn Asn Asn Leu Gly Gly Pro Leu Asp Pro Thr Ser Ile Ser Lys Tyr
                165                 170                 175

Asp Gln Leu Val Gln Gly Cys Leu Ser Leu Gly Val Tyr Cys Ile Val
        180                 185                 190

Asp Ile His Asn Tyr Ala Arg Trp Asn Gly Gly Ile Ile Gly Gln Gly
    195                 200                 205

Gly Pro Thr Asn Ala Gln Phe Thr Ser Leu Trp Ser Gln Leu Ala Ser
    210                 215                 220

Lys Tyr Ala Ser Gln Ser Arg Val Trp Phe Gly Ile Met Asn Glu Pro
225                 230                 235                 240

His Asp Val Asn Ile Asn Thr Trp Ala Ala Thr Val Gln Glu Val Val
                245                 250                 255

Thr Ala Ile Arg Asn Ala Gly Ala Thr Ser Gln Tyr Ile Ser Leu Pro
        260                 265                 270

Gly Asn Asp Trp Gln Ser Ala Ala Ala Phe Ile Ser Asp Gly Ser Ala
    275                 280                 285

Ala Ala Leu Ser Gln Val Thr Asn Pro Asp Gly Ser Thr Thr Asn Leu
    290                 295                 300

Ile Phe Asp Leu His Lys Tyr Leu Asp Ser Asp Asn Ser Gly Thr His
305                 310                 315                 320

Ala Glu Cys Thr Thr Asn Asn Ile Asp Gly Ala Phe Ala Pro Leu Ala
                325                 330                 335

Thr Trp Leu Arg Gln Asn Asn Arg Gln Ala Ile Leu Thr Glu Thr Gly
        340                 345                 350

Gly Gly Asn Val Gln Ser Cys Ile Gln Tyr Leu Cys Gln Gln Val Gln
        355                 360                 365

Tyr Leu Asn Gln Asn Ser Asp Val Tyr Leu Gly Tyr Val Gly Trp Gly
    370                 375                 380

Ala Gly Ser Phe Asp Asn Thr Tyr Val Leu Thr Glu Thr Pro Thr Gly
385                 390                 395                 400

Ser Gly Asn Ser Trp Thr Asp Thr Ser Leu Val Ser Ser Cys Leu Ala
                405                 410                 415

Arg Lys

<210>  58
<211>  419
<212>  PRT
<213>  Hypocrea virens

<400>  58

Met Asn Lys Pro Ile Ser Ser Leu Leu Leu Ala Ala Thr Leu Leu Ala
1               5                   10                  15

Ser Gly Ser Ile Ala Gln Gln Thr Val Trp Gly Gln Cys Gly Gly Ile
            20                  25                  30

Gly Tyr Ser Gly Pro Thr Asn Cys Ala Ala Gly Ser Ala Cys Ser Thr
        35                  40                  45

Leu Asn Pro Tyr Tyr Ala Gln Cys Ile Pro Gly Ala Thr Thr Met Ser
    50                  55                  60

Thr Ser Thr Lys Pro Thr Ser Val Pro Ala Ser Thr Thr Arg Ala Ser
65                  70                  75                  80

Ser Thr Ser Ser Ala Thr Pro Pro Ala Gly Ser Gly Leu Thr Arg Phe
                85                  90                  95

Ala Gly Val Asn Ile Ala Gly Phe Asp Phe Gly Cys Gly Thr Asp Gly
            100                 105                 110

Thr Cys Val Thr Ser Lys Val Tyr Pro Pro Leu Lys Asn Tyr Ala Gly
        115                 120                 125

Thr Asn Asn Tyr Pro Asp Gly Val Gly Gln Met Gln His Phe Val Asn
    130                 135                 140

Asp Asp Lys Leu Thr Ile Phe Arg Leu Pro Val Gly Trp Gln Tyr Leu
145                 150                 155                 160

Val Asn Asn Asn Leu Gly Gly Thr Leu Asp Ala Asn Asn Leu Ala Lys
                165                 170                 175

Tyr Asp Gln Leu Val Gln Ser Cys Leu Ser Leu Gly Val Tyr Cys Ile
            180                 185                 190

Ile Asp Ile His Asn Tyr Ala Arg Trp Asn Gly Gly Ile Ile Gly Gln
        195                 200                 205

Gly Gly Pro Thr Asn Asp Gln Phe Thr Ser Leu Trp Ser Gln Leu Ala
    210                 215                 220

Thr Lys Tyr Ala Ser Gln Ser Lys Val Trp Phe Gly Ile Met Asn Glu
225                 230                 235                 240

Pro His Asp Val Asn Ile Asn Thr Trp Ala Thr Thr Val Gln Ala Val
                245                 250                 255

Val Thr Ala Ile Arg Lys Ala Gly Ala Thr Ser Gln Phe Ile Ser Leu
            260                 265                 270

Pro Gly Asn Asp Trp Gln Ser Ala Gly Ala Phe Ile Ser Asp Gly Ser
        275                 280                 285

Ala Ala Ala Leu Ser Gln Val Lys Asn Pro Asp Gly Ser Thr Thr Asn
    290                 295                 300

Leu Ile Phe Asp Leu His Lys Tyr Leu Asp Ser Asp Asn Ser Gly Thr
305                 310                 315                 320

His Ala Asp Cys Val Thr Asn Asn Ile Asn Asp Ala Phe Ser Pro Val
                325                 330                 335

Ala Thr Trp Leu Arg Gln Asn Asn Arg Gln Ala Ile Leu Thr Glu Thr
            340                 345                 350

Gly Gly Gly Asn Thr Gln Ser Cys Ile Gln Tyr Leu Cys Gln Gln Phe
        355                 360                 365

Gln Tyr Leu Asn Gln Asn Ser Asp Val Tyr Leu Gly Tyr Val Gly Trp
    370                 375                 380

Gly Ala Gly Ser Phe Asp Ser Thr Tyr Ile Leu Thr Glu Thr Pro Thr
385                 390                 395                 400

Gly Ser Gly Ser Ser Trp Thr Asp Thr Ser Leu Val Ser Ser Cys Ile
                405                 410                 415

Ala Arg Lys

<210>  59
<211>  419
<212>  PRT
<213>  Trichoderma harzianum

<400>  59

Met Asn Lys Pro Met Ser Ser Leu Leu Leu Ala Ala Thr Leu Leu Ala
1               5                   10                  15

Gly Gly Ser Ile Ala Gln Gln Thr Val Trp Gly Gln Cys Gly Gly Gln
            20                  25                  30

Gly Trp Ser Gly Pro Thr Ser Cys Val Ala Gly Ser Ala Cys Ser Thr
        35                  40                  45

Leu Asn Pro Tyr Tyr Ala Gln Cys Ile Pro Gly Ala Thr Thr Met Ser
    50                  55                  60

Thr Thr Thr Lys Pro Thr Ser Val Ser Ala Ser Thr Thr Arg Ala Ser
65                  70                  75                  80

Ala Thr Ser Ser Ser Thr Pro Pro Ala Gly Ser Gly Leu Thr Arg Phe
                85                  90                  95

Ala Gly Ile Asn Ile Ala Gly Phe Asp Phe Gly Cys Gly Thr Asp Gly
            100                 105                 110

Thr Cys Val Thr Ser Lys Val Tyr Pro Pro Leu Lys Asn Tyr Ala Gly
        115                 120                 125

Thr Asn Asn Tyr Pro Asp Gly Val Gly Gln Met Gln His Phe Val Asn
    130                 135                 140

Asp Asp Lys Leu Thr Ile Phe Arg Leu Pro Val Gly Trp Gln Tyr Leu
145                 150                 155                 160

Val Asn Asn Asn Leu Gly Gly Thr Leu Asp Ser Asn Asn Leu Gly Lys
                165                 170                 175

Tyr Asp Gln Leu Val Gln Ala Cys Leu Ser Leu Gly Val Tyr Cys Ile
            180                 185                 190

Val Asp Ile His Asn Tyr Ala Arg Trp Asn Gly Gly Ile Ile Gly Gln
        195                 200                 205

Gly Gly Pro Thr Asn Asp Gln Phe Thr Ser Leu Trp Ser Gln Leu Ala
    210                 215                 220

Lys Lys Tyr Ala Ser Gln Ser Lys Val Trp Phe Gly Ile Met Asn Glu
225                 230                 235                 240

Pro His Asp Val Asn Ile Asn Thr Trp Ala Thr Thr Val Gln Ala Val
                245                 250                 255

Val Thr Ala Ile Arg Asn Ala Gly Ala Thr Ser Gln Phe Ile Ser Leu
            260                 265                 270

Pro Gly Asn Asp Trp Gln Ser Ala Gly Ala Phe Ile Ser Asp Gly Ser
        275                 280                 285

Ala Ala Ala Leu Ser Gln Val Lys Asn Pro Asp Gly Ser Thr Thr Asn
    290                 295                 300

Leu Ile Phe Asp Leu His Lys Tyr Leu Asp Ser Asp Asn Ser Gly Thr
305                 310                 315                 320

His Ala Asp Cys Val Thr Asn Asn Val Asn Asp Ala Phe Ser Pro Val
                325                 330                 335

Ala Thr Trp Leu Arg Gln Asn Asn Arg Gln Ala Ile Leu Thr Glu Thr
            340                 345                 350

Gly Gly Gly Asn Thr Gln Ser Cys Ile Gln Tyr Leu Cys Gln Gln Phe
        355                 360                 365

Gln Tyr Ile Asn Gln Asn Ser Asp Val Tyr Leu Gly Tyr Val Gly Trp
    370                 375                 380

Gly Ala Gly Ser Phe Asp Ser Thr Tyr Ile Leu Thr Glu Thr Pro Thr
385                 390                 395                 400

Gly Ser Gly Ser Ser Trp Thr Asp Thr Ser Leu Val Ser Ser Cys Ile
                405                 410                 415

Ser Arg Lys

<210> 60

<211>  418
<212>  PRT
<213>  Trichoderma asperellum

<400>  60

Met Asn Lys Pro Met Ala Pro Leu Leu Leu Ala Ala Thr Leu Met Ala
1               5                   10                  15

Ser Gly Ala Ile Ala Gln Thr Gln Thr Val Trp Gly Gln Cys Gly Gly
            20                  25                  30

Thr Gly Tyr Ser Gly Pro Thr Asn Cys Ala Ser Gly Ser Ala Cys Ser
        35                  40                  45

Thr Leu Asn Pro Tyr Tyr Ala Gln Cys Ile Pro Gly Ala Thr Ser Phe
    50                  55                  60

Thr Thr Ser Thr Thr Ser Thr Lys Pro Pro Gly Ser Gly Ser Thr Thr
65                  70                  75                  80

Thr Ser Ser Ala Ser Ala Pro Thr Gly Ser Gly Gln Thr Arg Phe Ala
                85                  90                  95

Gly Ile Asn Ile Ala Gly Phe Asp Phe Gly Cys Thr Thr Asp Gly Thr
            100                 105                 110

Cys Val Thr Ser Gln Val Tyr Pro Pro Leu Lys Asn Phe Asp Gly Ala
        115                 120                 125

Asn Asn Tyr Pro Asp Gly Ile Gly Gln Met Gln His Phe Val Asn Asn
    130                 135                 140

Asp Lys Leu Asn Ile Phe Arg Leu Pro Val Gly Trp Gln Tyr Leu Val
145                 150                 155                 160

Asn Asn Asn Leu Gly Gly Thr Leu Asp Ala Thr Asn Leu Gly Tyr Tyr
                165                 170                 175

Asp Gln Leu Val Gln Gly Cys Leu Ala Thr Gly Ala Tyr Cys Ile Val
            180                 185                 190

Asp Ile His Asn Tyr Ala Arg Trp Asn Gly Ala Ile Ile Gly Gln Gly
        195                 200                 205

Gly Pro Thr Asn Ala Gln Phe Thr Asn Leu Trp Thr Gln Leu Ala Thr
    210                 215                 220

Lys Tyr Ala Ser Gln Ser Lys Ile Trp Phe Gly Ile Val Asn Glu Pro

225                 230                 235                 240

His Asp Val Asp Ile Asn Thr Trp Ala Thr Thr Val Gln Ala Val Val
                245                 250                 255

Thr Ala Ile Arg Asn Ala Gly Ala Thr Thr Gln Phe Ile Ser Leu Pro
            260                 265                 270

Gly Thr Asp Tyr Gln Ser Ala Gly Asn Phe Leu Ser Asp Gly Ser Ser
        275                 280                 285

Thr Ala Leu Ser Gln Val Lys Asn Pro Asp Gly Ser Thr Thr Asn Leu
    290                 295                 300

Ile Phe Asp Leu His Lys Tyr Leu Asp Ser Asp Asn Ser Gly Thr Ser
305                 310                 315                 320

Thr Thr Cys Val Thr Asn Asn Ile Ala Thr Ala Phe Gln Pro Val Ala
                325                 330                 335

Thr Trp Leu Arg Gln Asn Lys Arg Gln Ala Ile Leu Thr Glu Thr Gly
            340                 345                 350

Gly Gly Asn Thr Gln Ser Cys Ile Gln Asp Val Cys Gln Gln Asn Gln
        355                 360                 365

Phe Leu Asn Gln Asn Ser Asp Val Phe Leu Gly Tyr Leu Gly Trp Gly
    370                 375                 380

Ala Gly Ser Phe Asp Ser Thr Tyr Ala Leu Thr Leu Thr Pro Thr Gln
385                 390                 395                 400

Asn Gly Asn Thr Trp Thr Asp Thr Ala Leu Ala Ala Ala Cys Phe Ser
                405                 410                 415

Arg Ala

<210>  61
<211>  418
<212>  PRT
<213>  Trichoderma gamsii

<400>  61

Met Asn Lys Pro Met Gly Pro Leu Leu Leu Ala Ala Thr Leu Met Ala
1               5                   10                  15

Ser Gly Ala Ile Ala Gln Thr Gln Ser Val Trp Gly Gln Cys Gly Gly

20                  25                  30

Thr Gly Tyr Ser Gly Pro Thr Asn Cys Ala Ser Gly Ser Ala Cys Ser
        35                  40                  45

Thr Leu Asn Pro Tyr Tyr Ala Gln Cys Ile Pro Gly Ala Thr Ser Phe
    50                  55                  60

Ile Thr Ser Thr Thr Ser Thr Lys Ser Pro Gly Ser Gly Ser Ser Thr
65                  70                  75                  80

Thr Ser Ser Ala Ser Gln Pro Thr Gly Ser Gly Gln Thr Arg Phe Ala
                85                  90                  95

Gly Ile Asn Ile Ala Gly Phe Asp Phe Gly Cys Thr Ile Asp Gly Thr
            100                 105                 110

Cys Val Thr Ser Gln Ile Tyr Pro Pro Leu Lys Asn Phe Gly Gly Thr
        115                 120                 125

Asn Asn His Pro Asp Gly Val Gly Gln Met Gln His Phe Val Asn Asp
    130                 135                 140

Asp Lys Leu Asn Ile Phe Arg Leu Pro Val Gly Trp Gln Tyr Leu Val
145                 150                 155                 160

Asn Asn Asn Leu Gly Gly Thr Leu Asp Ser Thr Ala Ile Ser Asn Tyr
                165                 170                 175

Asp Gln Leu Val Gln Gly Cys Leu Ala Thr Gly Ser Tyr Cys Ile Val
            180                 185                 190

Asp Ile His Asn Tyr Ala Arg Trp Asn Gly Ala Ile Ile Gly Gln Gly
        195                 200                 205

Gly Pro Thr Asn Ala Gln Phe Val Ser Leu Trp Thr Gln Leu Ala Thr
    210                 215                 220

Lys Tyr Ala Ser Gln Ser Lys Val Trp Phe Gly Ile Val Asn Glu Pro
225                 230                 235                 240

His Asp Val Asp Ile Asn Thr Trp Gly Gln Thr Val Gln Ala Val Val
                245                 250                 255

Thr Ala Ile Arg Asn Ala Gly Ala Thr Thr Gln Phe Ile Ser Leu Pro
            260                 265                 270

Gly Thr Asp Phe Gln Ser Ala Gly Ser Phe Leu Ser Asp Gly Ser Ser
        275                 280                 285

Thr Ala Leu Ser Gln Val Lys Asn Pro Asp Gly Ser Thr Thr Asn Leu
    290                 295                 300

Ile Phe Asp Phe His Lys Tyr Leu Asp Ser Asp Asn Ser Gly Thr His
305                 310                 315                 320

Thr Glu Cys Val Thr Asn Asn Ile Ala Thr Ala Phe Gln Pro Val Ala
                325                 330                 335

Thr Trp Leu Arg Gln Asn Lys Arg Gln Gly Ile Leu Thr Glu Thr Gly
            340                 345                 350

Gly Gly Asn Thr Gln Ser Cys Leu Thr Asp Met Cys Gln Gln Asn Gln
        355                 360                 365

Phe Leu Asn Gln Asn Ser Asp Val Phe Leu Gly Tyr Ile Gly Trp Gly
    370                 375                 380

Ala Gly Ser Phe Asp Ser Thr Tyr Glu Leu Thr Leu Thr Pro Thr Gln
385                 390                 395                 400

Asn Gly Asn Thr Trp Thr Asp Thr Ala Leu Ala Ala Ala Cys Phe Ser
                405                 410                 415

Arg Lys

<210>  62
<211>  416
<212>  PRT
<213>  Thielavia terrestris

<400>  62

Met Pro Ser Val Val Leu Ser Leu Ala Phe Leu Ala Gly Ser Ala Ile
1               5                   10                  15

Ala Gln Gln Ser Val Trp Gly Gln Cys Gly Gly Ile Gly Tyr Ser Gly
            20                  25                  30

Pro Thr Ser Cys Val Ser Gly Ser Cys Cys Val Thr Leu Asn Ala Tyr
        35                  40                  45

Tyr Ala Gln Cys Thr Pro Gly Thr Gly Cys Gly Gly Gly Gly Ser Gly
    50                  55                  60

Thr Thr Leu Thr Thr Lys Thr Thr Thr Thr Thr Ala Thr Ala Thr Pro
65                  70                  75                  80

Thr Gly Gly Gly Gly Val Gly Ser Gly Lys Thr Arg Phe Ala Gly Val
                85                  90                  95

Asn Ile Ala Gly Phe Asp Phe Gly Cys Gly Thr Asp Gly Thr Cys Asn
            100                 105                 110

Thr Ala Lys Val Tyr Pro Pro Val Lys Asn Tyr Pro Pro Asp Tyr Asn
        115                 120                 125

His Pro Asp Gly Ala Gly Gln Met Gln His Phe Tyr Arg Asp Asp Asn
    130                 135                 140

Met Asn Ile Phe Arg Leu Pro Val Gly Trp Gln Tyr Leu Val Asn Asn
145                 150                 155                 160

Asn Leu Gly Gly Ser Leu Asp Ser Thr Asn Phe Gly Tyr Tyr Asp Gln
                165                 170                 175

Leu Val Gln Ala Cys Leu Ser Thr Gly Ala Tyr Cys Ile Ile Asp Ile
            180                 185                 190

His Asn Tyr Ala Arg Trp Asn Gly Ala Ile Ile Gly Gln Gly Gly Pro
        195                 200                 205

Thr Asn Glu Gln Phe Ala Asp Leu Trp Arg Gln Ile Ala Thr Lys Tyr
    210                 215                 220

Ala Asn Gln Pro Arg Ile Trp Phe Gly Ile Met Asn Glu Pro His Asp
225                 230                 235                 240

Val Pro Asn Ile Ser Thr Trp Ala Ala Thr Val Gln Ala Ala Val Thr
                245                 250                 255

Ala Ile Arg Asn Ala Gly Ala Thr Ser Gln Phe Ile Ser Leu Pro Gly
            260                 265                 270

Asn Asp Trp Gln Ser Ala Ala Ala Phe Ile Ser Asp Gly Ser Ala Ala
        275                 280                 285

Ala Leu Ser Ala Val Lys Asn Pro Asp Gly Ser Thr Thr Asn Leu Ile
    290                 295                 300

Phe Asp Val His Lys Tyr Leu Asp Ser Asp Asn Ser Gly Thr His Thr
305                 310                 315                 320

Glu Cys Val Thr Asn Asn Ile Asp Ser Ala Phe Ala Pro Leu Ala Thr
                325                 330                 335

Trp Leu Arg Gln Asn Asn Arg Gln Ala Ile Leu Thr Glu Thr Gly Gly
            340                 345                 350

Gly Asn Thr Ala Ser Cys Gln Thr Tyr Val Cys Gln Gln Leu Ala Tyr
        355                 360                 365

Leu Asn Gln Asn Ser Asp Val Tyr Leu Gly Tyr Val Gly Trp Ala Ala
    370                 375                 380

Gly Ser Phe Asp Ser Thr Tyr Glu Leu Asp Leu Thr Pro Thr Gln Asn
385                 390                 395                 400

Gly Asn Ser Trp Thr Asp Thr Ala Leu Ala Lys Ala Cys Phe Ala Arg
                405                 410                 415

<210>  63
<211>  407
<212>  PRT
<213>  Phialophora sp. G5

<400>  63

Met Met Arg Ser Leu Leu Leu Ser Leu Thr Val Leu Gly Ala Gly Ala
1               5                   10                  15

Val Ala Gln Gln Ser Ala Trp Gly Gln Cys Gly Gly Ile Gly Trp Ser
            20                  25                  30

Gly Ser Thr Ser Cys Glu Ala Gly Ser Cys Cys Val Ser Gln Asn Ala
        35                  40                  45

Tyr Tyr Ser Gln Cys Val Pro Gly Thr Ala Cys Ser Gly Thr Ala Thr
    50                  55                  60

Thr Thr Ala Thr Pro Thr Pro Thr Ser Gly Gly Gly Ser Gly Arg Thr
65                  70                  75                  80

Arg Phe Ala Gly Val Asn Ile Ala Gly Phe Asp Phe Gly Cys Ala Thr
                85                  90                  95

Asp Gly Thr Cys Asn Thr Thr Ala Val Tyr Pro Pro Val Lys Asp Met
            100                 105                 110

Pro Pro Tyr Tyr Asn Asn Pro Asp Gly Ala Gly Gln Met Asp His Phe
        115                 120                 125

Ser Lys Asp Asp Asn Leu Asn Ile Phe Arg Leu Pro Val Gly Trp Gln
    130                 135                 140

Tyr Leu Val Asn Ser Asn Leu Gly Gly Thr Leu Asp Ser Thr Asn Leu
145                 150                 155                 160

Gly Tyr Tyr Asp Gln Leu Val Gln Ser Cys Leu Ser Thr Gly Ala Tyr
                165                 170                 175

Cys Ile Val Asp Ile His Asn Tyr Ala Arg Trp Asn Gly Ala Ile Ile
            180                 185                 190

Gly Gln Gly Gly Pro Thr Asn Glu Gln Phe Val Ser Val Trp Thr Gln
        195                 200                 205

Leu Ala Thr Lys Tyr Ala Ser Gln Ala Arg Val Trp Phe Gly Ile Met
    210                 215                 220

Asn Glu Pro His Asp Val Pro Ser Ile Thr Thr Trp Ala Ala Thr Val
225                 230                 235                 240

Gln Ala Val Val Thr Ala Ile Arg Asn Ala Gly Ala Thr Ser Gln Phe
                245                 250                 255

Ile Ser Leu Pro Gly Asn Asp Trp Gln Ser Ala Ala Ala Val Ile Ser
            260                 265                 270

Asp Gly Ser Ala Ala Ala Leu Ser Thr Val Thr Asn Pro Asp Gly Thr
        275                 280                 285

Thr Thr Asn Leu Ile Phe Asp Val His Lys Tyr Leu Asp Ser Asp Asn
    290                 295                 300

Ser Gly Thr His Thr Glu Cys Val Thr Asn Asn Ile Asp Asp Ala Phe
305                 310                 315                 320

Ala Pro Leu Ala Thr Trp Leu Arg Gln Asn Gly Arg Gln Ala Ile Leu
                325                 330                 335

Thr Glu Thr Gly Gly Gly Asn Thr Ala Ser Cys Glu Thr Tyr Leu Cys
            340                 345                 350

Gln Gln Ile Ala Tyr Leu Asn Ala Asn Ala Asp Val Tyr Leu Gly Tyr
        355                 360                 365

Val Gly Trp Gly Ala Gly Ser Phe Asp Ser Thr Tyr Ala Leu Asp Glu
    370                 375                 380

Thr Pro Thr Gly Ser Gly Ser Ser Trp Thr Asp Thr Pro Leu Val Lys
385                 390                 395                 400

Ala Cys Ile Ala Arg Ser Ser
                405

<210>  64
<211>  411
<212>  PRT
<213>  Botryotinia fuckeliana

<400>  64

Met Lys Leu Ser Thr Thr Val Tyr Thr Val Val Pro Phe Leu Ser Thr
1               5                   10                  15

Ala Thr Ala Gln Gly Val Ala Tyr Ala Gln Cys Gly Gly Asn Gly Trp
            20                  25                  30

Thr Gly Ser Thr Ala Cys Val Ser Gly Tyr Ala Cys Ser Tyr Val Asn
        35                  40                  45

Ala Tyr Tyr Ser Gln Cys Leu Pro Gly Thr Ala Thr Leu Thr Thr Val
    50                  55                  60

Thr Ser Ala Thr Thr Ser Ala Ser Ser Lys Thr Ser Thr Ala Ala Ala
65                  70                  75                  80

Pro Asn Ser Thr Gly Lys Thr Lys Tyr Ile Gly Thr Asn Ile Ala Gly
                85                  90                  95

Phe Asp Phe Gly Cys Thr Thr Asp Gly Thr Cys Leu Thr Asn Lys Val
            100                 105                 110

Tyr Pro Ala Leu Ser Ser Leu Asn Asn Gly Pro Asp Gly Leu Gly Gln
        115                 120                 125

Met Ala His Phe Val Ser Lys Thr Gly His Asn Ile Phe Arg Leu Pro
    130                 135                 140

Val Gly Trp Gln Tyr Leu Val Asn Asn Asn Leu Gly Gly Thr Leu Asp
145                 150                 155                 160

Ser Ser Asn Leu Ala Thr Tyr Asp Leu Leu Val Gln Gly Cys Leu Ala
                165                 170                 175

Thr Gly Ala Thr Cys Val Ile Asp Ile His Asn Tyr Ala Arg Trp Asn
            180                 185                 190

Gly Ala Ile Ile Gly Gln Gly Gly Pro Thr Asp Ala Gln Phe Ala Ser
        195                 200                 205

Leu Trp Ser Gln Leu Ala Thr Lys Tyr Lys Ser Asn Thr Lys Val Val
    210                 215                 220

Phe Gly Leu Met Asn Glu Pro His Asp Leu Asn Ser Ile Thr Thr Trp
225                 230                 235                 240

Ala Ala Thr Leu Gln Thr Val Val Thr Ala Ile Arg Gln Ala Gly Ala
                245                 250                 255

Thr Ser Thr Met Leu Leu Leu Pro Gly Ser Asp Tyr Thr Ser Ala Gly
            260                 265                 270

Ala Phe Ile Thr Asp Gly Ser Ala Ala Ala Leu Ser Lys Ile Thr Asn
        275                 280                 285

Leu Asp Gly Thr Thr Thr Asn Leu Ile Phe Asp Val His Lys Tyr Leu
    290                 295                 300

Asp Ser Asp Asn Ser Gly Thr His Ala Glu Cys Val Thr Asn Asn Ile
305                 310                 315                 320

Asp Ala Ala Phe Ala Pro Leu Ala Thr Trp Leu Arg Ala Asn Gly Arg
                325                 330                 335

Gln Ala Ile Leu Ser Glu Thr Gly Gly Gly Asn Thr Ala Ser Cys Gln
            340                 345                 350

Thr Tyr Leu Cys Gln Gln Val Ala Tyr Leu Asn Ala Asn Ser Asp Val
        355                 360                 365

Tyr Leu Gly Tyr Ile Gly Trp Ser Ala Gly Ser Phe Asp Ser Thr Tyr
    370                 375                 380

Ile Leu Thr Glu Thr Pro Asn Gly Ser Gly Ser Ser Met Thr Asp Gln
385                 390                 395                 400

Ala Leu Val Ala Ala Cys Leu Thr Arg Thr Ser
                405                 410

<210>  65
<211>  424
<212>  PRT
<213>  Jaapia argillacea

<400> 65

Met Lys Thr Leu Ile Ala Leu Gly Val Ala Gly Leu Ser Ala Leu Ser
1               5                   10                  15

Val Arg Ala Gln Gln Thr Leu Tyr Gly Gln Cys Gly Gly Ile Gly Trp
            20                  25                  30

Thr Gly Ala Thr Thr Cys Val Ser Gly Ala Thr Cys Thr Leu Leu Asn
        35                  40                  45

Ser Tyr Tyr Tyr Gln Cys Leu Pro Ala Ser Ser Thr Thr Ser His Ser
    50                  55                  60

Ser Thr Ser Ser His Ser Ser Ser Ser Thr Thr Thr Thr Pro Thr Thr
65                  70                  75                  80

Thr Pro Thr Thr Ile Ser Thr Thr Thr Ser Ser Thr Ser Ser Ser Pro
                85                  90                  95

Ser Ala Ser Ala Thr Gly Ser Leu Lys Phe Tyr Gly Val Asn Ile Ala
            100                 105                 110

Gly Phe Asp Phe Gly Cys Gly Thr Asp Gly Thr Cys Asn Thr Thr Gln
        115                 120                 125

Ala Tyr Pro Pro Leu Val Ser Leu Gly Gly Pro Asp Gly Ala Gly Gln
    130                 135                 140

Met Gln His Phe Val Ser Glu Gly Tyr Asn Ile Phe Arg Leu Pro Val
145                 150                 155                 160

Gly Trp Gln Tyr Leu Val Ala Asn Thr Leu Gly Gly Pro Leu Asp Pro
                165                 170                 175

Thr Asn Ser Gly Lys Tyr Asp Gln Leu Val Gln Ala Cys Leu Ser Thr
            180                 185                 190

Gly Ala Tyr Cys Ile Ile Asp Ile His Asn Tyr Ala Arg Trp Asn Gly
        195                 200                 205

Gly Ile Ile Gly Gln Gly Gly Pro Thr Asn Ala Gln Phe Thr Ser Leu
    210                 215                 220

Trp Ser Ala Leu Ala Thr Lys Tyr Lys Ala Gln Ser Lys Val Val Phe
225                 230                 235                 240

Gly Ile Met Asn Glu Pro His Asp Val Pro Asp Ile Asn Ala Trp Ala

245                 250                 255

Ala Thr Val Gln Ala Ala Val Thr Ala Ile Arg Gln Ala Gly Ala Thr
            260                 265                 270

Ser Gln Leu Ile Leu Leu Pro Gly Asn Asn Trp Thr Ser Ala Gln Thr
        275                 280                 285

Phe Val Ser Asn Gly Ser Gly Ala Ala Leu Ala Ala Val Thr Asn Leu
    290                 295                 300

Asp Gly Thr Thr Thr Asn Leu Ile Phe Asp Val His Lys Tyr Leu Asp
305                 310                 315                 320

Ser Asp Asn Ser Gly Thr His Ala Glu Cys Thr Thr Asp Asn Val Ser
                325                 330                 335

Ser Ser Trp Gln Pro Leu Ala Thr Trp Leu Arg Ser Val Gly Arg Gln
            340                 345                 350

Ala Leu Asn Thr Glu Thr Gly Gly Gly Asn Val Ala Ser Cys Val Gln
        355                 360                 365

Tyr Met Cys Ser Gln Val Ala Tyr Gln Thr Ser Val Asn Asp Val Ile
    370                 375                 380

Leu Gly Tyr Val Gly Trp Ala Ala Gly Ser Phe Ala Thr Ser Tyr Val
385                 390                 395                 400

Leu Ser Glu Thr Pro Thr Gln Ser Gly Ser Thr Trp Thr Asp Gln Leu
                405                 410                 415

Leu Val Ser Ser Cys Met Lys Pro
            420

<210>  66
<211>  413
<212>  PRT
<213>  Cylindrobasidium torrendii

<400>  66

Met Arg Leu Leu Thr Leu Arg Asn Leu Val Ala Ser Ser Leu Val Val
1               5                   10                  15

Val Ser Val Ser Ala Gln Gly Gln Leu Tyr Ala Gln Cys Gly Gly Ser
            20                  25                  30

Gly Trp Ser Gly Ser Thr Ala Cys Val Ser Gly Ala Thr Cys Thr Tyr

35                  40                  45

Ile Asn Asp Tyr Tyr Ser Gln Cys Leu Pro Gly Ser Ala Thr Val Ser
    50                  55                  60

Ser Thr Thr Val Lys Thr Ser Ser Thr Ser Ala Ser Ser Pro Ser Ser
65                  70                  75                  80

Thr Ala Ala Ala Leu Lys Tyr Val Gly Val Asn Ile Ala Gly Phe Asp
                85                  90                  95

Phe Gly Cys Ala Thr Asp Gly Thr Cys Val Ile Ala Asp Val Tyr Pro
            100                 105                 110

Pro Leu Ser Ser Gln Gly Gly Asn Asp Gly Glu Gly Gln Met Lys His
        115                 120                 125

Phe Phe Asn Asp Asp Gly Phe Asn Thr Phe Arg Leu Pro Val Gly Trp
    130                 135                 140

Gln Phe Leu Thr Asn Gly Val Val Gly Gly Thr Leu Asn Asp Ala Asn
145                 150                 155                 160

Phe Gln Lys Tyr Asp Gln Leu Val Gln Ala Cys Leu Arg Thr Gly Ala
                165                 170                 175

Thr Cys Ile Ile Asp Ile His Asn Tyr Ala Arg Trp Asn Gly Gly Val
            180                 185                 190

Ile Gly Gln Gly Gly Pro Thr Asn Glu Gln Phe Ala Ala Leu Trp Thr
        195                 200                 205

Ser Leu Ala Gly Lys Tyr Lys Ser Gln Ser Lys Val Trp Phe Gly Val
    210                 215                 220

Val Asn Glu Pro His Asp Val Ala Asp Ile Thr Ala Trp Gly Val Thr
225                 230                 235                 240

Val Gln Ala Val Val Thr Ala Ile Arg Asn Ala Gly Ala Thr Thr Gln
                245                 250                 255

Asn Ile Leu Leu Pro Gly Asn Asn Trp Ala Ser Ala Ala Ala Phe Val
            260                 265                 270

Ser Ser Gly Ser Ala Ala Ala Leu Lys Gly Ile Thr Asn Pro Asp Gly
        275                 280                 285

Thr Asn Thr Gly Leu Ile Phe Asp Val His Lys Tyr Leu Asp Ser Asp
    290                 295                 300

Asn Ser Gly Thr His Thr Glu Cys Thr Thr Asn Asn Ile Ser Glu Ala
305                 310                 315                 320

Trp Glu Pro Leu Ser Ala Trp Leu Arg Ala Asn Gly Asn Arg Gln Ile
                325                 330                 335

Ile Val Thr Glu Thr Gly Gly Gly Asn Thr Ala Ser Cys Val Lys Tyr
            340                 345                 350

Met Cys Glu Gln Ile Lys Tyr Gln Ala Lys Asn Ser Asp Val Ile Leu
        355                 360                 365

Gly Tyr Val Gly Trp Ser Ala Gly Asn Phe Ala Asn Thr Tyr Glu Leu
    370                 375                 380

Ser Glu Val Pro Thr Leu Ser Gly Ser Thr Trp Thr Asp Thr Leu Leu
385                 390                 395                 400

Val Lys Ser Cys Met Ala Pro Ile Ala Gly Ala Gln Asn
                405                 410

<210>  67
<211>  417
<212>  PRT
<213>  Polyporus arcularius

<400>  67

Met His Ser Phe Ser Gly Leu Leu Val Ala Val Ala Leu Ala Val Ser
1               5                   10                  15

Val Lys Ala Gln Thr Thr Leu Ser Pro Val Trp Gly Gln Cys Gly Gly
            20                  25                  30

Thr Gly Trp Thr Gly Pro Thr Ala Cys Ala Thr Gly Ser Val Cys Thr
        35                  40                  45

Lys Leu Asn Asp Ala His Phe Gln Cys Val Pro Gly Thr Ala Thr Thr
    50                  55                  60

Ala Pro Ser Ser Pro Ser Thr Thr Ser Ser Ala Pro Ala Pro Thr Ser
65                  70                  75                  80

Ser Cys Gln Ile Pro Ala Pro Ser Ala Gly Lys Leu Arg Phe Ala Gly
                85                  90                  95

Val Asn Ile Ala Gly Phe Asp Phe Gly Cys Ser Thr Asp Gly Thr Cys
        100                 105                 110

Ser Ala Ser Gly Ala Trp Pro Pro Leu Thr Gln Tyr Tyr Gly Asn Asp
    115                 120                 125

Gly Glu Gly Gln Met Gln His Phe Val Lys Asp Asp Gly Phe Asn Val
130                 135                 140

Phe Arg Leu Pro Val Gly Trp Gln Tyr Leu Thr Asp Gly Val Leu Gly
145                 150                 155                 160

Gly Thr Ile Asp Glu Asp Asn Phe Glu Glu Tyr Asp Ala Leu Val Gln
                165                 170                 175

Ala Cys Leu Asn Thr Gly Ala Ser Cys Ile Ile Asp Ile His Asn Tyr
        180                 185                 190

Ala Arg Trp Asn Gly Lys Ile Ile Gly Gln Gly Gly Pro Thr Asn Glu
    195                 200                 205

Gln Phe Ala Glu Leu Trp Ser Ser Val Ala Ala Lys Tyr Ala Asn Asn
210                 215                 220

Ser Lys Ile Ile Phe Gly Val Met Asn Glu Pro His Asp Val Pro Asp
225                 230                 235                 240

Ile Asn Leu Trp Ala Glu Ser Val Gln Ala Ala Val Thr Ala Ile Arg
                245                 250                 255

Lys Ala Gly Ala Thr Ser Gln Ile Ile Leu Leu Pro Gly Asn Asn Trp
        260                 265                 270

Thr Ser Ala Glu Thr Phe Ile Ser Asn Gly Ser Gly Ala Ala Leu Met
    275                 280                 285

Lys Val Thr Asn Pro Asp Gly Ser Thr Thr Asn Leu Val Phe Asp Val
290                 295                 300

His Lys Tyr Leu Asp Ser Asp Asn Ser Gly Thr His Glu Glu Cys Val
305                 310                 315                 320

Thr Asn Asn Ile Asp Asn Ala Trp Ala Pro Leu Ala Glu Trp Leu Arg
                325                 330                 335

Cys Asn Gly Arg Gln Ala Phe Asn Thr Glu Thr Gly Gly Gly Asn Val
        340                 345                 350

Ala Ser Cys Gln Gln Phe Met Cys Glu Gln Val Ala Phe Gln Asn Ala
        355                 360                 365

Asn Ser Asp Val Phe Leu Gly Tyr Val Gly Trp Ala Ala Gly Asn Phe
    370                 375                 380

Tyr Gln Gly Tyr Ala Leu Gly Glu Val Pro Thr Tyr Ser Asn Gly Gln
385                 390                 395                 400

Trp Val Asp Thr Ser Leu Val Ser Ser Cys Leu Ala Pro Asn Ala Arg
                405                 410                 415

Lys

<210>  68
<211>  430
<212>  PRT
<213>  Ceriporiopsis subvermispora

<400>  68

Met Lys Ser Val Leu Leu Phe Thr Cys Ala Thr Trp Tyr Ala Leu Ser
1               5                   10                  15

Val Arg Ala Gln Ala Ser Leu Tyr Gly Gln Cys Gly Gly Ile Gly Trp
            20                  25                  30

Thr Gly Pro Thr Thr Cys Ala Ser Gly Ser Val Cys Thr Val Ser Asn
        35                  40                  45

Ala Tyr Tyr Ser Gln Cys Leu Pro Gly Ala Ala Ser Ala Ser Asn Pro
    50                  55                  60

Thr Thr Ser Ser Ala Pro Thr Thr Ala Pro Gly Gly Gly Ser Pro Val
65                  70                  75                  80

Ser Ser Ser Thr Ala Ser Ala Pro Ser Ser Ala Pro Ser Ala Ser Cys
                85                  90                  95

Ser Asp Leu Pro Ala Asn Pro Gly Pro Leu Gln Leu Val Gly Val Asn
            100                 105                 110

Ile Pro Gly Phe Asp Phe Gly Cys Ser Thr Asp Gly Thr Cys Thr Val
        115                 120                 125

Ser Gly Ala Trp Pro Pro Leu Thr Gln Tyr Tyr Gly Ala Asp Gly Ala
    130                 135                 140

Gly Gln Met Thr His Phe Val Asn Asp Asp Gly Tyr Asn Val Phe Arg
145                 150                 155                 160

Leu Pro Val Gly Trp Gln Tyr Leu Thr Asn Asp Val Leu Gly Gly Asp
                165                 170                 175

Leu Asp Glu Ala Asn Ala Ala Glu Tyr Asp Glu Leu Val Gln Ala Cys
            180                 185                 190

Leu Ala Thr Gly Ala Ser Cys Ile Ile Asp Ile His Asn Tyr Ala Arg
        195                 200                 205

Trp Asn Gly Glu Ile Ile Gly Gln Gly Gly Pro Thr Asn Asp Gln Phe
    210                 215                 220

Ala Ala Ile Trp Ser Ser Leu Ala Ala Lys Tyr Ala Ser Glu Asp Lys
225                 230                 235                 240

Ile Ile Phe Gly Val Met Asn Glu Pro His Asp Ile Pro Asp Ile Asp
                245                 250                 255

Thr Trp Gly Gln Ser Val Gln Ala Ala Val Thr Ala Ile Arg Gln Ala
            260                 265                 270

Gly Ala Thr Thr Gln Leu Ile Leu Leu Pro Gly Asn Asp Tyr Thr Ser
        275                 280                 285

Ala Glu Glu Phe Ile Ser Asn Gly Ser Ala Ala Ala Leu Met Asn Val
    290                 295                 300

Thr Asn Pro Asp Gly Ser Phe Thr Asp Leu Ile Phe Asp Val His Lys
305                 310                 315                 320

Tyr Leu Asp Ser Asp Asn Ser Gly Thr Asn Ala Glu Cys Val Thr Asn
                325                 330                 335

Asn Ile Asp Asp Ala Trp Glu Pro Leu Thr Gln Trp Leu Arg Cys Asn
            340                 345                 350

Gly Arg Gln Ala Phe Asn Thr Glu Thr Gly Gly Gly Asn Thr Ala Ser
        355                 360                 365

Cys Asn Thr Tyr Met Cys Gln Gln Val Ala Tyr Gln Lys Gln Asn Ser
    370                 375                 380

Asp Val Leu Leu Gly Tyr Val Gly Trp Ala Ala Gly Asn Phe Met Asn
385                 390                 395                 400

Thr Tyr Val Leu Ser Glu Thr Pro Thr Glu Ser Gly Ser Thr Trp Thr
                405                 410                 415

Asp Ala Ala Leu Val Ser Ala Cys Met Ala Pro Asn Ala Ser
            420                 425                 430

<210>  69
<211>  414
<212>  PRT
<213>  Phlebiopsis gigantea

<400>  69

Met Ala Phe Asn Val Lys Ser Leu Tyr Ala Phe Thr Leu Leu Ser Val
1               5                   10                  15

Trp Ile Ala Ser Ala Arg Gly Gln Ala Gln Glu Trp Gln Gln Cys Gly
            20                  25                  30

Gly Ile Gly Phe Ser Gly Ala Thr Thr Cys Val Ser Gly Ser Val Cys
        35                  40                  45

Ser Glu Ile Asn Asp Tyr Tyr Ser Gln Cys Val Pro Gly Val Ala Ser
    50                  55                  60

Ser Ala Pro Ala Thr Ser Thr Ala Thr Ser Thr Ser Thr Ala Pro Ser
65                  70                  75                  80

Ser Thr Pro Ser Ser Gly Ser Ser Ser Gly Ser Leu Arg Phe Ala Gly
                85                  90                  95

Val Asn Ile Ser Gly Phe Asp Phe Gly Cys Asn Ser Asp Gly Ser Cys
            100                 105                 110

Thr Ala Ser Gly Ala Trp Pro Pro Leu Leu Gln Tyr Tyr Gly His Asp
        115                 120                 125

Gly Gln Gly Gln Met Gln His Phe Val Asn Asp Asp Gly Phe Asn Val
    130                 135                 140

Phe Arg Leu Pro Val Gly Trp Gln Phe Leu Thr Asn Asp Val Met Ser
145                 150                 155                 160

Gly Asp Ile Asp Glu Asp Asn Leu Ala Glu Tyr Asp Ala Leu Val Gln
                165                 170                 175

Ala Cys Leu Ala Thr Gly Ala Ser Cys Ile Val Asp Val His Asn Tyr
            180                 185                 190

Ala Arg Phe Asn Gly Glu Ile Ile Gly Gln Gly Gly Pro Ser Asn Asp
        195                 200                 205

Ile Phe Ala Thr Leu Trp Ser Asn Leu Ala Ala Lys Tyr Ala Thr Ser
    210                 215                 220

Asp Asn Val Ile Phe Gly Val Met Asn Glu Pro His Asp Ile Pro Asp
225                 230                 235                 240

Ile Val Ala Trp Ala Ala Ser Val Gln Ala Ala Val Thr Ala Ile Arg
                245                 250                 255

Gln Ala Gly Ala Thr Thr Gln Leu Ile Leu Leu Pro Gly Asn Asn Trp
            260                 265                 270

Thr Ser Ala Glu Thr Phe Val Ser Asn Gly Ser Ala Asp Ala Leu Ser
        275                 280                 285

Gln Val Thr Asn Pro Asp Gly Ser Ile Thr Asn Leu Ile Phe Asp Val
    290                 295                 300

His Lys Tyr Leu Asp Ser Asp Asn Ser Gly Thr Asn Ala Glu Cys Val
305                 310                 315                 320

Thr Asn Asn Ile Asp Asp Ala Phe Ser Pro Leu Ala Asp Tyr Leu Arg
                325                 330                 335

Thr Asn Gly Arg Gln Ala Phe Asn Thr Glu Thr Gly Gly Gly Asn Thr
            340                 345                 350

Ala Ser Cys Ala Gln Tyr Leu Cys Glu Gln Ile Ala Phe Met Gln Gln
        355                 360                 365

Asn Ser Asp Val Tyr Leu Gly Tyr Val Gly Trp Ala Ala Gly Asn Phe
    370                 375                 380

Asp Pro Thr Tyr Val Leu Gly Glu Thr Pro Thr Asp Asn Gly Gly Val
385                 390                 395                 400

Trp Thr Asp Thr Ser Leu Val Ser Ala Cys Leu Ala Pro Lys
                405                 410

<210>  70
<211>  16
<212>  PRT
<213>  Artificial Sequence

<220>
<223>  CBH1 First consensus domain sequence

<220>
<221>  VARIANT
<222>  (14)..(14)
<223>  Replace = Ser

<400>  70

Asn Lys Ala Gly Ala Lys Tyr Gly Thr Gly Tyr Cys Asp Ala Gln Cys
1               5                   10                  15

<210>  71
<211>  11
<212>  PRT
<213>  Artificial Sequence

<220>
<223>  CBH2 Second consensus domain sequence

<220>
<221>  VARIANT
<222>  (3)..(3)
<223>  Replace = Ala or Pro

<220>
<221>  VARIANT
<222>  (7)..(7)
<223>  Replace = Val

<400>  71

Cys Cys Ser Glu Met Asp Ile Trp Glu Ala Asn
1               5                   10

<210>  72
<211>  659
<212>  PRT
<213>  Neurospora tetrasperma

<400>  72

Met Ile Met Glu Glu Ala Glu Glu Lys Arg Thr Thr Ser Ser Thr Trp
1               5                   10                  15

Arg Pro Ser Arg Phe Ser Glu Trp Trp Ser Val His Thr Thr Pro Arg
            20                  25                  30

Pro Pro Pro Pro Pro Ser Ser Pro Ser Pro Ser Pro Ser Pro Arg Gly
        35                  40                  45

Asn Cys Ala Leu Gln Ser Tyr Gly Lys Arg Leu Glu Arg Gly Trp Arg
    50                  55                  60

Met Gln Thr Arg Glu Arg Gly Ala Ser Asn Pro Pro Ala Lys Pro Thr
65                  70                  75                  80

Ala Pro Arg Ser Leu Ala Arg Leu His Glu Thr Gly Val Ala Asn Glu
                85                  90                  95

Pro Trp Gly Ala Leu Arg Asp Ile Asn Ile Tyr Ile Ile Trp Gly Phe
            100                 105                 110

Gly Thr Ser Ser Asn Ser Phe Ser Ser Ile Ser Gln Ser Ser Ser Ser
        115                 120                 125

Ser Thr Pro Phe Ser Leu Ile Leu Thr Met Leu Ala Lys Phe Ala Ala
    130                 135                 140

Leu Ala Ala Leu Val Ala Ser Ala Asn Ala Gln Ala Val Cys Ser Leu
145                 150                 155                 160

Thr Ala Glu Thr His Pro Ser Leu Asn Trp Ser Lys Cys Thr Ser Ser
                165                 170                 175

Gly Cys Thr Asn Val Ala Gly Ser Ile Thr Val Asp Ala Asn Trp Arg
            180                 185                 190

Trp Thr His Ile Thr Ser Gly Ser Thr Asn Cys Tyr Ser Gly Asn Glu
        195                 200                 205

Trp Asp Thr Ser Leu Cys Ser Thr Asn Thr Asp Cys Ala Thr Lys Cys
    210                 215                 220

Cys Val Asp Gly Ala Glu Tyr Ser Ser Thr Tyr Gly Ile Gln Thr Ser
225                 230                 235                 240

Gly Asn Ser Leu Ser Leu Gln Phe Val Thr Lys Gly Ser Tyr Ser Thr
                245                 250                 255

Asn Ile Gly Ser Arg Thr Tyr Leu Met Asn Gly Ala Asp Ala Tyr Gln
            260                 265                 270

Gly Phe Glu Leu Leu Gly Asn Glu Phe Thr Phe Asp Val Asp Val Ser
        275                 280                 285

Gly Thr Gly Cys Gly Leu Asn Gly Ala Leu Tyr Phe Val Ser Met Asp
    290                 295                 300

Leu Asp Gly Gly Lys Ala Lys Tyr Thr Asn Asn Lys Ala Gly Ala Lys
305                 310                 315                 320

Tyr Gly Thr Gly Tyr Cys Asp Ala Gln Cys Pro Arg Asp Leu Lys Tyr
                325                 330                 335

Ile Asn Gly Val Ala Asn Val Glu Gly Trp Thr Pro Ser Thr Asn Asp
            340                 345                 350

Ala Asn Ala Gly Ile Gly Asn His Gly Thr Cys Cys Ser Glu Met Asp
        355                 360                 365

Ile Trp Glu Ala Asn Lys Val Ser Thr Ala Phe Thr Pro His Pro Cys
    370                 375                 380

Thr Thr Ile Glu Gln His Met Cys Glu Gly Asp Ser Cys Gly Gly Thr
385                 390                 395                 400

Tyr Ser Asp Asp Arg Tyr Gly Gly Thr Cys Asp Ala Asp Gly Cys Asp
                405                 410                 415

Phe Asn Ser Tyr Arg Met Gly Asn Thr Thr Phe Tyr Gly Glu Gly Lys
            420                 425                 430

Thr Val Asp Thr Ser Ser Lys Phe Thr Val Val Thr Gln Phe Ile Lys
        435                 440                 445

Asp Ser Ala Gly Asp Leu Ala Glu Ile Lys Arg Phe Tyr Val Gln Asn
    450                 455                 460

Gly Lys Val Ile Glu Asn Ser Gln Ser Asn Ile Asp Gly Val Ser Gly
465                 470                 475                 480

Asn Ser Ile Thr Gln Ser Phe Cys Asn Ala Gln Lys Thr Ala Phe Gly
                485                 490                 495

Asp Ile Asp Asp Phe Asn Lys Lys Gly Gly Leu Lys Gln Met Gly Lys
            500                 505                 510

Ala Leu Ala Lys Pro Met Val Leu Val Met Ser Ile Trp Asp Asp His
        515                 520                 525

Ala Ala Asn Met Leu Trp Leu Asp Ser Thr Tyr Pro Val Glu Gly Gly
    530                 535                 540

Ser Pro Gly Ala Tyr Arg Gly Glu Cys Pro Thr Thr Ser Gly Val Pro
545                 550                 555                 560

Ala Asp Val Glu Ala Asn Ala Pro Asn Ser Lys Val Ile Phe Ser Asn
                565                 570                 575

Ile Lys Phe Gly Pro Ile Gly Ser Thr Phe Ser Gly Gly Ser Ser Gly
        580                 585                 590

Thr Thr Pro Ser Asn Pro Ser Ser Ser Val Lys Pro Val Thr Ser Thr
    595                 600                 605

Ala Lys Pro Ser Ser Thr Ser Thr Ala Ser Asn Pro Ser Gly Thr Gly
    610                 615                 620

Ala Ala His Trp Ala Gln Cys Gly Gly Ile Gly Phe Ser Gly Pro Thr
625                 630                 635                 640

Thr Cys Gln Ser Pro Tyr Thr Cys Gln Lys Ile Asn Asp Tyr Tyr Ser
                645                 650                 655

Gln Cys Val

<210>  73
<211>  522
<212>  PRT
<213>  Sordaria macrospora

<400>  73

Met Leu Ala Lys Phe Ala Ala Leu Ala Ala Leu Val Ala Ser Ala Asn
1               5                   10                  15

Ala Gln Ala Val Cys Ser Leu Thr Ala Glu Thr His Pro Ser Leu Asn
            20                  25                  30

Trp Ser Lys Cys Thr Ser Ser Gly Cys Thr Asn Val Ala Gly Ser Val
        35                  40                  45

Thr Ile Asp Ala Asn Trp Arg Trp Thr His Thr Leu Ser Gly Ser Thr
    50                  55                  60

Asn Cys Tyr Asn Gly Asn Lys Trp Asp Thr Thr Ile Cys Ser Thr Asn
65                  70                  75                  80

Thr Asp Cys Ala Thr Lys Cys Cys Val Asp Gly Ala Glu Tyr Ser Ser
                85                  90                  95

Thr Tyr Gly Ile Gln Thr Ser Gly Asn Ser Leu Ser Leu Gln Phe Val
            100                 105                 110

Thr Lys Gly Gln Tyr Ser Thr Asn Ile Gly Ser Arg Thr Tyr Leu Met
        115                 120                 125

Asn Gly Asn Asp Ala Tyr Gln Gly Phe Glu Leu Leu Gly Asn Glu Phe
    130                 135                 140

Thr Phe Asp Val Asp Val Ser Gly Thr Gly Cys Gly Leu Asn Gly Ala
145                 150                 155                 160

Leu Tyr Phe Val Ser Met Asp Leu Asp Gly Gly Lys Ser Lys Tyr Ser
                165                 170                 175

Thr Asn Lys Ala Gly Ala Lys Tyr Gly Thr Gly Tyr Cys Asp Ala Gln
            180                 185                 190

Cys Pro Arg Asp Leu Lys Tyr Ile Asn Gly Val Gly Asn Val Glu Gly
        195                 200                 205

Trp Ser Ser Ser Thr Asn Asp Pro Asn Ala Gly Ile Gly Asn His Gly
    210                 215                 220

Thr Cys Cys Ser Glu Met Asp Ile Trp Glu Ala Asn Lys Val Ser Thr
225                 230                 235                 240

Ala Phe Thr Pro His Pro Cys Thr Thr Ile Asp Gln His Met Cys Glu
                245                 250                 255

Gly Asn Ser Cys Gly Gly Thr Tyr Ser Thr Asp Arg Tyr Gly Gly Thr
            260                 265                 270

Cys Asp Ala Asp Gly Cys Asp Phe Asn Ser Tyr Arg Met Gly Asn Thr
        275                 280                 285

Thr Phe Tyr Gly Glu Gly Lys Thr Val Asp Thr Arg Ser Lys Phe Thr
    290                 295                 300

Val Val Thr Gln Phe Ile Lys Asn Ser Ala Gly Asp Leu Gly Glu Ile
305                 310                 315                 320

Lys Arg Phe Tyr Val Gln Asn Gly Lys Val Ile Glu Asn Ser Gln Ser
                325                 330                 335

Asn Ile Ala Gly Val Ser Gly Asn Ser Ile Thr Gln Ser Phe Cys Asp
            340                 345                 350

Ala Gln Lys Thr Ala Phe Gly Asp Val Gly Asp Phe Asn Val Lys Gly
        355                 360                 365

Gly Leu Lys Gln Met Gly Lys Ala Leu Ala Lys Pro Met Val Leu Val

370                 375                 380

Met Ser Ile Trp Asp Asp His Ala Ala Asn Met Leu Trp Leu Asp Ser
385                 390                 395                 400

Thr Tyr Pro Val Glu Gly Gly Ser Pro Gly Ala Tyr Arg Gly Glu Cys
                405                 410                 415

Pro Thr Thr Ser Gly Val Pro Ala Glu Val Glu Ala Asn Ala Pro Asn
            420                 425                 430

Ser Lys Val Ile Phe Ser Asn Ile Lys Phe Gly Pro Ile Gly Ser Thr
        435                 440                 445

Phe Ser Gly Gly Ser Ser Gly Gly Thr Pro Ser Asn Pro Ser Ser Ser
    450                 455                 460

Val Lys Pro Val Thr Ser Thr Ala Lys Pro Thr Thr Thr Ser Thr Ala
465                 470                 475                 480

Ser Asn Pro Ser Gly Thr Gly Ala Ala His Trp Ala Gln Cys Gly Gly
                485                 490                 495

Ile Gly Phe Asn Gly Pro Thr Thr Cys Gln Ser Pro Tyr Thr Cys Gln
            500                 505                 510

Lys Ile Asn Asp Tyr Tyr Ser Gln Cys Leu
        515                 520

<210>  74
<211>  526
<212>  PRT
<213>  Thielavia terrestris

<400>  74

Met His Ala Lys Phe Ala Thr Leu Ala Ala Leu Val Ala Ser Ala Ala
1               5                   10                  15

Ala Gln Gln Ala Cys Thr Leu Thr Ala Glu Asn His Pro Thr Leu Ser
            20                  25                  30

Trp Ser Lys Cys Thr Ser Gly Gly Ser Cys Thr Ser Val Ser Gly Ser
        35                  40                  45

Val Thr Ile Asp Ala Asn Trp Arg Trp Thr His Gln Val Ser Ser Ser
    50                  55                  60

Thr Asn Cys Tyr Thr Gly Asn Glu Trp Asp Thr Ser Ile Cys Thr Asp

65                  70                  75                  80

Gly Ala Ser Cys Ala Ala Ala Cys Cys Leu Asp Gly Ala Asp Tyr Ser
                85                  90                  95

Gly Thr Tyr Gly Ile Thr Thr Ser Gly Asn Ala Leu Ser Leu Gln Phe
            100                 105                 110

Val Thr Gln Gly Pro Tyr Ser Thr Asn Ile Gly Ser Arg Thr Tyr Leu
        115                 120                 125

Met Ala Ser Asp Thr Lys Tyr Gln Met Phe Thr Leu Leu Gly Asn Glu
    130                 135                 140

Phe Thr Phe Asp Val Asp Val Ser Gly Leu Gly Cys Gly Leu Asn Gly
145                 150                 155                 160

Ala Leu Tyr Phe Val Ser Met Asp Glu Asp Gly Gly Leu Ser Lys Tyr
                165                 170                 175

Ser Gly Asn Lys Ala Gly Ala Lys Tyr Gly Thr Gly Tyr Cys Asp Ser
            180                 185                 190

Gln Cys Pro Arg Asp Leu Lys Phe Ile Asn Gly Glu Ala Asn Asn Val
        195                 200                 205

Gly Trp Thr Pro Ser Ser Asn Asp Lys Asn Ala Gly Leu Gly Asn Tyr
    210                 215                 220

Gly Ser Cys Cys Ser Glu Met Asp Val Trp Glu Ala Asn Ser Ile Ser
225                 230                 235                 240

Ala Ala Tyr Thr Pro His Pro Cys Thr Thr Ile Gly Gln Thr Arg Cys
                245                 250                 255

Glu Gly Asp Asp Cys Gly Gly Thr Tyr Ser Thr Asp Arg Tyr Ala Gly
            260                 265                 270

Glu Cys Asp Pro Asp Gly Cys Asp Phe Asn Ser Tyr Arg Met Gly Asn
        275                 280                 285

Thr Thr Phe Tyr Gly Lys Gly Met Thr Val Asp Thr Ser Lys Lys Phe
    290                 295                 300

Thr Val Val Thr Gln Phe Leu Thr Asp Ser Ser Gly Asn Leu Ser Glu
305                 310                 315                 320

Ile Lys Arg Phe Tyr Val Gln Asn Gly Val Val Ile Pro Asn Ser Asn
                325                 330                 335

Ser Asn Ile Ala Gly Val Ser Gly Asn Ser Ile Thr Gln Ala Phe Cys
            340                 345                 350

Asp Ala Gln Lys Thr Ala Phe Gly Asp Thr Asn Val Phe Asp Gln Lys
        355                 360                 365

Gly Gly Leu Ala Gln Met Gly Lys Ala Leu Ala Gln Pro Met Val Leu
    370                 375                 380

Val Met Ser Leu Trp Asp Asp His Ala Val Asn Met Leu Trp Leu Asp
385                 390                 395                 400

Ser Thr Tyr Pro Thr Asp Ala Ala Gly Lys Pro Gly Ala Ala Arg Gly
                405                 410                 415

Thr Cys Pro Thr Thr Ser Gly Val Pro Ala Asp Val Glu Ser Gln Ala
            420                 425                 430

Pro Asn Ser Lys Val Ile Tyr Ser Asn Ile Arg Phe Gly Pro Ile Gly
        435                 440                 445

Ser Thr Val Ser Gly Leu Pro Gly Gly Gly Ser Asn Pro Gly Gly Gly
    450                 455                 460

Ser Ser Ser Thr Thr Thr Thr Thr Arg Pro Ala Thr Ser Thr Thr Ser
465                 470                 475                 480

Ser Ala Ser Ser Gly Pro Thr Gly Gly Gly Thr Ala Ala His Trp Gly
                485                 490                 495

Gln Cys Gly Gly Ile Gly Trp Thr Gly Pro Thr Val Cys Ala Ser Pro
            500                 505                 510

Tyr Thr Cys Gln Lys Leu Asn Asp Trp Tyr Tyr Gln Cys Leu
        515                 520                 525

<210>  75
<211>  523
<212>  PRT
<213>  Acremonium thermophilum

<400>  75

Met Tyr Thr Lys Phe Ala Ala Leu Ala Ala Leu Val Ala Thr Val Arg
1               5                   10                  15

Gly Gln Ala Ala Cys Ser Leu Thr Ala Glu Thr His Pro Ser Leu Gln
            20                  25                  30

Trp Gln Lys Cys Thr Ala Pro Gly Ser Cys Thr Thr Val Ser Gly Gln
        35                  40                  45

Val Thr Ile Asp Ala Asn Trp Arg Trp Leu His Gln Thr Asn Ser Ser
    50                  55                  60

Thr Asn Cys Tyr Thr Gly Asn Glu Trp Asp Thr Ser Ile Cys Ser Ser
65                  70                  75                  80

Asp Thr Asp Cys Ala Thr Lys Cys Cys Leu Asp Gly Ala Asp Tyr Thr
                85                  90                  95

Gly Thr Tyr Gly Val Thr Ala Ser Gly Asn Ser Leu Asn Leu Lys Phe
            100                 105                 110

Val Thr Gln Gly Pro Tyr Ser Lys Asn Ile Gly Ser Arg Met Tyr Leu
        115                 120                 125

Met Glu Ser Glu Ser Lys Tyr Gln Gly Phe Thr Leu Leu Gly Gln Glu
    130                 135                 140

Phe Thr Phe Asp Val Asp Val Ser Asn Leu Gly Cys Gly Leu Asn Gly
145                 150                 155                 160

Ala Leu Tyr Phe Val Ser Met Asp Leu Asp Gly Gly Val Ser Lys Tyr
                165                 170                 175

Thr Thr Asn Lys Ala Gly Ala Lys Tyr Gly Thr Gly Tyr Cys Asp Ser
            180                 185                 190

Gln Cys Pro Arg Asp Leu Lys Phe Ile Asn Gly Gln Ala Asn Ile Asp
        195                 200                 205

Gly Trp Gln Pro Ser Ser Asn Asp Ala Asn Ala Gly Leu Gly Asn His
    210                 215                 220

Gly Ser Cys Cys Ser Glu Met Asp Ile Trp Glu Ala Asn Lys Val Ser
225                 230                 235                 240

Ala Ala Tyr Thr Pro His Pro Cys Thr Thr Ile Gly Gln Thr Met Cys
                245                 250                 255

Thr Gly Asp Asp Cys Gly Gly Thr Tyr Ser Ser Asp Arg Tyr Ala Gly
            260                 265                 270

Ile Cys Asp Pro Asp Gly Cys Asp Phe Asn Ser Tyr Arg Met Gly Asp
        275                 280                 285

Thr Ser Phe Tyr Gly Pro Gly Lys Thr Val Asp Thr Gly Ser Lys Phe
    290                 295                 300

Thr Val Val Thr Gln Phe Leu Thr Gly Ser Asp Gly Asn Leu Ser Glu
305                 310                 315                 320

Ile Lys Arg Phe Tyr Val Gln Asn Gly Lys Val Ile Pro Asn Ser Glu
                325                 330                 335

Ser Lys Ile Ala Gly Val Ser Gly Asn Ser Ile Thr Thr Asp Phe Cys
            340                 345                 350

Thr Ala Gln Lys Thr Ala Phe Gly Asp Thr Asn Val Phe Glu Glu Arg
        355                 360                 365

Gly Gly Leu Ala Gln Met Gly Lys Ala Leu Ala Glu Pro Met Val Leu
    370                 375                 380

Val Leu Ser Val Trp Asp Asp His Ala Val Asn Met Leu Trp Leu Asp
385                 390                 395                 400

Ser Thr Tyr Pro Thr Asp Ser Thr Lys Pro Gly Ala Ala Arg Gly Asp
                405                 410                 415

Cys Pro Ile Thr Ser Gly Val Pro Ala Asp Val Glu Ser Gln Ala Pro
            420                 425                 430

Asn Ser Asn Val Ile Tyr Ser Asn Ile Arg Phe Gly Pro Ile Asn Ser
        435                 440                 445

Thr Tyr Thr Gly Thr Pro Ser Gly Gly Asn Pro Pro Gly Gly Gly Thr
    450                 455                 460

Thr Thr Thr Thr Thr Thr Thr Thr Ser Lys Pro Ser Gly Pro Thr Thr
465                 470                 475                 480

Thr Thr Asn Pro Ser Gly Pro Gln Gln Thr His Trp Gly Gln Cys Gly
                485                 490                 495

Gly Gln Gly Trp Thr Gly Pro Thr Val Cys Gln Ser Pro Tyr Thr Cys
            500                 505                 510

Lys Tyr Ser Asn Asp Trp Tyr Ser Gln Cys Leu
        515                 520

<210>  76
<211>  526
<212>  PRT
<213>  Thielavia microspora

<400>  76

Met Tyr Gly Lys Lys Phe Ala Thr Leu Ala Ala Leu Val Ala Ser Ala
1               5                   10                  15

Thr Ala Gln Gln Val Cys Thr Leu Thr Thr Glu Thr His Pro Ser Leu
            20                  25                  30

Ser Trp Ser Arg Cys Thr Ser Gly Gly Ser Cys Thr Ser Val Ser Gly
        35                  40                  45

Ser Ile Val Ile Asp Ala Asn Trp Arg Trp Thr His Gln Leu Gln Gly
    50                  55                  60

Thr Thr Asn Cys Tyr Asn Gly Asn Glu Trp Asp Thr Ser Ile Cys Thr
65                  70                  75                  80

Asp Asp Gln Ser Cys Ala Ala Asn Cys Cys Val Asp Gly Ala Glu Tyr
                85                  90                  95

Ser Ser Thr Tyr Gly Ile Thr Thr Ser Gly Asn Ala Leu Asn Leu Lys
            100                 105                 110

Phe Val Thr Lys Gly Thr Tyr Ser Thr Asn Ile Gly Ser Arg Thr Tyr
        115                 120                 125

Leu Met Glu Ser Ala Thr Lys Tyr Gln Met Phe Asn Leu Leu Gly Asn
    130                 135                 140

Glu Phe Thr Phe Asp Val Asp Val Ser Asn Leu Gly Cys Gly Leu Asn
145                 150                 155                 160

Gly Ala Leu Tyr Phe Val Ser Met Asp Ala Asp Gly Gly Leu Ser Lys
                165                 170                 175

Tyr Ser Gly Asn Lys Ala Gly Ala Lys Tyr Gly Thr Gly Tyr Cys Asp
            180                 185                 190

Ser Gln Cys Ser Arg Asp Leu Lys Phe Ile Asn Gly Leu Ala Asn Val
        195                 200                 205

Glu Gly Trp Ser Gly Ser Thr Asn Asp Pro Asn Ala Gly Leu Gly His
    210                 215                 220

Met Gly Thr Cys Cys Ser Glu Met Asp Ile Trp Glu Ala Asn Lys Val
225                 230                 235                 240

Ser Thr Ala Phe Thr Pro His Pro Cys Thr Thr Ile Gly Gln Glu Ala
                245                 250                 255

Cys Glu Gly Asp Ser Cys Gly Gly Thr Tyr Ser Ser Asp Arg Tyr Gly
            260                 265                 270

Gly Thr Cys Asp Pro Asp Gly Cys Asp Phe Asn Ser Tyr Arg Gln Gly
        275                 280                 285

Asp Lys Thr Phe Tyr Gly Glu Gly Lys Thr Val Asp Thr Thr Lys Lys
    290                 295                 300

Phe Thr Val Val Thr Gln Phe Leu Lys Asn Ala Ala Gly Glu Leu Ser
305                 310                 315                 320

Glu Ile Lys Arg Phe Tyr Ala Gln Asn Gly Val Ile Ile Pro Asn Ser
                325                 330                 335

Glu Ser Lys Ile Ala Gly Val Pro Gly Asn Ser Ile Thr Gln Asp Tyr
            340                 345                 350

Cys Asp Ala Gln Lys Ala Ala Phe Gly Asp Ile Asp Asp Phe Ser Ala
        355                 360                 365

Lys Gly Gly Leu Val Gln Met Gly Lys Ala Leu Glu Ser Met Val Leu
    370                 375                 380

Val Met Ser Val Trp Asp Asp His Ala Ala Asn Met Leu Trp Leu Asp
385                 390                 395                 400

Ser Thr Tyr Pro Val Asp Gly Ala Gly Val Pro Gly Ala Glu Arg Gly
                405                 410                 415

Glu Cys Ala Thr Thr Ser Gly Val Pro Ala Glu Val Glu Ala Gln Ala
            420                 425                 430

Pro Asn Ser Asn Val Ile Phe Ser Asn Ile Arg Phe Gly Pro Ile Asn
        435                 440                 445

Ser Thr Val Pro Gly Leu Gly Ser Val Gly Gly Gly Asn Pro Pro Val
    450                 455                 460

Ser Ser Ser Thr Arg Leu Pro Ala Thr Thr Ser Thr Arg Thr Ser Thr

465                 470                 475                 480

Ser Ala Ser Ser Thr Pro Thr Asn Gly Gly Thr Ala Gly His Trp Met
                485                 490                 495

Gln Cys Gly Gly Gln Gly Tyr Thr Gly Pro Thr Arg Cys Glu Ser Pro
            500                 505                 510

Tyr Asn Cys Val Tyr Ser Asn Pro Trp Tyr Ser Gln Cys Leu
        515                 520                 525

<210>  77
<211>  526
<212>  PRT
<213>  Myceliophthora thermophila

<400>  77

Met Tyr Ala Lys Phe Ala Thr Leu Ala Ala Leu Val Ala Gly Ala Ala
1               5                   10                  15

Ala Gln Asn Ala Cys Thr Leu Thr Ala Glu Asn His Pro Ser Leu Thr
            20                  25                  30

Trp Ser Lys Cys Thr Ser Gly Gly Ser Cys Thr Ser Val Gln Gly Ser
        35                  40                  45

Ile Thr Ile Asp Ala Asn Trp Arg Trp Thr His Arg Thr Asp Ser Ala
    50                  55                  60

Thr Asn Cys Tyr Glu Gly Asn Lys Trp Asp Thr Ser Tyr Cys Ser Asp
65                  70                  75                  80

Gly Pro Ser Cys Ala Ser Lys Cys Cys Ile Asp Gly Ala Asp Tyr Ser
                85                  90                  95

Ser Thr Tyr Gly Ile Thr Thr Ser Gly Asn Ser Leu Asn Leu Lys Phe
            100                 105                 110

Val Thr Lys Gly Gln Tyr Ser Thr Asn Ile Gly Ser Arg Thr Tyr Leu
        115                 120                 125

Met Glu Ser Asp Thr Lys Tyr Gln Met Phe Gln Leu Leu Gly Asn Glu
    130                 135                 140

Phe Thr Phe Asp Val Asp Val Ser Asn Leu Gly Cys Gly Leu Asn Gly
145                 150                 155                 160

Ala Leu Tyr Phe Val Ser Met Asp Ala Asp Gly Gly Met Ser Lys Tyr

                165                 170                 175

Ser Gly Asn Lys Ala Gly Ala Lys Tyr Gly Thr Gly Tyr Cys Asp Ser
            180                 185                 190

Gln Cys Pro Arg Asp Leu Lys Phe Ile Asn Gly Glu Ala Asn Val Glu
        195                 200                 205

Asn Trp Gln Ser Ser Thr Asn Asp Ala Asn Ala Gly Thr Gly Lys Tyr
    210                 215                 220

Gly Ser Cys Cys Ser Glu Met Asp Val Trp Glu Ala Asn Asn Met Ala
225                 230                 235                 240

Ala Ala Phe Thr Pro His Pro Cys Thr Val Ile Gly Gln Ser Arg Cys
                245                 250                 255

Glu Gly Asp Ser Cys Gly Gly Thr Tyr Ser Thr Asp Arg Tyr Ala Gly
            260                 265                 270

Ile Cys Asp Pro Asp Gly Cys Asp Phe Asn Ser Tyr Arg Gln Gly Asn
        275                 280                 285

Lys Thr Phe Tyr Gly Lys Gly Met Thr Val Asp Thr Thr Lys Lys Ile
    290                 295                 300

Thr Val Val Thr Gln Phe Leu Lys Asn Ser Ala Gly Glu Leu Ser Glu
305                 310                 315                 320

Ile Lys Arg Phe Tyr Val Gln Asn Gly Lys Val Ile Pro Asn Ser Glu
                325                 330                 335

Ser Thr Ile Pro Gly Val Glu Gly Asn Ser Ile Thr Gln Asp Trp Cys
            340                 345                 350

Asp Arg Gln Lys Ala Ala Phe Gly Asp Val Thr Asp Phe Gln Asp Lys
        355                 360                 365

Gly Gly Met Val Gln Met Gly Lys Ala Leu Ala Gly Pro Met Val Leu
    370                 375                 380

Val Met Ser Ile Trp Asp Asp His Ala Val Asn Met Leu Trp Leu Asp
385                 390                 395                 400

Ser Thr Trp Pro Ile Asp Gly Ala Gly Lys Pro Gly Ala Glu Arg Gly
                405                 410                 415

Ala Cys Pro Thr Thr Ser Gly Val Pro Ala Glu Val Glu Ala Glu Ala
        420                 425                 430

Pro Asn Ser Asn Val Ile Phe Ser Asn Ile Arg Phe Gly Pro Ile Gly
        435                 440                 445

Ser Thr Val Ser Gly Leu Pro Asp Gly Gly Ser Gly Asn Pro Asn Pro
    450                 455                 460

Pro Val Ser Ser Ser Thr Pro Val Pro Ser Ser Ser Thr Thr Ser Ser
465                 470                 475                 480

Gly Ser Ser Gly Pro Thr Gly Gly Thr Gly Val Ala Lys His Tyr Glu
                485                 490                 495

Gln Cys Gly Gly Ile Gly Phe Thr Gly Pro Thr Gln Cys Glu Ser Pro
        500                 505                 510

Tyr Thr Cys Thr Lys Leu Asn Asp Trp Tyr Ser Gln Cys Leu
        515                 520                 525

<210>  78
<211>  525
<212>  PRT
<213>  Humicola grisea var. thermoida

<400>  78

Met Arg Thr Ala Lys Phe Ala Thr Leu Ala Ala Leu Val Ala Ser Ala
1               5                   10                  15

Ala Ala Gln Gln Ala Cys Ser Leu Thr Thr Glu Arg His Pro Ser Leu
            20                  25                  30

Ser Trp Lys Lys Cys Thr Ala Gly Gly Gln Cys Gln Thr Val Gln Ala
        35                  40                  45

Ser Ile Thr Leu Asp Ser Asn Trp Arg Trp Thr His Gln Val Ser Gly
    50                  55                  60

Ser Thr Asn Cys Tyr Thr Gly Asn Lys Trp Asp Thr Ser Ile Cys Thr
65                  70                  75                  80

Asp Ala Lys Ser Cys Ala Gln Asn Cys Cys Val Asp Gly Ala Asp Tyr
                85                  90                  95

Thr Ser Thr Tyr Gly Ile Thr Thr Asn Gly Asp Ser Leu Ser Leu Lys
            100                 105                 110

Phe Val Thr Lys Gly Gln Tyr Ser Thr Asn Val Gly Ser Arg Thr Tyr
        115                 120                 125

Leu Met Asp Gly Glu Asp Lys Tyr Gln Thr Phe Glu Leu Leu Gly Asn
    130                 135                 140

Glu Phe Thr Phe Asp Val Asp Val Ser Asn Ile Gly Cys Gly Leu Asn
145                 150                 155                 160

Gly Ala Leu Tyr Phe Val Ser Met Asp Ala Asp Gly Gly Leu Ser Arg
                165                 170                 175

Tyr Pro Gly Asn Lys Ala Gly Ala Lys Tyr Gly Thr Gly Tyr Cys Asp
            180                 185                 190

Ala Gln Cys Pro Arg Asp Ile Lys Phe Ile Asn Gly Glu Ala Asn Ile
        195                 200                 205

Glu Gly Trp Thr Gly Ser Thr Asn Asp Pro Asn Ala Gly Ala Gly Arg
    210                 215                 220

Tyr Gly Thr Cys Cys Ser Glu Met Asp Ile Trp Glu Ala Asn Asn Met
225                 230                 235                 240

Ala Thr Ala Phe Thr Pro His Pro Cys Thr Ile Ile Gly Gln Ser Arg
                245                 250                 255

Cys Glu Gly Asp Ser Cys Gly Gly Thr Tyr Ser Asn Glu Arg Tyr Ala
            260                 265                 270

Gly Val Cys Asp Pro Asp Gly Cys Asp Phe Asn Ser Tyr Arg Gln Gly
        275                 280                 285

Asn Lys Thr Phe Tyr Gly Lys Gly Met Thr Val Asp Thr Thr Lys Lys
    290                 295                 300

Ile Thr Val Val Thr Gln Phe Leu Lys Asp Ala Asn Gly Asp Leu Gly
305                 310                 315                 320

Glu Ile Lys Arg Phe Tyr Val Gln Asp Gly Lys Ile Ile Pro Asn Ser
                325                 330                 335

Glu Ser Thr Ile Pro Gly Val Glu Gly Asn Ser Ile Thr Gln Asp Trp
            340                 345                 350

Cys Asp Arg Gln Lys Val Ala Phe Gly Asp Ile Asp Asp Phe Asn Arg
        355                 360                 365

Lys Gly Gly Met Lys Gln Met Gly Lys Ala Leu Ala Gly Pro Met Val
    370                 375                 380

Leu Val Met Ser Ile Trp Asp Asp His Ala Ser Asn Met Leu Trp Leu
385                 390                 395                 400

Asp Ser Thr Phe Pro Val Asp Ala Ala Gly Lys Pro Gly Ala Glu Arg
                405                 410                 415

Gly Ala Cys Pro Thr Thr Ser Gly Val Pro Ala Glu Val Glu Ala Glu
            420                 425                 430

Ala Pro Asn Ser Asn Val Val Phe Ser Asn Ile Arg Phe Gly Pro Ile
        435                 440                 445

Gly Ser Thr Val Ala Gly Leu Pro Gly Ala Gly Asn Gly Gly Asn Asn
    450                 455                 460

Gly Gly Asn Pro Pro Pro Pro Thr Thr Thr Thr Ser Ser Ala Pro Ala
465                 470                 475                 480

Thr Thr Thr Thr Ala Ser Ala Gly Pro Lys Ala Gly Arg Trp Gln Gln
                485                 490                 495

Cys Gly Gly Ile Gly Phe Thr Gly Pro Thr Gln Cys Glu Glu Pro Tyr
            500                 505                 510

Thr Cys Thr Lys Leu Asn Asp Trp Tyr Ser Gln Cys Leu
        515                 520                 525

<210>  79
<211>  530
<212>  PRT
<213>  Chaetomium luteum

<400>  79

Met Tyr Ala Lys Phe Ala Thr Leu Ala Ala Leu Val Ala Ser Ala Ala
1               5                   10                  15

Ala Gln Gln Ala Cys Thr Leu Thr Ala Glu Thr His Pro Pro Leu Thr
            20                  25                  30

Trp Gln Lys Cys Ser Ala Gly Gly Ser Cys Thr Ser Val Gln Gly Ser
        35                  40                  45

Val Val Leu Asp Ala Asn Trp Arg Trp Thr His Gln Thr Ser Ser Ala
    50                  55                  60

Thr Asn Cys Tyr Ser Gly Asn Glu Trp Asp Thr Ser Ile Cys Thr Asp
65                  70                  75                  80

Gly Pro Ser Cys Ala Ser Lys Cys Cys Val Asp Gly Ala Asp Tyr Ser
                85                  90                  95

Gly Thr Tyr Gly Ile Thr Thr Ser Gly Asn Ser Leu Asn Leu Lys Phe
            100                 105                 110

Val Thr Lys Gly Pro Tyr Ser Thr Asn Val Gly Ser Arg Thr Tyr Leu
        115                 120                 125

Met Glu Ser Asp Thr Lys Tyr Gln Met Phe Thr Leu Leu Gly Asn Glu
    130                 135                 140

Phe Thr Phe Asp Val Asp Val Ser Asn Ile Gly Cys Gly Leu Asn Gly
145                 150                 155                 160

Ala Leu Tyr Phe Val Thr Met Asp Ala Asp Gly Gly Leu Ser Lys Tyr
                165                 170                 175

Thr Gly Asn Lys Ala Gly Ala Lys Tyr Gly Thr Gly Tyr Cys Asp Ser
            180                 185                 190

Gln Cys Pro Arg Asp Ile Lys Phe Ile Asn Gly Glu Ala Asn Val Glu
        195                 200                 205

Gly Trp Ser Gly Ser Thr Asn Asp Ala Asn Ala Gly Ala Gly Lys Tyr
    210                 215                 220

Gly Ser Cys Cys Asp Glu Met Asp Ile Trp Glu Ala Asn Asn Met Ala
225                 230                 235                 240

Thr Ala Phe Thr Pro His Pro Cys Thr Thr Ile Gly Gln Thr Arg Cys
                245                 250                 255

Glu Gly Glu Ser Cys Gly Gly Thr Tyr Ser Ser Asp Arg Tyr Gly Gly
            260                 265                 270

Ile Cys Asp Pro Asp Gly Cys Asp Phe Asn Ala Tyr Arg Gln Gly Asn
        275                 280                 285

Lys Thr Phe Tyr Gly Lys Gly Met Thr Val Asp Thr Ser Lys Lys Leu
    290                 295                 300

Thr Val Val Thr Gln Phe Leu Thr Asp Ala Ser Gly Asn Leu Ser Glu
305                 310                 315                 320

Ile Lys Arg Phe Tyr Ala Gln Asn Gly Val Val Ile Pro Asn Ser Glu
                325                 330                 335

Ser Thr Ile Ala Gly Val Pro Gly Asn Ser Ile Thr Gln Asp Tyr Cys
            340                 345                 350

Asp Lys Gln Lys Thr Ala Phe Gly Asp Val Asn Asp Phe Asn Lys Lys
        355                 360                 365

Gly Gly Leu Ala Gln Met Gly Lys Ala Leu Ala Lys Pro Met Val Leu
    370                 375                 380

Val Met Ser Ile Trp Asp Asp His Ala Ala Asn Met Leu Trp Leu Asp
385                 390                 395                 400

Ser Thr Tyr Pro Val Asp Ala Ala Gly Lys Pro Gly Ala Glu Arg Gly
                405                 410                 415

Ala Cys Pro Thr Thr Ser Gly Val Pro Ala Glu Val Glu Ala Gln Val
            420                 425                 430

Pro Asn Ser Asn Val Val Phe Ser Asn Ile Lys Phe Gly Pro Ile Gly
        435                 440                 445

Ser Thr Val Ser Gly Leu Pro Asn Gly Gly Gly Ser Pro Gly Gly Gly
    450                 455                 460

Ser Ser Ser Ser Ser Ala Arg Pro Ser Ser Thr Ser Thr Ala Arg Pro
465                 470                 475                 480

Ser Ser Thr Ser Thr Ser Ala Gly Gly Gly Pro Thr Gly Cys Thr Ala
                485                 490                 495

Ala Lys Tyr Ala Gln Cys Gly Gly Asn Gly Phe Thr Gly Cys Thr Asn
            500                 505                 510

Cys Pro Ala Gly Thr Thr Cys Arg Lys Gln Asn Asp Trp Tyr Ser Gln
        515                 520                 525

Cys Leu
    530

<210>  80
<211>  528
<212>  PRT
<213>  Podospora anserina

<400>  80

Met Val Ser Ala Lys Phe Ala Ala Leu Ala Ala Leu Val Ala Ser Ala
1               5                   10                  15

Ser Ala Gln Gln Val Cys Ser Leu Thr Pro Glu Ser His Pro Pro Leu
            20                  25                  30

Thr Trp Gln Arg Cys Ser Ala Gly Gly Ser Cys Thr Asn Val Ala Gly
        35                  40                  45

Ser Val Thr Leu Asp Ser Asn Trp Arg Trp Thr His Thr Leu Gln Gly
    50                  55                  60

Ser Thr Asn Cys Tyr Ser Gly Asn Glu Trp Asp Thr Ser Ile Cys Thr
65                  70                  75                  80

Thr Gly Thr Lys Cys Ala Gln Asn Cys Cys Val Glu Gly Ala Glu Tyr
                85                  90                  95

Ala Ala Thr Tyr Gly Ile Thr Thr Ser Gly Asn Gln Leu Asn Leu Lys
            100                 105                 110

Phe Val Thr Glu Gly Lys Tyr Ser Thr Asn Val Gly Ser Arg Thr Tyr
        115                 120                 125

Leu Met Glu Asn Ala Thr Lys Tyr Gln Gly Phe Asn Leu Leu Gly Asn
    130                 135                 140

Glu Phe Thr Phe Asp Val Asp Val Ser Asn Ile Gly Cys Gly Leu Asn
145                 150                 155                 160

Gly Ala Leu Tyr Phe Val Ser Met Asp Leu Asp Gly Gly Leu Ala Lys
                165                 170                 175

Tyr Ser Gly Asn Lys Ala Gly Ala Lys Tyr Gly Thr Gly Tyr Cys Asp
            180                 185                 190

Ala Gln Cys Pro Arg Asp Ile Lys Phe Ile Asn Gly Glu Ala Asn Ile
        195                 200                 205

Glu Gly Trp Asn Pro Ser Thr Asn Asp Val Asn Ala Gly Ala Gly Arg
    210                 215                 220

Tyr Gly Thr Cys Cys Ser Glu Met Asp Ile Trp Glu Ala Asn Asn Met
225                 230                 235                 240

Ala Thr Ala Tyr Thr Pro His Ser Cys Thr Ile Leu Asp Gln Ser Arg

245                 250                 255

Cys Glu Gly Glu Ser Cys Gly Gly Thr Tyr Ser Ser Asp Arg Tyr Gly
            260                 265                 270

Gly Val Cys Asp Pro Asp Gly Cys Asp Phe Asn Ser Tyr Arg Met Gly
        275                 280                 285

Asn Lys Glu Phe Tyr Gly Lys Gly Lys Thr Val Asp Thr Thr Lys Lys
    290                 295                 300

Met Thr Val Val Thr Gln Phe Leu Lys Asn Ala Ala Gly Glu Leu Ser
305                 310                 315                 320

Glu Ile Lys Arg Phe Tyr Val Gln Asn Gly Val Val Ile Pro Asn Ser
                325                 330                 335

Val Ser Ser Ile Pro Gly Val Pro Asn Gln Asn Ser Ile Thr Gln Asp
            340                 345                 350

Trp Cys Asp Ala Gln Lys Ile Ala Phe Gly Asp Pro Asp Asp Asn Thr
        355                 360                 365

Ala Lys Gly Gly Leu Arg Gln Met Gly Leu Ala Leu Asp Lys Pro Met
    370                 375                 380

Val Leu Val Met Ser Ile Trp Asn Asp His Ala Ala His Met Leu Trp
385                 390                 395                 400

Leu Asp Ser Thr Tyr Pro Val Asp Ala Ala Gly Arg Pro Gly Ala Glu
                405                 410                 415

Arg Gly Ala Cys Pro Thr Thr Ser Gly Val Pro Ser Glu Val Glu Ala
            420                 425                 430

Glu Ala Pro Asn Ser Asn Val Ala Phe Ser Asn Ile Lys Phe Gly Pro
        435                 440                 445

Ile Gly Ser Thr Phe Asn Ser Gly Ser Thr Asn Pro Asn Pro Ile Ser
    450                 455                 460

Ser Ser Thr Ala Thr Thr Pro Thr Ser Thr Arg Val Ser Ser Thr Ser
465                 470                 475                 480

Thr Ala Ala Gln Thr Pro Thr Ser Ala Pro Gly Gly Thr Val Pro Arg
                485                 490                 495

Trp Gly Gln Cys Gly Gly Gln Gly Tyr Thr Gly Pro Thr Gln Cys Val
            500                 505                 510

Ala Pro Tyr Thr Cys Val Val Ser Asn Gln Trp Tyr Ser Gln Cys Leu
        515                 520                 525

<210> 81
<211> 516
<212> PRT
<213> Scedosporium apiospermum

<400> 81

Met Phe Lys Lys Leu Ser Ala Leu Ser Met Ile Leu Gly Ala Val Ser
1               5                   10                  15

Ala Gln Gln Val Cys Thr Gln Thr Pro Glu Thr His Pro Ser Leu Thr
            20                  25                  30

Trp Gln Lys Cys Thr Ala Gly Gly Ser Cys Ser Asn Val Ala Gly Ser
        35                  40                  45

Val Val Ile Asp Ser Asn Trp Arg Trp Thr His Thr Val Ser Gly Ser
    50                  55                  60

Thr Asn Cys Tyr Asp Gly Asn Thr Trp Asp Ser Ser Ile Cys Ser Ser
65                  70                  75                  80

Gly Ala Thr Cys Ala Ser Gln Cys Cys Leu Glu Gly Ala Asp Tyr Thr
                85                  90                  95

Thr Thr Tyr Gly Ile Thr Ala Ser Gly Asn Ser Leu Asp Leu Lys Phe
            100                 105                 110

Val Thr Lys His Gln Tyr Gly Thr Asn Ile Gly Ser Arg Val Tyr Leu
        115                 120                 125

Met Glu Asn Asp Thr Lys Tyr Gln Met Phe Asn Leu Leu Gly Asn Glu
    130                 135                 140

Phe Thr Phe Asp Val Asp Val Ser Asn Ile Gly Cys Gly Leu Asn Gly
145                 150                 155                 160

Ala Leu Tyr Phe Val Ala Met Asp Gln Asp Gly Gly Met Ser Arg Tyr
                165                 170                 175

Ser Gly Asn Lys Ala Gly Ala Lys Tyr Gly Thr Gly Tyr Cys Asp Ala
            180                 185                 190

Gln Cys Pro Arg Asp Ile Lys Phe Ile Asn Gly Val Ala Asn Ser Glu
        195                 200                 205

Gly Trp Glu Pro Ser Ser Asn Asp Ala Asn Ala Gly Val Gly Gly Met
    210                 215                 220

Gly Ser Cys Cys Ala Glu Met Asp Ile Trp Glu Ala Asn Asn Ile Ala
225                 230                 235                 240

Thr Ala Tyr Thr Pro His Pro Cys Thr Asn Asn Ala Gln His Ser Cys
                245                 250                 255

Thr Gly Asp Ser Cys Gly Gly Thr Tyr Ser Asp Asp Arg Tyr Gly Gly
            260                 265                 270

Thr Cys Asp Pro Asp Gly Cys Asp Phe Asn Ser Tyr Arg Gln Gly Asn
        275                 280                 285

Lys Thr Phe Tyr Gly Lys Gly Met Thr Val Asp Thr Thr Lys Lys Met
    290                 295                 300

Thr Val Val Thr Gln Phe Leu Thr Asn Ser Ala Gly Thr Leu Ser Glu
305                 310                 315                 320

Ile Lys Arg Phe Tyr Val Gln Asn Gly Val Val Ile Pro Asn Ser Glu
                325                 330                 335

Ser Val Ile Pro Gly Val Pro Gly Asn Ser Ile Thr Gln Glu Phe Cys
            340                 345                 350

Asp Asn Gln Lys Thr Val Phe Gly Asp Val Asp Thr Phe Asn Ser Arg
        355                 360                 365

Gly Gly Phe Ala Gln Met Ser Lys Ala Leu Gln Gly Ser Met Val Leu
    370                 375                 380

Val Met Ser Leu Trp Asp Asp His Tyr Ala Asn Met Leu Trp Leu Asp
385                 390                 395                 400

Ser Thr Tyr Pro Val Asp Gly Thr Gly Tyr Gly Thr Glu Arg Gly Ser
                405                 410                 415

Cys Pro Thr Ser Ser Gly Val Pro Ser Glu Ile Glu Ala Ser Val Pro
            420                 425                 430

Asn Ser Asn Val Ile Phe Ser Asn Ile Lys Phe Gly Pro Ile Gly Ser
        435                 440                 445

Thr Phe Asn Gln Asn Gly Gly Gly Asn Pro Gln Pro Ser Ser Ser Ala
    450                 455                 460

Thr Val Arg Pro Thr Ser Thr Thr Ser Ala Thr Asn Pro Gly Gln Thr
465                 470                 475                 480

Gly Ala Asp His Trp Gly Gln Cys Gly Gly Ile Gly Trp Thr Gly Pro
                485                 490                 495

Thr Thr Cys Lys Ser Pro Tyr Thr Cys Thr Lys Leu Asn Asp Trp Tyr
            500                 505                 510

Ser Gln Cys Leu
        515


<210>  82
<211>  569
<212>  PRT
<213>  Togninia minima

<400>  82

Met Asn Met Thr His Ala Gly Leu Leu Ile Gly Pro Leu Val Thr Gly
1               5                   10                  15

Glu Ala Ile Ser Met Ala Pro Ser Val Trp Ala Gly Ile Pro Gly Leu
            20                  25                  30

Arg Thr Leu Ala Phe Arg Arg Tyr Thr Asp Thr Met Tyr Arg Lys Ile
        35                  40                  45

Ala Thr Leu Ser Ala Leu Val Ala Ala Ala Ser Ala Gln Gln Val Cys
    50                  55                  60

Thr Leu Thr Ala Glu Thr His Pro Thr Met Ser Trp Ser Lys Cys Ala
65                  70                  75                  80

Ala Gly Gly Thr Cys Thr Thr Val Ser Gly Ser Val Thr Ile Asp Ala
                85                  90                  95

Asn Trp Arg Trp Leu His Gln Thr Ser Thr Ser Thr Asn Cys Tyr Asp
            100                 105                 110

Gly Asn Glu Trp Asp Thr Ser Tyr Cys Ser Thr Gly Ala Asp Cys Ala
        115                 120                 125

Ala Lys Cys Cys Val Asp Gly Ser Asp Tyr Ser Gly Thr Tyr Gly Val
    130                 135                 140

Thr Thr Ser Gly Asn Ala Leu Asn Leu Lys Phe Val Thr Gln Gly Pro
145                 150                 155                 160

Tyr Ser Thr Asn Ile Gly Ser Arg Met Tyr Leu Met Glu Ser Asp Thr
                165                 170                 175

Lys Tyr Gln Met Phe Thr Leu Leu Gly Asn Glu Phe Thr Phe Asp Val
            180                 185                 190

Asp Met Ser Asn Leu Gly Cys Gly Leu Asn Gly Ala Leu Tyr Phe Val
        195                 200                 205

Ser Met Asp Ser Asp Gly Gly Ala Ser Lys Tyr Thr Thr Asn Lys Ala
    210                 215                 220

Gly Ala Lys Tyr Gly Thr Gly Tyr Cys Asp Ser Gln Cys Pro Arg Asp
225                 230                 235                 240

Leu Lys Phe Ile Asn Gly Ile Ala Asn Val Glu Gly Trp Glu Pro Ser
                245                 250                 255

Ser Asn Asp Ala Asn Ala Gly Ile Gly Asn Met Gly Ser Cys Cys Ser
            260                 265                 270

Glu Met Asp Ile Trp Glu Ala Asn Ser Val Ala Ala Ala Tyr Thr Pro
        275                 280                 285

His Pro Cys Gln Thr Ile Gly Gln Glu Met Cys Thr Gly Asp Ala Cys
    290                 295                 300

Gly Gly Thr Tyr Ser Ser Asp Arg Tyr Gly Gly Glu Cys Asp Pro Asp
305                 310                 315                 320

Gly Cys Asp Phe Asn Ser Tyr Arg Gln Gly Asn Thr Thr Phe Leu Gly
                325                 330                 335

Lys Gly Ser Ser Tyr Thr Ile Asp Thr Ser Gln Lys Ile Thr Val Val
            340                 345                 350

Thr Gln Phe Ile Thr Ser Gly Ser Thr Leu Ser Glu Ile Lys Arg Phe
        355                 360                 365

Tyr Val Gln Asn Gly Lys Val Ile Pro Asn Ser Glu Ser Thr Ile Ser
    370                 375                 380

Gly Val Pro Gly Asn Ser Ile Thr Thr Ala Tyr Cys Asp Ala Gln Lys
385                 390                 395                 400

Val Ala Phe Gly Asp Thr Asn Val Phe Glu Gln Lys Gly Gly Leu Ala
                405                 410                 415

Gln Met Gly Ala Ala Leu Ala Gln Pro Met Val Leu Val Met Ser Leu
            420                 425                 430

Trp Asp Asp His Tyr Ala Asn Met Leu Trp Leu Asp Ser Thr Tyr Pro
        435                 440                 445

Val Gly Ser Thr Lys Pro Gly Ala Ala Arg Gly Thr Cys Ala Thr Thr
    450                 455                 460

Ser Gly Val Pro Ser Asp Ile Glu Lys Ser Ala Gly Ser Ser Asn Val
465                 470                 475                 480

Ile Tyr Ser Asn Ile Lys Phe Gly Pro Ile Gly Ser Thr Phe Asn Ser
                485                 490                 495

Ala Gly Thr Gly Gly Gly Ser Ser Ser Ser Thr Thr Ala Arg Val Ser
            500                 505                 510

Ser Ser Thr Thr Arg Thr Ser Ala Thr Thr Ser Ser Thr Ser Gly Ser
        515                 520                 525

Gly Ser Gly Ser Gly Thr Ala Ala His Trp Ala Gln Cys Gly Gly Ile
    530                 535                 540

Gly Tyr Thr Gly Pro Thr Thr Cys Ala Ser Pro Tyr Thr Cys Thr Tyr
545                 550                 555                 560

Ser Asn Ala Tyr Tyr Ser Gln Cys Leu
                565

<210>  83
<211>  514
<212>  PRT
<213>  Gibberella moniliformis

<400>  83

Met Tyr Arg Val Ile Ala Thr Ala Ser Ala Leu Ile Ala Ala Ala Arg
1               5                   10                  15

Ala Gln Gln Val Cys Ser Leu Thr Thr Glu Thr Lys Pro Ala Leu Thr
            20                  25                  30

Trp Ser Lys Cys Thr Ser Ser Gly Cys Ser Asp Val Lys Gly Ser Val
        35                  40                  45

Gly Ile Asp Ala Asn Trp Arg Trp Thr His Gln Thr Ser Gly Ser Thr
    50                  55                  60

Asn Cys Tyr Thr Gly Asn Lys Trp Asp Thr Ser Ile Cys Thr Asp Gly
65                  70                  75                  80

Lys Thr Cys Ala Glu Lys Cys Cys Leu Asp Gly Ala Asp Tyr Ala Gly
                85                  90                  95

Thr Tyr Gly Ile Thr Ser Ser Gly Asn Gln Leu Ser Leu Gly Phe Val
            100                 105                 110

Thr Gln Gly Pro Tyr Ser Lys Asn Ile Gly Ser Arg Thr Tyr Leu Met
        115                 120                 125

Glu Asn Glu Asn Thr Tyr Gln Met Phe Gln Leu Leu Gly Asn Glu Phe
    130                 135                 140

Thr Phe Asp Val Asp Val Ser Gly Ile Gly Cys Gly Leu Asn Gly Ala
145                 150                 155                 160

Leu Tyr Phe Val Ser Met Asp Glu Asp Gly Gly Lys Ala Lys Tyr Ser
                165                 170                 175

Gly Asn Lys Ala Gly Ala Lys Tyr Gly Thr Gly Tyr Cys Asp Ala Gln
            180                 185                 190

Cys Pro Arg Asp Val Lys Phe Ile Asn Gly Val Ala Asn Ser Glu Gly
        195                 200                 205

Trp Glu Pro Ser Asp Ser Asp Ala Asn Ala Gly Val Gly Asn Leu Gly
    210                 215                 220

Thr Cys Cys Pro Glu Met Asp Ile Trp Glu Ala Asn Ser Ile Ser Thr
225                 230                 235                 240

Ala Phe Thr Pro His Pro Cys Thr Lys Leu Thr Gln His Ser Cys Thr
                245                 250                 255

Gly Asn Ser Cys Gly Gly Thr Tyr Ser Asp Asp Arg Tyr Gly Gly Thr
            260                 265                 270

Cys Asp Ala Asp Gly Cys Asp Phe Asn Ala Tyr Arg Gln Gly Asn Lys
        275                 280                 285

Thr Phe Tyr Gly Pro Gly Ser Asn Phe Asn Val Asp Thr Thr Lys Lys

290                 295                 300

Met Thr Val Val Thr Gln Phe His Lys Gly Ser Asp Gly Arg Leu Ser
305                 310                 315                 320

Glu Ile Thr Arg Leu Tyr Val Gln Asn Gly Lys Val Ile Ala Asn Ser
                325                 330                 335

Glu Ser Lys Ile Ala Gly Asn Pro Gly Ser Ser Leu Thr Ser Asp Phe
            340                 345                 350

Cys Ser Lys Gln Lys Ser Val Phe Gly Asp Ile Asp Asp Phe Thr Lys
        355                 360                 365

Lys Gly Gly Trp Asn Gly Met Ser Asp Ala Leu Ala Ala Pro Met Val
    370                 375                 380

Leu Val Met Ser Leu Trp His Asp His His Ser Asn Met Leu Trp Leu
385                 390                 395                 400

Asp Ser Thr Tyr Pro Thr Asp Ser Thr Lys Val Gly Ser Gln Arg Gly
                405                 410                 415

Ser Cys Ala Thr Thr Ser Gly Lys Pro Ser Asp Leu Glu Arg Asp Val
            420                 425                 430

Pro Asn Ser Lys Val Ser Phe Ser Asn Ile Lys Phe Gly Pro Ile Gly
        435                 440                 445

Ser Thr Tyr Lys Ser Asp Gly Thr Thr Pro Asn Pro Pro Ala Ser Ser
    450                 455                 460

Ser Thr Thr Gly Ser Ser Thr Pro Thr Asn Pro Pro Ala Gly Ser Val
465                 470                 475                 480

Asp Gln Trp Gly Gln Cys Gly Gly Gln Asn Tyr Ser Gly Pro Thr Thr
                485                 490                 495

Cys Lys Ser Pro Phe Thr Cys Lys Lys Ile Asn Asp Phe Tyr Ser Gln
            500                 505                 510

Cys Gln

<210>  84
<211>  514
<212>  PRT
<213>  Fusarium oxysporum sp. radicis-lycoperscici

<400>  84

Met Tyr Arg Ile Val Ala Thr Ala Ser Ala Leu Ile Ala Ala Ala Arg
1               5                   10                  15

Ala Gln Gln Val Cys Ser Leu Asn Thr Glu Thr Lys Pro Ala Leu Thr
            20                  25                  30

Trp Ser Lys Cys Thr Ser Ser Gly Cys Ser Asp Val Lys Gly Ser Val
        35                  40                  45

Val Ile Asp Ala Asn Trp Arg Trp Thr His Gln Thr Ser Gly Ser Thr
    50                  55                  60

Asn Cys Tyr Thr Gly Asn Lys Trp Asp Thr Ser Ile Cys Pro Asp Gly
65                  70                  75                  80

Lys Thr Cys Ala Asp Lys Cys Cys Leu Asp Gly Ala Asp Tyr Ala Gly
                85                  90                  95

Thr Tyr Gly Ile Thr Ser Ser Gly Asn Gln Leu Ser Leu Gly Phe Val
            100                 105                 110

Thr Asn Gly Pro Tyr Ser Lys Asn Ile Gly Ser Arg Thr Tyr Leu Met
        115                 120                 125

Glu Asn Glu Asn Thr Tyr Gln Met Phe Gln Leu Leu Gly Asn Glu Phe
    130                 135                 140

Thr Phe Asp Val Asp Val Ser Gly Ile Gly Cys Gly Leu Asn Gly Ala
145                 150                 155                 160

Leu Tyr Phe Val Ser Met Asp Glu Asp Gly Gly Lys Ala Lys Tyr Ser
                165                 170                 175

Gly Asn Lys Ala Gly Ala Lys Tyr Gly Thr Gly Tyr Cys Asp Ala Gln
            180                 185                 190

Cys Pro Arg Asp Val Lys Phe Ile Asn Gly Val Ala Asn Ser Glu Gly
        195                 200                 205

Trp Lys Pro Ser Ala Ser Asp Val Asn Ala Gly Val Gly Asn Leu Gly
    210                 215                 220

Thr Cys Cys Pro Glu Met Asp Ile Trp Glu Ala Asn Ser Ile Ser Thr
225                 230                 235                 240

Ala Phe Thr Pro His Pro Cys Thr Lys Leu Thr Gln His Ser Cys Thr
                245                 250                 255

Gly Asp Ser Cys Gly Gly Thr Tyr Ser Ser Asp Arg Tyr Gly Gly Thr
            260                 265                 270

Cys Asp Ala Asp Gly Cys Asp Phe Asn Ala Tyr Arg Gln Gly Asn Lys
        275                 280                 285

Thr Phe Tyr Gly Pro Gly Ser Asn Phe Asn Ile Asp Thr Thr Lys Lys
    290                 295                 300

Met Thr Val Val Thr Gln Phe His Lys Gly Ser Asn Gly Arg Leu Ser
305                 310                 315                 320

Glu Ile Thr Arg Leu Tyr Val Gln Asn Gly Lys Val Ile Ala Asn Ser
                325                 330                 335

Glu Ser Lys Ile Ala Gly Asn Pro Gly Ser Ser Leu Thr Ser Asp Phe
            340                 345                 350

Cys Ser Lys Gln Lys Ser Val Phe Gly Asp Ile Asp Asp Phe Thr Lys
        355                 360                 365

Lys Gly Gly Trp Asn Gly Met Ser Asp Ala Leu Ser Ala Pro Met Val
    370                 375                 380

Leu Val Met Ser Leu Trp His Asp His His Ser Asn Met Leu Trp Leu
385                 390                 395                 400

Asp Ser Thr Tyr Pro Thr Asp Ser Thr Lys Val Gly Ser Gln Arg Gly
                405                 410                 415

Ser Cys Ala Ile Thr Ser Gly Lys Pro Ser Asp Leu Glu Arg Asp Val
            420                 425                 430

Pro Asn Ser Lys Val Ser Phe Ser Asn Ile Lys Phe Gly Pro Ile Gly
        435                 440                 445

Ser Thr Tyr Asn Ser Asp Gly Thr Thr Pro Asn Pro Pro Ala Ser Ser
    450                 455                 460

Ser Thr Thr Gly Ser Ser Thr Pro Thr Asn Pro Pro Ala Gly Ser Val
465                 470                 475                 480

Asp Gln Trp Gly Gln Cys Gly Gly Gln Asn Tyr Ser Gly Pro Thr Thr
                485                 490                 495

Cys Lys Ser Pro Phe Thr Cys Lys Lys Ile Asn Asp Phe Tyr Ser Gln
            500                 505                 510

Cys Gln

<210>  85
<211>  598
<212>  PRT
<213>  Gaeumannomyces graminis

<400>  85

Met Gly Ile Cys Pro Asp Ser Arg Ala Arg Arg Glu Ala His Pro Leu
1               5                   10                  15

Ser Ala Thr Arg Ala Trp Gln Met Lys Ala Gly Val Met Leu Glu Pro
            20                  25                  30

Gly Gly Gly Cys Arg Pro Arg Pro Pro Arg Trp Thr Cys Ile Lys Pro
        35                  40                  45

Trp Arg Ala Pro Ser Ser Ala Leu Thr Asp Leu Arg Cys Thr Ser His
    50                  55                  60

Pro Arg Leu His Ser Ser Val Ser Pro Asp Leu Pro Trp Pro Ala His
65                  70                  75                  80

Ser Leu Lys Met Leu Arg Lys Ile Ala Thr Leu Ala Ala Leu Val Thr
                85                  90                  95

Ala Val Arg Gly Gln Ala Val Cys Ser Leu Thr Ala Glu Thr His Pro
            100                 105                 110

Asn Leu Gln Trp Ser Lys Cys Thr Ser Gly Gly Cys Ser Asn Val Ala
        115                 120                 125

Gly Ser Val Thr Ile Asp Ala Asn Trp Arg Trp Thr His Ala Val Ser
    130                 135                 140

Gly Ser Thr Asn Cys Tyr Thr Gly Asn Lys Trp Asp Ser Ser Ile Cys
145                 150                 155                 160

Ser Thr Asn Ala Asp Cys Ala Ala Lys Cys Cys Val Asp Gly Ala Ser
                165                 170                 175

Tyr Ala Ser Thr Tyr Gly Ala Val Thr Ser Gly Asn Ala Leu Ser Leu
            180                 185                 190

Lys Phe Val Thr Gln Gly Gly Ser Gly Lys Asn Val Gly Ser Arg Leu
        195                 200                 205

Tyr Leu Met Glu Ser Glu Ser Lys Tyr Gln Met Phe Asn Leu Leu Gly
    210                 215                 220

Asn Glu Phe Thr Phe Asp Val Asp Ala Ala Gln Leu Gly Cys Gly Leu
225                 230                 235                 240

Asn Gly Ala Val Tyr Phe Val Ser Met Asp Ala Asp Gly Gly Ala Ser
                245                 250                 255

Lys Tyr Pro Gly Asn Lys Ala Gly Ala Lys Tyr Gly Thr Gly Tyr Cys
            260                 265                 270

Asp Ser Gln Cys Pro Arg Asp Leu Lys Tyr Ile Asn Gly Ala Ala Asn
        275                 280                 285

Ile Glu Gly Trp Lys Pro Ser Asp Gly Asp Ala Asn Ser Gly Val Gly
    290                 295                 300

Thr Val Gly Ser Cys Cys Ala Glu Met Asp Ile Trp Glu Ala Asn Ser
305                 310                 315                 320

Met Ser Thr Ala Tyr Thr Pro His Pro Cys Ala Asn Asn Ala Gln His
                325                 330                 335

Ser Cys Ser Gly Asp Ala Cys Gly Gly Thr Tyr Ser Gln Thr Arg Tyr
            340                 345                 350

Ala Gly Asp Cys Asp Pro Asp Gly Cys Asp Phe Asn Ser Tyr Arg Gln
        355                 360                 365

Gly Asn Thr Ser Phe Tyr Gly Arg Gly Ala Ser Asn Thr Val Asp Ser
    370                 375                 380

Thr Lys Lys Val Thr Val Val Thr Gln Phe Ile Lys Asn Ser Ala Gly
385                 390                 395                 400

Gln Leu Ser Glu Ile Lys Arg Phe Tyr Val Gln Gly Gly Lys Val Ile
                405                 410                 415

Pro Asn Ser Gln Ser Thr Val Ala Gly Leu Pro Gly Asn Ser Ile Thr
            420                 425                 430

Gln Asp Trp Cys Asp Lys Gln Lys Val Ala Phe Gly Asp Pro Asn Val
        435                 440                 445

His Lys Glu Lys Gly Gly Leu Val Gln Met Gly Lys Ala Leu Glu Lys
    450                 455                 460

Gly Met Val Leu Val Leu Ser Leu Trp Ala Asp His Asn Ser Asn Met
465                 470                 475                 480

Leu Trp Leu Asp Ser Thr Tyr Pro Val Asp Arg Thr Gly Pro Gly Ala
                485                 490                 495

Ala Arg Gly Ser Cys Ser Thr Thr Ser Gly Lys Pro Ala Asp Leu Glu
            500                 505                 510

Arg Asp Val Pro Gly Ser Thr Val Ile Tyr Ser Asn Ile Lys Phe Gly
        515                 520                 525

Pro Ile Gly Ser Thr Phe Ser Ser Gly Gly Ala Gln Pro Glu Pro Ser
    530                 535                 540

Gly Thr Ala Thr Thr Asn Pro Pro Gln Pro Thr Asn Thr Gly Thr Asn
545                 550                 555                 560

Pro Gly Thr Val Pro Arg Tyr Gly Gln Cys Gly Gly Asn Asp Trp Asn
                565                 570                 575

Gly Pro Thr Val Cys Val Ser Pro Tyr Thr Cys Thr Lys Ala Asn Asn
            580                 585                 590

Trp Tyr Ser Gln Cys Leu
        595

<210>  86
<211>  514
<212>  PRT
<213>  Fusarium oxysporum sp. conglutinans race 2

<400>  86

Met Tyr Arg Ile Val Ala Thr Ala Ser Ala Leu Ile Ala Ala Ala Arg
1               5                   10                  15

Ala Gln Gln Val Cys Ser Leu Asn Thr Glu Thr Lys Pro Ala Leu Thr
            20                  25                  30

Trp Ser Lys Cys Thr Ser Ser Gly Cys Ser Asp Val Lys Gly Ser Val
        35                  40                  45

Val Ile Asp Ala Asn Trp Arg Trp Thr His Gln Thr Ser Gly Ser Thr
    50                  55                  60

Asn Cys Tyr Thr Gly Asn Lys Trp Asp Thr Ser Ile Cys Pro Asp Gly
65                  70                  75                  80

Lys Thr Cys Ala Asp Lys Cys Cys Leu Asp Gly Ala Asp Tyr Ala Gly
                85                  90                  95

Thr Tyr Gly Ile Thr Ser Ser Gly Asn Gln Leu Ser Leu Gly Phe Val
            100                 105                 110

Thr Asn Gly Pro Tyr Ser Lys Asn Ile Gly Ser Arg Thr Tyr Leu Met
        115                 120                 125

Glu Asn Glu Asn Thr Tyr Gln Met Phe Gln Leu Leu Gly Asn Glu Phe
    130                 135                 140

Thr Phe Asp Val Asp Val Ser Gly Ile Gly Cys Gly Leu Asn Gly Ala
145                 150                 155                 160

Leu Tyr Phe Val Ser Met Asp Glu Asp Gly Gly Lys Ala Lys Tyr Ser
                165                 170                 175

Gly Asn Lys Ala Gly Ala Lys Tyr Gly Thr Gly Tyr Cys Asp Ala Gln
            180                 185                 190

Cys Pro Arg Asp Val Lys Phe Ile Asn Gly Val Ala Asn Ser Glu Gly
        195                 200                 205

Trp Lys Pro Ser Asp Ser Asp Val Asn Ala Gly Val Gly Asn Leu Gly
    210                 215                 220

Thr Cys Cys Pro Glu Met Asp Ile Trp Glu Ala Asn Ser Ile Ser Thr
225                 230                 235                 240

Ala Phe Thr Pro His Pro Cys Thr Lys Leu Thr Gln His Ser Cys Thr
                245                 250                 255

Gly Asp Ser Cys Gly Gly Thr Tyr Ser Ser Asp Arg Tyr Gly Gly Thr
            260                 265                 270

Cys Asp Ala Asp Gly Cys Asp Phe Asn Ala Tyr Arg Gln Gly Asn Lys
        275                 280                 285

Thr Phe Tyr Gly Pro Gly Ser Asn Phe Asn Ile Asp Thr Thr Lys Lys
    290                 295                 300

Met Thr Val Val Thr Gln Phe His Lys Gly Ser Asn Gly Arg Leu Ser

305                 310                 315                 320

Glu Ile Thr Arg Leu Tyr Val Gln Asn Gly Lys Val Ile Ala Asn Ser
                325                 330                 335

Glu Ser Lys Ile Ala Gly Asn Pro Gly Ser Ser Leu Thr Ser Asp Phe
            340                 345                 350

Cys Ser Lys Gln Lys Ser Val Phe Gly Asp Ile Asp Asp Phe Thr Lys
        355                 360                 365

Lys Gly Gly Trp Asn Gly Met Ser Asp Ala Leu Ser Ala Pro Met Val
    370                 375                 380

Leu Val Met Ser Leu Trp His Asp His His Ser Asn Met Leu Trp Leu
385                 390                 395                 400

Asp Ser Thr Tyr Pro Thr Asp Ser Thr Lys Val Gly Ser Gln Arg Gly
                405                 410                 415

Ser Cys Ala Ile Thr Ser Gly Lys Pro Ser Asp Leu Glu Arg Asp Val
            420                 425                 430

Pro Asn Ser Lys Val Ser Phe Ser Asn Ile Lys Phe Gly Pro Ile Gly
        435                 440                 445

Ser Thr Tyr Lys Ser Asp Gly Thr Thr Pro Asn Pro Pro Ala Ser Ser
    450                 455                 460

Ser Thr Thr Gly Ser Ser Thr Pro Thr Asn Pro Pro Ala Gly Ser Val
465                 470                 475                 480

Asp Gln Trp Gly Gln Cys Gly Gly Gln Asn Tyr Ser Gly Pro Thr Thr
                485                 490                 495

Cys Lys Ser Pro Phe Thr Cys Lys Lys Ile Asn Asp Phe Tyr Ser Gln
            500                 505                 510

Cys Gln

<210>  87
<211>  14
<212>  PRT
<213>  Artificial Sequence

<220>
<223>  CBH2 Fist consensus domain sequence

<220>
<221>  VARIANT
<222>  (3)..(3)
<223>  Replace = Glu

<220>
<221>  VARIANT
<222>  (9)..(9)
<223>  Replace = Leu

<400>  87

Leu Pro Asp Arg Asp Cys Ala Ala Ala Ala Ser Asn Gly Glu
1               5                   10

<210>  88
<211>  11
<212>  PRT
<213>  Artificial Sequence

<220>
<223>  CBH2 Second consensus domain sequence

<220>
<221>  VARIANT
<222>  (8)..(8)
<223>  Replace = Met

<400>  88

Glu Pro Asp Ser Leu Ala Asn Leu Val Thr Asn
1               5                   10

<210>  89
<211>  471
<212>  PRT
<213>  Hypocrea rufa

<400>  89

Met Ile Val Gly Ile Leu Thr Thr Leu Ala Thr Leu Ala Thr Leu Ala
1               5                   10                  15

Ala Ser Val Pro Leu Glu Glu Arg Gln Ala Cys Ser Ser Val Trp Gly
            20                  25                  30

Gln Cys Gly Gly Gln Asn Trp Ser Gly Pro Thr Cys Cys Ala Ser Gly
        35                  40                  45

Ser Thr Cys Val Tyr Ser Asn Asp Tyr Tyr Ser Gln Cys Leu Pro Gly
    50                  55                  60

Ala Ala Ser Ser Ser Ser Ser Thr Arg Ala Ala Ser Thr Thr Ser Arg
65                  70                  75                  80

Val Ser Pro Thr Thr Ser Arg Ser Ser Ser Ala Thr Pro Pro Pro Gly
                85                  90                  95

Ser Thr Thr Thr Arg Val Pro Pro Val Gly Ser Gly Thr Ala Thr Tyr
            100                 105                 110

Ser Gly Asn Pro Phe Val Gly Val Thr Pro Trp Ala Asn Ala Tyr Tyr
        115                 120                 125

Ala Ser Glu Val Ser Ser Leu Ala Ile Pro Ser Leu Thr Gly Ala Met
    130                 135                 140

Ala Thr Ala Ala Ala Ala Val Ala Lys Val Pro Ser Phe Met Trp Leu
145                 150                 155                 160

Asp Thr Leu Asp Lys Thr Pro Leu Met Glu Gln Thr Leu Ala Asp Ile
                165                 170                 175

Arg Thr Ala Asn Lys Asn Gly Gly Asn Tyr Ala Gly Gln Phe Val Val
            180                 185                 190

Tyr Asp Leu Pro Asp Arg Asp Cys Ala Ala Leu Ala Ser Asn Gly Glu
        195                 200                 205

Tyr Ser Ile Ala Asp Gly Gly Val Ala Lys Tyr Lys Asn Tyr Ile Asp
    210                 215                 220

Thr Ile Arg Gln Ile Val Val Glu Tyr Ser Asp Ile Arg Thr Leu Leu
225                 230                 235                 240

Val Ile Glu Pro Asp Ser Leu Ala Asn Leu Val Thr Asn Leu Gly Thr
                245                 250                 255

Pro Lys Cys Ala Asn Ala Pro Ser Ala Tyr Leu Glu Cys Ile Asn Tyr
            260                 265                 270

Ala Val Thr Gln Leu Asn Leu Pro Asn Val Ala Met Tyr Leu Asp Ala
        275                 280                 285

Gly His Ala Gly Trp Leu Gly Trp Pro Ala Asn Gln Asp Pro Ala Ala
    290                 295                 300

Gln Leu Phe Ala Asn Val Tyr Lys Asn Ala Ser Ser Pro Arg Ala Leu
305                 310                 315                 320

Arg Gly Leu Ala Thr Asn Val Ala Asn Tyr Asn Gly Trp Asn Ile Thr
                325                 330                 335

Ser Pro Pro Ser Tyr Thr Gln Gly Asn Ala Val Tyr Asn Glu Lys Leu
            340                 345                 350

Tyr Ile His Ala Ile Gly Pro Leu Leu Ala Asn His Gly Trp Ser Asn
        355                 360                 365

Ala Phe Phe Ile Thr Asp Gln Gly Arg Ser Gly Lys Gln Pro Thr Gly
    370                 375                 380

Gln Gln Gln Trp Gly Asp Trp Cys Asn Val Ile Gly Thr Gly Phe Gly
385                 390                 395                 400

Ile Arg Pro Ser Ala Asn Thr Gly Asp Ser Leu Leu Asp Ser Phe Val
                405                 410                 415

Trp Val Lys Pro Gly Gly Glu Cys Asp Gly Thr Ser Asp Ser Ser Ala
            420                 425                 430

Pro Arg Phe Asp Ser His Cys Ala Leu Pro Asp Ala Leu Gln Pro Ala
        435                 440                 445

Pro Gln Ala Gly Ala Trp Phe Gln Ala Tyr Phe Val Gln Leu Leu Thr
    450                 455                 460

Asn Ala Asn Pro Ser Phe Leu
465                 470

<210>  90
<211>  471
<212>  PRT
<213>  Trichoderma koningii

<400>  90

Met Ile Val Gly Ile Leu Thr Thr Leu Ala Thr Leu Ala Thr Leu Ala
1               5                   10                  15

Ala Ser Val Pro Leu Glu Glu Arg Gln Ala Cys Ser Ser Val Trp Gly
            20                  25                  30

Gln Cys Gly Gly Gln Asn Trp Ser Gly Pro Thr Cys Cys Ala Ser Gly
        35                  40                  45

Ser Thr Cys Val Tyr Ser Asn Asp Tyr Tyr Ser Gln Cys Leu Pro Gly
    50                  55                  60

Ala Ala Ser Ser Ser Ser Ser Thr Arg Ala Ala Ser Thr Thr Ser Arg
65                  70                  75                  80

Val Ser Pro Thr Thr Ser Arg Ser Ser Ser Ala Thr Pro Pro Pro Gly
                85                  90                  95

Ser Thr Thr Thr Arg Val Pro Pro Val Gly Ser Gly Thr Ala Thr Tyr
            100                 105                 110

Ser Gly Asn Pro Phe Val Gly Val Thr Pro Trp Ala Asn Ala Tyr Tyr
        115                 120                 125

Ala Ser Glu Val Ser Ser Leu Ala Ile Pro Ser Leu Thr Gly Ala Met
    130                 135                 140

Ala Thr Ala Ala Ala Ala Val Ala Lys Val Pro Ser Phe Met Trp Leu
145                 150                 155                 160

Asp Thr Leu Asp Lys Thr Pro Leu Met Glu Gln Thr Leu Ala Asp Ile
                165                 170                 175

Arg Thr Ala Asn Lys Asn Gly Gly Asn Tyr Ala Gly Gln Phe Val Val
            180                 185                 190

Tyr Asp Leu Pro Asp Arg Asp Cys Ala Ala Leu Ala Ser Asn Gly Glu
        195                 200                 205

Tyr Ser Ile Ala Asp Gly Gly Val Ala Lys Tyr Lys Asn Tyr Ile Asp
    210                 215                 220

Thr Ile Arg Gln Ile Val Val Glu Tyr Ser Asp Ile Arg Thr Leu Leu
225                 230                 235                 240

Val Ile Glu Pro Asp Ser Leu Ala Asn Leu Val Thr Asn Leu Gly Thr
                245                 250                 255

Pro Lys Cys Ala Asn Ala Gln Ser Ala Tyr Leu Glu Cys Ile Asn Tyr
            260                 265                 270

Ala Val Thr Gln Leu Asn Leu Pro Asn Val Ala Met Tyr Leu Asp Ala
        275                 280                 285

Gly His Ala Gly Trp Leu Gly Trp Pro Ala Asn Gln Asp Pro Ala Ala
    290                 295                 300

Gln Leu Phe Ala Asn Val Tyr Lys Asn Ala Ser Ser Pro Arg Ala Leu
305                 310                 315                 320

Arg Gly Leu Ala Thr Asn Val Ala Asn Tyr Asn Gly Trp Asn Ile Thr

325                 330                 335

Ser Pro Pro Ser Tyr Thr Gln Gly Asn Ala Val Tyr Asn Glu Lys Leu
            340                 345                 350

Tyr Ile His Ala Ile Gly Arg Leu Leu Ala Asn His Gly Trp Ser Asn
        355                 360                 365

Ala Phe Phe Ile Thr Asp Gln Gly Arg Ser Gly Lys Gln Pro Thr Gly
    370                 375                 380

Gln Gln Gln Trp Gly Asp Trp Cys Asn Val Ile Gly Thr Gly Phe Gly
385                 390                 395                 400

Ile Arg Pro Ser Ala Asn Thr Gly Asp Ser Leu Leu Asp Ser Phe Val
                405                 410                 415

Trp Val Lys Pro Gly Gly Glu Cys Asp Gly Thr Ser Asp Ser Ser Ala
            420                 425                 430

Pro Arg Phe Asp Ser His Cys Ala Leu Pro Asp Ala Leu Gln Pro Ala
        435                 440                 445

Pro Gln Ala Gly Ala Trp Phe Gln Ala Tyr Phe Val Gln Leu Leu Thr
    450                 455                 460

Asn Ala Asn Pro Ser Phe Leu
465                 470

<210>  91
<211>  470
<212>  PRT
<213>  Trichoderma longibrachiatum

<400>  91

Met Ile Val Gly Ile Leu Thr Thr Leu Ala Thr Leu Ala Thr Leu Ala
1               5                   10                  15

Ala Ser Val Pro Leu Glu Glu Arg Gln Ala Cys Ser Ser Val Trp Gly
            20                  25                  30

Gln Cys Gly Gly Gln Asn Trp Ser Gly Pro Thr Cys Cys Ala Ser Gly
        35                  40                  45

Ser Thr Cys Val Tyr Ser Asn Asp Tyr Tyr Ser Gln Cys Leu Pro Gly
    50                  55                  60

Ala Ala Ser Ser Ser Ser Ser Thr Arg Ala Ser Ser Thr Thr Ala Arg

65                  70                  75                  80

Ala Ser Ser Thr Thr Ser Arg Ser Ser Ala Thr Pro Pro Pro Gly Ser
                85                  90                  95

Ser Thr Thr Arg Val Pro Pro Val Gly Ser Gly Thr Ala Thr Tyr Ser
            100                 105                 110

Gly Asn Pro Phe Val Gly Val Thr Pro Trp Ala Asn Ala Tyr Tyr Ala
        115                 120                 125

Ser Glu Val Ser Ser Leu Ala Ile Pro Ser Leu Thr Gly Ala Met Ala
    130                 135                 140

Thr Ala Ala Ala Ala Val Ala Lys Val Pro Ser Phe Met Trp Leu Asp
145                 150                 155                 160

Thr Phe Asp Lys Thr Pro Leu Met Glu Gln Thr Leu Ala Asp Ile Arg
                165                 170                 175

Thr Ala Asn Lys Asn Gly Gly Asn Tyr Ala Gly Gln Phe Val Val Tyr
            180                 185                 190

Asp Leu Pro Asp Arg Asp Cys Ala Ala Leu Ala Ser Asn Gly Glu Tyr
        195                 200                 205

Ser Ile Ala Asp Gly Gly Val Asp Lys Tyr Lys Asn Tyr Ile Asp Thr
    210                 215                 220

Ile Arg Gln Ile Val Val Glu Tyr Ser Asp Ile Arg Thr Leu Leu Val
225                 230                 235                 240

Ile Glu Pro Asp Ser Leu Ala Asn Leu Val Thr Asn Leu Gly Thr Pro
                245                 250                 255

Lys Cys Ala Asn Ala Gln Ser Ala Tyr Leu Glu Cys Ile Asn Tyr Ala
            260                 265                 270

Val Thr Gln Leu Asn Leu Pro Asn Val Ala Met Tyr Leu Asp Ala Gly
        275                 280                 285

His Ala Gly Trp Leu Gly Trp Pro Ala Asn Gln Asp Pro Ala Ala Gln
    290                 295                 300

Leu Phe Ala Asn Val Tyr Lys Asn Ala Ser Ser Pro Arg Ala Leu Arg
305                 310                 315                 320

Gly Leu Ala Thr Asn Val Ala Asn Tyr Asn Gly Trp Asn Ile Thr Ser
                325                 330                 335

Pro Pro Ser Tyr Thr Gln Gly Asn Ala Val Tyr Asn Glu Gln Leu Tyr
            340                 345                 350

Ile His Ala Ile Gly Pro Leu Leu Ala Asn His Gly Trp Ser Asn Ala
        355                 360                 365

Phe Phe Ile Thr Asp Gln Gly Arg Ser Gly Lys Gln Pro Thr Gly Gln
    370                 375                 380

Gln Gln Trp Gly Asp Trp Cys Asn Val Ile Gly Thr Gly Phe Gly Ile
385                 390                 395                 400

Arg Pro Ser Ala Asn Thr Gly Asp Ser Leu Leu Asp Ser Phe Val Trp
                405                 410                 415

Ile Lys Pro Gly Gly Glu Cys Asp Gly Thr Ser Asp Ser Ser Ala Pro
            420                 425                 430

Arg Phe Asp Ser His Cys Ala Leu Pro Asp Ala Leu Gln Pro Ala Pro
        435                 440                 445

Gln Ala Gly Ala Trp Phe Gln Ala Tyr Phe Val Gln Leu Leu Thr Asn
    450                 455                 460

Ala Asn Pro Ser Phe Leu
465                 470

<210>  92
<211>  470
<212>  PRT
<213>  Trichoderma orientale

<400>  92

Met Ile Val Gly Ile Leu Thr Thr Leu Ala Thr Leu Ala Thr Leu Ala
1               5                   10                  15

Ala Ser Val Pro Leu Glu Glu Arg Gln Ala Cys Ser Ser Val Trp Gly
            20                  25                  30

Gln Cys Gly Gly Gln Asn Trp Ser Gly Pro Thr Cys Cys Ala Ser Gly
        35                  40                  45

Ser Thr Cys Val Tyr Ser Asn Asp Tyr Tyr Ser Gln Cys Leu Pro Gly
    50                  55                  60

Ala Ala Ser Ser Ser Ser Ser Thr Arg Ala Ser Ser Thr Thr Ala Arg
65                  70                  75                  80

Ala Ser Ser Thr Thr Ser Arg Ser Ser Ala Thr Pro Pro Pro Gly Ser
                85                  90                  95

Ser Thr Thr Arg Val Pro Pro Val Gly Ser Gly Thr Ala Thr Tyr Ser
            100                 105                 110

Gly Asn Pro Phe Val Gly Val Thr Pro Trp Ala Asn Ala Tyr Tyr Ala
        115                 120                 125

Ser Glu Val Ser Ser Leu Ala Ile Pro Ser Leu Thr Gly Ala Met Ala
    130                 135                 140

Thr Ala Ala Ala Ala Val Ala Lys Val Pro Ser Phe Met Trp Leu Asp
145                 150                 155                 160

Thr Phe Asp Lys Thr Pro Leu Met Glu Gln Thr Leu Ala Asp Ile Arg
                165                 170                 175

Thr Ala Asn Lys Asn Gly Gly Asn Tyr Ala Gly Gln Phe Val Val Tyr
            180                 185                 190

Asp Leu Pro Asp Arg Asp Cys Ala Ala Leu Ala Ser Asn Gly Glu Tyr
        195                 200                 205

Ser Ile Ala Asp Gly Gly Val Asp Lys Tyr Lys Asn Tyr Ile Asp Thr
    210                 215                 220

Ile Arg Gln Ile Val Val Glu Tyr Ser Asp Ile Arg Thr Leu Leu Val
225                 230                 235                 240

Ile Glu Pro Asp Ser Leu Ala Asn Leu Val Thr Asn Leu Gly Thr Pro
                245                 250                 255

Lys Cys Ala Asn Ala Gln Ser Ala Tyr Leu Glu Cys Ile Asn Tyr Ala
            260                 265                 270

Val Thr Gln Leu Asn Leu Pro Asn Val Ala Met Tyr Leu Asp Ala Gly
        275                 280                 285

His Ala Gly Trp Leu Gly Trp Pro Ala Asn Gln Asp Pro Ala Ala Gln
    290                 295                 300

Leu Phe Ala Asn Val Tyr Lys Asn Ala Ser Ser Pro Arg Ala Leu Arg
305                 310                 315                 320

Gly Leu Ala Thr Asn Val Ala Asn Tyr Asn Gly Trp Asn Ile Thr Ser
                325                 330                 335

Pro Pro Ser Tyr Thr Gln Gly Asn Ala Val Tyr Asn Glu Gln Leu Tyr
            340                 345                 350

Ile His Ala Ile Gly Pro Leu Leu Ala Asn His Gly Trp Ser Asn Ala
        355                 360                 365

Phe Phe Ile Thr Asp Gln Gly Arg Ser Gly Lys Gln Pro Thr Gly Gln
    370                 375                 380

Gln Gln Trp Gly Asp Trp Cys Asn Val Ile Gly Thr Gly Phe Gly Ile
385                 390                 395                 400

Arg Pro Ser Ala Asn Thr Gly Asp Ser Leu Leu Asp Ser Phe Val Trp
                405                 410                 415

Ile Lys Pro Gly Gly Glu Cys Asp Gly Thr Ser Asp Ser Ser Ala Pro
            420                 425                 430

Arg Phe Asp Ser His Cys Ala Leu Pro Asp Ala Leu Gln Pro Ala Pro
        435                 440                 445

Gln Ala Gly Ala Trp Phe Gln Ala Tyr Phe Val Gln Leu Leu Thr Asn
    450                 455                 460

Ala Asn Pro Ser Phe Leu
465                 470

<210>  93
<211>  470
<212>  PRT
<213>  Trichoderma ghanense

<400>  93

Met Ile Val Gly Ile Leu Thr Thr Leu Ala Thr Leu Ala Thr Leu Ala
1               5                   10                  15

Ala Ser Val Pro Leu Glu Glu Arg Gln Ala Cys Ser Ser Val Trp Gly
            20                  25                  30

Gln Cys Gly Gly Gln Asn Trp Ser Gly Pro Thr Cys Cys Ala Ala Gly
        35                  40                  45

Ser Thr Cys Val Tyr Ser Asn Asp Tyr Tyr Ser Gln Cys Pro Pro Gly
    50                  55                  60

Ala Ala Ser Ser Ser Ser Ser Thr Arg Ala Ser Ser Thr Thr Asn Arg
65                  70                  75                  80

Val Ser Ser Thr Thr Ser Thr Ser Ser Ala Thr Pro Pro Pro Gly Ser
                85                  90                  95

Thr Thr Thr Arg Val Pro Pro Val Gly Ser Gly Thr Ala Thr Tyr Ser
            100                 105                 110

Gly Asn Pro Phe Val Gly Val Thr Pro Trp Ala Asn Ala Tyr Tyr Ala
        115                 120                 125

Ser Glu Val Ser Ser Leu Ala Ile Pro Ser Leu Thr Gly Ala Met Ala
    130                 135                 140

Thr Ala Ala Ala Ala Val Ala Lys Val Pro Ser Phe Met Trp Leu Asp
145                 150                 155                 160

Thr Leu Asp Lys Thr Pro Leu Met Glu Gln Thr Leu Ala Asp Ile Arg
                165                 170                 175

Thr Ala Asn Lys Asn Gly Gly Asn Tyr Ala Gly Gln Phe Val Val Tyr
            180                 185                 190

Asp Leu Pro Asp Arg Asp Cys Ala Ala Leu Ala Ser Asn Gly Glu Tyr
        195                 200                 205

Ser Ile Ala Asp Gly Gly Val Ala Lys Tyr Lys Asn Tyr Ile Asp Thr
    210                 215                 220

Ile Arg Gln Ile Val Val Glu Tyr Ser Asp Ile Arg Thr Ile Leu Val
225                 230                 235                 240

Ile Glu Pro Asp Ser Leu Ala Asn Leu Val Thr Asn Leu Gly Thr Pro
                245                 250                 255

Lys Cys Ala Asn Ala Gln Ser Ala Tyr Leu Glu Cys Ile Asn Tyr Ala
            260                 265                 270

Ile Thr Gln Leu Asn Leu Pro Asn Ile Ala Met Tyr Leu Asp Ala Gly
        275                 280                 285

His Ala Gly Trp Leu Gly Trp Pro Ala Asn Gln Asp Pro Ala Ala Gln
    290                 295                 300

Leu Phe Ala Asn Val Tyr Lys Asn Ala Ser Ser Pro Ser Ala Leu Arg
305                 310                 315                 320

Gly Leu Ala Thr Asn Val Ala Asn Tyr Asn Gly Trp Asn Ile Thr Ser
                325                 330                 335

Pro Pro Ser Tyr Thr Gln Gly Asn Ala Val Tyr Asn Glu Lys Leu Tyr
            340                 345                 350

Ile His Ala Ile Gly Pro Leu Leu Ala Asn His Gly Trp Ser Asn Ala
        355                 360                 365

Phe Phe Ile Thr Asp Gln Gly Arg Ser Gly Lys Gln Pro Thr Gly Gln
    370                 375                 380

Gln Gln Trp Gly Asp Trp Cys Asn Val Ile Gly Thr Gly Phe Gly Ile
385                 390                 395                 400

Arg Pro Ser Ser Asn Thr Gly Asp Ser Leu Leu Asp Ser Phe Val Trp
                405                 410                 415

Val Lys Pro Gly Gly Glu Cys Asp Gly Thr Ser Asp Ser Ser Ala Pro
            420                 425                 430

Arg Phe Asp Ser His Cys Ala Leu Pro Asp Ala Leu Gln Pro Ala Pro
        435                 440                 445

Gln Ala Gly Ala Trp Phe Gln Ala Tyr Phe Val Gln Leu Leu Thr Asn
    450                 455                 460

Ala Asn Pro Ser Phe Leu
465                 470

<210>  94
<211>  471
<212>  PRT
<213>  Hypocrea virens

<400>  94

Met Val Ala Gly Ile Leu Val Ser Leu Ala Ala Leu Ala Thr Leu Ala
1               5                   10                  15

Ala Ser Val Pro Val Glu Glu Arg Gln Ala Cys Ser Gly Val Trp Gly
            20                  25                  30

Gln Cys Gly Gly Gln Asn Trp Ala Gly Pro Phe Cys Cys Val Ser Gly
        35                  40                  45

Ser Thr Cys Val Phe Ser Asn Asp Tyr Tyr Ser Gln Cys Leu Pro Gly
    50                  55                  60

Ala Ala Ser Ser Ser Ser Ser Thr Arg Ala Ser Ser Thr Thr Ser Arg
65                  70                  75                  80

Ala Ser Ser Thr Ser Thr Arg Ser Ser Thr Thr Thr Pro Pro Pro Gly
                85                  90                  95

Ser Ser Thr Thr Ser Ala Pro Pro Val Gly Ser Gly Thr Val Thr Trp
            100                 105                 110

Gln Gly Asn Pro Phe Ser Gly Val Thr Pro Trp Ala Asn Ala Tyr Tyr
        115                 120                 125

Ala Ser Glu Val Ser Thr Leu Ala Ile Pro Ser Leu Thr Gly Ala Met
    130                 135                 140

Ala Thr Ala Ala Ala Ala Val Ala Lys Val Pro Ser Phe Met Trp Leu
145                 150                 155                 160

Asp Thr Leu Asp Lys Thr Pro Leu Met Ser Gln Thr Leu Ser Asp Ile
                165                 170                 175

Arg Ala Ala Asn Lys Asn Gly Gly Asn Tyr Ala Gly Gln Phe Val Val
            180                 185                 190

Tyr Asp Leu Pro Asp Arg Asp Cys Ala Ala Leu Ala Ser Asn Gly Glu
        195                 200                 205

Tyr Thr Ile Ala Asp Gly Gly Val Ala Lys Tyr Lys Asn Tyr Ile Asp
    210                 215                 220

Thr Ile Arg Gly Ile Val Thr Thr Phe Ser Asp Ile Arg Ile Leu Leu
225                 230                 235                 240

Val Ile Glu Pro Asp Ser Leu Ala Asn Leu Val Thr Asn Leu Gly Thr
                245                 250                 255

Pro Lys Cys Ala Asn Ala Gln Ser Ala Tyr Leu Asp Cys Ile Asn Tyr
            260                 265                 270

Ala Ile Thr Arg Leu Asn Leu Pro Asn Val Ala Met Tyr Leu Asp Ala
        275                 280                 285

Gly His Ala Gly Trp Leu Gly Trp Pro Ala Asn Gln Asp Pro Ala Ala
    290                 295                 300

Gln Leu Phe Ala Asn Val Tyr Lys Asn Ala Ser Ser Pro Gln Ala Val

305                 310                 315                 320

Arg Gly Leu Ala Thr Asn Val Ala Asn Tyr Asn Ala Trp Asn Ile Thr
                325                 330                 335

Ser Pro Pro Pro Tyr Thr Gln Gln Asn Ser Val Tyr Asn Glu Lys Leu
            340                 345                 350

Tyr Ile His Ala Ile Gly Pro Leu Leu Ala Asn His Gly Trp Ser Asn
        355                 360                 365

Ala Phe Phe Ile Thr Asp Gln Gly Arg Ser Gly Lys Gln Pro Thr Gly
    370                 375                 380

Gln Leu Glu Trp Gly Asn Trp Cys Asn Val Ile Gly Thr Gly Phe Gly
385                 390                 395                 400

Ile Arg Pro Ser Ala Asn Thr Gly Asp Ser Leu Leu Asp Ala Phe Val
                405                 410                 415

Trp Val Lys Pro Gly Gly Glu Cys Asp Gly Thr Ser Asn Ser Ser Ala
            420                 425                 430

Pro Arg Phe Asp Ser His Cys Ala Ser Pro Asp Ala Leu Gln Pro Ala
        435                 440                 445

Pro Gln Ala Gly Ser Trp Phe Gln Ala Tyr Phe Val Gln Leu Leu Thr
    450                 455                 460

Asn Ala Asn Pro Ser Phe Leu
465                 470

<210>  95
<211>  472
<212>  PRT
<213>  Trichoderma harzianum

<400>  95

Met Val Val Gly Ile Leu Ala Thr Leu Ala Thr Leu Ala Thr Leu Ala
1               5                   10                  15

Ala Ser Val Pro Leu Glu Glu Arg Gln Ser Cys Ser Ser Val Trp Gly
            20                  25                  30

Gln Cys Gly Gly Gln Asn Trp Ala Gly Pro Phe Cys Cys Ala Ser Gly
        35                  40                  45

Ser Thr Cys Val Tyr Ser Asn Asp Phe Tyr Ser Gln Cys Leu Pro Gly

50                  55                  60

Ala Ala Ser Ser Ser Ser Ser Thr Arg Ala Ser Ser Thr Thr Ser Arg
65                  70                  75                  80

Val Ser Ser Ala Thr Ser Thr Arg Ser Ser Ser Ser Thr Pro Pro Pro
                85                  90                  95

Ala Ser Ser Thr Thr Pro Ala Pro Pro Val Gly Ser Gly Thr Ala Thr
            100                 105                 110

Tyr Ser Gly Asn Pro Phe Ala Gly Val Thr Pro Trp Ala Asn Ser Phe
        115                 120                 125

Tyr Ala Ser Glu Val Ser Thr Leu Ala Ile Pro Ser Leu Thr Gly Ala
    130                 135                 140

Met Ala Thr Ala Ala Ala Ala Val Ala Lys Val Pro Ser Phe Met Trp
145                 150                 155                 160

Leu Asp Thr Leu Asp Lys Thr Pro Leu Met Ser Ser Thr Leu Ser Asp
                165                 170                 175

Ile Arg Ala Ala Asn Lys Ala Gly Gly Asn Tyr Ala Gly Gln Phe Val
            180                 185                 190

Val Tyr Asp Leu Pro Asp Arg Asp Cys Ala Ala Ala Ala Ser Asn Gly
        195                 200                 205

Glu Tyr Ser Ile Ala Asp Gly Gly Val Ala Lys Tyr Lys Asn Tyr Ile
    210                 215                 220

Asp Thr Ile Arg Gly Ile Val Thr Thr Phe Ser Asp Val Arg Ile Leu
225                 230                 235                 240

Leu Val Ile Glu Pro Asp Ser Leu Ala Asn Leu Val Thr Asn Leu Ala
                245                 250                 255

Thr Pro Lys Cys Ser Asn Ala Gln Ser Ala Tyr Leu Glu Cys Ile Asn
            260                 265                 270

Tyr Ala Ile Thr Gln Leu Asn Leu Pro Asn Val Ala Met Tyr Leu Asp
        275                 280                 285

Ala Gly His Ala Gly Trp Leu Gly Trp Pro Ala Asn Gln Asp Pro Ala
    290                 295                 300

Ala Gln Leu Phe Ala Asn Val Tyr Lys Asn Ala Ser Ser Pro Arg Ala
305                 310                 315                 320

Val Arg Gly Leu Ala Thr Asn Val Ala Asn Tyr Asn Ala Trp Asn Ile
                325                 330                 335

Thr Thr Pro Pro Ser Tyr Thr Gln Gly Asn Ala Val Tyr Asn Glu Lys
            340                 345                 350

Leu Tyr Ile His Ala Leu Gly Pro Leu Leu Ala Asn His Gly Trp Ser
        355                 360                 365

Asn Ala Phe Phe Ile Thr Asp Gln Gly Arg Ser Gly Lys Gln Pro Thr
    370                 375                 380

Gly Gln Leu Glu Trp Gly Asn Trp Cys Asn Ala Val Gly Thr Gly Phe
385                 390                 395                 400

Gly Ile Arg Pro Ser Ala Asn Thr Gly Asp Ser Leu Leu Asp Ser Phe
                405                 410                 415

Val Trp Ile Lys Pro Gly Gly Glu Cys Asp Gly Thr Ser Asn Ser Ser
            420                 425                 430

Ala Pro Arg Phe Asp Tyr His Cys Ala Ser Ala Asp Ala Leu Gln Pro
        435                 440                 445

Ala Pro Gln Ala Gly Ser Trp Phe Gln Ala Tyr Phe Val Gln Leu Leu
    450                 455                 460

Thr Asn Ala Asn Pro Ser Phe Leu
465                 470

<210>  96
<211>  470
<212>  PRT
<213>  Hypocrea atroviridis

<400>  96

Met Ala Gly Arg Val Phe Ala Thr Leu Ala Thr Leu Ala Ala Leu Ala
1               5                   10                  15

Ala Ser Ala Pro Val Val Glu Glu Arg Gln Ala Cys Ala Ser Val Trp
            20                  25                  30

Gly Gln Cys Gly Gly Gln Gly Trp Ser Gly Ala Thr Cys Cys Ala Ser
        35                  40                  45

Gly Ser Ser Cys Val Val Ser Asn Pro Tyr Tyr Ser Gln Cys Leu Pro
    50                  55                  60

Gly Ser Gly Gly Gly Ser Ser Ser Ser Thr Leu Ala Ser Ser Thr Arg
65                  70                  75                  80

Ala Ser Ser Thr Thr Val Arg Ser Ser Ser Thr Thr Pro Pro Pro Ser
                85                  90                  95

Ser Ser Thr Pro Pro Pro Pro Val Gly Ser Gly Thr Ala Thr Tyr Gln
            100                 105                 110

Gly Asn Pro Phe Ser Gly Ile Asn Pro Trp Ala Asn Ser Phe Tyr Ala
        115                 120                 125

Gln Glu Val Ser Ser Ser Ala Ile Pro Ser Leu Ser Gly Ala Met Ala
    130                 135                 140

Thr Ala Ala Ala Ala Ala Ala Lys Val Pro Ser Phe Met Trp Leu Asp
145                 150                 155                 160

Thr Leu Ser Lys Thr Ser Leu Leu Ser Ser Thr Leu Ser Asp Ile Arg
                165                 170                 175

Ala Ala Asn Lys Ala Gly Gly Asn Tyr Ala Gly Gln Phe Val Val Tyr
            180                 185                 190

Asp Leu Pro Asp Arg Asp Cys Ala Ala Ala Ala Ser Asn Gly Glu Tyr
        195                 200                 205

Ser Ile Ala Asp Asn Gly Val Ala Asn Tyr Lys Asn Tyr Ile Asp Thr
    210                 215                 220

Ile Val Gly Ile Leu Lys Thr Tyr Ser Asp Ile Arg Thr Ile Leu Val
225                 230                 235                 240

Ile Glu Pro Asp Ser Leu Ala Asn Leu Val Thr Asn Leu Ser Val Ala
                245                 250                 255

Lys Cys Ser Asn Ala Gln Ala Ala Tyr Leu Glu Cys Ile Asn Tyr Ala
            260                 265                 270

Ile Thr Gln Leu Asn Leu Pro Asn Val Ala Met Tyr Leu Asp Ala Gly
        275                 280                 285

His Ala Gly Trp Leu Gly Trp Pro Ala Asn Gln Gln Pro Ala Ala Gln
    290                 295                 300

Leu Phe Ala Ser Val Tyr Lys Asn Ala Ser Ser Pro Arg Ala Val Arg
305                 310                 315                 320

Gly Leu Ala Thr Asn Val Ala Asn Tyr Asn Gly Trp Asn Ile Thr Ser
                325                 330                 335

Ala Pro Ser Tyr Thr Gln Gly Asn Ser Val Tyr Asn Glu Gln Leu Tyr
            340                 345                 350

Ile His Ala Ile Ser Pro Leu Leu Thr Gln Gln Gly Trp Ser Asn Thr
        355                 360                 365

Tyr Phe Ile Thr Asp Gln Gly Arg Ser Gly Lys Gln Pro Thr Gly Gln
    370                 375                 380

Gln Ala Trp Gly Asp Trp Cys Asn Val Ile Gly Thr Gly Phe Gly Ile
385                 390                 395                 400

Arg Pro Ser Ala Asn Thr Gly Asp Ser Leu Leu Asp Ala Phe Thr Trp
                405                 410                 415

Ile Lys Pro Gly Gly Glu Cys Asp Gly Thr Ser Asn Thr Ser Ala Thr
            420                 425                 430

Arg Tyr Asp Tyr His Cys Gly Leu Ser Asp Ala Leu Gln Pro Ala Pro
        435                 440                 445

Glu Ala Gly Ser Trp Phe Gln Ala Tyr Phe Val Gln Leu Leu Thr Asn
    450                 455                 460

Ala Asn Pro Ser Phe Leu
465                 470

<210>  97
<211>  471
<212>  PRT
<213>  Trichoderma gamsii

<400>  97

Met Ala Gly Arg Val Phe Ala Thr Leu Ala Thr Leu Ala Ala Leu Ala
1               5                   10                  15

Ala Ser Ala Pro Val Val Glu Glu Arg Gln Ala Cys Ala Thr Val Trp
            20                  25                  30

Gly Gln Cys Gly Gly Gln Gly Trp Ser Gly Ala Thr Cys Cys Ala Ser
        35                  40                  45

Gly Ser Ser Cys Val Val Ser Asn Gln Tyr Tyr Ser Gln Cys Leu Pro
    50                  55                  60

Gly Ser Gly Gly Gly Ser Ser Ser Ser Thr Val Ala Ser Ser Thr Arg
65                  70                  75                  80

Val Ser Ser Thr Thr Val Arg Ser Ser Ser Thr Thr Pro Pro Pro Thr
                85                  90                  95

Ser Ser Thr Gly Ser Pro Pro Pro Val Gly Ser Gly Thr Ala Thr Tyr
            100                 105                 110

Gln Gly Asn Pro Phe Ser Gly Val Asn Leu Trp Ala Asn Lys Phe Tyr
        115                 120                 125

Gln Asp Glu Val Thr Ser Ser Ala Ile Pro Ser Leu Ser Gly Ala Met
    130                 135                 140

Ala Thr Ala Ala Ala Ala Ala Ala Lys Val Pro Ala Phe Met Trp Leu
145                 150                 155                 160

Asp Thr Leu Ser Lys Thr Thr Leu Leu Ser Ser Thr Leu Ala Asp Ile
                165                 170                 175

Arg Ala Ala Asn Lys Ala Gly Gly Asn Tyr Ala Gly Gln Phe Val Val
            180                 185                 190

Tyr Asp Leu Pro Asp Arg Asp Cys Ala Ala Ala Ala Ser Asn Gly Glu
        195                 200                 205

Tyr Ser Ile Ala Asn Asn Gly Val Ala Asn Tyr Lys Asn Tyr Ile Asp
    210                 215                 220

Thr Ile Val Gly Ile Leu Lys Thr Tyr Ser Asp Ile Arg Thr Ile Leu
225                 230                 235                 240

Val Val Glu Pro Asp Ser Leu Ala Asn Leu Val Thr Asn Leu Asn Val
                245                 250                 255

Thr Lys Cys Ala Asn Ala Gln Ser Ala Tyr Leu Glu Ser Ile Asn Tyr
            260                 265                 270

Ala Ile Thr Lys Leu Asn Leu Pro Asn Val Ala Met Tyr Leu Asp Ala
        275                 280                 285

Gly His Ala Gly Trp Leu Gly Trp Pro Ala Asn Gln Gln Pro Ala Ala
    290                 295                 300

Gln Leu Phe Ala Ser Val Tyr Lys Asn Ala Ser Ser Pro Arg Ala Val
305                 310                 315                 320

Arg Gly Leu Ala Thr Asn Val Ala Asn Tyr Asn Gly Trp Asn Ile Thr
                325                 330                 335

Ser Ala Pro Ser Tyr Thr Gln Gly Asn Ala Val Tyr Asn Glu Gln Leu
            340                 345                 350

Tyr Ile His Ala Leu Ser Pro Leu Leu Ala Gln Gln Gly Trp Ser Gly
        355                 360                 365

Ala Gln Phe Ile Thr Asp Gln Gly Arg Ser Gly Arg Gln Pro Thr Gly
    370                 375                 380

Gln Gln Ala Trp Gly Asp Trp Cys Asn Val Ile Gly Thr Gly Phe Gly
385                 390                 395                 400

Ile Arg Pro Ser Ala Asn Thr Gly Asp Ser Leu Leu Asp Ala Phe Thr
                405                 410                 415

Trp Ile Lys Pro Gly Gly Glu Cys Asp Gly Thr Ser Asn Thr Ser Ala
            420                 425                 430

Thr Arg Tyr Asp Tyr His Cys Gly Leu Ser Asp Ala Leu Gln Pro Ala
        435                 440                 445

Pro Glu Ala Gly Ser Trp Phe Gln Ala Tyr Phe Val Gln Leu Leu Thr
    450                 455                 460

Asn Ala Asn Pro Ser Phe Leu
465                 470

<210>  98
<211>  469
<212>  PRT
<213>  Bionectria ochroleuca

<400>  98

Met Phe Ala Lys Ser Val Ile Leu Ser Ala Tyr Leu Ala Leu Gly Val
1               5                   10                  15

Leu Gly Ala Pro Leu Glu Glu Arg Ala Cys Ser Ala Ala Trp Ala Gln
            20                  25                  30

Cys Gly Gly Asn Gly Tyr Thr Gly Glu Ala Cys Cys Val Ser Gly Asn
        35                  40                  45

Val Cys Thr Tyr Val Asn Asp Trp Tyr Ser Gln Cys Ile Pro Gly Thr
    50                  55                  60

Ala Thr Ser Ser Ser Ser Thr Leu Ala Thr Ser Thr Thr Thr Ser Arg
65                  70                  75                  80

Thr Thr Ser Ala Thr Ser Ala Thr Ser Thr Ser Gln Ile Thr Ser Thr
                85                  90                  95

Ser Thr Thr Ser Val Pro Val Gly Thr Gly Thr Ala Thr Trp Ser Gly
            100                 105                 110

Asn Pro Tyr Ser Gly Val Asn Leu Tyr Ala Asn Ser Tyr Tyr Ala Ser
        115                 120                 125

Glu Val Ser Thr Leu Ala Ile Pro Ser Leu Ser Gly Ala Met Ala Thr
    130                 135                 140

Ala Ala Ala Lys Val Ala Asn Val Pro Ser Tyr Met Trp Leu Asp Thr
145                 150                 155                 160

Phe Ser Lys Thr Pro Leu Met Arg Ser Thr Leu Ala Asp Ile Arg Thr
                165                 170                 175

Lys Asn Arg Ala Gly Gly Asn Tyr Ala Gly Gln Phe Val Val Tyr Asp
            180                 185                 190

Leu Pro Asp Arg Asp Cys Ala Ala Ala Ala Ser Asn Gly Glu Tyr Ser
        195                 200                 205

Ile Ile Asp Gly Gly Val Ala Lys Tyr Lys Thr Tyr Ile Asp Thr Ile
    210                 215                 220

Arg Asn Ile Ile Val Glu Tyr Ser Asp Ile Arg Ile Leu Leu Val Ile
225                 230                 235                 240

Glu Pro Asp Ser Leu Ala Asn Leu Val Thr Asn Leu Asn Val Ala Lys
                245                 250                 255

Cys Ser Asn Ala Gln Ser Ala Tyr Met Glu Cys Thr Asn Tyr Ala Val
            260                 265                 270

Lys Gln Leu Asn Leu Pro Asn Val Ala Met Tyr Leu Asp Ala Gly His
        275                 280                 285

Ala Gly Trp Leu Gly Trp Pro Ala Asn Gln Ala Pro Ala Ala Gln Leu

290                 295                 300

Tyr Ala Lys Val Tyr Lys Asp Ala Gly Ser Pro Lys Ser Leu Arg Gly
305                 310                 315                 320

Leu Ala Thr Asn Val Ala Asn Tyr Asn Ala Trp Ser Leu Ser Ser Ala
                325                 330                 335

Pro Ser Tyr Thr Ser Pro Asn Pro Ile Tyr Asp Glu Gln Lys Tyr Ile
            340                 345                 350

Asn Ala Leu Glu Pro Gln Leu Ala Ser Asn Gly Trp Thr Gly Cys Lys
        355                 360                 365

Phe Ile Thr Asp Gln Gly Arg Ser Gly Lys Gln Pro Thr Gly Gln Thr
    370                 375                 380

Glu Trp Gly His Trp Cys Asn Ala Lys Gly Thr Gly Phe Gly Ile Arg
385                 390                 395                 400

Pro Ser Ala Asn Thr Gly Asp Ala Leu Leu Asp Ala Phe Val Trp Val
                405                 410                 415

Lys Pro Gly Gly Glu Cys Asp Gly Thr Ser Asp Thr Ser Ala Ala Arg
            420                 425                 430

Tyr Asp Tyr His Cys Gly Leu Ser Asp Ala Leu Gln Pro Ala Pro Glu
        435                 440                 445

Ala Gly Thr Trp Phe Gln Ala Tyr Phe Glu Gln Leu Leu Thr Asn Ala
    450                 455                 460

Asn Pro Ser Phe Leu
465

<210>  99
<211>  476
<212>  PRT
<213>  Colletotrichum incanum

<400>  99

Met Ala Ala Arg Thr Leu Leu Ala Ala Ala Leu Leu Gly Thr Ala Ala
1               5                   10                  15

Phe Ala Val Pro Ile Glu Glu Arg Gln Ser Cys Ala Ser Val Trp Ser
            20                  25                  30

Gln Cys Gly Gly Gln Thr Trp Thr Gly Pro Thr Cys Cys Ala Ala Gly

35                  40                  45

Ser Thr Cys Val Val Gly Asn Pro Phe Tyr Ser Gln Cys Ile Pro Gly
    50                  55                  60

Ser Ala Ala Pro Ser Ser Ser Ser Thr Val Val Ser Ser Thr Val Arg
65                  70                  75                  80

Thr Ser Ser Thr Pro Val Val Ser Pro Ser Arg Thr Ser Thr Thr Ala
                85                  90                  95

Gly Pro Val Thr Thr Thr Ser Ala Gly Thr Gly Thr Thr Pro Pro Pro
            100                 105                 110

Val Ser Gly Gly Ala Thr Tyr Thr Gly Asn Pro Phe Ala Gly Val Asp
        115                 120                 125

Leu Trp Ala Asn Ser Tyr Tyr Ala Ser Glu Ile Thr Thr Leu Ala Ile
    130                 135                 140

Pro Ser Leu Ser Pro Ala Leu Ala Thr Ala Ala Ala Lys Val Ala Lys
145                 150                 155                 160

Val Pro Thr Phe Met Trp Met Asp Thr Arg Ala Lys Val Pro Leu Val
                165                 170                 175

Asp Ser Thr Leu Ala Asp Ile Arg Arg Ala Asn Gln Ala Gly Gly Asn
            180                 185                 190

Tyr Ala Gly Gln Phe Val Val Tyr Asp Leu Pro Asp Arg Asp Cys Ala
        195                 200                 205

Ala Ala Ala Ser Asn Gly Glu Phe Ala Ile Ala Asn Gly Gly Val Ala
    210                 215                 220

Lys Tyr Lys Glu Tyr Ile Asp Ala Ile Arg Ala Met Ile Ile Lys Tyr
225                 230                 235                 240

Ser Asp Ile Arg Ile Leu Leu Val Ile Glu Pro Asp Ser Leu Ala Asn
                245                 250                 255

Leu Val Thr Asn Leu Asn Val Pro Lys Cys Ala Gly Ala Gln Ala Ala
            260                 265                 270

Tyr Leu Glu Cys Thr Asn Tyr Ala Val Thr Gln Leu Asn Leu Pro Asn
        275                 280                 285

Val Ala Met Tyr Leu Asp Gly Gly His Ala Gly Trp Leu Gly Trp Thr
    290                 295                 300

Ala Asn Leu Pro Pro Ala Ala Gln Met Tyr Ala Lys Val Tyr Lys Asp
305                 310                 315                 320

Ala Gly Ser Pro Lys Ala Leu Arg Gly Leu Val Thr Asn Val Ala Asn
                325                 330                 335

Tyr Asn Ala Trp Ser Val Ser Ser Pro Pro Pro Tyr Thr Gln Gly Asn
            340                 345                 350

Ser Asn Tyr Asp Glu Lys His Tyr Ile Glu Ala Leu Gly Pro Leu Leu
        355                 360                 365

Ala Ala Gln Gly Trp Asn Ala Gln Phe Ile Val Asp Gln Gly Arg Ser
    370                 375                 380

Gly Lys Gln Pro Thr Gly Gln Glu Ala Trp Gly Asp Trp Cys Asn Ala
385                 390                 395                 400

Ile Gly Thr Gly Phe Gly Leu Arg Pro Ser Thr Asn Thr Gly Ser Ser
                405                 410                 415

Leu Val Asp Ser Phe Val Trp Val Lys Pro Gly Gly Glu Ser Asp Gly
            420                 425                 430

Thr Ser Asp Thr Thr Ala Ala Arg Tyr Asp His Asn Cys Gly Lys Asn
        435                 440                 445

Asp Ala Leu Lys Pro Ala Pro Glu Ala Gly Thr Trp Phe Gln Ala Tyr
    450                 455                 460

Phe Glu Gln Leu Leu Lys Asn Ala Asn Pro Ala Phe
465                 470                 475

<210>  100
<211>  486
<212>  PRT
<213>  Stachybotrys chartarum

<400>  100

Met Ala Ala Arg Thr Leu Leu Ile Ala Ala Ala Phe Ala Thr Ala Ala
1               5                   10                  15

Ile Ala Ala Pro Leu Glu Val Glu Glu Arg Gln Ala Cys Ala Ser Val
            20                  25                  30

Trp Ser Gln Cys Gly Gly Asn Asn Trp Asn Gly Ala Thr Cys Cys Val
        35                  40                  45

Ala Gly Asn Thr Cys Ser Val Ile Asn Glu Trp Tyr His Gln Cys Ile
    50                  55                  60

Pro Gly Ala Ala Ala Thr Thr Ser Ser Ala Arg Pro Thr Ser Ser Ser
65                  70                  75                  80

Ser Val Arg Pro Thr Ser Ser Ser Ser Val Arg Pro Thr Thr Ser Ser
                85                  90                  95

Ala Val Thr Arg Thr Ser Ser Ala Thr Ser Thr Arg Ala Thr Gly Ser
            100                 105                 110

Thr Ser Thr Thr Arg Ala Pro Val Gly Ser Gly Thr Ala Thr Trp Ser
        115                 120                 125

Gly Asn Pro Tyr Ser Gly Val Asp Leu Trp Ala Asn Asn Tyr Tyr Arg
    130                 135                 140

Ser Glu Val Leu Asn Leu Ala Val Pro Ser Leu Thr Gly Ala Met Ala
145                 150                 155                 160

Thr Ala Ala Ala Arg Val Ala Glu Val Pro Ser Phe Met Trp Leu Asp
                165                 170                 175

Thr Phe Ser Lys Thr Pro Leu Met Arg Glu Thr Leu Ala Asp Ile Arg
            180                 185                 190

Ala Ala Asn Ala Ala Gly Gly Asn Tyr Ala Gly Gln Phe Val Val Tyr
        195                 200                 205

Asp Leu Pro Asp Arg Asp Cys Ala Ala Ala Ala Ser Asn Gly Glu Tyr
    210                 215                 220

Ser Leu Asp Asn Asn Gly Ile Ala Asn Tyr Arg Asn Tyr Ile Asp Thr
225                 230                 235                 240

Ile Arg Glu Ile Ile Val Glu Tyr Ser Asp Ile Arg Met Leu Leu Val
                245                 250                 255

Ile Glu Pro Asp Ser Leu Ala Asn Met Val Thr Asn Met Asn Val Pro
            260                 265                 270

Lys Cys Ala Asn Ala Ala Asp Ala Tyr His Glu Cys Thr Leu Tyr Ala
        275                 280                 285

Val Thr Gln Leu Asn Leu Pro Asn Val Ala Met Tyr Leu Asp Ala Gly
    290                 295                 300

His Ala Gly Trp Leu Gly Trp Pro Ala Asn Gln Ala Gly Ala Ala Glu
305                 310                 315                 320

Thr Phe Ala Ser Val Tyr Thr Glu Ala Gly Arg Pro Arg Ala Leu Arg
                325                 330                 335

Gly Leu Val Thr Asn Val Ser Asn Tyr Asn Ala Trp Leu Leu Ser Ser
            340                 345                 350

Pro Pro Ser Tyr Thr Thr Pro Asn Pro Val Tyr Asp Glu Ser Lys Tyr
        355                 360                 365

Val His Ala Leu Ala Pro Leu Leu Glu Ala Arg Gly Trp Thr Gly Val
    370                 375                 380

Arg Phe Ile Thr Asp Gln Gly Arg Ser Gly Arg Gln Pro Thr Gly Gln
385                 390                 395                 400

Leu Glu Trp Gly His Trp Cys Asn Ala Arg Gly Thr Gly Phe Gly Arg
                405                 410                 415

Arg Pro Thr Ala Asn Thr Gly Asp Glu Leu Leu Asp Ala Phe Val Trp
            420                 425                 430

Val Lys Pro Gly Gly Glu Ala Asp Gly Thr Ser Asp Thr Thr Ala Asp
        435                 440                 445

Arg Tyr Asp His Phe Cys Gly Ser Ala Ser Ser Val Ile Pro Ala Pro
    450                 455                 460

Glu Ala Gly Thr Trp Phe Gln Ala Tyr Phe Val Gln Leu Leu Glu Asn
465                 470                 475                 480

Ala Asn Pro Ser Phe Met
                485

<210>  101
<211>  468
<212>  PRT
<213>  Nectria haematococca

<400>  101

Met Thr Ala Tyr Lys Leu Leu Phe Thr Ala Ala Leu Ala Gly Thr Ala
1               5                   10                  15

Phe Ala Ala Pro Leu Val Glu Glu Arg Gln Ala Cys Ala Ala Gln Trp
            20                  25                  30
Ala Gln Cys Gly Gly Phe Ser Trp Asn Gly Ala Thr Cys Cys Gln Ser
        35                  40                  45
Gly Ser Tyr Cys Ser Lys Ile Asn Asp Tyr Tyr Ser Gln Cys Ile Pro
    50                  55                  60
Gly Glu Gly Pro Ala Thr Ser Lys Ser Ser Thr Leu Pro Ala Ser Thr
65                  70                  75                  80
Thr Thr Thr Gln Pro Thr Ser Thr Ser Thr Ala Gly Thr Ser Ser Thr
                85                  90                  95
Thr Lys Pro Pro Pro Ala Gly Ser Gly Thr Ala Thr Tyr Ser Gly Asn
            100                 105                 110
Pro Tyr Ser Gly Val Asn Leu Trp Ala Asn Ser Tyr Tyr Arg Ser Glu
        115                 120                 125
Val Thr Asn Leu Ala Ile Pro Lys Leu Ser Gly Ala Met Ala Thr Ala
    130                 135                 140
Ala Ala Lys Val Ala Asp Val Pro Ser Tyr Gln Trp Met Asp Ser Phe
145                 150                 155                 160
Asp His Ile Ser Leu Met Glu Asp Thr Leu Val Asp Ile Arg Lys Ala
                165                 170                 175
Asn Leu Ala Gly Gly Asn Tyr Ala Gly Gln Phe Val Val Tyr Asp Leu
            180                 185                 190
Pro Asp Arg Asp Cys Ala Ala Ala Ala Ser Asn Gly Glu Tyr Ser Leu
        195                 200                 205
Asp Asn Asp Gly Ala Asn Lys Tyr Lys Asn Tyr Ile Gln Thr Ile Lys
    210                 215                 220
Lys Ile Ile Gln Ser Tyr Ser Asp Ile Arg Ile Leu Leu Val Ile Glu
225                 230                 235                 240
Pro Asp Ser Leu Ala Asn Leu Val Thr Asn Met Asp Val Ala Lys Cys
                245                 250                 255
Ala Lys Ala His Asp Ala Tyr Ile Ser Leu Thr Asn Tyr Ala Val Thr
            260                 265                 270

Glu Leu Asn Leu Pro Asn Val Ala Met Tyr Leu Asp Ala Gly His Ala
        275                 280                 285

Gly Trp Leu Gly Trp Pro Ala Asn Gln Gly Pro Ala Ala Lys Leu Phe
    290                 295                 300

Ala Ser Ile Tyr Lys Asp Ala Gly Lys Pro Ala Ala Leu Arg Gly Leu
305                 310                 315                 320

Ala Thr Asn Val Ala Asn Tyr Asn Ala Trp Ser Leu Ser Ser Ala Pro
                325                 330                 335

Pro Tyr Thr Gln Gly Ala Ser Ile Tyr Asp Glu Lys Ser Phe Ile His
            340                 345                 350

Ala Met Gly Pro Leu Leu Glu Gln Asn Gly Trp Pro Gly Ala His Phe
        355                 360                 365

Ile Thr Asp Gln Gly Arg Ser Gly Lys Gln Pro Thr Gly Gln Ile Gln
    370                 375                 380

Trp Gly Asp Trp Cys Asn Ser Lys Gly Thr Gly Phe Gly Ile Arg Pro
385                 390                 395                 400

Ser Ala Asn Thr Gly Asp Ser Leu Leu Asp Ala Phe Val Trp Val Lys
                405                 410                 415

Pro Gly Gly Glu Ser Asp Gly Thr Ser Asp Thr Ser Ala Thr Arg Tyr
            420                 425                 430

Asp Tyr His Cys Gly Ala Ser Ala Ala Leu Gln Pro Ala Pro Glu Ala
        435                 440                 445

Gly Thr Trp Phe Gln Ala Tyr Phe Glu Gln Leu Leu Thr Asn Ala Asn
    450                 455                 460

Pro Ser Phe Leu
465

<210>  102
<211>  470
<212>  PRT
<213>  Neonectria ditissima

<400>  102

Met Thr Thr Cys Lys Leu Leu Leu Ala Ala Ala Leu Ala Gly Ala Ala
1               5                   10                  15

Val Ala Ile Pro Leu Ala Glu Arg Gln Ser Cys Pro Leu Val Lys Arg
            20                  25                  30

Gln Ser Cys Ser Ser Gln Trp Ala Gln Cys Gly Gly Ile Ser Trp Thr
        35                  40                  45

Gly Ala Thr Cys Cys Asp Ser Gly Ser Ser Cys Thr Lys Val Asn Asp
    50                  55                  60

Tyr Tyr Phe Gln Cys Gln Pro Gly Thr Ala Ala Ser Ala Ser Ser Thr
65                  70                  75                  80

Gly Ser Leu Pro Ala Ser Thr Thr Lys Ala Thr Val Thr Thr Ser Ala
                85                  90                  95

Gly Ser Ala Thr Thr Ala Pro Pro Ala Thr Gly Thr Ala Val Ala Ser
            100                 105                 110

Gly Asn Pro Phe Ser Gly Val Asp Leu Trp Ala Asn Asp Tyr Tyr Arg
        115                 120                 125

Ser Glu Val Ser Asn Leu Ala Ile Pro Lys Leu Ser Gly Ala Met Ala
    130                 135                 140

Thr Ala Ala Ala Lys Val Ala Asp Val Pro Ser Phe Gln Trp Met Asp
145                 150                 155                 160

Ser Phe Asp His Ile Ser Leu Met Glu Asp Thr Leu Val Glu Ile Lys
                165                 170                 175

Ala Ala Asn Lys Ala Gly Gly Asn Tyr Ala Gly Gln Phe Val Val Tyr
            180                 185                 190

Asp Leu Pro Glu Arg Asp Cys Ala Ala Ala Ala Ser Asn Gly Glu Tyr
        195                 200                 205

Ser Leu Asp Asn Asp Gly Ala Asn Lys Tyr Lys Asn Tyr Ile Asp Thr
    210                 215                 220

Ile Lys Lys Ile Ile Gln Ala Tyr Ser Asp Ile Arg Ile Ile Leu Val
225                 230                 235                 240

Ile Glu Pro Asp Ser Leu Ala Asn Leu Val Thr Asn Met Asn Val Ala
                245                 250                 255

Lys Cys Ala Lys Ala His Asp Ala Tyr Leu Thr Leu Thr Ser Tyr Ala

            260                 265                 270

Val Thr Ala Leu Asn Leu Pro Asn Val Ala Met Tyr Leu Asp Ala Gly
        275                 280                 285

His Ala Gly Trp Leu Gly Trp Pro Ala Asn Gln Gly Pro Ala Ala Gln
    290                 295                 300

Leu Phe Ala Gly Val Tyr Lys Asp Ala Gly Lys Pro Ala Ala Leu Arg
305                 310                 315                 320

Gly Leu Ala Thr Asn Val Ala Asn Tyr Asn Ala Trp Ser Ile Ser Ser
                325                 330                 335

Pro Pro Ser Tyr Thr Ser Gly Asn Ser Ile Tyr Asp Glu Lys Gly Phe
            340                 345                 350

Val His Ala Met Gly Pro Leu Leu Glu Gln Asn Gly Trp Pro Gly Ala
        355                 360                 365

Lys Phe Ile Thr Asp Gln Gly Arg Ser Gly Lys Gln Pro Thr Gly Gln
    370                 375                 380

Ala Gln Trp Gly Asp Trp Cys Asn Ala Lys Gly Thr Gly Phe Gly Ile
385                 390                 395                 400

Arg Pro Ser Ala Asn Thr Gly Asp Ser Leu Leu Asp Ala Phe Val Trp
                405                 410                 415

Ile Lys Pro Gly Gly Glu Ser Asp Gly Thr Ser Asp Thr Ser Ala Ala
            420                 425                 430

Arg Tyr Asp Tyr His Cys Gly Tyr Ser Asp Ala Leu Gln Pro Ala Pro
        435                 440                 445

Glu Ala Gly Thr Trp Phe Gln Ala Tyr Phe Glu Gln Leu Leu Thr Asn
    450                 455                 460

Ala Asn Pro Ser Phe Leu
465                 470

<210> 103
<211> 474
<212> PRT
<213> Pestalotiopsis fici

<400> 103

Met Ala Val Lys Ile Trp Ile Leu Thr Val Leu Ser Ala Leu Ala Leu

1               5                   10                  15

Ala Ile Pro Leu Asp Gly Pro Ile Glu Glu Arg Gln Ser Cys Ala Ala
            20                  25                  30

Leu Tyr Gly Gln Cys Gly Gly Thr Gly Trp Ser Gly Ala Thr Cys Cys
        35                  40                  45

Ala Ser Gly Thr Cys Val Leu Ser Asn Glu Tyr Tyr Ser Gln Cys Leu
    50                  55                  60

Pro Ser Ser Gly Thr Thr Thr Ala Gly Thr Thr Arg Ser Thr Thr Thr
65                  70                  75                  80

Ser Ala Ala Thr Ser Thr Lys Thr Ser Thr Thr Thr Ser Lys Ala Thr
                85                  90                  95

Thr Ser Ala Ser Ser Ala Ser Thr Lys Thr Thr Thr Ala Pro Val Gly
            100                 105                 110

Ser Gly Thr Ala Thr Trp Ser Gly Asn Pro Phe Ser Gly Val Ala Gln
        115                 120                 125

Trp Ala Asn Asn Tyr Tyr Ala Ser Glu Val Thr Ala Tyr Ala Val Pro
    130                 135                 140

Thr Leu Gly Ala Lys Ala Ala Ala Val Ala Asp Val Pro Ser Phe Met
145                 150                 155                 160

Trp Leu Asp Thr Leu Ser Lys Thr Asp Leu Met Glu Asp Thr Leu Ala
                165                 170                 175

Asp Ile Arg Thr Ala Asn Gln Ala Gly Ala Ser Pro Ala Asn Ala Gly
            180                 185                 190

Ile Phe Val Val Tyr Asp Leu Pro Asp Arg Asp Cys Ala Ala Ala Ala
        195                 200                 205

Ser Asn Gly Glu Tyr Ser Ile Ala Asn Asn Gly Val Ala Asn Tyr Lys
    210                 215                 220

Asn Tyr Ile Asp Thr Ile Val Ala Ile Val Lys Lys Tyr Ser Asp Val
225                 230                 235                 240

Arg Ile Leu Leu Val Ile Glu Pro Asp Ser Leu Ala Asn Leu Val Thr
                245                 250                 255

Asn Leu Ser Val Ser Lys Cys Ser Asn Ala Gln Ser Ala Tyr Leu Glu
            260                 265                 270

Cys Ile Asn Tyr Ala Leu Thr Asn Leu Asn Leu Pro Asn Val Ala Met
        275                 280                 285

Tyr Leu Asp Ala Gly His Ala Gly Trp Leu Gly Trp Pro Ala Asn Leu
    290                 295                 300

Ser Pro Ala Ala Thr Leu Phe Ala Gln Val Tyr Ser Asp Ala Gly Ser
305                 310                 315                 320

Pro Ala Ala Leu Arg Gly Leu Ala Thr Asn Val Ala Asn Tyr Asn Gly
                325                 330                 335

Trp Ser Val Ser Ser Ala Pro Ser Tyr Thr Gln Gly Asn Ala Asn Tyr
            340                 345                 350

Asp Glu Ser His Tyr Val Ala Ala Ile Ala Pro Leu Leu Ser Ala Ala
        355                 360                 365

Gly Phe Asp Ala His Phe Ile Thr Asp Thr Gly Arg Ser Gly Lys Gln
    370                 375                 380

Pro Thr Gly Gln Leu Ala Trp Gly Asp Trp Cys Asn Val Ile Gly Thr
385                 390                 395                 400

Gly Phe Gly Thr Arg Pro Thr Ala Asn Thr Gly Ser Asp Leu Leu Asp
                405                 410                 415

Ala Phe Val Trp Val Lys Pro Gly Gly Glu Cys Asp Gly Thr Ser Asn
            420                 425                 430

Thr Thr Ala Thr Arg Tyr Asp Tyr His Cys Gly Leu Ala Asp Ala Leu
        435                 440                 445

Gln Pro Ala Pro Glu Ala Gly Thr Trp Phe Glu Ala Tyr Phe Glu Gln
    450                 455                 460

Leu Phe Thr Asn Ala Asn Pro Ala Phe Thr
465                 470

<210>  104
<211>  455
<212>  PRT
<213>  Rosellinia necatrix

<400>  104

Met Ser Thr Lys Ser Phe Leu Ile Ala Ala Leu Ser Thr Val Ala Leu
1               5                   10                  15

Ala Val Pro Phe Glu Gly Arg Val Glu Glu Arg Gln Ser Cys Ala Ala
            20                  25                  30

Gln Trp Ala Gln Cys Gly Gly Asn Gly Trp Ser Gly Ala Thr Cys Cys
        35                  40                  45

Val Ser Gly Thr Thr Cys Val Lys Gln Asn Asp Tyr Tyr Ser Gln Cys
    50                  55                  60

Met Pro Gly Ser Gly Ala Thr Thr Thr Thr Thr Ala Thr Arg Thr Thr
65                  70                  75                  80

Thr Ala Gly Ser Gly Thr Thr Thr Thr Thr Thr Ala Gly Gly Gly Thr
                85                  90                  95

Thr Ala Val Pro Ser Asp Asn Pro Phe Ser Gly Val Gln Met Trp Ala
            100                 105                 110

Asn Ser Tyr Tyr Ala Ser Gln Val Ser Ala Tyr Ala Val Pro Thr Met
        115                 120                 125

Gly Ala Ala Ala Ala Ala Val Ala Lys Val Pro Ser Phe Met Trp Met
    130                 135                 140

Asp Thr Leu Ser Lys Val Pro Leu Met Glu Ser Thr Leu Ala Asp Ile
145                 150                 155                 160

Lys Ala Ala Asn Ala Gly Gly Gly Lys Asn Ala Gly Leu Phe Val Ile
                165                 170                 175

Tyr Asp Leu Pro Asp Arg Asp Cys Ala Ala Ser Ala Ser Asn Gly Glu
            180                 185                 190

Tyr Ser Ile Ala Asp Gly Gly Val Ala Lys Tyr Lys Ala Tyr Ile Asp
        195                 200                 205

Ala Val Val Thr Val Leu Lys Lys Tyr Ser Asp Thr Arg Val Ile Leu
    210                 215                 220

Val Ile Glu Pro Asp Ser Leu Ala Asn Leu Val Thr Asn Leu Ser Val
225                 230                 235                 240

Gln Lys Cys Ala Asn Ala Lys Ala Ala Tyr Leu Glu Cys Thr Asn Tyr
                245                 250                 255

Ala Ile Lys Gln Leu Asn Leu Pro Asn Val Ser Met Tyr Leu Asp Ala
            260                 265                 270

Gly His Ala Gly Trp Leu Gly Trp Pro Ala Asn Leu Ser Pro Ala Ala
        275                 280                 285

Thr Leu Tyr Ala Gln Val Tyr Lys Asp Ala Gly Ser Pro Ala Ala Leu
    290                 295                 300

Arg Gly Leu Ala Thr Asn Val Ala Asn Tyr Asn Ala Trp Lys Ile Thr
305                 310                 315                 320

Gly Ser Cys Pro Ser Tyr Thr Gln Gly Asn Ser Val Cys Asp Glu Ser
                325                 330                 335

Gly Tyr Ile His Asn Phe Tyr Pro Leu Leu Lys Ser Leu Gly Trp Asp
            340                 345                 350

Ala Arg Phe Ile Thr Asp Thr Gly Arg Ser Gly Lys Gln Pro Thr Gly
        355                 360                 365

Gln Gln Gln Trp Gly Asp Trp Cys Asn Ser Lys Gly Thr Gly Phe Gly
    370                 375                 380

Leu Arg Pro Ser Ala Ser Thr Gly Asp Glu Leu Leu Asp Ala Phe Val
385                 390                 395                 400

Trp Val Lys Pro Gly Gly Glu Cys Asp Gly Thr Ser Asp Thr Ser Ala
                405                 410                 415

Ala Arg Tyr Asp Tyr His Cys Gly Leu Pro Ala Ala Leu Gln Pro Ala
            420                 425                 430

Pro Glu Ala Gly Ser Trp Phe Gln Ala Tyr Phe Lys Gln Leu Tyr Asp
        435                 440                 445

Asn Ala Ser Pro Ala Phe Ala
    450                 455

<210>  105
<211>  479
<212>  PRT
<213>  Verticillium dahliae

<400>  105

Met Ala Gly Arg Leu Phe Leu Ser Ala Ala Leu Leu Ser Thr Ala Ala
1               5                   10                  15

Phe Ala Ala Pro Leu Glu Glu Arg Gln Ala Cys Ala Ser Gln Trp Gly
            20                  25                  30

Gln Cys Gly Gly Gln Gly Trp Ser Gly Pro Thr Cys Cys Pro Ser Gly
        35                  40                  45

Thr Thr Cys Gln Leu Gln Asn Ala Trp Tyr Ser Gln Cys Leu Pro Gly
    50                  55                  60

Ala Ala Pro Pro Pro Ala Val Thr Thr Thr Arg Pro Ala Thr Thr Ala
65                  70                  75                  80

Ala Ser Ser Thr Arg Pro Ala Thr Thr Ser Ser Val Arg Ser Thr Thr
                85                  90                  95

Val Val Asn Pro Pro Thr Thr Thr Val Ala Pro Pro Pro Gly Thr Thr
            100                 105                 110

Val Ala Pro Pro Pro Gly Gly Ala Thr Tyr Thr Gly Asn Pro Phe Ala
        115                 120                 125

Gly Val Asn Gln Trp Ala Asn Ala Tyr Tyr Arg Ser Glu Val Ser Ser
    130                 135                 140

Leu Ala Val Pro Ser Leu Ser Gly Pro Leu Ala Thr Ala Ala Ala Lys
145                 150                 155                 160

Val Ala Asp Val Pro Thr Phe Gln Trp Met Asp Thr Thr Ala Lys Val
                165                 170                 175

Pro Leu Ile Asp Gly Ala Leu Ala Asp Ile Arg Arg Ala Asn Ala Ala
            180                 185                 190

Gly Gly Asn Tyr Ala Gly Ile Phe Val Val Tyr Asn Leu Pro Asp Arg
        195                 200                 205

Asp Cys Ala Ala Ala Ala Ser Asn Gly Glu Leu Ser Ile Ala Asn Asp
    210                 215                 220

Gly Ile Asn Lys Tyr Lys Ala Tyr Ile Asp Ser Ile Arg Ala Val Leu
225                 230                 235                 240

Leu Lys Tyr Asn Asp Ile Arg Thr Leu Leu Val Ile Glu Pro Asp Ser
                245                 250                 255

Leu Ala Asn Met Val Thr Asn Met Gly Val Ala Lys Cys Ser Asn Ala
            260                 265                 270

Ala Ala Ala Tyr Lys Glu Cys Thr Lys Tyr Ala Val Gln Gln Leu Asp
        275                 280                 285

Leu Pro His Val Ala Gln Tyr Leu Asp Ala Gly His Ala Gly Trp Leu
    290                 295                 300

Gly Trp Pro Ala Asn Ile Gly Pro Ala Ala Thr Ile Phe Thr Asp Ile
305                 310                 315                 320

Tyr Lys Glu Ala Gly Arg Pro Lys Ser Leu Arg Gly Leu Ala Thr Asn
                325                 330                 335

Val Ser Asn Tyr Asn Ala Trp Asn Ala Thr Ser Pro Ala Pro Tyr Thr
            340                 345                 350

Ser Pro Asn Pro Asn Tyr Asp Glu Lys His Tyr Val Asp Ala Phe Ala
        355                 360                 365

Pro Leu Leu Arg Gln Asn Gly Trp Asp Ala Lys Phe Ile Ile Asp Gln
    370                 375                 380

Gly Arg Ser Gly Lys Gln Pro Thr Gly Gln Gln Glu Trp Gly His Trp
385                 390                 395                 400

Cys Asn Ala Leu Gly Thr Gly Phe Gly Leu Arg Pro Thr Ser Asn Thr
                405                 410                 415

Gly His Pro Asp Val Asp Ala Phe Val Trp Val Lys Pro Gly Gly Glu
            420                 425                 430

Ala Asp Gly Thr Ser Asp Thr Thr Ala Val Arg Tyr Asp His Phe Cys
        435                 440                 445

Gly Ser Ala Ser Ser Met Lys Pro Ala Pro Glu Ala Gly Thr Trp Phe
    450                 455                 460

Gln Ala Tyr Phe Glu Gln Leu Leu Arg Asn Ala Asn Pro Ser Phe
465                 470                 475

<210>  106
<211>  481
<212>  PRT
<213>  Thielavia terrestris

<400>  106

Met Ala Gln Lys Leu Leu Leu Ala Ala Ala Leu Ala Ala Ser Ala Leu
1               5                   10                  15

Ala Ala Pro Val Val Glu Glu Arg Gln Asn Cys Gly Ser Val Trp Ser
            20                  25                  30

Gln Cys Gly Gly Ile Gly Trp Ser Gly Ala Thr Cys Cys Ala Ser Gly
        35                  40                  45

Asn Thr Cys Val Glu Leu Asn Pro Tyr Tyr Ser Gln Cys Leu Pro Asn
    50                  55                  60

Ser Gln Val Thr Thr Ser Thr Ser Lys Thr Thr Ser Thr Thr Thr Arg
65                  70                  75                  80

Ser Ser Thr Thr Ser His Ser Ser Gly Pro Thr Ser Thr Ser Thr Thr
                85                  90                  95

Thr Thr Ser Ser Pro Val Val Thr Thr Pro Pro Ser Thr Ser Ile Pro
            100                 105                 110

Gly Gly Ala Ser Ser Thr Ala Ser Trp Ser Gly Asn Pro Phe Ser Gly
        115                 120                 125

Val Gln Met Trp Ala Asn Asp Tyr Tyr Ala Ser Glu Val Ser Ser Leu
    130                 135                 140

Ala Ile Pro Ser Met Thr Gly Ala Met Ala Thr Lys Ala Ala Glu Val
145                 150                 155                 160

Ala Lys Val Pro Ser Phe Gln Trp Leu Asp Arg Asn Val Thr Ile Asp
                165                 170                 175

Thr Leu Phe Ala His Thr Leu Ser Gln Ile Arg Ala Ala Asn Gln Lys
            180                 185                 190

Gly Ala Asn Pro Pro Tyr Ala Gly Ile Phe Val Val Tyr Asp Leu Pro
        195                 200                 205

Asp Arg Asp Cys Ala Ala Ala Ala Ser Asn Gly Glu Phe Ser Ile Ala
    210                 215                 220

Asn Asn Gly Ala Ala Asn Tyr Lys Thr Tyr Ile Asp Ala Ile Arg Ser
225                 230                 235                 240

Leu Val Ile Gln Tyr Ser Asp Ile Arg Ile Ile Phe Val Ile Glu Pro
                245                 250                 255

Asp Ser Leu Ala Asn Met Val Thr Asn Leu Asn Val Ala Lys Cys Ala

260                 265                 270

Asn Ala Glu Ser Thr Tyr Lys Glu Leu Thr Val Tyr Ala Leu Gln Gln
        275                 280                 285

Leu Asn Leu Pro Asn Val Ala Met Tyr Leu Asp Ala Gly His Ala Gly
    290                 295                 300

Trp Leu Gly Trp Pro Ala Asn Ile Gln Pro Ala Ala Asn Leu Phe Ala
305                 310                 315                 320

Glu Ile Tyr Thr Ser Ala Gly Lys Pro Ala Ala Val Arg Gly Leu Ala
                325                 330                 335

Thr Asn Val Ala Asn Tyr Asn Gly Trp Ser Leu Ala Thr Pro Pro Ser
            340                 345                 350

Tyr Thr Gln Gly Asp Pro Asn Tyr Asp Glu Ser His Tyr Val Gln Ala
        355                 360                 365

Leu Ala Pro Leu Leu Thr Ala Asn Gly Phe Pro Ala His Phe Ile Thr
    370                 375                 380

Asp Thr Gly Arg Asn Gly Lys Gln Pro Thr Gly Gln Arg Gln Trp Gly
385                 390                 395                 400

Asp Trp Cys Asn Val Ile Gly Thr Gly Phe Gly Val Arg Pro Thr Thr
                405                 410                 415

Asn Thr Gly Leu Asp Ile Glu Asp Ala Phe Val Trp Val Lys Pro Gly
            420                 425                 430

Gly Glu Cys Asp Gly Thr Ser Asn Thr Thr Ser Pro Arg Tyr Asp Tyr
        435                 440                 445

His Cys Gly Leu Ser Asp Ala Leu Gln Pro Ala Pro Glu Ala Gly Thr
    450                 455                 460

Trp Phe Gln Ala Tyr Phe Glu Gln Leu Leu Thr Asn Ala Asn Pro Pro
465                 470                 475                 480

Phe

<210>  107
<211>  11
<212>  PRT
<213>  Artificial Sequence

<220>
<223>  LPMOA consensus domain sequence

<220>
<221>  VARIANT
<222>  (7)..(7)
<223>  Replace = Leu, Met or Thr

<400>  107

Tyr Val Leu Arg His Glu Ile Ile Ala Leu His
1               5                   10

<210>  108
<211>  346
<212>  PRT
<213>  Trichoderma harzianum

<400>  108

Met Ala Gln Lys Leu Ser Asn Leu Leu Ala Thr Ala Leu Thr Val Ala
1               5                   10                  15

Thr Gly Val Val Gly His Gly His Val Asn Asn Ile Ile Val Asn Gly
            20                  25                  30

Val Tyr Tyr Gln Gly Tyr Asp Pro Thr Ser Phe Pro Tyr Met Ser Asp
        35                  40                  45

Pro Pro Ile Val Ile Gly Trp Thr Ala Ala Asp Leu Asp Asn Gly Phe
    50                  55                  60

Val Ser Pro Asp Ala Tyr Gln Ser Pro Asp Ile Val Cys His Lys Asn
65                  70                  75                  80

Ala Thr Asn Ala Lys Gly His Ala Ser Val Lys Ala Gly Asp Ser Ile
                85                  90                  95

Leu Leu Gln Trp Leu Pro Leu Pro Trp Pro His Pro Gly Pro Ile Val
            100                 105                 110

Asp Tyr Leu Ala Asn Cys Asn Gly Asp Cys Glu Thr Val Asp Lys Thr
        115                 120                 125

Thr Leu Glu Phe Phe Lys Ile Asp Gly Val Gly Leu Ile Ser Gly Ala
    130                 135                 140

Asp Pro Gly Asn Trp Ala Gln Asn Val Leu Val Thr Asn Asn Asn Thr
145                 150                 155                 160

Trp Val Val Gln Ile Pro Lys Asp Leu Gln Thr Gly Asn Tyr Val Leu
                165                 170                 175

Arg His Glu Leu Ile Ala Leu His Ser Ser Gly Gln Pro Asp Gly Ala
            180                 185                 190

Gln Asn Tyr Pro Gln Cys Phe Asn Leu Ala Val Thr Gly Thr Gly Ser
        195                 200                 205

Leu Gln Pro Thr Gly Val Leu Ala Thr Asp Leu Tyr His Glu Ser Asp
    210                 215                 220

Pro Gly Ile Leu Val Asn Ile Tyr Thr Ser Ser Leu Ala Tyr Thr Met
225                 230                 235                 240

Pro Gly Pro Ser Val Val Ala Gly Leu Pro Ser Ser Val Ala Gln Gly
                245                 250                 255

Asn Ser Ala Ala Thr Ala Thr Ser Ser Ala Thr Ile Pro Gly Gly Gly
            260                 265                 270

Gly Ser Ser Ser Ser Arg Ala Ser Ser Ala Thr Arg Thr Ser Ser Gly
        275                 280                 285

Ala Thr Ser Arg Pro Thr Ser Ser Ser Ile Arg Thr Thr Ser Ala Pro
    290                 295                 300

Ala Gly Gly Pro Thr Gln Thr Leu Tyr Gly Gln Cys Gly Gly Ser Gly
305                 310                 315                 320

Tyr Ser Gly Pro Thr Ile Cys Ala Ser Pro Ala Val Cys Thr Thr Leu
                325                 330                 335

Asn Pro Tyr Tyr Ala Gln Cys Val Ser Arg
            340                 345

<210>  109
<211>  349
<212>  PRT
<213>  Hypocrea atroviridis

<400>  109

Met Ala Gln Lys Leu Ser Asn Leu Phe Ala Ile Ala Leu Thr Val Ala
1               5                   10                  15

Thr Gly Val Val Gly His Gly His Val Asn Asn Ile Val Val Asn Gly
            20                  25                  30

Val Tyr Tyr Gln Gly Tyr Asp Pro Thr Ser Phe Pro Tyr Met Pro Asp
        35                  40                  45

Pro Pro Ile Val Val Gly Trp Thr Ala Ala Asp Thr Asp Asn Gly Phe
    50                  55                  60

Val Ser Pro Asp Ala Tyr Gln Thr Pro Asp Ile Val Cys His Lys Asn
65                  70                  75                  80

Gly Thr Asn Ala Lys Gly His Ala Ser Val Lys Ala Gly Asp Ser Val
                85                  90                  95

Leu Phe Gln Trp Val Pro Val Pro Trp Pro His Lys Ser Thr Val Val
            100                 105                 110

Asp Tyr Leu Ala Asn Cys Asn Gly Pro Cys Glu Thr Val Asp Lys Thr
        115                 120                 125

Thr Leu Glu Phe Phe Lys Ile Asp Gly Ile Gly Leu Leu Ser Gly Gly
    130                 135                 140

Asn Pro Gly Thr Trp Gly Ser Asp Val Leu Ile Gly Asn Asn Asn Thr
145                 150                 155                 160

Trp Val Ile Gln Ile Pro Glu Asp Leu Gln Thr Gly Asn Tyr Val Leu
                165                 170                 175

Arg His Glu Leu Ile Ala Leu His Ser Ala Glu Gln Ala Asp Gly Ala
            180                 185                 190

Gln Asn Tyr Pro Gln Cys Phe Asn Leu Ala Val Thr Gly Thr Gly Ser
        195                 200                 205

Leu Gln Pro Ser Gly Val Leu Ala Thr Asp Leu Tyr His Glu Thr Asp
    210                 215                 220

Pro Gly Ile Leu Phe Asn Ile Tyr Thr Ser Pro Leu Thr Tyr Ile Ile
225                 230                 235                 240

Pro Gly Pro Thr Val Val Ser Gly Leu Pro Ser Ser Val Ala Gln Ala
                245                 250                 255

Ser Ser Ala Ala Thr Ala Thr Ser Ser Ala Thr Val Ser Gly Gly Gly
            260                 265                 270

Gly Gly Ser Ser Thr Gly Gly Ser Thr Ser Lys Thr Thr Thr Val Val
        275                 280                 285

Arg Ser Thr Thr Ser Val Thr Ser Lys Ala Ser Ser Ser Thr Ala Val
    290                 295                 300

Thr Thr Pro Pro Pro Ala Gly Gly Thr Gln Thr Leu Tyr Gly Gln Cys
305                 310                 315                 320

Gly Gly Ser Gly Tyr Ser Gly Pro Thr Lys Cys Ala Ser Pro Ala Val
                325                 330                 335

Cys Thr Thr Leu Asn Pro Tyr Tyr Ala Gln Cys Leu Asn
            340                 345

<210>  110
<211>  347
<212>  PRT
<213>  Hypocrea virens

<400>  110

Met Thr Gln Lys Leu Thr Ser Leu Leu Val Thr Ala Leu Thr Val Ala
1               5                   10                  15

Thr Gly Val Ile Gly His Gly His Val Asn Asn Ile Val Ile Asn Gly
            20                  25                  30

Ala Tyr Tyr Gln Gly Tyr Asp Pro Thr Leu Phe Pro Tyr Glu Pro Asn
        35                  40                  45

Pro Pro Ile Val Val Gly Trp Thr Ala Ser Asp Thr Asp Asn Gly Phe
    50                  55                  60

Val Ala Pro Asp Ala Tyr Gln Ser Pro Asp Ile Ile Cys His Arg Asn
65                  70                  75                  80

Ala Thr Asn Ala Arg Gly His Ala Ser Val Met Ala Gly Ser Ser Val
                85                  90                  95

Leu Ile Gln Trp Val Pro Ile Pro Trp Pro His Pro Gly Pro Val Leu
            100                 105                 110

Asp Tyr Leu Ala Asn Cys Asn Gly Asp Cys Glu Thr Val Asp Lys Thr
        115                 120                 125

Thr Leu Glu Phe Phe Lys Ile Asp Gly Ile Gly Leu Ile Ser Gly Gly
    130                 135                 140

Asn Pro Gly Arg Trp Ala Ser Asp Val Leu Ile Gly Asn Asn Gly Thr
145                 150                 155                 160

Trp Val Val Gln Ile Pro Ala Asp Leu Glu Thr Gly Asn Tyr Val Leu
                165                 170                 175

Arg His Glu Leu Ile Ala Leu His Ser Ala Gly Ser Val Asp Gly Ala
            180                 185                 190

Gln Asn Tyr Pro Gln Cys Phe Asn Leu Ala Val Thr Gly Thr Gly Ser
        195                 200                 205

Leu Gln Pro Thr Gly Val Leu Gly Thr Lys Leu Tyr Gln Glu Ser Asp
    210                 215                 220

Pro Gly Ile Leu Phe Asn Ile Tyr Thr Ser Pro Leu Thr Tyr Thr Ile
225                 230                 235                 240

Pro Gly Pro Thr Val Val Ser Gly Leu Pro Ser Ser Val Thr Gln Arg
                245                 250                 255

Ser Ser Thr Ala Thr Ala Thr Ser Ile Ala Thr Val Pro Gly Ser Val
            260                 265                 270

Ser Thr Gly Gly Thr Ser Ser Lys Thr Thr Thr Val Pro Arg Ser Thr
        275                 280                 285

Ser Ser Ala Thr Thr Arg Arg Ser Ser Ser Ser Ala Ile Thr Thr Ser
    290                 295                 300

Ala Pro Ala Gly Pro Ser Gln Thr Leu Tyr Gly Gln Cys Gly Gly Ser
305                 310                 315                 320

Gly Tyr Ser Gly Pro Thr Ile Cys Ala Ser Pro Ala Val Cys Ser Thr
                325                 330                 335

Leu Asn Pro Tyr Tyr Ala Gln Cys Leu Thr Arg
            340                 345

<210>  111
<211>  350
<212>  PRT
<213>  Trichoderma gamsii

<400>  111

Met Ala Gln Lys Leu Ser Asn Leu Phe Ala Ile Ala Leu Thr Val Ala
1               5                   10                  15

Thr Gly Val Val Gly His Gly His Val Asn Asn Ile Val Val Asn Gly
            20                  25                  30

Val Tyr Tyr Gln Gly Tyr Asp Pro Thr Ser Phe Pro Tyr Met Gln Asn
        35                  40                  45

Pro Pro Ile Val Val Gly Trp Thr Ala Ala Asp Thr Asp Asn Gly Phe
    50                  55                  60

Val Ser Pro Asp Ala Tyr Gln Ser Pro Asp Ile Val Cys His Arg Ser
65                  70                  75                  80

Ser Ala Asn Ala Lys Gly His Ala Ser Val Lys Ala Gly Asp Ser Val
                85                  90                  95

Leu Leu Gln Trp Val Pro Val Pro Trp Pro His Lys Ser Ala Val Val
            100                 105                 110

Asp Tyr Leu Ala Asn Cys Asn Gly Pro Cys Glu Thr Val Asp Lys Thr
        115                 120                 125

Thr Leu Glu Phe Phe Lys Ile Asp Ala Val Gly Leu Leu Ser Gly Ala
    130                 135                 140

Asn Pro Gly Thr Trp Gly Ser Asp Val Leu Ile Gly Asn Asn Asn Thr
145                 150                 155                 160

Trp Val Val Gln Ile Pro Glu Asp Leu Gln Thr Gly Asn Tyr Val Leu
                165                 170                 175

Arg His Glu Ile Ile Ala Leu His Ser Ala Ala Gln Val Asp Gly Ala
            180                 185                 190

Gln Asn Tyr Pro Gln Cys Phe Asn Leu Ala Val Thr Gly Thr Gly Ser
        195                 200                 205

Leu Gln Pro Ser Gly Val Leu Ala Thr Asp Leu Tyr Gln Glu Thr Asp
    210                 215                 220

Pro Gly Ile Leu Phe Asn Leu Tyr Thr Ser Pro Leu Thr Tyr Thr Ile
225                 230                 235                 240

Pro Gly Pro Thr Val Val Ala Gly Leu Pro Ser Ser Val Ala Gln Gly
                245                 250                 255

Ser Ser Thr Ala Thr Ala Thr Ser Ser Ala Thr Val Ser Gly Gly Gly
            260                 265                 270

Gly Gly Gly Ser Ser Ser Thr Gly Gly Ser Thr Ser Lys Thr Thr Thr
        275                 280                 285

Val Lys Thr Thr Ser Ser Val Thr Ser Lys Ala Ser Ser Ser Thr Ala
    290                 295                 300

Val Thr Thr Pro Pro Pro Ala Gly Gly Thr Gln Thr Leu Tyr Gly Gln
305                 310                 315                 320

Cys Gly Gly Ser Gly Tyr Ser Gly Pro Thr Lys Cys Ala Ser Pro Ala
                325                 330                 335

Val Cys Thr Thr Leu Asn Pro Tyr Tyr Ala Gln Cys Val Thr
            340                 345                 350

<210>  112
<211>  330
<212>  PRT
<213>  Pochonia chlamydosporia

<400>  112

Met Leu Glu Thr His His Leu Leu Thr Leu Leu Ala Tyr Ala Ile Thr
1               5                   10                  15

Ala Ser Ala His Gly His Ile Ser Asn Val Val Ile Asn Gly Val Ser
            20                  25                  30

Tyr Pro Gly Tyr Asp Ser Pro Ala Trp Tyr Gly Asn Pro Asn Ala Pro
        35                  40                  45

Asn Val Phe Gly Trp Thr Ile Ser Gln Gln Asp Asn Gly Phe Val Ser
    50                  55                  60

Pro Asp Ala Phe Gly Ser Ser Asp Ile Ile Cys His Arg Glu Ala Val
65                  70                  75                  80

Pro Ala Lys Ser His Val Gln Val Ala Ala Gly Asp Ile Leu Thr Leu
                85                  90                  95

Gln Trp Thr Pro Trp Pro Asp Ser His His Gly Pro Val Ile Asp Tyr
            100                 105                 110

Leu Ala Asn Cys Asn Gly Pro Cys Glu Ser Val Asp Lys Thr Thr Leu
        115                 120                 125

Glu Phe Phe Lys Ile Asp Gly Val Gly Ile Ile Ser Gln Gly Ser Pro
    130                 135                 140

Gly Thr Tyr Ala Asp Asp Val Leu Ile Ser Lys Asn Asn Thr Trp Glu
145                 150                 155                 160

Val Lys Ile Pro Ala Asn Ile Ala Pro Gly Asn Tyr Val Leu Arg His
                165                 170                 175

Glu Ile Ile Ala Leu His Ser Ala Gly Ser Glu Asn Gly Ala Gln Ala
            180                 185                 190

Tyr Pro Gln Cys Phe Asn Leu Lys Ile Thr Gly Ser Gly Ser Leu Lys
        195                 200                 205

Pro Ala Gly Val Lys Gly Thr Ala Leu Tyr Lys Ala Thr Asp Pro Gly
    210                 215                 220

Ile Leu Val Asn Ile Tyr Thr Ser Ser Val Asn Tyr Ile Val Pro Gly
225                 230                 235                 240

Pro Thr Leu Val Ala Gly Leu Pro Ser Ser Val Ala Gln Asn Pro Ser
                245                 250                 255

Gln Ile Thr Ala Ser Ala Thr Ala Thr Pro Gly Asp Gln Thr Gly Pro
            260                 265                 270

Gly Thr Pro Thr Thr Thr Leu Ala Thr Ser Val Lys Thr Ser Ser Gly
        275                 280                 285

Ser Ser Gly Gly Leu Gln Thr Val Tyr Gly Gln Cys Gly Gly Gln Ser
    290                 295                 300

Trp Thr Gly Pro Thr Ala Cys Val Gly Gly Ala Lys Cys Ala Thr Gln
305                 310                 315                 320

Asn Ala Tyr Tyr Ala Gln Cys Thr Pro Ala
                325                 330

<210> 113
<211> 342
<212> PRT
<213> Myceliophthora thermophila

<400> 113

Met Ser Lys Ala Ser Ala Leu Leu Ala Gly Leu Thr Gly Ala Ala Leu
1               5                   10                  15

Val Ala Ala His Gly His Val Ser His Ile Val Val Asn Gly Val Tyr
            20                  25                  30

Tyr Arg Asn Tyr Asp Pro Thr Thr Asp Trp Tyr Gln Pro Asn Pro Pro
        35                  40                  45

Thr Val Ile Gly Trp Thr Ala Ala Asp Gln Asp Asn Gly Phe Val Glu
    50                  55                  60

Pro Asn Ser Phe Gly Thr Pro Asp Ile Ile Cys His Lys Ser Ala Thr
65                  70                  75                  80

Pro Gly Gly Gly His Ala Thr Val Ala Ala Gly Asp Lys Ile Asn Ile
                85                  90                  95

Val Trp Thr Pro Glu Trp Pro Glu Ser His Ile Gly Pro Val Ile Asp
            100                 105                 110

Tyr Leu Ala Ala Cys Asn Gly Asp Cys Glu Thr Val Asp Lys Ser Ser
        115                 120                 125

Leu Arg Trp Phe Lys Ile Asp Gly Ala Gly Tyr Asp Lys Ala Ala Gly
    130                 135                 140

Arg Trp Ala Ala Asp Ala Leu Arg Ala Asn Gly Asn Ser Trp Leu Val
145                 150                 155                 160

Gln Ile Pro Ser Asp Leu Lys Ala Gly Asn Tyr Val Leu Arg His Glu
                165                 170                 175

Ile Ile Ala Leu His Gly Ala Gln Ser Pro Asn Gly Ala Gln Ala Tyr
            180                 185                 190

Pro Gln Cys Ile Asn Leu Arg Val Thr Gly Gly Gly Ser Asn Leu Pro
        195                 200                 205

Ser Gly Val Ala Gly Thr Ser Leu Tyr Lys Ala Thr Asp Pro Gly Ile
    210                 215                 220

Leu Phe Asn Pro Tyr Val Ser Ser Pro Asp Tyr Thr Val Pro Gly Pro
225                 230                 235                 240

Ala Leu Ile Ala Gly Ala Ala Ser Ser Ile Ala Gln Ser Thr Ser Val
                245                 250                 255

Ala Thr Ala Thr Gly Thr Ala Thr Val Pro Gly Gly Gly Gly Ala Asn
            260                 265                 270

Pro Thr Ala Thr Thr Thr Ala Ala Thr Ser Ala Ala Pro Ser Thr Thr
        275                 280                 285

Leu Arg Thr Thr Thr Thr Ser Ala Ala Gln Thr Thr Ala Pro Pro Ser

290                 295                 300

Gly Asp Val Gln Thr Lys Tyr Gly Gln Cys Gly Gly Asn Gly Trp Thr
305                 310                 315                 320

Gly Pro Thr Val Cys Ala Pro Gly Ser Ser Cys Ser Val Leu Asn Glu
                325                 330                 335

Trp Tyr Ser Gln Cys Leu
            340

<210>  114
<211>  355
<212>  PRT
<213>  Penicillium oxalicum

<400>  114

Met Phe Lys Ala Leu Ala Thr Ile Leu Leu Ala Glu Leu Val Ala Gly
1               5                   10                  15

His Gly His Val Ser Asn Ile Val Val Asn Gly Val Ser Tyr Gln Gly
            20                  25                  30

Trp Asp Ile Gly Ser Phe Pro Tyr Leu Ser Asn Pro Pro Val Val Ala
        35                  40                  45

Ala Trp Gly Thr Pro Asn Thr Gly Asn Gly Phe Val Thr Pro Ser Asn
    50                  55                  60

Phe Gly Ser Pro Asp Ile Ile Cys His Gln Asp Ala Lys Asn Ala Lys
65                  70                  75                  80

Gly His Val Val Val Ala Ala Gly Asp Lys Ile Asn Leu Gln Trp Thr
                85                  90                  95

Thr Trp Pro Glu Thr His His Gly Pro Val Ile Thr Tyr Leu Ala Asp
            100                 105                 110

Cys Gly Gly Ser Cys Glu Thr Val Asp Lys Thr Thr Leu Lys Phe Phe
        115                 120                 125

Lys Ile Asp Gly Val Gly Leu Val Asp Asp Ser Thr Pro Pro Gly Thr
    130                 135                 140

Trp Gly Asp Asp Gln Met Ile Ala Asn Gly Asn Ser Trp Met Val Glu
145                 150                 155                 160

Ile Pro Pro Lys Leu Ala Pro Gly Asn Tyr Val Leu Arg His Glu Leu

165                 170                 175

Ile Ala Leu His Ser Ala Gly Ser Glu Asp Gly Ala Gln Asn Tyr Pro
            180                 185                 190

Gln Cys Phe Asn Leu Gln Val Thr Gly Ser Gly Ser Asp Lys Pro Ser
        195                 200                 205

Gly Val Leu Gly Thr Glu Leu Tyr Arg Ala Thr Asp Pro Gly Ile Leu
    210                 215                 220

Val Asn Ile Tyr Gln Ser Leu Ala Ser Tyr Ala Ile Pro Gly Pro Ala
225                 230                 235                 240

Pro Tyr Thr Gly Ala Val Ser Ile Ala Gln Ala Thr Ser Ala Ile Thr
                245                 250                 255

Ala Ser Gly Thr Pro Val Thr Gly Ser Gly Ala Ala Pro Thr Thr Thr
            260                 265                 270

Ala Gly Gly Ala Gly Pro Gly Leu Gly Gln Ser Ser Thr Thr Thr Ser
        275                 280                 285

Ser Thr Thr Leu Ile Thr Lys Thr Thr Thr Thr Thr Ala Pro Arg Thr
    290                 295                 300

Thr Thr Ser Ser Gly Ser Gly Ser Gly Ser Gly Gly Thr Gln Thr Val
305                 310                 315                 320

Tyr Gly Gln Cys Gly Gly Phe Ser Trp Ser Gly Pro Thr Ala Cys Ala
                325                 330                 335

Ala Lys Ala Thr Cys Thr Ser Tyr Asn Pro Tyr Tyr Ala Gln Cys Val
            340                 345                 350

Pro Thr Ser
        355

<210>  115
<211>  437
<212>  PRT
<213>  Rosellinia necatrix

<400>  115

Met Asn Ser Leu Arg Ser Ser Ser Leu Leu Ala Ala Leu Thr Thr Ala
1               5                   10                  15

Val Gly Val Ala Ala His Gly His Val Thr Asn Leu Val Ile Asn Gly

            20                  25                  30

Val Ser Tyr Gln Thr Tyr Asp Pro Thr Val Phe Pro Tyr Met Ser Asp
        35                  40                  45

Pro Pro Val Val Val Gly Trp Thr Ala Ser Asn Thr Asp Asn Gly Phe
    50                  55                  60

Val Glu Pro Asn Ser Phe Asp Ser Gly Asp Ile Val Cys His Lys Ala
65                  70                  75                  80

Ala Thr Pro Ala Gly Gly Tyr Ala Gln Val Ala Ala Gly Asp Ser Ile
                85                  90                  95

Met Ile Lys Trp Asp Thr Trp Pro Glu Ser His Lys Gly Pro Met Met
            100                 105                 110

Asp Tyr Leu Ala Ala Cys Asn Gly Pro Cys Asp Thr Val Asp Lys Met
        115                 120                 125

Ala Leu Glu Phe Phe Lys Ile Asp Glu Ala Gly Ile Leu Asp Ser Ser
    130                 135                 140

Ser Ala Pro Gly Arg Trp Ala Ser Asp Val Leu Ile Glu Asn Asn Asn
145                 150                 155                 160

Ser Trp Leu Val Gln Ile Pro Ala Ser Leu Lys Pro Gly Asn Tyr Val
                165                 170                 175

Leu Arg His Glu Thr Ile Ala Leu His Ser Ala Gly Gln Ala Asn Gly
            180                 185                 190

Ala Gln Asn Tyr Pro Gln Cys Phe Asn Leu Glu Val Thr Gly Ser Gly
        195                 200                 205

Thr Asp Val Pro Ala Gly Val Leu Ala Thr Glu Leu Tyr Thr Pro Thr
    210                 215                 220

Asp Pro Gly Val Glu Val Asn Ile Tyr Val Ser Gly Leu Asp Tyr Gln
225                 230                 235                 240

Ile Pro Gly Pro Thr Leu Met Ala Gly Val Ser Ser Thr Val Ala Gln
                245                 250                 255

Ala Thr Ser Ala Ile Thr Ala Ser Ala Ser Ala Thr Val Gly Ser Gly
            260                 265                 270

Ser Asp Ser Gly Ser Gly Ser Gly Ser Gly Thr Ser Ala Val Ala Thr
        275                 280                 285

Thr Thr Thr Thr Ala Glu Ala Thr Thr Ala Pro Ala Asn Asp Ala Thr
    290                 295                 300

Ser Ser Ser Ala Ser Ser Ala Ala Gln Ala Thr Thr Ser Ala Ala Ala
305                 310                 315                 320

Pro Ser Ser Thr Ala Ala Gln Gln Thr Thr Thr Leu Val Thr Ser Thr
                325                 330                 335

Arg Ala Ala Ser Ser Ser Ala Ser Ala Pro Ala Thr Thr Asp Ala Ala
            340                 345                 350

Thr Thr Ala Lys Pro Thr Thr Thr Ala Ala Glu Ala Thr Thr Arg Thr
        355                 360                 365

Gln Thr Gly Asp Val Ala Ala Gln Thr Leu Tyr Gly Gln Cys Gly Gly
    370                 375                 380

Ser Asn Trp Thr Gly Pro Val Ala Cys Ala Lys Gly Thr Cys Thr Glu
385                 390                 395                 400

Tyr Asn Pro Tyr Tyr Ser Gln Cys Val Glu Asp Glu Val Ala Gln Ala
                405                 410                 415

Gln Ala Gln Ala Gln Ala Gln Ala Ser Gln Ala Gly Ser Ala Cys Asn
            420                 425                 430

Leu Ser Gly Ser Lys
        435

<210>  116
<211>  361
<212>  PRT
<213>  Penicillium expansum

<400>  116

Met Tyr Ser Leu Leu Gly Pro Val Leu Phe Ser Ser Leu Val Ala Ala
1               5                   10                  15

His Gly His Val Thr Asn Ile Val Ile Asn Gly Val Ser Phe Asn Gly
            20                  25                  30

Trp Asp Ile Asn Ser Tyr Pro Tyr Glu Ser Ser Pro Pro Thr Val Val
        35                  40                  45

Ala Trp Gly Thr Pro Asn Thr Gly Asn Gly Phe Ile Ala Pro Asp Ala
    50                  55                  60

Tyr Gln Thr Ser Asp Ile Ile Cys His Leu Asn Ala Thr Asn Ala Lys
65                  70                  75                  80

Gly His Ala Val Val Ala Ala Gly Asp Arg Val Phe Leu Gln Trp Thr
                85                  90                  95

Asp Trp Pro Glu Ser His His Gly Pro Val Ile Asp Tyr Leu Ala Ser
            100                 105                 110

Cys Gly Ser Ser Gly Cys Glu Thr Val Asp Lys Thr Thr Leu Lys Phe
        115                 120                 125

Phe Lys Ile Asp Gly Val Gly Leu Val Asp Asp Thr Thr Val Pro Gly
    130                 135                 140

Thr Trp Gly Asp Asp Gln Leu Ile Ala Gln Lys Ser Gly Trp Met Val
145                 150                 155                 160

Glu Ile Pro His Thr Ile Ala Pro Gly His Tyr Val Leu Arg His Glu
                165                 170                 175

Leu Ile Ala Leu His Ser Ala Gly Thr Glu Asp Gly Ala Gln Asn Tyr
            180                 185                 190

Pro Gln Cys Phe Asn Leu Gln Val Thr Gly Ser Gly Ser Asp Gln Pro
        195                 200                 205

Ala Gly Val Leu Gly Thr Asn Leu Tyr Arg Pro Thr Asp Pro Gly Ile
    210                 215                 220

Ile Val Asn Ile Tyr Thr Ser Leu Ser Thr Tyr Val Val Pro Gly Pro
225                 230                 235                 240

Thr Leu Tyr Ser Gly Ala Val Ser Ile Thr Gln Thr Thr Ser Ala Ile
                245                 250                 255

Thr Ala Ser Ser Thr Pro Gly Ala Gly Thr Gly Ser Ala Thr Ala Gly
            260                 265                 270

Ser Thr Thr Gly Ser Ala Ser Lys Ala Thr Thr Thr Thr Leu Val Thr
        275                 280                 285

Thr Thr Arg Pro Thr Ser Thr Thr Thr Thr Ser Lys Lys Ile Ala Ile
    290                 295                 300

Ala Ser Thr Thr Thr Thr Thr Thr Ser Ala Ala Ala Gly Gly Ser Gly
305                 310                 315                 320

Ala Thr Gln Thr Leu Tyr Gly Gln Cys Gly Gly Thr Gly Trp Thr Gly
                325                 330                 335

Ala Thr Ala Cys Ala Ser Pro Ala Ala Cys Ser Ser Ser Asn Pro Trp
            340                 345                 350

Tyr Ser Gln Cys Val Ser Thr Ala Val
        355                 360

<210>  117
<211>  355
<212>  PRT
<213>  Penicillium patulum

<400>  117

Met Phe Thr Phe Leu Arg Pro Ala Leu Phe Ala Ser Leu Val Ala Ala
1               5                   10                  15

His Gly His Val Thr Asn Ile Val Ile Asn Gly Val Ser Phe Asp Gly
            20                  25                  30

Trp Asp Ile Gly Ser Tyr Pro Tyr Thr Asp Asn Pro Pro Asp Val Val
        35                  40                  45

Ala Trp Gly Thr Pro Asn Thr Ser Asn Gly Phe Val Ala Pro Asp Ala
    50                  55                  60

Tyr Gly Thr Ser Asp Ile Ile Cys His Leu Glu Ala Lys Asn Ala Lys
65                  70                  75                  80

Gly His Ala Val Val Ala Ala Gly Asp Arg Val Phe Leu Gln Trp Thr
                85                  90                  95

Pro Asp Trp Pro Glu Ser His His Gly Pro Ile Ile Asp Tyr Leu Ala
            100                 105                 110

Ser Cys Gly Glu Ser Gly Cys Glu Thr Val Asp Lys Asn Thr Leu Lys
        115                 120                 125

Phe Phe Lys Ile Asp Gly Val Gly Leu Val Asp Gly Ser Lys Pro Pro
    130                 135                 140

Gly Phe Trp Ala Asp Asp Gln Leu Ile Ala Gln Asn Ser Gly Trp Met
145                 150                 155                 160

Val Glu Ile Pro Ala Asn Ile Ala Pro Gly His Tyr Val Leu Arg His
                165                 170                 175

Glu Met Ile Ala Leu His Gly Ala Gly Ser Val Gly Gly Thr Gln Asn
            180                 185                 190

Tyr Pro Gln Cys Phe Asn Leu Gln Ile Thr Gly Ser Gly Thr Asp Lys
        195                 200                 205

Pro Ala Gly Val Leu Gly Thr Asp Leu Tyr Ser Pro Thr Asp Pro Gly
    210                 215                 220

Ile Leu Val Asn Ile Tyr Thr Ser Leu Ala Ser Tyr Ser Leu Ala Asn
225                 230                 235                 240

Ala Phe Gly Gln Val Val Pro Gly Pro Thr Leu Tyr Ser Gly Ala Val
                245                 250                 255

Ser Ile Thr Gln Thr Thr Ser Ala Ile Thr Ala Ser Gly Thr Pro Ile
            260                 265                 270

Thr Gly Thr Gly Gly Gly Gly Thr Gly Pro Thr Thr Thr Ser Lys Val
        275                 280                 285

Thr Thr Thr Thr Leu Ala Thr Thr Thr Lys Pro Asn Thr Thr Thr Lys
    290                 295                 300

Thr Thr Thr Thr Thr Ala Gly Gly Gly Gly Ala Ala Gln Thr Pro Tyr
305                 310                 315                 320

Gly Gln Cys Gly Gly Ser Gly Trp Thr Gly Pro Thr Ala Cys Ala Pro
                325                 330                 335

Pro Ala Ala Cys Ser Ser Val Asn Glu Trp Tyr Ser Gln Cys Leu Pro
            340                 345                 350

Thr Gly Ala
        355

<210>  118
<211>  377
<212>  PRT
<213>  Eutypa lata

<400>  118

Met Ser Asn Asn Met Lys Thr Ser Ser Leu Ile Ala Ala Leu Ala Ser
1               5                   10                  15

Ala Ala Thr Val Ala Ala His Gly His Val Ser Asn Ile Val Val Asn
            20                  25                  30

Gly Val Ser Tyr Gln Gly Tyr Asp Pro Thr Thr Met Pro Tyr Met Thr
        35                  40                  45

Asp Pro Pro Asp Val Ile Gly Trp Thr Ala Asp Asp Thr Asp Asn Gly
    50                  55                  60

Phe Val Ser Pro Ser Ser Tyr Gly Ala Ala Asp Ile Val Cys His Lys
65                  70                  75                  80

Ser Ala Thr Pro Gly Gly Ala His Val Glu Val Ala Ala Gly Asp Ser
                85                  90                  95

Ile Ser Ile Gln Trp Asp Thr Trp Pro Glu Ser His Lys Gly Pro Met
            100                 105                 110

Ile Asp Tyr Leu Ala Ala Cys Ser Gly Asn Cys Glu Asp Ser Asp Lys
        115                 120                 125

Thr Ala Leu Glu Phe Phe Lys Ile Asp Gly Ala Gly Tyr Asp Ala Ala
    130                 135                 140

Ser Ser Thr Trp Ala Ser Asp Val Leu Ile Ala Asn Asn Asn Thr Trp
145                 150                 155                 160

Leu Val Gln Ile Pro Ser Ser Ile Ala Pro Gly Asn Tyr Val Leu Arg
                165                 170                 175

His Glu Thr Ile Ala Leu His Ser Ala Gly Ser Glu Asp Gly Ala Gln
            180                 185                 190

Asn Tyr Pro Gln Cys Phe Asn Leu Ala Val Thr Gly Ser Gly Thr Glu
        195                 200                 205

Val Pro Ala Gly Thr Leu Gly Thr Glu Leu Tyr Lys Ile Asp Asp Ala
    210                 215                 220

Gly Ile Val Val Asp Ile Tyr Asn Gly Leu Ser Ser Tyr Ala Val Pro
225                 230                 235                 240

Gly Pro Thr Leu Ile Ala Gly Ala Ser Ser Ser Val Ala Gln Ser Ser
                245                 250                 255

Ser Ala Val Thr Ala Ser Gly Thr Ala Thr Val Gly Ser Gly Ser Gly
            260                 265                 270

Ala Thr Thr Thr Ala Glu Gln Ala Thr Ser Gln Asp Pro Thr Ser Glu
        275                 280                 285

Ala Thr Ser Val Ala Gln Pro Thr Ser Thr Ala Glu Pro Thr Thr Thr
    290                 295                 300

Ala Gln Pro Thr Thr Thr Ala Pro Ser Thr Leu Val Thr Ala Thr Arg
305                 310                 315                 320

Ala Pro Thr Thr Thr Thr Ala Ala Pro Thr Thr Thr Ala Ala Pro Thr
                325                 330                 335

Gly Gly Val Gly Ala Ala Ala Leu Tyr Gly Gln Cys Gly Gly Asn Gly
            340                 345                 350

Trp Thr Gly Ala Thr Thr Cys Ala Glu Gly Thr Cys Val Leu Gln Asn
        355                 360                 365

Asp Tyr Tyr Ser Gln Cys Ile Asn Ala
    370                 375

<210>  119
<211>  344
<212>  PRT
<213>  Madurella mycetomatis

<400>  119

Met Ser Lys Thr Ser Ala Val Leu Ala Ser Leu Ala Gly Ala Ala Leu
1               5                   10                  15

Val Ala Ala His Gly His Val Ser His Ile Ile Val Asn Gly Val Tyr
            20                  25                  30

Tyr Glu Asn Tyr Asp Pro Thr Thr His Ser Tyr Gln Pro Asn Pro Pro
        35                  40                  45

Thr Val Ile Gly Trp Ser Ala Asp Gln Gln Asp Asn Gly Phe Val Glu
    50                  55                  60

Pro Asn Asn Phe Gly Thr Ser Asp Ile Ile Cys His Lys Ala Gly Ala
65                  70                  75                  80

Pro Gly Gly Gly His Ala Thr Val Asn Ala Gly Asp Lys Ile Ser Ile
                85                  90                  95

Val Trp Thr Pro Glu Trp Pro Glu Ser His Ile Gly Pro Val Ile Asp
            100                 105                 110

Tyr Leu Ala Ala Cys Asn Gly Asp Cys Glu Thr Val Asn Lys Glu Ser
        115                 120                 125

Leu Arg Trp Phe Lys Ile Asp Gly Ala Gly Tyr Asn Ser Asp Thr Gly
    130                 135                 140

Thr Trp Ala Ala Asp Asp Leu Arg Ala Asn Gly Asn Ser Trp Leu Val
145                 150                 155                 160

Gln Ile Pro Ser Asp Leu Ser Asp Gly Asn Tyr Val Leu Arg His Glu
                165                 170                 175

Ile Ile Ala Leu His Gly Ala Ser Ser Pro Asn Gly Ala Gln Ala Tyr
            180                 185                 190

Pro Gln Cys Ile Asn Leu Arg Val Thr Gly Gly Gly Ser Asn Ser Pro
        195                 200                 205

Ser Gly Val Ala Gly Thr Ser Leu Tyr Ser Ala Asn Asp Pro Gly Ile
    210                 215                 220

Leu Phe Asn Pro Tyr Ser Gly Ser Val Asn Tyr Pro Val Pro Gly Pro
225                 230                 235                 240

Ala Leu Ile Ala Gly Ala Val Ser Ser Ile Ala Gln Ser Gln Ser Ala
                245                 250                 255

Ala Thr Arg Thr Ala Ser Ala Thr Ala Pro Gly Gly Gly Gly Gly Asn
            260                 265                 270

Pro Glu Pro Thr Asn Pro Ala Pro Thr Thr Thr Arg Gly Ala Val Thr
        275                 280                 285

Thr Thr Leu Gln Thr Val Thr Gln Ala Pro Glu Pro Thr Thr Pro Pro
    290                 295                 300

Ala Gly Gly Ala Glu Gln Thr Arg Trp Gly Gln Cys Gly Gly Ser Gly
305                 310                 315                 320

Trp Val Ser Pro Thr Ala Cys Val Ala Gly Ser Ser Cys Ser Val Ile
                325                 330                 335

Asn Ala Tyr Tyr His Gln Cys Val
            340

<210>  120

<211>  368
<212>  PRT
<213>  Thielavia terrestris

<400>  120
   Met Pro Ser Phe Ala Ser Lys Thr Leu Leu Ser Thr Leu Ala Gly Ala
   1               5                   10                  15
   Ala Ser Val Ala Ala His Gly His Val Ser Asn Ile Val Ile Asn Gly
               20                  25                  30
   Val Ser Tyr Gln Gly Tyr Asp Pro Thr Ser Phe Pro Tyr Met Gln Asn
           35                  40                  45
   Pro Pro Ile Val Val Gly Trp Thr Ala Ala Asp Thr Asp Asn Gly Phe
       50                  55                  60
   Val Ala Pro Asp Ala Phe Ala Ser Gly Asp Ile Ile Cys His Lys Asn
   65                  70                  75                  80
   Ala Thr Asn Ala Lys Gly His Ala Val Val Ala Ala Gly Asp Lys Ile
                   85                  90                  95
   Phe Ile Gln Trp Asn Thr Trp Pro Glu Ser His His Gly Pro Val Ile
               100                 105                 110
   Asp Tyr Leu Ala Ser Cys Gly Ser Ala Ser Cys Glu Thr Val Asp Lys
           115                 120                 125
   Thr Lys Leu Glu Phe Phe Lys Ile Asp Glu Val Gly Leu Val Asp Gly
       130                 135                 140
   Ser Ser Ala Pro Gly Val Trp Gly Ser Asp Gln Leu Ile Ala Asn Asn
   145                 150                 155                 160
   Asn Ser Trp Leu Val Glu Ile Pro Pro Thr Ile Ala Pro Gly Asn Tyr
                   165                 170                 175
   Val Leu Arg His Glu Ile Ile Ala Leu His Ser Ala Glu Asn Ala Asp
               180                 185                 190
   Gly Ala Gln Asn Tyr Pro Gln Cys Phe Asn Leu Gln Ile Thr Gly Thr
           195                 200                 205
   Gly Thr Ala Thr Pro Ser Gly Val Pro Gly Thr Ser Leu Tyr Thr Pro
       210                 215                 220
   Thr Asp Pro Gly Ile Leu Val Asn Ile Tyr Ser Ala Pro Ile Thr Tyr
   225                 230                 235                 240
   Thr Val Pro Gly Pro Ala Leu Ile Ser Gly Ala Val Ser Ile Ala Gln
                   245                 250                 255
   Ser Ser Ser Ala Ile Thr Ala Ser Gly Thr Ala Leu Thr Gly Ser Ala
               260                 265                 270
   Thr Ala Pro Ala Ala Ala Ala Ala Thr Thr Thr Ser Thr Thr Asn Ala
           275                 280                 285
   Ala Ala Ala Ala Thr Ser Ala Ala Ala Ala Ala Gly Thr Ser Thr Thr
       290                 295                 300
   Thr Thr Ser Ala Ala Ala Val Val Gln Thr Ser Ser Ser Ser Ser Ser
   305                 310                 315                 320
   Ala Pro Ser Ser Ala Ala Ala Ala Ala Thr Thr Thr Ala Ala Ala Ser
                   325                 330                 335
   Ala Arg Pro Thr Gly Cys Ser Ser Gly Arg Ser Arg Lys Gln Pro Arg
               340                 345                 350
   Arg His Ala Arg Asp Met Val Val Ala Arg Gly Ala Glu Glu Ala Asn
           355                 360                 365

<210>  121
<211>  33
<212>  PRT
<213>  Yarrowia lipolytica

<400>  121

Met Lys Leu Ser Thr Ile Leu Phe Thr Ala Cys Ala Thr Leu Ala Ala
1               5                   10                  15

Ala Leu Pro Ser Pro Ile Thr Pro Ser Glu Ala Ala Val Leu Gln Lys
            20                  25                  30

Arg

<210>  122
<211>  847
<212>  DNA
<213>  Yarrowia lipolytica

<400>  122
gccgccgcaa ggaatggtgc atgctgaggt gtctcacaag tgccgtgcag tcccgccccc      60

acttgcttct ctttgtgtgt agtgtacgta cattatcgag accgttgttc ccgcccacct     120

cgatccggca tgctgaggtg tctcacaagt gccgtgcagt cccgccccca cttgcttctc 180

tttgtgtgta gtgtacgtac attatcgaga ccgttgttcc cgcccacctc gatccggcat 240

gctgaggtgt ctcacaagtg ccgtgcagtc ccgcccccac ttgcttctct ttgtgtgtag 300

tgtacgtaca ttatcgagac cgttgttccc gcccacctcg atccggcatg ctgaggtgtc 360

tcacaagtgc cgtgcagtcc cgcccccact tgcttctctt tgtgtgtagt gtacgtacat 420

tatcgagacc gttgttcccg cccacctcga tccggcatgc actgatcacg ggcaaaagtg 480

cagagaccgg gttggcggcg catttgtgtc ccaaaaaaca gccccaattg ccccaattga 540

ccccaaattg acccagtagc ggacccaacc ccggcgagag cccccttcac cccacatatc 600

aaacctcccc cggttcccac acttgccgtt aagggcgtag ggtactgcag tctggaatct 660

acgcttgttc agactttgta ctagtttctt tgtctggcca tccgggtaac ccatgccgga 720

cgcaaaatag actactgaaa atttttttgc tttgtggttg ggactttagc caagggtata 780

aaagaccacc gtccccgaat tacctttcct cttcttttct ctctctcctt gtcaactcac 840

acccgaa 847

<210> 123
<211> 366
<212> DNA
<213> Yarrowia lipolytica

<400> 123
agagaccggg ttggcggcgc atttgtgtcc caaaaaacag ccccaattgc cccaattgac 60

cccaaattga cccagtagcg gacccaaccc cggcgagagc ccccttcacc ccacatatca 120

aacctccccc ggttcccaca cttgccgtta agggcgtagg gtactgcagt ctggaatcta 180

cgcttgttca gactttgtac tagtttcttt gtctggccat ccgggtaacc catgccggac 240

gcaaaataga ctactgaaaa tttttttgct ttgtggttgg gactttagcc aagggtataa 300

aagaccaccg tccccgaatt acctttcctc ttcttttctc tctctccttg tcaactcaca 360

cccgaa 366

<210> 124
<211> 493
<212> PRT
<213> Hypocrea jecorina

<400> 124

Met Ala Gly Lys Leu Ile Leu Val Ala Leu Ala Ser Leu Val Ser Leu
1               5                   10                  15

Ser Ile Gln Gln Asn Cys Ala Ala Leu Phe Gly Gln Cys Gly Gly Ile
            20                  25                  30

Gly Trp Ser Gly Thr Thr Cys Cys Val Ala Gly Ala Gln Cys Ser Phe
        35                  40                  45

Val Asn Asp Trp Tyr Ser Gln Cys Leu Ala Ser Thr Gly Gly Asn Pro
    50                  55                  60

Pro Asn Gly Thr Thr Ser Ser Ser Leu Val Ser Arg Thr Ser Ser Ala
65                  70                  75                  80

Ser Ser Ser Val Gly Ser Ser Ser Pro Gly Gly Asn Ser Pro Thr Gly
                85                  90                  95

Ser Ala Ser Thr Tyr Thr Thr Thr Asp Thr Ala Thr Val Ala Pro His
            100                 105                 110

Ser Gln Ser Pro Tyr Pro Ser Ile Ala Ala Ser Ser Cys Gly Ser Trp
        115                 120                 125

Thr Leu Val Asp Asn Val Cys Cys Pro Ser Tyr Cys Ala Asn Asp Asp
    130                 135                 140

Thr Ser Glu Ser Cys Ser Gly Cys Gly Thr Cys Thr Thr Pro Pro Ser
145                 150                 155                 160

Ala Asp Cys Lys Ser Gly Thr Met Tyr Pro Glu Val His His Val Ser
                165                 170                 175

Ser Asn Glu Ser Trp His Tyr Ser Arg Ser Thr His Phe Gly Leu Thr
            180                 185                 190

Ser Gly Gly Ala Cys Gly Phe Gly Leu Tyr Gly Leu Cys Thr Lys Gly
        195                 200                 205

Ser Val Thr Ala Ser Trp Thr Asp Pro Met Leu Gly Ala Thr Cys Asp
    210                 215                 220

Ala Phe Cys Thr Ala Tyr Pro Leu Leu Cys Lys Asp Pro Thr Gly Thr
225                 230                 235                 240

Thr Leu Arg Gly Asn Phe Ala Ala Pro Asn Gly Asp Tyr Tyr Thr Gln
                245                 250                 255

Phe Trp Ser Ser Leu Pro Gly Ala Leu Asp Asn Tyr Leu Ser Cys Gly
            260                 265                 270

Glu Cys Ile Glu Leu Ile Gln Thr Lys Pro Asp Gly Thr Asp Tyr Ala
        275                 280                 285

Val Gly Glu Ala Gly Tyr Thr Asp Pro Ile Thr Leu Glu Ile Val Asp
    290                 295                 300

Ser Cys Pro Cys Ser Ala Asn Ser Lys Trp Cys Cys Gly Pro Gly Ala
305                 310                 315                 320

Asp His Cys Gly Glu Ile Asp Phe Lys Tyr Gly Cys Pro Leu Pro Ala
                325                 330                 335

Asp Ser Ile His Leu Asp Leu Ser Asp Ile Ala Met Gly Arg Leu Gln
            340                 345                 350

Gly Asn Gly Ser Leu Thr Asn Gly Val Ile Pro Thr Arg Tyr Arg Arg
        355                 360                 365

Val Gln Cys Pro Lys Val Gly Asn Ala Tyr Ile Trp Leu Arg Asn Gly
    370                 375                 380

Gly Gly Pro Tyr Tyr Phe Ala Leu Thr Ala Val Asn Thr Asn Gly Pro
385                 390                 395                 400

Gly Ser Val Thr Lys Ile Glu Ile Lys Gly Ala Asp Thr Asp Asn Trp
                405                 410                 415

Val Ala Leu Val His Asp Pro Asn Tyr Thr Ser Ser Arg Pro Gln Glu
            420                 425                 430

Arg Tyr Gly Ser Trp Val Ile Pro Gln Gly Ser Gly Pro Phe Asn Leu
        435                 440                 445

Pro Val Gly Ile Arg Leu Thr Ser Pro Thr Gly Glu Gln Ile Val Asn
    450                 455                 460

Glu Gln Ala Ile Lys Thr Phe Thr Pro Pro Ala Thr Gly Asp Pro Asn
465                 470                 475                 480

Phe Tyr Tyr Ile Asp Ile Gly Val Gln Phe Ser Gln Asn
                485                 490

<210>  125
<211>  1428
<212>  DNA
<213>  Hypocrea jecorina

<400>  125
cagcagaatt gcgcagcatt atttggccaa tgtggaggca tagggtggtc cggcaccaca      60

tgttgcgttg ctggcgccca gtgcagtttt gtcaatgact ggtactccca gtgccttgcg     120

tcaaccggcg gaaacccccc aaacggaaca acttcctcta gcttggtttc acggacgtcg 180

tcagcatcct catccgtcgg ctcgtcttca cccggcggca actcaccaac tggcagtgct 240

tccacctaca caaccacaga tacagctacc gtggctcctc attcgcagtc tccttacccc 300

agcattgccg catccagttg cggatcgtgg accctcgtgg ataatgtttg ctgcccatca 360

tattgtgcta atgatgacac atccgagtca tgctcaggct gcggtacctg cactacgccg 420

ccctcggcgg actgcaaatc cggaaccatg tatccagagg tccatcacgt atccagcaac 480

gagagctggc actacagtag atcaacccac tttggcctaa cgagcggcgg ggcctgtggc 540

tttggcctgt acggtctctg cacaaagggc agtgttacag ccagctggac ggatcccatg 600

cttggcgcga cgtgtgacgc tttttgtaca gcgtatcccc tgctttgcaa agaccctacc 660

ggcactaccc ttcgtggcaa cttcgcagct ccaaacggcg attactacac ccaattctgg 720

tcctcgttgc caggagccct cgataactac ctgtcctgcg gcgagtgcat tgagctgata 780

caaacaaagc ccgatgggac cgattatgct gtcggagaag ccggctacac ggatccaatt 840

actctcgaga ttgtggacag ctgcccgtgc agcgcgaact ccaagtggtg ctgtggtccg 900

ggcgccgatc attgcggaga gatcgacttc aaatacggct gtcctcttcc tgctgacagc 960

attcatctcg acctgtcaga cattgccatg ggccgtttgc agggcaatgg atcactaacc 1020

aatggcgtca tcccgactcg atatagaaga gtccaatgcc ccaaagttgg gaacgcctac 1080

atttggcttc gaaatggcgg agggccttac tattttgctc tcacggcagt caacaccaac 1140

ggaccgggct cagtcaccaa aatcgagatc aagggcgcag acaccgacaa ctgggttgcc 1200

ttggtccatg acccaaacta tacgagtagc cgcccacaag aacgctatgg cagttgggta 1260

atcccacagg gatcagggcc ctttaacttg cctgttggaa ttcgtctgac tagcccaacg 1320

ggggaacaga ttgtgaatga acaggccatc aagacattca ctcctccggc cacaggtgac 1380

cccaattttt actacattga cattggtgtg cagtttagcc agaattga 1428

<210> 126
<211> 494
<212> PRT
<213> Trichoderma pseudokoningii

<400> 126

Met Ala Arg Ile Leu Ser Val Val Ala Leu Ala Ser Leu Val Ser Val
1               5                   10                  15

Ser Val Gln Gln Asn Cys Ala Ala Leu Phe Gly Gln Cys Gly Gly Thr
            20                  25                  30

Gly Trp Ser Gly Ala Thr Cys Cys Val Asp Gly Ala Gln Cys Thr Tyr
        35                  40                  45

Val Asn Asp Trp Tyr Ser Gln Cys Leu Ala Ser Thr Gly Gly Ser Pro
    50                  55                  60

Pro Gly Gly Thr Thr Ser Ser Ser Ser Ser Val Ser Arg Ser Ser Ser
65                  70                  75                  80

Ala Ser Ser Ser Gly Gly Ser Ser Ser Pro Gly Gly Gly Ser Pro Thr
                85                  90                  95

Gly Gly Ala Ser Thr Tyr Thr Thr Thr Asp Thr Ala Thr Val Ala Pro
            100                 105                 110

His Ser Gln Ser Pro Tyr Pro Ser Ile Ala Ala Ser Ser Cys Gly Ser
        115                 120                 125

Trp Thr Leu Val Asp Asn Val Cys Cys Pro Ser Tyr Cys Ala Asn Asp
    130                 135                 140

Asp Thr Ser Glu Phe Cys Ser Gly Cys Asp Thr Cys Thr Thr Pro Pro
145                 150                 155                 160

Ser Ala Asp Cys Lys Ser Gly Thr Met Tyr Pro Glu Val His His Val
                165                 170                 175

Ser Ser Asn Glu Ser Trp His Ser Ser Arg Ser Thr His Phe Gly Leu
            180                 185                 190

Thr Ser Gly Gly Ala Cys Gly Phe Gly Leu Tyr Gly Leu Cys Thr Lys
        195                 200                 205

Gly Ser Val Thr Ala Asn Trp Thr Asp Pro Met Leu Gly Ser Thr Cys
    210                 215                 220

Asp Ala Phe Cys Thr Ala Tyr Pro Leu Leu Cys Gln Asp Pro Thr Gly
225                 230                 235                 240

Thr Thr Leu Arg Gly Asn Phe Ala Ala Pro Asn Gly Asp Tyr Tyr Thr
                245                 250                 255

Gln Phe Trp Ser Ser Leu Pro Gly Ala Leu Asp Asn Tyr Leu Ser Cys
            260                 265                 270

Gly Glu Cys Met Glu Leu Ile Gln Thr Lys Pro Asp Gly Ser Asp Tyr
        275                 280                 285

Ala Val Gly Glu Ala Gly Tyr Thr Asp Pro Ile Thr Leu Glu Ile Val

290                 295                 300

Asp Ser Cys Pro Cys Ser Ala Asn Ser Lys Trp Cys Cys Gly Pro Gly
305                 310                 315                 320

Ala Asp His Cys Gly Glu Ile Asp Phe Lys Tyr Gly Cys Pro Leu Pro
                325                 330                 335

Ala Asp Ser Ile His Leu Asp Leu Ser Asp Ile Ala Met Gly Arg Leu
            340                 345                 350

Gln Gly Asn Gly Ser Leu Thr Asn Gly Val Ile Pro Thr Arg Tyr Arg
        355                 360                 365

Arg Val Pro Cys Pro Lys Val Gly Asn Ala Tyr Ile Trp Leu Arg Ser
    370                 375                 380

Gly Gly Gly Pro Tyr Tyr Phe Ala Leu Thr Ala Val Asn Thr Asn Gly
385                 390                 395                 400

Pro Gly Ser Val Thr Lys Ile Glu Ile Lys Ser Ala Asp Ser Asp Asp
                405                 410                 415

Trp Val Ala Leu Val His Asp Pro Asn Tyr Thr Ser Ser Arg Pro Gln
            420                 425                 430

Glu Arg Tyr Gly Ser Trp Val Val Pro Gln Gly Ala Gly Pro Phe Asn
        435                 440                 445

Leu Pro Val Gly Ile Arg Leu Thr Ser Pro Thr Gly Glu Gln Ile Val
    450                 455                 460

Asn Glu Gln Ala Ile Lys Thr Phe Thr Pro Pro Ala Thr Gly Asp Pro
465                 470                 475                 480

Asn Phe Tyr Tyr Ile Asp Ile Gly Val Gln Phe Gly Gln Asn
                485                 490

<210>  127
<211>  491
<212>  PRT
<213>  Hypocrea virens

<400>  127

Met Ala Arg Lys Leu Ser Leu Leu Ala Leu Ala Ser Leu Val Ser Leu
1               5                   10                  15

Ser Leu Gln Gln Asn Cys Ala Ala Leu Phe Gly Gln Cys Gly Gly Ile

20                  25                  30

Gly Trp Thr Ser Pro Thr Cys Cys Val Ala Gly Ala Gln Cys Ile Tyr
        35                  40                  45

Val Asn Asp Phe Tyr Ser Gln Cys Leu Ala Ser Thr Gly Gly Gly Gly
    50                  55                  60

Ser Thr Thr Arg Ser Ser Thr Ser Ser Thr Ser Val Ser Arg Ser Ser
65                  70                  75                  80

Ser Val Gly Ser Ser Ser Gly Gly Gly Gly Ser Pro Thr Gly Gly Ala
                85                  90                  95

Ser Thr Tyr Thr Thr Thr Asp Thr Ala Thr Val Ala Pro His Ser Gln
            100                 105                 110

Ser Pro Tyr Pro Ser Ile Ala Ala Ser Ser Cys Gly Ser Trp Thr Leu
        115                 120                 125

Val Asp Asn Val Cys Cys Pro Ser Tyr Cys Ala Asn Asp Asn Thr Ser
    130                 135                 140

Glu Phe Cys Ser Gly Cys Gly Thr Cys Thr Thr Pro Pro Ser Ala Asp
145                 150                 155                 160

Cys Lys Ser Gly Thr Met Tyr Pro Glu Val His His Val Thr Asn Asn
                165                 170                 175

Glu Thr Trp His Tyr Ser Arg Ser Thr His Phe Gly Leu Thr Ser Gly
            180                 185                 190

Gly Ala Cys Gly Phe Gly Leu Tyr Gly Leu Cys Thr Lys Gly Ser Val
        195                 200                 205

Thr Ala Asn Trp Thr Asp Pro Met Leu Gly Ser Thr Cys Asn Ala Phe
    210                 215                 220

Cys Thr Ala Tyr Pro Leu Leu Cys Lys Asp Pro Ala Gly Thr Thr Leu
225                 230                 235                 240

Arg Gly Asn Phe Ala Ala Pro Asn Gly Asp Tyr Tyr Ser Gln Phe Trp
                245                 250                 255

Ser Ser Leu Pro Gly Ala Leu Asp Asn Tyr Leu Ser Cys Gly Glu Cys
            260                 265                 270

Ile Glu Leu Ile Gln Thr Lys Pro Asp Gly Thr Asp Tyr Val Val Gly
        275                 280                 285

Glu Ala Gly Tyr Thr Asp Pro Ile Thr Leu Glu Ile Val Asp Ser Cys
    290                 295                 300

Pro Cys Ser Ala Asn Ser Lys Trp Cys Cys Gly Pro Gly Ala Asp His
305                 310                 315                 320

Cys Gly Glu Ile Asn Phe Lys Tyr Gly Cys Pro Leu Pro Ala Asp Ser
                325                 330                 335

Ile His Leu Asp Leu Ser Asp Ile Ala Met Gly Arg Leu Gln Gly Asn
            340                 345                 350

Gly Ser Leu Thr Asn Gly Val Ile Pro Thr Arg Tyr Lys Arg Val Pro
        355                 360                 365

Cys Pro Lys Val Gly Asn Val Tyr Ile Trp Leu Arg Asn Gly Gly Gly
    370                 375                 380

Pro Tyr Tyr Phe Ala Leu Thr Ala Val Asn Thr Asn Gly Pro Gly Ser
385                 390                 395                 400

Val Thr Lys Ile Glu Ile Lys Gly Ala Asp Thr Ser Thr Trp Val Ala
                405                 410                 415

Leu Val His Asp Pro Asn Tyr Thr Ser Ser Arg Pro Gln Glu Arg Tyr
            420                 425                 430

Gly Ser Trp Val Ile Pro Gln Gly Ser Gly Pro Phe Asn Leu Pro Ile
        435                 440                 445

Ala Ile Arg Leu Thr Ser Pro Thr Gly Glu Gln Ile Val Asn Glu Gln
    450                 455                 460

Ala Ile Lys Thr Phe Thr Pro Pro Ala Thr Ala Asp Pro Asn Phe Tyr
465                 470                 475                 480

Tyr Ile Asp Ile Gly Val Gln Phe Ser Gln Asn
                485                 490

<210>  128
<211>  493
<212>  PRT
<213>  Hypocrea atroviridis

<400>  128

Met Leu Arg Lys Leu Ser Leu Leu Ala Leu Ala Gly Leu Val Ser His
1               5                   10                  15

Ser Val Gln Gln Ser Cys Ala Ala Leu Tyr Gly Gln Cys Gly Gly Ser
            20                  25                  30

Gly Trp Ser Gly Ala Thr Cys Cys Val Ser Gly Ala Gln Cys Ser Ala
        35                  40                  45

Leu Asn Gln Tyr Tyr Ser Gln Cys Val Pro Ser Thr Gly Ser Ser Gly
    50                  55                  60

Pro Thr Gly Ser Ser Ser Ser Thr Thr Ser Ile Pro His Ser Gln Ser
65                  70                  75                  80

Ser Ser Ala Ser His Ser Ser Gly Gly Gln Ser Ser Val Gly Gln Ser
                85                  90                  95

Thr Gly Val Ser Thr Tyr Thr Thr Thr Asp Thr Ser Thr Val Ala Leu
            100                 105                 110

His Ser Gln Ser Pro Tyr Pro Ser Ile Ala Ala Ser Ser Cys Gly Ala
        115                 120                 125

Trp Thr Leu Val Asp Asn Val Cys Cys Pro Ser Tyr Cys Ala Thr Asp
    130                 135                 140

Asp Thr Ser Glu Ser Cys Thr Cys Ser Asp Cys Thr Thr Pro Pro Ser
145                 150                 155                 160

Ala Asp Cys Lys Ser Gly Thr Met Tyr Pro Glu Val His Thr Val Ser
                165                 170                 175

Thr Asn Glu Thr Trp His Tyr Ser Arg Ser Thr His Phe Gly Leu Thr
            180                 185                 190

Ser Gly Gly Ala Cys Gly Phe Gly Leu Tyr Gly Leu Cys Thr Lys Gly
        195                 200                 205

Ser Val Thr Ala Ser Trp Thr Asp Pro Met Leu Gly Thr Thr Cys Asp
    210                 215                 220

Ala Phe Cys Thr Ala Tyr Pro Leu Leu Cys Lys Asp Pro Ala Gly Thr
225                 230                 235                 240

Thr Leu Arg Gly Asn Phe Ala Ala Pro Asn Gly Asp Tyr Tyr Thr Gln
                245                 250                 255

Phe Trp Pro Ser Leu Pro Gly Asp Leu Asp Asn Tyr Leu Ser Cys Gly
            260                 265                 270

Glu Cys Ile Glu Leu Ile Gln Thr Lys Pro Asp Gly Thr Asp Tyr Ala
        275                 280                 285

Val Gly Glu Ala Gly Tyr Thr Asp Pro Ile Thr Leu Glu Ile Val Asp
    290                 295                 300

Ser Cys Pro Cys Ser Ala Asn Ser Lys Trp Cys Cys Gly Pro Gly Ala
305                 310                 315                 320

Asp His Cys Gly Glu Ile Asp Phe Lys Tyr Gly Cys Pro Leu Pro Ala
                325                 330                 335

Asp Ser Met His Leu Asp Leu Ser Asp Ile Ala Met Gly Arg Leu Gln
            340                 345                 350

Gly Asn Gly Ser Leu Thr Asn Gly Val Ile Pro Thr Arg Tyr Arg Arg
        355                 360                 365

Val Gln Cys Pro Lys Leu Gly Asn Val Tyr Ile Trp Leu Arg Asn Gly
    370                 375                 380

Gly Gly Pro Tyr Tyr Phe Ala Leu Thr Ala Val Asn Ala Asn Gly Pro
385                 390                 395                 400

Gly Ser Val Thr Lys Ile Glu Val Arg Gly Ala Gly Ser Ser Thr Trp
                405                 410                 415

Ile Pro Leu Val His Asp Pro Asn Tyr Thr Ser Ser Arg Pro Gln Glu
            420                 425                 430

Arg Tyr Gly Ser Trp Val Ile Pro Gln Gly Ser Gly Pro Phe Asn Leu
        435                 440                 445

Pro Val Ala Val Arg Leu Thr Ser Pro Thr Gly Glu Gln Ile Val Asn
    450                 455                 460

Glu Gln Ala Ile Lys Thr Phe Thr Pro Pro Ser Thr Ala Asp Pro Asn
465                 470                 475                 480

Phe Tyr Tyr Ile Asp Ile Gly Val Gln Phe Ser Lys Asn
                485                 490

<210>  129
<211>  495

<212>  PRT
<213>  Trichoderma asperellum

<400>  129

Met Leu Arg Arg Leu Ser Leu Leu Val Leu Ala Gly Leu Val Ser Arg
1               5                   10                  15

Ser Ala Gln Gln Ser Cys Ala Ala Leu Tyr Gly Gln Cys Gly Gly Ile
            20                  25                  30

Gly Trp Ser Gly Ala Thr Cys Cys Val Ser Gly Ala Gln Cys Asn Ala
        35                  40                  45

Leu Asn Asp Tyr Tyr Ser Gln Cys Val Leu Ser Thr Gly Gly Ser Gly
    50                  55                  60

Pro Thr Gly Ser Ser Ser Ser Ala Thr Ser Ile Pro His Ser Gln Ser
65                  70                  75                  80

Leu Ser Ser Ala Ser Ser Ile Pro His Ser Gln Ser Ser Ser Val Gly
                85                  90                  95

Gln Ser Thr Gly Val Ser Thr Tyr Thr Thr Thr Asp Thr Ser Thr Val
            100                 105                 110

Ala Leu His Ser Gln Ser Pro Tyr Pro Ser Ile Ala Ala Ser Ser Cys
        115                 120                 125

Gly Ala Trp Thr Leu Val Asp Asn Val Cys Cys Pro Ser Tyr Cys Ala
    130                 135                 140

Thr Asp Asp Thr Ser Glu Ser Cys Thr Cys Ser Asp Cys Thr Thr Pro
145                 150                 155                 160

Pro Ser Ala Asp Cys Lys Ser Gly Thr Met Tyr Pro Glu Val His Thr
                165                 170                 175

Val Ser Thr Asn Glu Thr Trp His Tyr Ser Arg Ser Thr His Phe Gly
            180                 185                 190

Leu Thr Ser Gly Gly Ala Cys Gly Phe Gly Leu Tyr Gly Leu Cys Thr
        195                 200                 205

Lys Gly Ser Val Thr Ala Asn Trp Thr Asp Pro Met Leu Gly Thr Thr
    210                 215                 220

Cys Asp Ala Phe Cys Thr Ala Tyr Pro Leu Leu Cys Lys Asp Pro Ala
225                 230                 235                 240

Gly Thr Thr Leu Arg Gly Asn Phe Ala Ala Pro Asn Gly Asp Tyr Tyr
                245                 250                 255

Thr Gln Phe Trp Pro Ser Leu Thr Gly Asp Leu Asp Asn Tyr Leu Ser
            260                 265                 270

Cys Gly Glu Cys Ile Glu Leu Ile Gln Thr Lys Pro Asp Gly Thr Asp
        275                 280                 285

Tyr Ala Val Gly Glu Ala Gly Tyr Thr Asp Pro Ile Thr Leu Glu Ile
    290                 295                 300

Val Asp Ser Cys Pro Cys Ser Ala Asn Ser Lys Trp Cys Cys Gly Pro
305                 310                 315                 320

Gly Ala Asp His Cys Gly Glu Ile Asp Phe Lys Tyr Gly Cys Pro Leu
                325                 330                 335

Pro Ala Asp Ser Ile His Leu Asp Leu Ser Asp Ile Ala Met Gly Arg
            340                 345                 350

Leu Gln Gly Asn Gly Ser Leu Asn Asn Gly Val Ile Pro Thr Arg Tyr
        355                 360                 365

Arg Arg Val Gln Cys Pro Lys Leu Gly Asn Val Tyr Ile Trp Leu Arg
    370                 375                 380

Asn Gly Gly Gly Pro Tyr Tyr Phe Ala Leu Thr Ala Val Asn Thr Asn
385                 390                 395                 400

Gly Pro Gly Ser Val Thr Lys Ile Glu Val Lys Gly Ser Asp Ser Ser
                405                 410                 415

Thr Trp Val Pro Leu Glu His Asp Pro Asn Tyr Thr Ser Ser Arg Pro
            420                 425                 430

Gln Glu Arg Tyr Gly Ser Trp Val Ile Pro Gln Gly Ser Gly Pro Phe
        435                 440                 445

Asn Leu Pro Val Gly Val Arg Leu Thr Ser Pro Thr Gly Glu Gln Ile
    450                 455                 460

Val Asn Glu Gln Ala Ile Lys Thr Phe Thr Pro Pro Ala Thr Ala Asp
465                 470                 475                 480

Pro Asn Phe Tyr Tyr Ile Asp Ile Gly Val Gln Phe Ser Arg Asn

                485                 490                 495

<210> 130
<211> 499
<212> PRT
<213> Penicillium oxalicum

<400> 130

Met Ala Gln Leu Ser Phe Leu Thr Leu Ala Val Leu Gly Leu Ser Gly
1               5                   10                  15

Thr Val Ala Ala Gln Ser Ala Cys Ala Ser Ala Trp Ala Gln Cys Gly
            20                  25                  30

Gly Ser Asn Phe Asn Gly Pro Thr Cys Cys Val Ser Gly Ser Ser Cys
        35                  40                  45

Ser Val Leu Asn Pro Tyr Tyr Ser Gln Cys Val Pro Gly Thr Gln Val
    50                  55                  60

Pro Gly Ser Gly Ser Gly Ser Ser Ser Thr Arg Ser Ser Ser Thr Thr
65                  70                  75                  80

Thr Ala Gly Gly Thr Gly Pro Ser Thr Thr Ala Thr Ala Gly Gly Gly
                85                  90                  95

Ala Ala Gly Thr Ser Lys Tyr Thr Thr Ser Thr Ser Phe Pro Ala Ala
            100                 105                 110

Thr Ala Thr Gln Pro Ala Tyr Pro Ser Ala Asp Pro Ser Ser Cys Gly
        115                 120                 125

Ala Trp Thr Leu Val Asp Asn Val Cys Cys Pro Glu Tyr Cys Leu Ser
    130                 135                 140

Asn Asn Lys Ser Glu Ser Cys Thr Ser Ser Cys Ser Gly Gly Cys Gly
145                 150                 155                 160

Thr Pro Pro Ser Ser Met Cys Lys Ser Gly Thr Met Trp Gly Glu Gln
                165                 170                 175

Leu Glu Val Gly Thr Asp Glu Asp Trp His Tyr Ser Arg Ser Thr His
            180                 185                 190

Phe Gly Leu Thr Ser Gly Gly Ala Cys Gly Phe Gly Leu Tyr Gly Leu
        195                 200                 205

Cys Thr Lys Gly Ser Lys Thr Ala Ser Trp Thr Asp Pro Met Leu Gly

210                 215                 220

Ser Thr Cys Asp Ala Phe Cys Thr Ala Tyr Pro Leu Leu Cys Lys Asp
225                 230                 235                 240

Pro Ser Asp Val Thr Leu Arg Gly Asn Phe Ala Ala Pro Asn Gly Asp
                245                 250                 255

Tyr Tyr Thr Gln Phe Trp Pro Ser Leu Ile Gly Ala Leu Pro Gly Ser
            260                 265                 270

Pro Asp Asn Tyr Leu Ser Cys Gly Glu Cys Phe Glu Leu Ile Arg Thr
        275                 280                 285

Arg Ala Asp Gly Thr Glu Tyr Gln Val Gly Asp Ser Gly Tyr Thr Asp
    290                 295                 300

Pro Ile Tyr Leu Glu Val Val Asp Ser Cys Pro Cys Ser Ala Asn Ser
305                 310                 315                 320

Lys Trp Cys Cys Gly Ser Gly Ala Asp His Cys Gly Glu Ile Asp Phe
                325                 330                 335

Lys Tyr Gly Cys Pro Leu Pro Glu Gly Ser His His Met Asp Leu Ser
            340                 345                 350

Asp Ile Ala Met Gly Arg Leu Gln Gly Asn Gly Ser Leu Thr Asp Gly
        355                 360                 365

Val Ile Pro Ile Arg Tyr Arg Arg Val Pro Cys Pro Lys Pro Gly Asn
    370                 375                 380

Ala Tyr Val Trp Leu Arg Asp Gly Gly Ser Glu Tyr Tyr Phe Ala Leu
385                 390                 395                 400

Ser Val Val Asn Thr Asn Gly Val Gly Ser Ile Thr Thr Val Glu Val
                405                 410                 415

Lys Gly Lys Gly Ser Asn Ser Trp Val Ala Leu Lys Arg Asp Pro Asn
            420                 425                 430

Tyr Thr Ser Ser Arg Pro Gln Glu Arg Tyr Gly Ala Trp Val Val Pro
        435                 440                 445

Gln Gly Lys Gly Pro Phe Ser Leu Pro Val Gly Ile Arg Val Thr Ser
    450                 455                 460

Pro Asp Gly Gln Gln Ile Val Asn Glu Asn Ala Ile Thr Ser Phe Thr
465                 470                 475                 480

Ala Pro Ala Gly Ser Val Glu Gly Phe Tyr Tyr Ile Asp Leu Gly Val
                485                 490                 495

Gln Phe Thr

<210>  131
<211>  478
<212>  PRT
<213>  Neosartorya fumigata strain CEA10

<400>  131

Met Thr Leu Leu Leu Gly Ile Phe Leu Ala Arg Leu Ala Val Ala Ala
1               5                   10                  15

Ala Gln Ser Ser Cys Val Ser Ala Trp Gly Gln Cys Gly Gly Ile Ser
            20                  25                  30

Tyr Thr Gly Ser Thr Cys Cys Gln Ser Gly Asn Thr Cys Ala Glu Ile
        35                  40                  45

Asn Ser Tyr Tyr Tyr Gln Cys Ile Pro Ala Thr Ala Val Ser Thr Ser
    50                  55                  60

Thr Thr Thr Val Thr Ser Pro Ser Ser Ser Gly Ser Ser Ser Ala Asn
65                  70                  75                  80

Thr Ser Gly Tyr Thr Thr Thr Thr Val Leu Pro Ser Pro Thr Ala Thr
                85                  90                  95

Gln Asn Pro Tyr Pro Pro Ala Asn Ala Ser Ser Cys Gly Ser Trp Ala
            100                 105                 110

Leu Val Asp Asn Val Cys Cys Pro Tyr Tyr Cys Leu Ser Asp Asn Glu
        115                 120                 125

Ser Glu Ser Cys Thr Ser Ser Cys Thr Gly Gly Cys Gly Ser Pro Asp
    130                 135                 140

Ser Ser Met Cys Lys Ser Gly Thr Met Trp Gly Glu Gln His Thr Val
145                 150                 155                 160

Thr Ser Asn Glu Asp Trp His Tyr Ser Arg Ser Thr His Phe Gly Leu
                165                 170                 175

Thr Ser Gly Gly Ala Cys Gly Phe Gly Leu Tyr Gly Leu Cys Thr Lys
            180                 185                 190

Asn Ser Val Thr Ala Ser Trp Thr Asp Pro Met Leu Gly Ser Thr Cys
        195                 200                 205

Asp Ala Phe Cys Thr Ala Tyr Pro Leu Leu Cys Lys Asp Pro Ala Asn
    210                 215                 220

Val Thr Leu Arg Gly Asn Phe Ala Ala Pro Asn Gly Asp Tyr Tyr Thr
225                 230                 235                 240

Gln Phe Trp Pro Ser Leu Ala Ala Ser Gly Asn Pro Asp Asn Tyr Leu
                245                 250                 255

Ser Cys Gly Glu Cys Phe Glu Val Ile Arg Thr Lys Pro Asp Gly Thr
            260                 265                 270

Asp Tyr Ala Val Gly Glu Ser Gly Tyr Thr Asp Pro Ile Val Leu Glu
        275                 280                 285

Val Val Asp Ser Cys Pro Cys Asn Ala Asn Ser Lys Trp Cys Cys Gly
    290                 295                 300

Ser Gly Ala Asp His Cys Gly Glu Ile Asp Phe Lys Tyr Gly Cys Pro
305                 310                 315                 320

Leu Pro Glu Gly Ser His His Met Asp Leu Ser Asp Ile Ala Met Gly
                325                 330                 335

Arg Leu Gln Gly Asn Gly Ser Leu Ala Asp Gly Val Ile Pro Ile Arg
            340                 345                 350

Tyr Arg Arg Val Pro Cys Pro Lys Pro Gly Asn Ile Tyr Ile Trp Leu
        355                 360                 365

His Asp Gly Ala Gly Pro Tyr Tyr Phe Ala Leu Ser Ala Val Asn Thr
    370                 375                 380

Asn Gly Pro Gly Ser Val Ile Ser Ile Glu Val Gln Ala Gly Gly Ser
385                 390                 395                 400

Gly Pro Trp Val Ala Leu Glu His Asp Pro Asn Tyr Thr Ser Ser Arg
                405                 410                 415

Pro Gln Glu Arg Tyr Gly Ser Trp Val Leu Pro Gln Gly Lys Gly Pro
            420                 425                 430

Ile Thr Val Pro Val Ser Met Arg Leu Thr Ala Pro Asn Gly Gln Gln
        435                 440                 445

Val Val Lys Glu Gly Ala Ile Thr Ser Tyr Thr Pro Pro Ala Thr Ala
    450                 455                 460

Val Glu Gly Phe Trp Tyr Ile Asp Leu Gly Val Gln Phe Thr
465                 470                 475

<210>  132
<211>  476
<212>  PRT
<213>  Neosartorya fumigata strain AF210

<400>  132

Met Thr Leu Leu Phe Gly Ile Phe Leu Ala Arg Leu Ala Val Ala Ala
1               5                   10                  15

Ala Gln Ser Ser Cys Val Ser Ala Trp Gly Gln Cys Gly Gly Ile Ser
            20                  25                  30

Tyr Thr Gly Ser Thr Cys Cys Gln Ser Gly Asn Thr Cys Ala Glu Ile
        35                  40                  45

Asn Ser Tyr Tyr Tyr Gln Cys Ile Pro Ala Thr Ala Thr Ser Thr Thr
    50                  55                  60

Thr Val Thr Ser Pro Ser Ser Ser Gly Ser Ser Ser Ala Asn Thr Ser
65                  70                  75                  80

Gly Tyr Thr Thr Thr Thr Val Leu Pro Ser Pro Thr Ala Thr Gln Asn
                85                  90                  95

Pro Tyr Pro Pro Ala Asn Ala Ser Ser Cys Gly Ser Trp Ala Leu Val
            100                 105                 110

Asp Asn Val Cys Cys Pro Tyr Tyr Cys Leu Ser Asp Asn Glu Ser Glu
        115                 120                 125

Ser Cys Thr Ser Ser Cys Thr Gly Gly Cys Gly Ser Pro Asp Ser Ser
    130                 135                 140

Met Cys Lys Ser Gly Thr Met Trp Gly Glu Gln His Thr Val Thr Ser
145                 150                 155                 160

Asn Glu Asp Trp His Tyr Ser Arg Ser Thr His Phe Gly Leu Thr Ser
                165                 170                 175

Gly Gly Ala Cys Gly Phe Gly Leu Tyr Gly Leu Cys Thr Lys Asn Ser
            180                 185                 190

Val Thr Ala Ser Trp Thr Asp Pro Met Leu Gly Ser Thr Cys Asp Ala
        195                 200                 205

Phe Cys Thr Ala Tyr Pro Leu Leu Cys Lys Asp Pro Ala Asn Val Thr
    210                 215                 220

Leu Arg Gly Asn Phe Ala Ala Pro Asn Gly Asp Tyr Tyr Thr Gln Phe
225                 230                 235                 240

Trp Pro Ser Leu Ala Ala Ser Gly Asn Pro Asp Asn Tyr Leu Ser Cys
                245                 250                 255

Gly Glu Cys Phe Glu Val Ile Arg Thr Lys Pro Asp Gly Thr Asp Tyr
            260                 265                 270

Ala Val Gly Glu Ser Gly Tyr Thr Asp Pro Ile Val Leu Glu Val Val
        275                 280                 285

Asp Ser Cys Pro Cys Asn Ala Asn Ser Lys Trp Cys Cys Gly Ser Gly
    290                 295                 300

Ala Asp His Cys Gly Glu Ile Asp Phe Lys Tyr Gly Cys Pro Leu Pro
305                 310                 315                 320

Glu Gly Ser His His Met Asp Leu Ser Asp Ile Ala Met Gly Arg Leu
                325                 330                 335

Gln Gly Asn Gly Ser Leu Ala Asp Gly Val Ile Pro Ile Arg Tyr Arg
            340                 345                 350

Arg Val Pro Cys Pro Lys Pro Gly Asn Ile Tyr Ile Trp Leu His Asp
        355                 360                 365

Gly Ala Gly Pro Tyr Tyr Phe Ala Leu Ser Ala Val Asn Thr Asn Gly
    370                 375                 380

Pro Gly Ser Val Ile Ser Ile Glu Val Gln Ala Gly Gly Ser Gly Pro
385                 390                 395                 400

Trp Val Ala Leu Glu His Asp Pro Asn Tyr Thr Ser Ser Arg Pro Gln
                405                 410                 415

Glu Arg Tyr Gly Ser Trp Val Leu Pro Gln Gly Lys Gly Pro Ile Thr
            420                 425                 430

Val Pro Val Ser Met Arg Leu Thr Ala Pro Asn Gly Gln Gln Val Val
        435                 440                 445

Lys Glu Gly Ala Ile Thr Ser Tyr Thr Pro Pro Ala Thr Ala Val Glu
    450                 455                 460

Gly Phe Trp Tyr Ile Asp Leu Gly Val Gln Phe Thr
465                 470                 475

<210>  133
<211>  499
<212>  PRT
<213>  Penicillium decumbens

<400>  133

Met Ala Gln Leu Ser Phe Leu Thr Leu Ala Val Leu Gly Leu Ser Gly
1               5                   10                  15

Thr Val Ala Ala Gln Ser Ala Cys Ala Ser Ala Trp Ala Gln Cys Gly
            20                  25                  30

Gly Ser Asn Phe Asn Gly Pro Thr Cys Cys Val Ser Gly Ser Ser Cys
        35                  40                  45

Ser Val Leu Asn Pro Tyr Tyr Ser Gln Cys Val Pro Gly Thr Gln Val
    50                  55                  60

Pro Gly Ser Gly Ser Gly Ser Ser Ser Thr Arg Ser Ser Ser Thr Thr
65                  70                  75                  80

Thr Ala Gly Gly Thr Gly Pro Ser Thr Thr Ala Thr Ala Gly Gly Gly
                85                  90                  95

Ala Ala Gly Thr Ser Lys Tyr Thr Thr Ser Thr Ser Phe Pro Ala Ala
            100                 105                 110

Thr Ala Thr Gln Pro Ala Tyr Pro Ser Ala Asp Pro Ser Ser Cys Gly
        115                 120                 125

Ala Trp Thr Leu Val Asp Asn Val Cys Cys Pro Glu Tyr Cys Leu Ser
    130                 135                 140

Asn Asn Lys Ser Glu Ser Cys Thr Ser Ser Cys Ser Gly Gly Cys Gly
145                 150                 155                 160

Thr Pro Pro Ser Ser Met Cys Lys Ser Gly Thr Met Trp Gly Glu Gln
                165                 170                 175

Leu Glu Val Gly Thr Asp Glu Asp Trp His Tyr Ser Arg Ser Thr His
            180                 185                 190

Phe Gly Leu Thr Ser Gly Gly Ala Cys Gly Phe Gly Leu Tyr Gly Pro
        195                 200                 205

Cys Thr Lys Gly Ser Lys Thr Ala Ser Trp Thr Asp Pro Met Leu Gly
    210                 215                 220

Ser Thr Cys Asp Ala Phe Cys Thr Ala Tyr Pro Leu Leu Cys Lys Asp
225                 230                 235                 240

Pro Ser Asp Val Thr Leu Arg Gly Asn Phe Ala Ala Pro Asn Gly Asp
                245                 250                 255

Tyr Tyr Thr Gln Phe Trp Pro Ser Leu Ile Gly Ala Leu Pro Gly Ser
            260                 265                 270

Pro Asp Asn Tyr Leu Ser Cys Gly Glu Cys Phe Glu Leu Ile Arg Thr
        275                 280                 285

Arg Ala Asp Gly Thr Glu Tyr Gln Val Gly Asp Ser Gly Tyr Thr Asp
    290                 295                 300

Pro Ile Tyr Leu Glu Val Val Asp Ser Cys Pro Cys Ser Ala Asn Ser
305                 310                 315                 320

Lys Trp Cys Cys Gly Ser Gly Ala Asp His Cys Gly Glu Ile Asp Phe
                325                 330                 335

Lys Tyr Gly Cys Pro Leu Pro Glu Gly Ser His His Met Asp Leu Ser
            340                 345                 350

Asp Ile Ala Met Gly Arg Leu Gln Gly Asn Gly Ser Leu Thr Asp Gly
        355                 360                 365

Val Ile Pro Ile Arg Tyr Arg Arg Val Pro Cys Pro Lys Pro Gly Asn
    370                 375                 380

Ala Tyr Val Trp Leu Arg Asp Gly Gly Ser Glu Tyr Tyr Phe Ala Leu
385                 390                 395                 400

Ser Val Val Asn Thr Asn Gly Val Gly Ser Ile Thr Thr Val Glu Val
                405                 410                 415

Lys Gly Lys Gly Ser Asn Ser Trp Val Ala Leu Lys Arg Asp Pro Asn

420                         425                         430

Tyr Thr Ser Ser Arg Pro Gln Glu Arg Tyr Gly Ala Trp Val Ile Pro
            435                 440                 445

Gln Gly Lys Gly Pro Phe Ser Leu Pro Val Gly Ile Arg Val Thr Ser
    450                 455                 460

Pro Asp Gly Gln Gln Ile Val Asn Glu Asn Ala Ile Thr Ser Phe Thr
465                 470                 475                 480

Ala Pro Ala Gly Ser Val Glu Gly Phe Tyr Tyr Ile Asp Leu Gly Val
                485                 490                 495

Gln Phe Thr

<210>  134
<211>  500
<212>  PRT
<213>  Neosartorya fumigata strain ATCC MYA-4609

<400>  134

Met Thr Leu Leu Phe Gly Ile Phe Leu Ala Arg Leu Ala Val Ala Ala
1               5                   10                  15

Ala Gln Ser Ser Cys Val Ser Ala Trp Gly Gln Cys Gly Gly Ile Ser
            20                  25                  30

Tyr Thr Gly Ser Thr Cys Cys Gln Ser Gly Asn Thr Cys Ala Glu Ile
        35                  40                  45

Asn Ser Tyr Tyr Tyr Gln Cys Ile Pro Ala Thr Ala Val Ser Thr Ser
    50                  55                  60

Thr Thr Thr Val Thr Ser Pro Ser Ser Ser Gly Ser Ser Ser Ala Asn
65                  70                  75                  80

Thr Ser Gly Tyr Thr Thr Thr Thr Val Leu Pro Ser Pro Thr Ala Thr
                85                  90                  95

Gln Asn Pro Tyr Pro Pro Ala Asn Ala Ser Ser Cys Gly Ser Trp Ala
            100                 105                 110

Leu Val Asp Asn Val Cys Cys Pro Tyr Tyr Cys Leu Ser Asp Asn Glu
        115                 120                 125

Ser Glu Ser Cys Thr Ser Ser Cys Thr Gly Gly Cys Gly Ser Pro Asp

130                 135                 140

Ser Ser Met Cys Lys Ser Gly Thr Met Trp Gly Glu Gln His Thr Val
145                 150                 155                 160

Thr Ser Asn Glu Asp Trp His Tyr Ser Arg Ser Thr His Phe Gly Leu
                165                 170                 175

Thr Ser Gly Gly Ala Cys Gly Phe Gly Leu Tyr Gly Leu Cys Thr Lys
            180                 185                 190

Asn Ser Val Thr Ala Ser Trp Thr Asp Pro Met Leu Gly Ser Thr Cys
        195                 200                 205

Asp Ala Phe Cys Thr Ala Tyr Pro Leu Leu Cys Lys Asp Pro Ala Asn
    210                 215                 220

Val Thr Leu Arg Gly Asn Phe Ala Ala Pro Asn Gly Asp Tyr Tyr Thr
225                 230                 235                 240

Gln Phe Trp Pro Ser Leu Ala Ala Ser Gly Asn Pro Asp Asn Tyr Leu
                245                 250                 255

Ser Cys Gly Glu Cys Phe Glu Val Ile Arg Thr Lys Pro Asp Gly Thr
            260                 265                 270

Asp Tyr Ala Val Gly Glu Ser Gly Tyr Thr Asp Pro Ile Val Leu Glu
        275                 280                 285

Val Val Asp Ser Cys Pro Cys Asn Ala Asn Ser Lys Trp Cys Cys Lys
    290                 295                 300

Arg Pro Pro Pro Pro Pro Phe Phe Ser Pro Pro Ser Asn Gly Gln Asn
305                 310                 315                 320

Leu Thr Arg Pro Gly Gly Ser Gly Ala Asp His Cys Gly Glu Ile Asp
                325                 330                 335

Phe Lys Tyr Gly Cys Pro Leu Pro Glu Gly Ser His His Met Asp Leu
            340                 345                 350

Ser Asp Ile Ala Met Gly Arg Leu Gln Gly Asn Gly Ser Leu Ala Asp
        355                 360                 365

Gly Val Ile Pro Ile Arg Tyr Arg Arg Val Pro Cys Pro Lys Pro Gly
    370                 375                 380

Asn Ile Tyr Ile Trp Leu His Asp Gly Ala Gly Pro Tyr Tyr Phe Ala
385                 390                 395                 400

Leu Ser Ala Val Asn Thr Asn Gly Pro Gly Ser Val Ile Ser Ile Glu
                405                 410                 415

Val Gln Ala Gly Gly Ser Gly Pro Trp Val Ala Leu Glu His Asp Pro
            420                 425                 430

Asn Tyr Thr Ser Ser Arg Pro Gln Glu Arg Tyr Gly Ser Trp Val Leu
        435                 440                 445

Pro Gln Gly Lys Gly Pro Ile Thr Val Pro Val Ser Met Arg Leu Thr
    450                 455                 460

Ala Pro Asn Gly Gln Gln Val Val Lys Glu Gly Ala Ile Thr Ser Tyr
465                 470                 475                 480

Thr Pro Pro Ala Thr Ala Val Glu Gly Phe Trp Tyr Ile Asp Leu Gly
                485                 490                 495

Val Gln Phe Thr
            500

<210>  135
<211>  483
<212>  PRT
<213>  Talaromyces stipitatus

<400>  135

Met Ala Thr Thr Leu Ile Phe Val Met Gly Leu Trp Ser Gly Leu Leu
1               5                   10                  15

Trp Ser Thr Thr Val Ala Gln Ala Ser Cys Ala Ala Ile Trp Ala Gln
            20                  25                  30

Cys Gly Gly Ser Gly Phe Ser Gly Pro Ser Cys Cys Glu Thr Gly Ser
        35                  40                  45

Thr Cys Thr Gln Leu Asn Ser Phe Tyr Ser Gln Cys Thr Pro Thr Thr
    50                  55                  60

Gln Ile Ser Ser Ala Ser Pro Thr Gly Thr Asn Pro Ser Ser Val Thr
65                  70                  75                  80

Ser Leu Val Pro Ser Gly Ser Ser Tyr Thr Ser Thr Thr Ser Leu Ser
                85                  90                  95

Thr Ala Thr Ala Thr Gln Val Pro Tyr Pro Ala Val Asn Ser Ser Ser
        100                 105                 110

Cys Gly Ala Trp Ala Leu Val Glu Asn Val Cys Cys Pro Tyr Tyr Cys
    115                 120                 125

Thr Ser Asn Asn Glu Ser Glu Ser Cys Thr Ser Ser Cys Ser Gly Gly
130                 135                 140

Cys Gly Ser Pro Asp Ala Ser Met Cys Lys Ser Gly Thr Met Trp Gly
145                 150                 155                 160

Glu Gln Thr Thr Val Gly Thr Asp Glu Ser Trp His Tyr Ser Arg Ser
                165                 170                 175

Thr His Phe Gly Leu Thr Ser Gly Gly Ala Cys Gly Phe Gly Leu Tyr
        180                 185                 190

Gly Leu Cys Thr Lys Gly Ser Ser Thr Ala Asn Trp Thr Asp Pro Met
    195                 200                 205

Leu Gly Asp Thr Cys Asn Ala Phe Cys Thr Ala Tyr Pro Leu Leu Cys
210                 215                 220

Arg Asp Pro Ser Asn Val Thr Leu Arg Gly Asn Phe Ala Ala Pro Asn
225                 230                 235                 240

Gly Asp Tyr Tyr Thr Gln Phe Trp Pro Ser Leu Ala Ala Glu Gly Asn
                245                 250                 255

Pro Asp Asn Tyr Leu Ser Cys Gly Glu Cys Phe Glu Leu Ile Arg Thr
        260                 265                 270

Lys Ala Asp Gly Thr Asp Tyr Thr Val Gly Glu Asp Gly Tyr Thr Asp
    275                 280                 285

Pro Ile Tyr Leu Glu Ile Val Asp Ser Cys Pro Cys Ser Ala Asn Ala
290                 295                 300

Lys Trp Cys Cys Gly Ser Gly Ala Asp His Cys Gly Glu Ile Asp Phe
305                 310                 315                 320

Lys Tyr Gly Cys Pro Ile Pro Lys Asp Ser His His Met Asp Leu Ser
                325                 330                 335

Asp Ile Ala Met Gly Arg Leu Gln Gly Asn Gly Ser Leu Ala Asp Gly
        340                 345                 350

Val Ile Pro Ile Arg Tyr Lys Arg Val Pro Cys Pro Lys Pro Gly Asn
        355                 360                 365

Val Tyr Ile Trp Leu Arg Asp Gly Ala Gly Pro Tyr Tyr Phe Ser Leu
    370                 375                 380

Ser Ala Val Asn Thr Asn Gly Val Gly Ser Val Ile Asn Val Glu Val
385                 390                 395                 400

Gln Gly Ser Gly Gln Thr Gly Trp Thr Ala Leu Glu Arg Asp Pro Asn
                405                 410                 415

Tyr Ser Ser Ser Arg Pro Gln Glu Arg Tyr Gly Ala Trp Val Val Pro
            420                 425                 430

Gln Gly Ala Gly Pro Phe Asn Val Pro Ile Gly Ile Arg Ile Thr Ser
        435                 440                 445

Pro Asp Gly Gln Gln Ile Val Asn Thr Glu Met Ile Thr Ser Phe Thr
    450                 455                 460

Ala Pro Ala Ser Ala Pro Ser Gly Phe Trp Tyr Ile Asp Met Gly Val
465                 470                 475                 480

Gln Phe Thr

<210>  136
<211>  483
<212>  PRT
<213>  Talaromyces marneffei strain PM1

<400>  136

Met Ala Thr Arg Phe Met Leu Val Ala Gly Leu Leu Ser Ala Ser Leu
1               5                   10                  15

Trp Ser Ser Thr Ile Ala Gln Thr Ser Cys Ala Ala Gln Trp Ala Gln
            20                  25                  30

Cys Gly Gly Ser Gly Phe Ser Gly Pro Ser Cys Cys Gln Thr Gly Ser
        35                  40                  45

Ser Cys Val Ser Leu Asn Gln Tyr Tyr Ser Gln Cys Thr Pro Thr Thr
    50                  55                  60

Gln Ile Ser Ser Ala Pro Pro Thr Gly Thr His Gly Ser Ser Val Thr
65                  70                  75                  80

Ser Ile Ala Phe Ser Gly Ser Ser Tyr Thr Ser Thr Thr Ser Leu Gly
                85                  90                  95

Thr Ser Thr Ala Thr Gln Val Pro Tyr Pro Ala Ala Lys Ala Ser Asp
            100                 105                 110

Cys Gly Ser Trp Ala Leu Val Asp Asn Val Cys Cys Pro Tyr Tyr Cys
        115                 120                 125

Thr Ser Asn Asn Glu Ser Glu Ser Cys Thr Ser Ser Cys Ser Gly Gly
    130                 135                 140

Cys Ser Ser Pro Asp Ser Ser Met Cys Lys Ser Gly Thr Met Trp Gly
145                 150                 155                 160

Glu Gln Val Thr Val Gly Ala Asp Glu Ala Trp His Tyr Ser Arg Ser
                165                 170                 175

Thr His Phe Gly Leu Thr Ser Gly Gly Ala Cys Gly Phe Gly Leu Tyr
            180                 185                 190

Gly Leu Cys Thr Lys Gly Ser Lys Thr Ala Ser Trp Thr Asp Pro Met
        195                 200                 205

Leu Gly His Thr Cys Asp Ala Phe Cys Thr Ala Tyr Pro Leu Leu Cys
    210                 215                 220

Gln Asp Pro Ser Asn Val Thr Leu Arg Gly Asn Phe Ala Ala Pro Asn
225                 230                 235                 240

Gly Asp Tyr Tyr Thr Gln Phe Trp Pro Ser Leu Ala Ser Glu Gly Asn
                245                 250                 255

Pro Asp Asn Tyr Leu Ser Cys Gly Glu Cys Phe Glu Leu Ile Arg Thr
            260                 265                 270

Lys Ala Asp Gly Thr Asp Tyr Ala Val Gly Glu Ser Gly Tyr Thr Asp
        275                 280                 285

Pro Val Tyr Leu Glu Ile Val Asp Ser Cys Pro Cys Asn Ala Asn Ser
    290                 295                 300

Lys Trp Cys Cys Gly Ser Gly Ala Asp His Cys Gly Glu Ile Asp Phe
305                 310                 315                 320

Thr Tyr Gly Cys Pro Ile Pro Glu Lys Ser His His Met Asp Leu Ser
                325                 330                 335

Asp Ile Ala Met Gly Arg Leu Gln Gly Asn Gly Ser Leu Ala Asp Gly
            340                 345                 350

Val Ile Pro Ile Arg Tyr Lys Arg Val Pro Cys Pro Lys Pro Gly Asn
        355                 360                 365

Val Tyr Leu Trp Ile Arg Asp Gly Ala Ala Pro Tyr Tyr Phe Ser Leu
    370                 375                 380

Ser Ala Val Asn Thr Asn Gly Val Gly Ser Ile Ile Ser Ile Glu Val
385                 390                 395                 400

Gln Gly Ser Gly Gln Ser Asn Trp Thr Ala Leu Glu Arg Asp Pro Asn
                405                 410                 415

Tyr Ser Ser Ser Arg Pro Gln Glu Arg Tyr Gly Ala Trp Val Val Pro
            420                 425                 430

Gln Gly Ser Gly Pro Phe Asn Val Pro Ile Ser Ile Arg Ile Thr Ser
        435                 440                 445

Pro Asp Gly Gln Gln Ile Val Ser Thr Asp Val Ile Thr Ser Phe Thr
    450                 455                 460

Pro Pro Ala Ser Ala Pro Ser Gly Phe Trp Tyr Val Asp Thr Gly Val
465                 470                 475                 480

Gln Phe Thr

<210>  137
<211>  438
<212>  PRT
<213>  Aspergillus fumigatus

<400>  137

Met Thr Leu Leu Leu Gly Ile Phe Leu Ala Arg Leu Ala Val Ala Ala
1               5                   10                  15

Ala Gln Ser Ser Cys Val Ser Ala Cys Asn Ser Leu Thr Ser Pro Ser
            20                  25                  30

Ser Ser Gly Ser Ser Ser Ala Asn Thr Ser Gly Tyr Thr Thr Thr Thr
        35                  40                  45

Val Leu Pro Ser Pro Thr Ala Thr Gln Asn Pro Tyr Pro Pro Ala Asn
    50                  55                  60

Ala Ser Ser Cys Gly Ser Trp Ala Leu Val Asp Asn Val Cys Cys Pro
65                  70                  75                  80

Tyr Tyr Cys Leu Ser Asn Asn Glu Ser Glu Ser Cys Thr Ser Ser Cys
                85                  90                  95

Thr Gly Gly Cys Gly Ser Pro Asp Ser Ser Met Cys Lys Ser Gly Thr
            100                 105                 110

Met Trp Gly Glu Gln His Thr Val Thr Ser Asn Glu Asp Trp His Tyr
        115                 120                 125

Ser Arg Ser Thr His Phe Gly Leu Thr Ser Gly Gly Ala Cys Gly Phe
    130                 135                 140

Gly Leu Tyr Gly Leu Cys Thr Lys Asn Ser Val Thr Ala Ser Trp Thr
145                 150                 155                 160

Asp Pro Met Leu Gly Ser Thr Cys Asp Ala Phe Cys Thr Ala Tyr Pro
                165                 170                 175

Leu Leu Cys Lys Asp Pro Ala Asn Val Thr Leu Arg Gly Asn Phe Ala
            180                 185                 190

Ala Pro Asn Gly Asp Tyr Tyr Thr Gln Phe Trp Pro Ser Leu Ala Ala
        195                 200                 205

Ser Gly Asn Pro Asp Asn Tyr Leu Ser Cys Gly Glu Cys Phe Glu Val
    210                 215                 220

Ile Arg Thr Lys Pro Asp Gly Thr Asp Tyr Ala Val Gly Glu Ser Gly
225                 230                 235                 240

Tyr Thr Asp Pro Ile Val Leu Glu Val Val Asp Ser Cys Pro Cys Asn
                245                 250                 255

Ala Asn Ser Lys Trp Cys Cys Gly Ser Gly Ala Asp His Cys Gly Glu
            260                 265                 270

Ile Asp Phe Lys Tyr Gly Cys Pro Leu Pro Glu Gly Ser His His Met
        275                 280                 285

Asp Leu Ser Asp Ile Ala Met Gly Arg Leu Gln Gly Asn Gly Ser Leu
    290                 295                 300

Ala Asp Gly Val Ile Pro Ile Arg Tyr Arg Arg Val Pro Cys Pro Lys

305                 310                 315                 320

Pro Gly Asn Ile Tyr Ile Trp Leu His Asp Gly Ala Gly Pro Tyr Tyr
                325                 330                 335

Phe Ala Leu Ser Ala Val Asn Thr Asn Gly Pro Gly Ser Val Ile Ser
            340                 345                 350

Ile Glu Val Gln Ala Gly Gly Ser Gly Pro Trp Val Ala Leu Glu His
        355                 360                 365

Asp Pro Asn Tyr Thr Ser Ser Arg Pro Gln Glu Arg Tyr Gly Ser Trp
    370                 375                 380

Val Leu Pro Gln Gly Lys Gly Pro Ile Thr Ala Pro Val Ser Met Arg
385                 390                 395                 400

Leu Thr Ala Pro Asn Gly Gln Gln Val Val Lys Glu Gly Ala Ile Thr
                405                 410                 415

Ser Tyr Thr Pro Pro Ala Thr Ala Val Glu Gly Phe Arg Tyr Ile Asp
            420                 425                 430

Leu Gly Val Gln Phe Thr
        435

<210>  138
<211>  331
<212>  PRT
<213>  Aspergillus udagawae

<400>  138

Met Ala Asp Ile Thr Thr Gly Gly Glu Gln His Thr Val Thr Ser Asn
1               5                   10                  15

Glu Asn Trp His Tyr Ser Arg Ser Thr His Phe Gly Leu Thr Ser Gly
            20                  25                  30

Gly Ala Cys Gly Phe Gly Leu Tyr Gly Leu Cys Thr Lys Asn Ser Val
        35                  40                  45

Thr Ala Ser Trp Thr Asp Pro Met Leu Gly Ser Thr Cys Asp Ala Phe
    50                  55                  60

Cys Lys Ala Tyr Pro Leu Leu Cys Lys Asp Pro Ala Asn Val Thr Leu
65                  70                  75                  80

Arg Gly Asn Phe Ala Ala Pro Asn Gly Asp Tyr Tyr Thr Gln Phe Trp

                85                  90                  95

Pro Ser Leu Ala Ala Ser Gly Asn Pro Asp Asn Tyr Leu Ser Cys Gly
            100                 105                 110

Glu Cys Phe Glu Leu Ile Arg Thr Lys Ala Asp Gly Thr Asp Tyr Ala
        115                 120                 125

Val Gly Glu Ser Gly Tyr Thr Asp Pro Ile Val Leu Glu Val Val Asp
    130                 135                 140

Ser Cys Pro Cys Asp Ala Asn Ser Lys Trp Cys Cys Gly Ser Gly Ala
145                 150                 155                 160

Asp His Cys Gly Glu Ile Asp Phe Lys Tyr Gly Cys Pro Leu Pro Glu
                165                 170                 175

Gly Ser His His Met Asp Leu Ser Asp Ile Ala Met Gly Arg Leu Gln
            180                 185                 190

Gly Asn Gly Ser Leu Thr Asp Gly Val Ile Pro Ile Arg Tyr Arg Arg
        195                 200                 205

Val Pro Cys Pro Lys Pro Gly Asn Ile Tyr Ile Trp Leu His Asp Gly
    210                 215                 220

Ala Gly Pro Tyr Tyr Phe Ala Leu Ser Ala Val Asn Thr Asn Gly Pro
225                 230                 235                 240

Gly Ser Val Ile Ser Ile Glu Val Gln Ala Gly Gly Ser Gly Pro Trp
                245                 250                 255

Val Ala Leu Glu His Asp Pro Asn Tyr Ser Ser Ser Arg Pro Gln Glu
            260                 265                 270

Arg Tyr Gly Ser Trp Val Leu Pro Gln Gly Lys Gly Pro Ile Thr Val
        275                 280                 285

Pro Val Ala Met Arg Leu Thr Ala Pro Asp Gly Gln Gln Val Val Lys
    290                 295                 300

Gln Gly Ala Ile Thr Ser Phe Thr Pro Pro Ala Thr Ala Val Gln Gly
305                 310                 315                 320

Phe Trp Tyr Ile Asp Leu Gly Val Gln Phe Thr
                325                 330

<210>  139
<211>  374
<212>  PRT
<213>  Talaromyces marneffei strain ATCC 18224

<400>  139

Met Tyr Thr Asn Asn Thr Asp Phe Ile Arg Thr Ala Asn Arg Asp Thr
1               5                   10                  15

Trp Leu Leu Gly Tyr Ile Asn Cys Phe Leu Trp Phe Ile Ile His Lys
            20                  25                  30

Tyr Asn Phe Ser Ser Ser Pro Asp Ser Ser Met Cys Lys Ser Gly Thr
        35                  40                  45

Met Trp Gly Glu Gln Val Thr Val Gly Ala Asp Glu Ala Trp His Tyr
    50                  55                  60

Ser Arg Ser Thr His Phe Gly Leu Thr Ser Gly Gly Ala Cys Gly Phe
65                  70                  75                  80

Gly Leu Tyr Gly Leu Cys Thr Lys Gly Ser Lys Thr Ala Ser Trp Thr
                85                  90                  95

Asp Pro Met Leu Gly His Thr Cys Asp Ala Phe Cys Thr Ala Tyr Pro
            100                 105                 110

Leu Leu Cys Gln Asp Pro Ser Asn Val Thr Leu Arg Gly Asn Phe Ala
        115                 120                 125

Ala Pro Asn Gly Asp Tyr Tyr Thr Gln Phe Trp Pro Ser Leu Ala Ser
    130                 135                 140

Glu Gly Asn Pro Asp Asn Tyr Leu Ser Cys Gly Glu Cys Phe Glu Leu
145                 150                 155                 160

Ile Arg Thr Lys Ala Asp Gly Thr Asp Tyr Ala Val Gly Glu Ser Gly
                165                 170                 175

Tyr Thr Asp Pro Val Tyr Leu Glu Ile Val Asp Ser Cys Pro Cys Asn
            180                 185                 190

Ala Asn Ser Lys Trp Cys Cys Gly Ser Gly Ala Asp His Cys Gly Glu
        195                 200                 205

Ile Asp Phe Thr Tyr Gly Cys Pro Ile Pro Glu Lys Ser His His Met
    210                 215                 220

Asp Leu Ser Asp Ile Ala Met Gly Arg Leu Gln Gly Asn Gly Ser Leu
225                 230                 235                 240

Ala Asp Gly Val Ile Pro Ile Arg Tyr Lys Arg Val Pro Cys Pro Lys
                245                 250                 255

Pro Gly Asn Val Tyr Leu Trp Ile Arg Asp Gly Ala Ala Pro Tyr Tyr
            260                 265                 270

Phe Ser Leu Ser Ala Val Asn Thr Asn Gly Val Gly Ser Ile Ile Ser
        275                 280                 285

Ile Glu Val Gln Gly Ser Gly Gln Ser Asn Trp Thr Ala Leu Glu Arg
    290                 295                 300

Asp Pro Asn Tyr Ser Ser Ser Arg Pro Gln Glu Arg Tyr Gly Ala Trp
305                 310                 315                 320

Val Val Pro Gln Gly Ser Gly Pro Phe Asn Val Pro Ile Ser Ile Arg
                325                 330                 335

Ile Thr Ser Pro Asp Gly Gln Gln Ile Val Ser Thr Asp Val Ile Thr
            340                 345                 350

Ser Phe Thr Pro Pro Ala Ser Ala Pro Ser Gly Phe Trp Tyr Val Asp
        355                 360                 365

Thr Gly Val Gln Phe Thr
    370

<210>  140
<211>  389
<212>  PRT
<213>  Talaromyces cellulotyticus

<400>  140

Met Pro Asn Ala Ser Val Ala Arg Thr Gly Thr Ser Pro Val Thr Thr
1               5                   10                  15

Thr Ser Met Pro Pro Thr Thr Arg Thr Thr Pro Tyr Arg Thr Ser Thr
            20                  25                  30

Thr Phe Leu Thr Gly Thr Ala Leu Ala Ser His Thr Gln Val Pro Tyr
        35                  40                  45

Thr Ser Leu Glu Cys Asp Asn Ala Asp Cys Gly Ser Gly Thr Ile Tyr
    50                  55                  60

Pro Asp Ile Thr Thr Val Asn Asp Ser Met Pro Trp His Tyr Ser Arg
65                  70                  75                  80

Ser Thr His Phe Gly Glu Thr Pro Ala Gly Ala Cys Gly Phe Gly Leu
                85                  90                  95

Tyr Gly Met Cys Thr Thr Lys Ser Val Ser Gly Ile Asp Leu Gly Asp
            100                 105                 110

Asp Cys Ala Gln Phe Cys Thr Met Tyr Pro Asp Leu Cys Thr Asp Pro
        115                 120                 125

Ala Asn Val Thr Leu Arg Gly Asn Phe Ala Ala Pro Asn Gly Asn Tyr
    130                 135                 140

Tyr Thr Gln Phe Trp Pro Met Ile Pro Ser Leu Asp Thr Ser Ala Glu
145                 150                 155                 160

Gln Asp Asn Tyr Leu Ser Cys Gly Glu Cys Phe Glu Leu Val Arg Thr
                165                 170                 175

Tyr Pro Asn Gly Thr Leu Tyr Ala Thr Asn Asp Ala Gly Tyr Ser Pro
            180                 185                 190

Pro Leu Val Leu Glu Ile Val Asp Ser Cys Pro Cys Ala Ala Asn Thr
        195                 200                 205

Lys Trp Cys Cys Gly Pro Gly Val Asp Gln Cys Gln Glu Val Ser Asp
    210                 215                 220

Phe Thr Tyr Gly Cys Pro Leu Pro Asn Gly Ser Ile His Leu Asp Leu
225                 230                 235                 240

Ser Asp Val Ala Met Thr Arg Ala Gln Ala Gly Thr Leu Val Glu Gly
                245                 250                 255

Val Ile Pro Thr Gln Tyr Lys Arg Val Pro Cys Pro Lys Val Gly Asn
            260                 265                 270

Val Tyr Leu Trp Leu Arg Gly Ser Ala Gly Pro Tyr Phe Phe Gln Ile
        275                 280                 285

Thr Ala Val Asn Ala Gly Gly Leu Gly Ser Ile Val Gly Phe Glu Ile
    290                 295                 300

Arg Pro Asp Gly Gln Ser Thr Trp Leu Ser Leu Val Gln Glu Ala Asp
305                 310                 315                 320

Tyr Pro Glu Gly His Pro Gln Glu Arg Tyr Gly Ala Trp Val Leu Pro
                325                 330                 335

Ala Asn Ala Gly Pro Ile Asn Leu Pro Met Gly Val Arg Leu Thr Ser
            340                 345                 350

Ala Thr Gly Glu Gln Ile Val Asn Glu Ala Val Ile Thr Asn Trp Thr
        355                 360                 365

Ala Pro Ala Thr Ala Ile Ser Gly Tyr Trp Tyr Ile Asp Leu Gly Ile
    370                 375                 380

Asn Phe Ser Gln Leu
385

<210>  141
<211>  35
<212>  DNA
<213>  Artificial Sequence

<220>
<223>  Primer F1

<400>  141
gttctccaga agcgacagca accgggtacc agcac                                35

<210>  142
<211>  41
<212>  DNA
<213>  Artificial Sequence

<220>
<223>  Primer R1

<400>  142
cacagacacc ctaggctaaa ggcattgcga gtagtagtcg t                         41

<210>  143
<211>  37
<212>  DNA
<213>  Artificial Sequence

<220>
<223>  Primer F2

<400>  143
gttctccaga agcgagcaca gcagactgtc tggggcc                              37

<210>  144
<211>  42
<212>  DNA
<213>  Artificial Sequence

<220>

<223>  Primer R2

<400>  144
cacagacacc ctaggctact ttcttgcgag acacgagctg ac                          42


<210>  145
<211>  36
<212>  DNA
<213>  Artificial Sequence

<220>
<223>  Primer F3

<400>  145
gttctccaga agcgagccca ggcttgctca agcgtc                                 36


<210>  146
<211>  38
<212>  DNA
<213>  Artificial Sequence

<220>
<223>  Primer R3

<400>  146
cacagacacc ctaggttaca ggaacgatgg gtttgcgt                               38


<210>  147
<211>  46
<212>  DNA
<213>  Artificial Sequence

<220>
<223>  Primer F4

<400>  147
gttctccaga agcgacatgg acatattaat gacattgtca tcaacg                      46


<210>  148
<211>  42
<212>  DNA
<213>  Artificial Sequence

<220>
<223>  Primer R4

<400>  148
cacagacacc ctaggctagt taaggcactg ggcgtagtag gg                          42


<210>  149
<211>  35
<212>  DNA
<213>  Artificial Sequence

<220>
<223>  Primer F5

<400>  149

gttctccaga agcgacaggc gtcggagtgg ggaca 35

<210> 150
<211> 35
<212> DNA
<213> Artificial Sequence

<220>
<223> Primer R5

<400> 150
cacagacacc ctaggctaca gcggcgggtt ggcag 35

<210> 151
<211> 29
<212> DNA
<213> Artificial Sequence

<220>
<223> Primer JMP1F

<400> 151
cctagggtgt ctgtggtatc taagctatt 29

<210> 152
<211> 20
<212> DNA
<213> Artificial Sequence

<220>
<223> Primer JMP1R

<400> 152
tcgcttctgg agaactgcgg 20

<210> 153
<211> 38
<212> DNA
<213> Artificial Sequence

<220>
<223> Primer F6

<400> 153
acacccgaag gatcccacaa tgaagctttc caccatcc 38

<210> 154
<211> 38
<212> DNA
<213> Artificial Sequence

<220>
<223> Primer R6

<400> 154
atggtgatga tggtgaaggc attgcgagta gtagtcgt 38

<210>  155
<211>  39
<212>  DNA
<213>  Artificial Sequence

<220>
<223>  Primer R7

<400>  155
atggtgatga tggtgctttc ttgcgagaca cgagctgac                              39


<210>  156
<211>  35
<212>  DNA
<213>  Artificial Sequence

<220>
<223>  Primer R8

<400>  156
atggtgatga tggtgcagga acgatgggtt tgcgt                                  35


<210>  157
<211>  33
<212>  DNA
<213>  Artificial Sequence

<220>
<223>  Primer R9

<400>  157
atggtgatga tggtgcagcg gcgggttggc agc                                    33


<210>  158
<211>  39
<212>  DNA
<213>  Artificial Sequence

<220>
<223>  Primer R10

<400>  158
atggtgatga tggtggttaa ggcactgggc gtagtaggg                              39


<210>  159
<211>  25
<212>  DNA
<213>  Artificial Sequence

<220>
<223>  Primer JMP2F

<400>  159
caccatcatc accatcatta aaact                                            25


<210>  160
<211>  21
<212>  DNA

<213>  Artificial Sequence

<220>
<223>  Primer JMP2R

<400>  160
ggatccttcg ggtgtgagtt g                                                21


<210>  161
<211>  32
<212>  DNA
<213>  Artificial Sequence

<220>
<223>  Primer HTF

<400>  161
atccctagaa tcgatgccgc cgcaaggaat gg                                    32


<210>  162
<211>  37
<212>  DNA
<213>  Artificial Sequence

<220>
<223>  Primer HTR

<400>  162
gccaacccgg tctctgcact tttgcccgtg atcagtg                               37


<210>  163
<211>  17
<212>  DNA
<213>  Artificial Sequence

<220>
<223>  Primer JMP3F

<400>  163
agagaccggg ttggcgg                                                     17


<210>  164
<211>  27
<212>  DNA
<213>  Artificial Sequence

<220>
<223>  Primer JMP3R

<400>  164
atcgattcta gggataacag ggtaatt                                          27


<210>  165
<211>  1593
<212>  DNA
<213>  Hypocrea jecorina

<400>  165

atgaagcttt ccaccatcct tttcacagcc tgcgctaccc tggctgccgc cctcccttcc 60

cccatcactc cttctgaggc cgcagttctc cagaagcgac agtcggcctg cactctccag 120

tccgagaccc accctcccct gacttggcag aagtgctcct ctggcggtac ttgtacccag 180

cagaccggtt ccgtcgtgat cgacgctaac tggcgatgga ctcatgccac caactcgtcc 240

accaactgtt acgacggtaa cacttggtct tcgaccctct gccccgataa cgagacctgt 300

gctaagaact gctgtctgga cggagccgct tacgcctcca cctacggagt caccacttct 360

ggcaactccc tctctattgg cttcgtcacc cagtccgccc agaagaacgt gggtgctcga 420

ctgtacctca tggcctctga caccacttac caggagttta ccctgctcgg taacgagttc 480

tcttttgacg tggatgtttc gcagctgccc tgcggtctca acggagctct gtacttcgtt 540

tccatggacg ccgatggagg cgtctctaag taccctacta acaccgccgg cgctaagtac 600

ggtaccggat actgcgactc tcagtgtccc cgagatctga agttcatcaa cggccaggct 660

aacgttgagg gttgggagcc ttcctctaac aacgccaaca ccggtattgg tggacacgga 720

tcttgctgtt cggagatgga catctgggag gctaactcga tttccgaggc cctcacccct 780

cacccttgca ccactgtggg ccaggagatc tgcgagggcg acggttgtgg cggtacctac 840

tccgataacc gatacggagg cacttgcgac cccgatggat gtgattggaa cccttaccga 900

ctgggcaaca cctctttcta cggacccggc tcgtccttta ctctcgacac cactaagaag 960

ctgactgttg tcacccagtt cgagacctcc ggtgctatta accgatacta cgttcagaac 1020

ggagtcacct ttcagcagcc caacgccgag ctcggatctt actcgggcaa cgagctgaac 1080

gacgattact gcaccgccga ggaagccgag ttcggtggat cttcgttttc cgacaagggc 1140

ggtctgaccc agttcaagaa ggctacttcc ggaggcatgg tcctcgtgat gtctctgtgg 1200

gacgattact acgccaacat gctgtggctc gactctactt accccaccaa cgagacttcc 1260

tctacccctg gtgccgtccg aggctcctgt tctacctcgt ccggtgtgcc cgctcaggtt 1320

gagtcgcagt cccctaacgc caaggtgacc ttctctaaca tcaagtttgg ccccattggt 1380

tccaccggaa acccttctgg tggaaaccct cccggcggca accctcccgg aaccactacc 1440

actcgacgac ccgctaccac taccggctct tctcctggtc ctacccagtc ccactacgga 1500

cagtgtggag gcattggata ctctggtcct accgtgtgtg cttctggaac tacctgtcag 1560

gttctcaacc cttactactc tcagtgtctg taa 1593

<210> 166
<211> 1614
<212> DNA
<213> Penicillium funiculosum

<400> 166
atgaagcttt ccaccatcct tttcacagcc tgcgctaccc tggctgccgc cctcccttcc 60

cccatcactc cttctgaggc cgcagttctc cagaagcgac agcagatcgg cacctacact 120

gccgagaccc acccctccct ctcttggtcg acctgcaagt ccggcggttc ttgtaccact 180

aactccggtg ccattaccct ggacgctaac tggcgatggg tgcatggcgt taacacctct 240

actaactgct acactggtaa cacctggaac tcggccatct gtgacaccga tgcctcttgc 300

gctcaggact gtgccctcga cggtgctgat tactccggca cctacggtat taccacttcg 360

ggaaactccc tgcgactcaa cttcgtcacc ggctcgaacg tgggttcccg aacttacctg 420

atggctgaca acacccacta ccagatcttt gatctgctca accaggagtt cacctttact 480

gtcgacgtgt ctcatctgcc ctgcggactc aacggcgccc tgtacttcgt gaccatggac 540

gctgatggag gcgtttccaa gtaccctaac aacaaggccg gcgctcagta cggtgtcgga 600

tactgcgact ctcagtgtcc ccgagatctc aagttcatcg ccggccaggc taacgtggag 660

ggttggaccc cttccgccaa caacgctaac accggcattg gtaaccacgg agcctgctgt 720

gctgagctgg acatctggga ggccaactcc atttctgagg ctctcactcc ccatccttgt 780

gatacccccg gtctgtctgt ctgcaccact gacgcctgtg gtggaaccta ctcctctgac 840

cgatacgctg gaacctgcga ccccgatggc tgtgacttca acccttaccg actcggcgtc 900

accgactttt acggctccgg caagaccgtg gataccacta agcccttcac tgtcgtgacc 960

cagtttgtta ccaacgacgg aacctctact ggctcgctgt ccgagatccg acgatactac 1020

gtccagaacg gtgttgtcat tccccagcct tcgtccaaga tctctggtat ttcgggaaac 1080

gtgatcaact ccgactactg cgccgctgag atttccactt tcggcggtac cgcctctttt 1140

aacaagcacg gaggcctcac caacatggcc gctggtatgg aggccggaat ggttctcgtc 1200

atgtccctgt gggacgatta cgctgttaac atgctgtggc tcgactctac ttaccctacc 1260

aacgctaccg gaactcctgg tgctgctcga ggtacctgtg ctaccacttc cggagacccc 1320

aagaccgtgg agtctcagtc gggatcttcg tacgttacct tctctgatat tcgagtcggc 1380

cctttcaact ctaccttctc cggcggctcc tctaccggcg gttctaccac taccactgcc 1440

tcgcgaacca ctaccacttc tgcttcgtcc acctcgactt cttcgacctc cactggaacc 1500

ggagtggctg gacactgggg acagtgtgga ggccagggtt ggaccggacc taccacttgc 1560

gtttccggca ccacttgtac cgtggttaac ccttactact ctcagtgtct gtaa 1614

<210> 167
<211> 1614
<212> DNA
<213> Neurospora crassa

<400> 167
atgaagcttt ccaccatcct tttcacagcc tgcgctaccc tggctgccgc cctcccttcc 60

cccatcactc cttctgaggc cgcagttctc cagaagcgac aggccgtctg ctccctgacc 120

gctgagactc acccctccct caactggtct aagtgcacct cctctggttg tactaacgtt 180

gccggctcca tcaccgtcga cgctaactgg cgatggaccc atattacttc tggatcgacc 240

aactgttact ctggcaacga gtgggacacc tccctgtgct ctaccaacac tgactgtgcc 300

accaagtgct gtgtggatgg tgctgagtac tcgtccacct acggcattca gacttccggt 360

aactccctgt ctctccagtt cgttaccaag ggttcgtact ccactaacat cggatctcga 420

acctacctca tgaacggcgc cgacgcttac cagggatttg agctgctcgg caacgagttc 480

acttttgacg ttgatgtctc tggaaccgga tgtggtctga acggtgctct ctacttcgtg 540

tctatggacc tggatggcgg caaggctaag tacaccaaca acaaggccgg tgctaagtac 600

ggaaccggat actgtgacgc tcagtgtccc cgagatctca agtacatcaa cggcattgct 660

aacgttgagg gttggacccc ttctactaac gacgccaacg ctggtatcgg agatcacgga 720

acctgctgtt cggagatgga catttgggag gccaacaagg tctctaccgc tttcactccc 780

cacccttgca ccactattga gcagcatatg tgcgagggcg actcttgtgg aggcacctac 840

tcggacgacc gatacggtgg aacctgcgac gccgatggct gtgacttcaa ctcctaccga 900

atgggtaaca ccacttttta cggcgagggc aagaccgttg atacttcttc gaagttcacc 960

gtcgtgactc agtttatcaa ggactctgcc ggagatctgg ctgagattaa gcgattttac 1020

gtccagaacg gcaaggtcat cgagaactct cagtcgaacg tcgacggtgt gtcgggaaac 1080

tccattaccc agtccttctg caacgcccag aagaccgctt tcggtgacat cgacgatttt 1140

aacaagaagg gcggtctgaa gcagatggga aaggccctgg ctaagcccat ggtgctcgtt 1200

atgtccattt gggacgatca cgccgctaac atgctgtggc tcgactctac ctaccccgtg 1260

gagggaggcc ctggtgctta ccgaggagag tgtcccacca cttctggtgt gcctgccgag 1320

gttgaggcca acgctcccaa ctcgaaggtc atcttctcca acattaagtt tggccctatc 1380

ggttctacct tctccggcgg ctcctctgga acccctccct ccaacccctc gtcctctgtt 1440

aagcctgtca cctctactgc caagccctcg tccacctcca ctgcttctaa cccctctgga 1500

accggtgctg ctcattgggc tcagtgtggc ggtattggat tctccggccc caccacttgc 1560

cagtctcctt acacctgtca gaagatcaac gactactact cccagtgtgt gtaa 1614

<210> 168
<211> 21
<212> DNA
<213> Artificial Sequence

<220>
<223> Primer VBGL1A

<400> 168
ttgacccagt agcggaccca a 21

<210>  169
<211>  23
<212>  DNA
<213>  Artificial Sequence

<220>
<223>  Primer VBGL1B

<400>  169
gccgacatta gccctaacag cat                                               23


<210>  170
<211>  18
<212>  DNA
<213>  Artificial Sequence

<220>
<223>  Primer VBGL2A

<400>  170
ggttggcggc gcatttgt                                                     18


<210>  171
<211>  22
<212>  DNA
<213>  Artificial Sequence

<220>
<223>  Primer VBGL2B

<400>  171
tgtcgtccac tcggctttca tc                                                22


<210>  172
<211>  24
<212>  DNA
<213>  Artificial Sequence

<220>
<223>  Primer VEGIA

<400>  172
cacttgccgt taagggcgta gggt                                              24


<210>  173
<211>  25
<212>  DNA
<213>  Artificial Sequence

<220>
<223>  Primer VEGIB

<400>  173
ctggctgttg tcgttccaaa tgctg                                             25


<210>  174
<211>  21
<212>  DNA

<213>  Artificial Sequence

<220>
<223>  Primer VEGIIA

<400>  174
cttgccgtta agggcgtagg g                                                21


<210>  175
<211>  25
<212>  DNA
<213>  Artificial Sequence

<220>
<223>  Primer VEGIIB

<400>  175
ccatcgagca taattgtgga tgtcg                                            25


<210>  176
<211>  25
<212>  DNA
<213>  Artificial Sequence

<220>
<223>  Primer VCBHIA

<400>  176
tttgctttgt ggttgggact ttagc                                            25


<210>  177
<211>  27
<212>  DNA
<213>  Artificial Sequence

<220>
<223>  Primer VCBHIB

<400>  177
ggttcgagat ccgatgttag tggagta                                          27


<210>  178
<211>  26
<212>  DNA
<213>  Artificial Sequence

<220>
<223>  Primer VCBHIIA

<400>  178
tttgctttgt ggttgggact ttagcc                                           26


<210>  179
<211>  20
<212>  DNA
<213>  Artificial Sequence

<220>

<223>  Primer VCBHIIB

<400>  179
aggcgttgga ccagccgtga                                                    20


<210>  180
<211>  19
<212>  DNA
<213>  Artificial Sequence

<220>
<223>  Primer V4UASCBHIA

<400>  180
cgccgcaagg aatggtgca                                                     19


<210>  181
<211>  27
<212>  DNA
<213>  Artificial Sequence

<220>
<223>  Primer V4UASCBHIB

<400>  181
ggttcgagat ccgatgttag tggagta                                            27


<210>  182
<211>  19
<212>  DNA
<213>  Artificial Sequence

<220>
<223>  Primer V4UASCBHIIA

<400>  182
cgccgcaagg aatggtgca                                                     19


<210>  183
<211>  27
<212>  DNA
<213>  Artificial Sequence

<220>
<223>  Primer V4UASCBHIIB

<400>  183
ccgatccgac tggaggtact ctggtag                                            27


<210>  184
<211>  24
<212>  DNA
<213>  Artificial Sequence

<220>
<223>  Primer VLPMOA

<400>  184

ttgacccagt agcggaccca accc                                          24


<210>  185
<211>  25
<212>  DNA
<213>  Artificial Sequence

<220>
<223>  Primer VLPMOB

<400>  185
tgatctcgtg gcggagcacg taatt                                         25


<210>  186
<211>  1530
<212>  DNA
<213>  Hypocrea jecorina

<400>  186
atgaagcttt ccaccatcct tttcacagcc tgcgctaccc tggctgccgc cctcccttcc      60

cccatcactc cttctgaggc cgcagttctc cagaagcgag cccagcagaa ctgtgccgct     120

ctctttggac agtgtggcgg aatcggttgg tcgggtacta cctgttgcgt tgccggtgcc     180

cagtgctcct tcgtgaacga ctggtactcg cagtgtctcg cttccaccgg aggtaaccct     240

cctaacggta ccacttcctc ttcgctggtg tcccgaacct cctctgcttc gtcctctgtt     300

ggctcgtcct ctcccggagg caactctcct actggttctg cctcgaccta caccactacc     360

gacactgcca ccgttgctcc ccactcccag tctccctacc cttccatcgc cgcttcgtcc     420

tgtggatctt ggaccctcgt cgacaacgtg tgctgtcctt cttactgcgc taacgacgat     480

acctcggagt cctgctctgg ctgtggtacc tgcactaccc ccccttctgc cgactgtaag      540

tcgggtacca tgtaccccga ggttcaccat gtctcttcga acgagtcctg gcactactcg     600

cgatccactc atttcggact gacctctggt ggagcttgtg gatttggcct gtacggactc     660

tgcaccaagg gctccgtcac tgcctcttgg accgacccca tgctcggagc cacttgtgat     720

gctttctgca ccgcctaccc cctgctctgc aaggacccta ccggtactac cctgcgagga     780

aacttcgccg ctcccaacgg cgactactac acccagtttt ggtcctctct gcctggtgcc     840

ctcgataact acctgtcctg tggagagtgc atcgagctca ttcagactaa gcccgacggt     900

accgattacg ctgttggcga ggccggttac actgacccca tcaccctgga gattgtcgat     960

tcttgccctt gttctgctaa ctcgaagtgg tgctgtggtc ctggagctga ccactgtgga    1020

gagattgatt tcaagtacgg ttgccccctg cctgctgact ccatccatct ggacctctct     1080

gatattgcca tgggacgact ccagggcaac ggttccctga ctaacggcgt catccccacc    1140

cgataccgac gagtccagtg ccctaaggtc ggcaacgctt acatttggct ccgaaacggc    1200

ggtggaccct actactttgc cctgactgct gttaacacca acggacctgg ctctgtcacc    1260

aagatcgaga ttaagggcgc tgacaccgat aactgggtgg ccctggttca cgaccccaac 1320

tacacctcgt cccgacctca ggagcgatac ggttcctggg tcatccccca gggttctgga 1380

cccttcaacc tccctgtggg aattcgactg acttccccca ccggcgagca gatcgtgaac 1440

gagcaggcca ttaagacttt tacccctcct gccaccggag accccaactt ttactacatt 1500

gacatcggcg tccagttttc ccagaactaa 1530

<210> 187
<211> 21
<212> DNA
<213> Artificial Sequence

<220>
<223> Primer VBGL1A

<400> 187
ttgacccagt agcggaccca a 21

<210> 188
<211> 23
<212> DNA
<213> Artificial Sequence

<220>
<223> Primer VBGL1B

<400> 188
gccgacatta gccctaacag cat 23

<210> 189
<211> 18
<212> DNA
<213> Artificial Sequence

<220>
<223> Primer VBGL2A

<400> 189
ggttggcggc gcatttgt 18

<210> 190
<211> 22
<212> DNA
<213> Artificial Sequence

<220>
<223> Primer VBGL2B

<400> 190
tgtcgtccac tcggctttca tc 22

<210> 191
<211> 24
<212> DNA
<213> Artificial Sequence

<220>
<223>  Primer VEGIA

<400>  191
cacttgccgt taagggcgta gggt                                              24


<210>  192
<211>  25
<212>  DNA
<213>  Artificial Sequence

<220>
<223>  Primer VEGIB

<400>  192
ctggctgttg tcgttccaaa tgctg                                             25


<210>  193
<211>  21
<212>  DNA
<213>  Artificial Sequence

<220>
<223>  Primer VEGIIA

<400>  193
cttgccgtta agggcgtagg g                                                 21


<210>  194
<211>  25
<212>  DNA
<213>  Artificial Sequence

<220>
<223>  Primer VEGIIB

<400>  194
ccatcgagca taattgtgga tgtcg                                             25


<210>  195
<211>  25
<212>  DNA
<213>  Artificial Sequence

<220>
<223>  Primer VCBHIA

<400>  195
tttgctttgt ggttgggact ttagc                                             25


<210>  196
<211>  27
<212>  DNA
<213>  Artificial Sequence

<220>
<223>  Primer VCBHIB

<400>  196
ggttcgagat ccgatgttag tggagta                                          27


<210>  197
<211>  26
<212>  DNA
<213>  Artificial Sequence

<220>
<223>  Primer VCBHIIA

<400>  197
tttgctttgt ggttgggact ttagcc                                           26


<210>  198
<211>  20
<212>  DNA
<213>  Artificial Sequence

<220>
<223>  Primer VCBHIIB

<400>  198
aggcgttgga ccagccgtga                                                  20


<210>  199
<211>  19
<212>  DNA
<213>  Artificial Sequence

<220>
<223>  Primer V4UASCBHIA

<400>  199
cgccgcaagg aatggtgca                                                   19


<210>  200
<211>  27
<212>  DNA
<213>  Artificial Sequence

<220>
<223>  Primer V4UASCBHIB

<400>  200
ggttcgagat ccgatgttag tggagta                                          27


<210>  201
<211>  19
<212>  DNA
<213>  Artificial Sequence

<220>
<223>  Primer V4UASCBHIIA

<400>  201
cgccgcaagg aatggtgca                                                   19

<210>  202
<211>  27
<212>  DNA
<213>  Artificial Sequence

<220>
<223>  Primer V4UASCBHIIB

<400>  202
ccgatccgac tggaggtact ctggtag                                          27


<210>  203
<211>  24
<212>  DNA
<213>  Artificial Sequence

<220>
<223>  Primer VLPMOA

<400>  203
ttgacccagt agcggaccca accc                                             24


<210>  204
<211>  25
<212>  DNA
<213>  Artificial Sequence

<220>
<223>  Primer VLPMOB

<400>  204
tgatctcgtg gcggagcacg taatt                                            25


<210>  205
<211>  27
<212>  DNA
<213>  Artificial Sequence

<220>
<223>  Primer VSWOA

<400>  205
ttttgctttg tggttgggac tttagcc                                          27


<210>  206
<211>  25
<212>  DNA
<213>  Artificial Sequence

<220>
<223>  Primer VSWOB

<400>  206
gggtgtagta gtcgccgttg ggagc                                            25


<210>  207

<211>  66
<212>  DNA
<213>  Hypocrea jecorina

<400>  207
atggcgccct cagttacact gccgttgacc acggccatcc tggccattgc ccggctcgtc        60

gccgcc                                                                    66


<210>  208
<211>  60
<212>  DNA
<213>  Hypocrea jecorina

<400>  208
atgaacaagt ccgtggctcc attgctgctt gcagcgtcca tactatatgg cggcgccgtc        60


<210>  209
<211>  51
<212>  DNA
<213>  Neurospora crassa

<400>  209
atgctcgcca agttcgctgc ccttgcggcc cttgtggcct ctgccaacgc c                 51


<210>  210
<211>  54
<212>  DNA
<213>  Hypocrea jecorina

<400>  210
atgattgtcg gcattctcac cacgctggct acgctggcca cactcgcagc tagt              54


<210>  211
<211>  63
<212>  DNA
<213>  Hypocrea jecorina

<400>  211
atgatccaga agctttccaa cctccttgtc accgcactgg cggtggctac tggcgttgtc        60

gga                                                                       63


<210>  212
<211>  54
<212>  DNA
<213>  Hypocrea jecorina

<400>  212
atggctggta agcttatcct cgtggctcta gcaagccttg tatcactctc tatt              54


<210>  213
<211>  99
<212>  DNA
<213>  Yarrowia lipolytica

<400>  213

atgaagcttt ccaccatcct tttcacagcc tgcgctaccc tggctgccgc cctcccttcc 60

cccatcactc cttctgaggc cgcagttctc cagaagcga 99

## Description

The present invention relates to mutant yeast strains, such as *Yarrowia lipolytica* strains, capable of growing on cellulose as carbon source and means for obtaining such mutant strains.

The production of second-generation biofuels and platform molecules for the chemical industry from lignocellulosic biomass (LCB) is viewed as crucial part of the bio-economy. Cellulose, the main component of LCB, is a polymer composed of β-1,4-linked glucose subunits usually embedded in an amorphous matrix of hemicellulose and lignin. It may exist in two forms, a tightly packed crystalline form where individual chains are organized in microfibrils via hydrogen bonding and van der Waals interactions, or less-ordered amorphous form. The depolymerisation of cellulose requires a variety of enzymes, including endoglucanases (EGs) (EC 3.2.1.4) that hydrolyse internal β-glucosidic bonds, cellobiohydrolases (CBHs) (EC 3.2.1.91) that remove cello-oligosaccharides in a processive manner from chain termini, and β-glucosidases (BGLs) (EC 3.2.1.21) that degrade cello-oligosaccharides to glucose (Menon and Rao, 2012). However, the condensed structure of crystalline cellulose and its intimate proximity with hemicelluloses and lignin combine to make LCB very resistant to enzymatic hydrolysis and render biochemical processing of raw LCB economically unreasonable. To overcome this recalcitrance, biomass pretreatment is necessry. However, this step constitutes a significant cost driver in the overall economics of LCB biorefinery processes. Presently it is widely recognized that significant technological advances, including better pretreatments, lower cost enzymes and efficient process design will be required in order to make LCB biorefining competitive within the current economic framework (Menon and Rao, 2012).

It is known a pretreatment process which belongs to the so-called organosolv technology family, because it uses organic solvents to dissolve lignin and hemicelluloses, and yields a pure and relatively amorphous cellulose fraction that is quite amenable to enzyme action (Delmas, 2008; International Application WO 2012/049054). This process produces functionalized lignins (Biolignin™) and an essentially furfural-free pentose-rich fraction, making this pretreatment technology an interesting platform for the design of a new LCB biorefinery concept.

Consolidated bioprocessing (CBP), featuring microbial enzyme production and concomitant microbial conversion of suitable feedstock into value-added products in a single step, offers great potential for cost-effective lignocellulosic bioconversion (den Haan *et al.,* 2015; Olson *et al.,* 2012). This is because it is predicted that CBP will reduce both capital investment and operating costs and possibly procure higher enzymatic hydrolysis rates. However, the main challenge for CBP technology is to develop a purpose built microorganism that will simultaneously convert cellulose pulp into sugars and ferment these to target products. To date, no naturally-occurring organism has been described with such capabilities (den Haan *et al.,* 2015). Nevertheless, using microbial engineering approaches and focusing on hosts such as *Escherichia coli* (Ryu and Karim, 2015), *Saccharomyces cerevisiae* (reviewed in La Grange DC, *et al..* 2010) and *Kluyveromyces marxianus* (Yanase *et al.,* 2010), considerable progress has been made, although so far most studies have used model cellulose substrates and achieved relatively low titres of product. Importantly, none of the engineered strains reported so far have convincingly hydrolysed the cellulose feedstock, and currently no commercially viable CBP organism has been reported (Brethauer and Studer, 2014).

The so-called oleaginous yeast *Yarrowia lipolytica* can accumulate lipids up to 50% of its dry weight depending on culture conditions, making this a promising platform for the production of biodiesel precursors (Thevenieau and Nicaud, 2013; Blazeck *et al.*, 2014). Advantageously, *Y. lipolytica* is already widely used in detergent, food, pharmaceutical and environmental industries and has been classified by the FDA (Food and Drug Administration) as "Generally Recognized as Safe" (GRAS) for numerous processes (Groenewald *et al.,* 2014). Moreover, *Y. lipolytica* is a suitable host for heterologous expression, since it displays high secretion ability and performs a wide-range of post-translational modifications (Madzak *et al.,* 2004; Nicaud *et al.,* 2002). However, regarding LCB biorefining, *Y. lipolytica* is unable to metabolize cellulose.

Recently, several reports have illustrated how cellulolytic capability can be conferred to *Y. lipolytica,* with single gene expression and co-culturing being used as a pragmatic way to design a *Y. lipolytica*-based CBP system (Boonvitthya *et al.,* 2013; Wei *et al.,* 2014). However, the use of co-culturing is not a feasible solution for industrial implementation, and a fully viable system can only be achieved if β-glucosidases (BGL) activity is present (Wei *et al.,* 2014). In this respect, the inventors have recently described the over-expression of endogenous BGLs in *Y. lipolytica* and the use of cello-oligosaccharides by the recombinant yeast strain to support growth (Guo *et al.,* 2015).

In pursuit of a more ambitious goal, the inventors have built on this platform strain, adding other cellulolytic enzyme-encoding genes and exploring different combinations in order to procure a *Y. lipolytica* strain that is able to grow on cellulose as the sole carbon source. The inventors have found that the co-expression of the previously described *Yarrowia lipolytica* β-glucosidases, a cellobiohydrolase (CBH) II, and endoglucanases (EG) I and II from *Trichoderma reesei* (syn. *Hypocrea jecorina*), and the *Neurospora crassa* CBH I procured an engineered *Y. lipolytica* strain that was able to grow both on model cellulose substrates, such as highly crystalline Avicel, and on industrial cellulose pulp, such as that obtained using an organosolv process. The inventors have also found that depending on the origin of the cellobiohydrolase and endoglucanase to express in *Y. lipolytica,* the secretion yield of this expressed enzyme may vary.

Accordingly, the present invention provides a method for obtaining an oleaginous yeast strain capable of growing on cellulose as carbon source, wherein said method comprises overexpressing in said strain:
- a β-glucosidase 1 (BGL 1; EC 3.2.1.21) having at least 80% identity, or by order of increasing preference at least 83%, 85%, 90%, 92%, 95%, 96%, 97%, 98%, 99% or 100% identity, with the polypeptide of sequence SEQ ID NO: 1 (mature YALI_BGL1) further comprising a N-terminal signal peptide,
- a β-glucosidase 2 (BGL 2; EC 3.2.1.21) having at least 80% identity, or by order of increasing preference at least 82%, 85%, 90%, 92%, 95%, 96%, 97%, 98%, 99% or 100% identity, with the polypeptide of sequence SEQ ID NO: 2 (mature YALI_BGL2) further comprising a N-terminal signal peptide,
- an endoglucanase I (EG I; EC 3.2.1.4) having at least 55% identity, or by order of increasing preference at least 59%, 60%, 65%, 70%, 75%, 82%, 85%, 90%, 92%, 95%, 96%, 97%, 98%, 99% or 100% identity, with the polypeptide of sequence SEQ ID NO: 3 (*Tr*EG I),
- an endoglucanase II (EG II; EC 3.2.1.4) having at least 55% identity, or by order of increasing preference at least 59%, 60%, 65%, 70%, 75%, 82%, 85%, 90%, 92%, 95%, 96%, 97%, 98%, 99% or 100% identity, with the polypeptide of sequence SEQ ID NO: 4 (*Tr*EG II),
- a cellobiohydrolase I (CBH I; EC 3.2.1.91) having at least 65% identity, or by order of increasing preference at least 70%, 75%, 82%, 85%, 90%, 92%, 95%, 96%, 97%, 98%, 99% or 100% identity, with the polypeptide of sequence SEQ ID NO: 5 (*Nc*CBH I), and
- a cellobiohydrolase II (CBH II; EC 3.2.1.91) having at least 60% identity, or by order of increasing preference at least 65%, 70%, 75%, 82%, 85%, 90%, 92%, 95%, 96%, 97%, 98%, 99% identity or 100%, with the polypeptide of sequence SEQ ID NO: 6 (*Tr*CBH II).

The term overexpressing an enzyme (or protein) in a yeast strain, herein refers to artificially increasing the quantity of said enzyme produced in a yeast strain compared to a reference (control) yeast strain wherein said enzyme is not overexpressed. This term also encompasses expression of an enzyme (or protein) in a yeast strain which does not naturally contain a gene encoding said enzyme.

An advantageous method for overexpressing these six enzymes (*i.e.,* BGL I, BGL II, EG I, EG II, CBH I, CBH II) comprises introducing into the genome of said yeast strain one or several DNA constructs comprising a nucleotide sequence encoding said enzymes, placed under the control of a promoter.

Unless otherwise specified, the percent of identity between two sequences which are mentioned herein is calculated from an alignment of the two sequences over their whole length, not comprising the signal sequence. One can use the BLAST program (Tatusova and Madden, 1999) with the default parameters (open gap penalty = 2; extension gap penalty = 5; matrix = BLOSUM 62).

The cellulose can be microcrystalline cellulose (*e.g*., Avicel®), phosphoric acid swollen cellulose (PASC) or cellulose pulp (*i.e..,* obtained using an organosolv process operated by CIMV S.A.).

The N-terminal signal peptide (or signal sequence) drives secretion of the β-glucosidase into the extracellular space (*e.g*., periplasm, external medium) of the yeast. After secretion, the N-terminal signal peptide is usually cleaved (removed) by a peptidase leading to a mature β-glucosidase. The N-terminal signal peptide can further allow glycosylation in the case of β-glucosidase bearing potential sites of glycosylation.

The N-terminal signal peptides are well known in the art (see for review von Heijne, 1985). In particular, N-terminal signal peptides able to drive secretion of a protein into the extracellular space in yeast and methods to perform such a secretion are well known in the art (see Sreekrishna *et al.,* 1997; Hashimoto *et al.,* 1998; Koganesawa *et al*.; 2001; Gasmi *et al.,* 2011; Madzak and Beckerich, 2013). Methods for identifying a N-terminal signal peptide (signal sequence) are also well known in the art. One can use the programs Signal-BLAST (Franck and Sippl, 2008) and/or SignalP 4.1 Server (Petersen *et al*., 2011).

The N-terminal signal peptide can be from a protein from any organism, such as mammal, bacteria, yeast, preferably from a protein form a yeast, more preferably from *Yarrowia.*

The N-terminal signal peptide of the β-glucosidase 1 having at least 80% identity with the polypeptide of sequence SEQ ID NO: 1 and the N-terminal signal peptide of the β-glucosidase 2 having at least 80% identity with the polypeptide of sequence SEQ ID NO: 2 can be identical or different, in terms of space of secretion (periplasm or external medium) and/or in terms of amino acid sequence. By way of example, the N-terminal signal peptide of the β-glucosidase 1 having at least 80% identity with the polypeptide of sequence SEQ ID NO: 1 drives secretion of said β-glucosidase 1 into the periplasm space or the external medium and the N-terminal signal peptide of the β-glucosidase 2 having at least 80% identity with the polypeptide of sequence SEQ ID NO: 2 drives secretion of said β-glucosidase 2 into the periplasm space or the external medium.

Advantageously, the N-terminal signal peptide of the β-glucosidase 1 having at least 80% identity with the polypeptide of sequence SEQ ID NO: 1 drives secretion of said β-glucosidase 1 into the periplasm space. It can be chosen from the N-terminal signal peptides SEQ ID NO: 26 or SEQ ID NO: 27.

Advantageously, the N-terminal signal peptide of the β-glucosidase 2 having at least 80% identity with the polypeptide of sequence SEQ ID NO: 2 drives secretion of said β-glucosidase 2 into the external medium. It can be chosen from the N-terminal signal peptides SEQ ID NO: 28, SEQ ID NO: 29, SEQ ID NO: 30 or SEQ ID NO: 31.

The N-terminal signal peptide can be the naturally occurring signal sequence of the β-glucosidase to overexpress or a modified signal sequence, such as the *Yarrowia* alkaline extracellular protease (Aep; Fabre *et al.,* 1991) or the extracellular lipase (Lip2p) signal sequences (Pignède *et al.,* 2000; Nicaud *et al.,* 2002).

Methods for determining the presence of a signal sequence (N-terminal signal peptide) in a protein are well known in the art. One can use the programs Signal-BLAST (Franck and Sippl, 2008) and/or SignalP 4.1 Server (Petersen *et al.,* 2011).

Nucleotide sequences encoding YALI_BGL1 and YALI_BGL2 are provided in SEQ ID NO: 8 and SEQ ID NO: 9 respectively.

Methods for determining whether an enzyme has a β-glucosidase activity (EC 3.2.1.21) are known in the art. By way of example, β-glucosidase activity can be measured by quantifying the release of *p*NP (*p*-nitrophenol) from *p*NPGlc as described in Guo *et al.,* 2011.

The β-glucosidase 1 having at least 80% identity with the polypeptide of sequence SEQ ID NO: 1 (mature YALI_BGL1) can be a heterologous or endogenous β-glucosidase of the oleaginous yeast strain.

Advantageously, the β-glucosidase 1 having at least 80% identity with the polypeptide of sequence SEQ ID NO: 1 (mature YALI_BGL1) is from a yeast strain, preferably an oleaginous yeast strain, such as *Candida, Cryptoccocus, Lipomyces*, *Rhodosporidium (e.g., Rhodosporidium toruloides), Rhodotorula* (*e.g., Rhodotorula glutinis), Trichosporon* or *Yarrowia,* more preferably a *Yarrowia* strain. According to this embodiment, the *Yarrowia* strain is preferably selected from *Y. lipolytica* and *Y. galli,* more preferably a *Y. lipolytica* strain.

In a preferred embodiment, the β-glucosidase 1 having at least 80% identity with the polypeptide of sequence SEQ ID NO: 1 (mature YALI_BGL1) is selected from the group consisting of the β-glucosidase 1 of SEQ ID NO: 1 (mature YALI_BGL1), and SEQ ID NO: 15 (YAGA_BGL1 without its naturally occurring N-terminal signal sequence), preferably SEQ ID NO: 1 (mature YALI_BGL1).

In another preferred embodiment, the β-glucosidase having at least 80% identity with the polypeptide of sequence SEQ ID NO: 1 (mature YALI_BGL1) further comprising a N-terminal signal peptide is selected from the group consisting of SEQ ID NO: 20 (YALI_BGL1; BGL1 from *Yarrowia lipolytica* CLIB122, comprising its naturally occurring N-terminal signal sequence) and SEQ ID NO: 21 (YAGA_BGL1; BGL1 from *Yarrowia galli* CBS 9722, comprising its naturally occurring N-terminal signal sequence), preferably SEQ ID NO: 20.

The β-glucosidases included in sequences SEQ ID NO: 20 (YALI_BGL1) and SEQ ID NO: 21 (YAGA_BGL1) have respectively 100% and 84.08% identity with the polypeptide of sequence SEQ ID NO: 1 (mature YALI_BGL1).

The β-glucosidase 2 having at least 80% identity with the polypeptide of sequence SEQ ID NO: 2 (mature YALI_BGL2) can be a heterologous or endogenous β-glucosidase of the oleaginous yeast strain.

Advantageously, the β-glucosidase 2 having at least 80% identity with the polypeptide of sequence SEQ ID NO: 2 (mature YALI_BGL2) is from a yeast strain, preferably an oleaginous yeast strain, such as *Candida, Cryptoccocus, Lipomyces, Rhodosporidium* (*e.g*., *Rhodosporidium toruloides), Rhodotorula (e.g., Rhodotorula glutinis*), *Trichosporon* or *Yarrowia,* more preferably a *Yarrowia* strain. According to this embodiment, the *Yarrowia* strain is preferably selected from a *Y. lipolytica, Y. galli, Y. yakushimensis* or *Yarrowia alimentaria* strain, more preferably a *Y. lipolytica* strain.

In a preferred embodiment, the β-glucosidase 2 having at least 80% identity with the polypeptide of sequence SEQ ID NO: 2 (mature YALI_BGL2) has the amino acid sequence SEQ ID NO: 16. This sequence SEQ ID NO: 16 corresponds to the consensus amino acid sequence obtained from mature YALI_BGL2, YAGA_BGL2, YAYA_BGL2 and YAAL_BGL2 (as described above).

In another preferred embodiment, the β-glucosidase 2 having at least 80% identity with the polypeptide of sequence SEQ ID NO: 2 (mature YALI_BGL2) is selected from the group consisting of the β-glucosidase of SEQ ID NO: 2 (mature YALI_BGL2), SEQ ID NO: 17 (YAGA_BGL2 without its naturally occurring N-terminal signal sequence), SEQ ID NO: 18 (YAYA_BGL2 without its naturally occurring N-terminal signal sequence) and SEQ ID NO: 19 (YAAL_BGL2 without its naturally occurring N-terminal signal sequence), preferably SEQ ID NO: 2 (mature YALI_BGL2).

In another preferred embodiment, the β-glucosidase 2 having at least 80% identity with the polypeptide of sequence SEQ ID NO: 2 (mature YALI_BGL2) further comprising a N-terminal signal peptide is selected from the group consisting of SEQ ID NO: 22 (YALI_BGL2; BGL2 from *Yarrowia lipolytica* CLIB122, comprising its naturally occurring N-terminal signal sequence), SEQ ID NO: 23 (YAGA_BGL2; BGL2 from *Yarrowia galli* CBS 9722, comprising its naturally occurring N-terminal signal sequence), SEQ ID NO: 24 (YAYA_BGL2; BGL2 from *Yarrowia yakushimensis* CBS 10253, comprising its naturally occurring N-terminal signal sequence) and SEQ ID NO: 25 (YAAL_BGL2; BGL2 from *Yarrowia alimentaria* CBS 10151, comprising its naturally occurring N-terminal signal sequence), preferably SEQ ID NO: 22.

The β-glucosidases 2 included in sequences SEQ ID NO: 22 (YALI_BGL2), SEQ ID NO: 23 (YAGA_BGL2), SEQ ID NO: 24 (YAYA_BGL2) and SEQ ID NO: 25 (YAAL_BGL2) have respectively 100%, 93.55%, 85.21% and 82.14% identity with the polypeptide of sequence SEQ ID NO: 2 (mature YALI_BGL2).

In another preferred embodiment, both β-glucosidases (the β-glucosidase 1 having at least 80% identity with the polypeptide of sequence SEQ ID NO: 1 and the β-glucosidase 2 having at least 80% identity with the polypeptide of sequence SEQ ID NO: 2) are from a yeast strain, preferably an oleaginous yeast strain, such as *Candida, Cryptoccocus, Lipomyces, Rhodosporidium (e.g., Rhodosporidium toruloides*), *Rhodotorula (e.g., Rhodotorula glutinis*), *Trichosporon* or *Yarrowia,* more preferably a *Yarrowia* strain. According to this embodiment, the *Yarrowia* strain is preferably selected from a *Y. lipolytica* or *Y. galli* strain, and more preferably a *Y. lipolytica* strain.

Methods for overexpressing BGL 1 and BGL 2 enzymes in an oleaginous yeast strain are described in Guo *et al.,* 2015 and International Application WO 2016/170155.

Endoglucanase I (EC 3.2.1.4), which is classified in glycoside hydrolase family 7 (GH7), has activity on cellulosic substrates. This enzyme randomly cleaves internal bonds at amorphous sites of cellulose that create new chain ends. Methods for determining whether an enzyme has an endoglucanase I activity are known in the art. By way of example, one can use the method described in Ghose *et al.* 1987.

Advantageously, the endoglucanase I is from a fungus or yeast strain, preferably from the genus *Trichoderma, Hypocrea, Thielavia, Dactylellina, Myceliophthora, Aspergillus*, *Neosartorya, Penicillium, Aspergillus* or *Rosellinia.*

In a preferred embodiment, the endoglucanase I has three conserved domains in catalytic core domain, defined by amino acid sequences SEQ ID NO: 32, SEQ ID NO: 33 and SEQ ID NO: 34; SEQ ID NO: 32 and SEQ ID NO: 33 being consensus amino acid sequences. These consensus amino acid sequences SEQ ID NO: 32 and SEQ ID NO: 33 correspond to the consensus amino acid sequences obtained by aligning the endoglucanase I from: *Trichoderma reesei* of SEQ ID NO: 3 (*Tr*EG I), *Hypocrea rufa* of SEQ ID NO: 35, *Trichoderma longibrachiatum* of SEQ ID NO: 36, *Trichoderma orientale* of SEQ ID NO: 37, *Trichoderma pseudokoningii* of SEQ ID NO: 38, *Hypocrea atroviridis* of SEQ ID NO: 39, *Trichoderma gamsii* of SEQ ID NO: 40, *Trichoderma harzianum* of SEQ ID NO: 41, *Thielavia terrestris* of SEQ ID NO: 42, *Dactylellina haptotyla* of SEQ ID NO: 43, *Myceliophthora thermophila* of SEQ ID NO: 44, *Aspergillus udagawae* of SEQ ID NO: 45, *Neosartorya fumigata* of SEQ ID NO: 46, *Neosartorya fischeri* of SEQ ID NO: 47, *Aspergillus fischeri* of SEQ ID NO: 48, *Penicillium brasilianum* of SEQ ID NO: 49, *Aspergillus fumigatus* Z5 of SEQ ID NO: 50, *Aspergillus lentulus* of SEQ ID NO: 51, and *Rosellinia necatrix* of SEQ ID NO: 52.

In another preferred embodiment, the endoglucanase I is selected from the group consisting of the EG I from: *Trichoderma reesei* of SEQ ID NO: 3 (*Tr*EG I), *Hypocrea rufa* of SEQ ID NO: 35 (which possesses 99.8% identity with the amino acid sequence of *Tr*EG I), *Trichoderma longibrachiatum* of SEQ ID NO: 36 (which possesses 94.6% identity with the amino acid sequence of *Tr*EG I), *Trichoderma orientale* of SEQ ID NO: 37 (which possesses 94.6% identity with the amino acid sequence of *Tr*EG I), *Trichoderma pseudokoningii* of SEQ ID NO: 38 (which possesses 94.4% identity with the amino acid sequence of *Tr*EG I), *Hypocrea atroviridis* of SEQ ID NO: 39 (which possesses 84.4% identity with the amino acid sequence of *Tr*EG I), *Trichoderma gamsii* of SEQ ID NO: 40 (which possesses 80.9% identity with the amino acid sequence of *Tr*EG I), *Trichoderma harzianum* of SEQ ID NO: 41 (which possesses 80.9% identity with the amino acid sequence of *Tr*EG I), *Thielavia terrestris* of SEQ ID NO: 42 (which possesses 69% identity with the amino acid sequence of *Tr*EG I), *Dactylellina haptotyla* of SEQ ID NO: 43 (which possesses 60.4% identity with the amino acid sequence of *Tr*EG I), *Myceliophthora thermophila* of SEQ ID NO: 44 (which possesses 62.9% identity with the amino acid sequence of *Tr*EG I), *Aspergillus udagawa* of SEQ ID NO: 45 (which possesses 62.2% identity with the amino acid sequence of *Tr*EG I), *Neosartorya fumigata* of SEQ ID NO: 46 (which possesses 63.3% identity with the amino acid sequence of *Tr*EG *I), Neosartorya fischeri* of SEQ ID NO: 47 (which possesses 61.9% identity with the amino acid sequence of *Tr*EG I), *Aspergillus fischeri* of SEQ ID NO: 48 (which possesses 62.2% identity with the amino acid sequence of *Tr*EG I), *Penicillium brasilianum* of SEQ ID NO: 49 (which possesses 59.8% identity with the amino acid sequence of *Tr*EG I), *Aspergillus fumigatus Z5* of SEQ ID NO: 50 (which possesses 63.0% identity with the amino acid sequence of *Tr*EG I), *Aspergillus lentulus* of SEQ ID NO: 51 (which possesses 61.2% identity with the amino acid sequence of *Tr*EG I) and *Rosellinia necatrix* of SEQ ID NO: 52 (which possesses 59% identity with the amino acid sequence of *Tr*EG I), preferably the endoglucanase I from *Trichoderma reesei* of SEQ ID NO: 3.

Endoglucanase II (EC 3.2.1.4), which belongs to glycoside hydrolase family 5 (GH5), has activity on both cellulose and hemicellulose. This enzyme randomly cleaves internal bonds at amorphous sites of cellulose as well as xylan that create new chain ends. Methods for determining whether an enzyme has an endoglucanase II activity are known in the art. By way of example, one can use the method described in Ghose *et al*., 1987.

Advantageously, the endoglucanase II is from a fungus or yeast strain, preferably from the genus *Trichoderma, Hypocrea, Thielavia, Phialophora, Batryotinia, Jaapia, Cylindrobasidium, Polyporus, Ceriporiopsis* or *Phlebiopsis.*

In a preferred embodiment, the endoglucanase II has two conserved domains in catalytic core domain, defined by the consensus amino acid sequences SEQ ID NO: 53 and SEQ ID NO: 54. These consensus amino acid sequences SEQ ID NO: 53 and SEQ ID NO: 54 correspond to the consensus amino acid sequences obtained by aligning the endoglucanase II from: *Trichoderma reesei* of SEQ ID NO: 4 (*Tr*EG II), *Hypocrea rufa* of SEQ ID NO: 55, *Hypocrea atroviridis* of SEQ ID NO: 56, *Trichoderma orientale* of SEQ ID NO: 57, *Hypocrea virens* of SEQ ID NO: 58, *Trichoderma harzianum* of SEQ ID NO: 59, *Trichoderma asperellum* of SEQ ID NO: *60, Trichoderma gamsii* of SEQ ID NO: 61, *Thielavia terrestris* of SEQ ID NO: 62, *Phialophora* sp. G5 of SEQ ID NO: 63, *Botryotinia fuckeliana* of SEQ ID NO: 64, *Jaapia argillacea* of SEQ ID NO: 65, *Cylindrobasidium torrendii* of SEQ ID NO: 66, *Polyporus arcularius* of SEQ ID NO: 67, *Ceriporiopsis subvermispora* of SEQ ID NO: 68, and *Phlebiopsis gigantea* of SEQ ID NO: 69.

In another preferred embodiment, the endoglucanase II is selected from the group consisting of the EG II from: *Trichoderma reesei* of SEQ ID NO: 4 (*Tr*EG II), *Hypocrea rufa* of SEQ ID NO: 55 (which possesses 99.3% identity with the amino acid sequence of *Tr*EG II), *Hypocrea atroviridis* of SEQ ID NO: 56 (which possesses 94.3% identity with the amino acid sequence of *Tr*EG II), *Trichoderma orientale* of SEQ ID NO: 57 (which possesses 94% identity with the amino acid sequence of *Tr*EG II), *Hypocrea virens* of SEQ ID NO: 58 (which possesses 85% identity with the amino acid sequence of *Tr*EG II), *Trichoderma harzianum* of SEQ ID NO: 59 (which possesses 84% identity with the amino acid sequence of *Tr*EG II), *Trichoderma asperellum* of SEQ ID NO: 60 (which possesses 78% identity with the amino acid sequence of *Tr*EG II), *Trichoderma gamsii* of SEQ ID NO: 61 (which possesses 76.4% identity with the amino acid sequence of *Tr*EG II), *Thielavia terrestris* of SEQ ID NO: 62 (which possesses 73.4% identity with the amino acid sequence of *Tr*EG II), *Phialophora* sp. G5 of SEQ ID NO: 63 (which possesses 72.6% identity with the amino acid sequence of *Tr*EG II), *Botryotinia fuckeliana* of SEQ ID NO: 64 (which possesses 67.6% identity with the amino acid sequence of *Tr*EG II), *Jaapia argillacea* of SEQ ID NO: 65 (which possesses 66% identity with the amino acid sequence of *Tr*EG II), *Cylindrobasidium torrendii* of SEQ ID NO: 66 (which possesses 61.1% identity with the amino acid sequence of *Tr*EG II), *Polyporus arcularius* of SEQ ID NO: 67 (which possesses 62.9% identity with the amino acid sequence of *Tr*EG II), *Ceriporiopsis subvermispora* of SEQ ID NO: 68 (which possesses 59.6% identity with the amino acid sequence of *Tr*EG II), and *Phlebiopsis gigantea* of SEQ ID NO: 69 (which possesses 61.9% identity with the amino acid sequence of *Tr*EG II), preferably the EG II from: *Trichoderma reesei* of SEQ ID NO: 4.

Nucleotide sequences (mature sequences) encoding *Tr*EG I and *Tr*EG II are provided in SEQ ID NO: 10 and SEQ ID NO: 11, respectively.

Nucleotide sequences encoding the native signal peptides of *Tr*EG I and *Tr*EG II are provided in SEQ ID NO: 207 and SEQ ID NO: 208, respectively.

Methods for overexpressing EG I or EG II enzymes in an oleaginous yeast strain are described in Boonvitthya *et al.,* 2013 and Park *et al.,* 2000.

Cellobiohydrolase I (EC 3.2.1.91) cleaves two to four units from the reducing end of the cellulose chain. Methods for determining whether an enzyme has an cellobiohydrolase I activity are known in the art. By way of example, one can use the method described in Zhang and Lynd, 2006.

Advantageously, the cellobiohydrolase I is from a fungus or yeast strain, preferably from the genus *Neurospora, Sordaria, Thielavia, Acremonium, Myceliophthora, Humicola, Chaetomium, Podospora, Scedosporium, Togninia, Gibberella, Gaeumannomyces* or *Fusarium.*

In a preferred embodiment, the cellobiohydrolase I has two conserved domains in catalytic core domain, defined by the consensus amino acid sequences SEQ ID NO: 70 and SEQ ID NO: 71. These consensus amino acid sequences SEQ ID NO: 70 and SEQ ID NO: 71 correspond to the consensus amino acid sequences obtained by aligning the cellobiohydrolase I from: *Neurospora crassa* of SEQ ID NO: 5 (*Nc*CBH *I), Neurospora tetrasperma* of SEQ ID NO: 72, *Sordaria macrospora* of SEQ ID NO: *73, Thielavia terrestris* of SEQ ID NO: 74, *Acremonium thermophilum* of SEQ ID NO: *75, Thielavia microspore* of SEQ ID NO: 76, *Myceliophthora thermophila* of SEQ ID NO: 77, *Humicola grisea* var. *thermoidea* of SEQ ID NO: 78, *Chaetomium luteum* of SEQ ID NO: 79, *Podospora anserina* of SEQ ID NO: 80, *Scedosporium apiospermum* of SEQ ID NO: 81, *Togninia minima* of SEQ ID NO: 82, *Gibberella moniliformis* of SEQ ID NO: 83, *Fusarium oxysporum sp. Radices-lycopersici* of SEQ ID NO: 84, *Gaeumannomyces graminis* of SEQ ID NO: 85, and *Fusarium oxysporum sp. Conglutinans race* 2 of SEQ ID NO: 86.

In another preferred embodiment, the cellobiohydrolase I is selected from the group consisting of the CBH I from: *Neurospora crassa* of SEQ ID NO: 5 (*Nc*CBH I), *Neurospora tetrasperma* of SEQ ID NO: 72 (which possesses 98.9% identity with the amino acid sequence of *Nc*CBH I), *Sordaria macrospora* of SEQ ID NO: 73 (which possesses 92.7% identity with the amino acid sequence of *Nc*CBH I), *Thielavia terrestris* of SEQ ID NO: 74 (which possesses 72.8% identity with the amino acid sequence of *Nc*CBH I), *Acremonium thermophilum* of SEQ ID NO: 75 (which possesses 71.1% identity with the amino acid sequence of *Nc*CBH I), *Thielavia microspore* of SEQ ID NO: 76 (which possesses 73.1% identity with the amino acid sequence of *Nc*CBH I), *Myceliophthora thermophila* of SEQ ID NO: 77 (which possesses 72.5% identity with the amino acid sequence of *Nc*CBH I), *Humicola grisea* var. *thermoi* of SEQ ID NO: 78 (which possesses 71.3% identity with the amino acid sequence of *Nc*CBH I), *Chaetomium luteum* of SEQ ID NO: 79 (which possesses 72.1% identity with the amino acid sequence of *Nc*CBH I), *Podospora anserina* of SEQ ID NO: 80 (which possesses 71.4% identity with the amino acid sequence of *Nc*CBH I), *Scedosporium apiospermum* of SEQ ID NO: 81 (which possesses 68.1% identity with the amino acid sequence of *Nc*CBH I), *Togninia minima* of SEQ ID NO: 82 (which possesses 69.4% identity with the amino acid sequence of *Nc*CBH I), *Gibberella moniliformis* of SEQ ID NO: 83 (which possesses 66.9% identity with the amino acid sequence of *Nc*CBH I), *Fusarium oxysporum* of SEQ ID NO: 84 (which possesses 66.4% identity with the amino acid sequence of *Nc*CBH I), *Gaeumannomyces graminis* of SEQ ID NO: 85 (which possesses 66.8% identity with the amino acid sequence of *Nc*CBH I), and *Fusarium oxysporum* of SEQ ID NO: 86 (which possesses 66.4% identity with the amino acid sequence of *Nc*CBH I), preferably the CBH I from *Neurospora crassa* of SEQ ID NO: 5.

Cellobiohydrolase II (EC 3.2.1.91) cleaves two to four units from the non-reducing ends of the exposed cellulose chains produced by endoglucanase. Methods for determining whether an enzyme has an cellobiohydrolase II activity are known in the art. By way of example, one can use the method described in Zhang and Lynd,2006.

Advantageously, the cellobiohydrolase II is from a fungus or yeast strain, preferably from the genus *Trichoderma, Hypocrea, Bionectria, Colletotrchum, Stachybotrys, Nectria, Neonectria, Pestalotiopsis, Rosellinia, Verticillium* or *Thielavia.*

In a preferred embodiment, the cellobiohydrolase II has two conserved domains in catalytic core domain, defined by the consensus amino acid sequence SEQ ID NO: 87 and SEQ ID NO: 88. These consensus amino acid sequences SEQ ID NO: 87 and SEQ ID NO: 88 correspond to the consensus amino acid sequences obtained by aligning the cellobiohydrolase II from: *Trichoderma reesei* of SEQ ID NO: 6 (*Tr*CBH II), *Hypocrea rufa* of SEQ ID NO: 89, *Trichoderma koningii* of SEQ ID NO: 90, *Trichoderma longibrachiatum* of SEQ ID NO: 91, *Trichoderma orientale* of SEQ ID NO: 92, *Trichoderma ghanense* of SEQ ID NO: 93, *Hypocrea virens* of SEQ ID NO: 94, *Trichoderma harzianum* of SEQ ID NO: 95, *Hypocrea atroviridis* of SEQ ID NO: 96, *Trichoderma gamsii* of SEQ ID NO: 97, *Bionectria ochroleuca* of SEQ ID NO: 98, *Colletotrichum incanum* of SEQ ID NO: 99, *Stachybotrys chartarum* of SEQ ID NO: 100, *Nectria haematococca* of SEQ ID NO: 101, *Neonectria ditissima* of SEQ ID NO: 102, *Pestalotiopsis fici* W106-1 of SEQ ID NO: 103, *Rosellinia necatrix* of SEQ ID NO: 104, *Verticillium dahlia* of SEQ ID NO: 105, and *Thielavia terrestris* of SEQ ID NO: 106.

In another preferred embodiment, the cellobiohydrolase II is selected from the group consisting of the CBH II from: *Trichoderma reesei* of SEQ ID NO: 6 (*Tr*CBH II), *Hypocrea rufa* of SEQ ID NO: 89 (which possesses 99.8% identity with the amino acid sequence of *Tr*CBH II), *Trichoderma koningii* of SEQ ID NO: 90 (which possesses 99.8% identity with the amino acid sequence of *Tr*CBH II), *Trichoderma longibrachiatum* of SEQ ID NO: 91 (which possesses 97.9% identity with the amino acid sequence of *Tr*CBH II), *Trichoderma orientale* of SEQ ID NO: 92 (which possesses 97.9% identity with the amino acid sequence of *Tr*CBH II), *Trichoderma ghanense* of SEQ ID NO: 93 (which possesses 97.5% identity with the amino acid sequence of *Tr*CBH II), *Hypocrea virens* of SEQ ID NO: 94 (which possesses 89.2% identity with the amino acid sequence of *Tr*CBH II), *Trichoderma harzianum* of SEQ ID NO: 95 (which possesses 89.6% identity with the amino acid sequence of *Tr*CBH II), *Hypocrea atroviridis* of SEQ ID NO: 96 (which possesses 81.0% identity with the amino acid sequence of *Tr*CBH II), *Trichoderma gamsii* of SEQ ID NO: 97 (which possesses 80.1% identity with the amino acid sequence of *Tr*CBH II), *Bionectria ochroleuca* of SEQ ID NO: 98 (which possesses 72.6% identity with the amino acid sequence of *Tr*CBH II), *Colletotrichum incanum* of SEQ ID NO: 99 (which possesses 69.2% identity with the amino acid sequence of *Tr*CBH II), *Stachybotrys chartarum* of SEQ ID NO: 100 (which possesses 67.3% identity with the amino acid sequence of *Tr*CBH II), *Nectria haematococca* of SEQ ID NO: 101 (which possesses 69.1% identity with the amino acid sequence of *Tr*CBH II), *Neonectria ditissima* of SEQ ID NO: 102 (which possesses 67.6% identity with the amino acid sequence of *Tr*CBH II), *Pestalotiopsis fici* W106-1 of SEQ ID NO: 103 (which possesses 68.5% identity with the amino acid sequence of *Tr*CBH II), *Rosellinia necatrix* of SEQ ID NO: 104 (which possesses 66.2% identity with the amino acid sequence of *Tr*CBH II), *Verticillium dahlia* of SEQ ID NO: 105 (which possesses 62.8% identity with the amino acid sequence of *Tr*CBH II), and *Thielavia terrestris* of SEQ ID NO: 106 (which possesses 64.8% identity with the amino acid sequence of *Tr*CBH II), preferably the CBH II from *Trichoderma reesei* of SEQ ID NO: 6.

Nucleotide sequences (mature sequences) encoding *Nc*CBH I and *Tr*CBH II are provided in SEQ ID NO: 12 and 13 respectively.

Nucleotide sequences encoding the native signal peptides of *Nc*CBH I and *Tr*CBH II are provided in SEQ ID NO: 209 and SEQ ID NO: 210, respectively.

Methods for overexpressing CBH I or CBH II enzymes in an oleaginous yeast strain are described in Boonvitthya *et al.,* 2013 and Wei *et al.,* 2014.

According to an advantageous embodiment of the invention, the method further comprises overexpressing in said strain, a lytic polysaccharide monooxygenase (LPMOA) having at least 51% identity or by order of increasing preference at least 52%, 53%, 54%, 55%, 56%, 60%, 65%, 70%, 75%, 80%, 85%, 90%, 92%, 95%, 96%, 97%, 98% or 99% identity, with the polypeptide of sequence SEQ ID NO: 7 (*Tr*LPMOA).

Lytic polysaccharide monooxygenase which is classified in the CAZy system as auxiliary activity family 9 (AA9, formerly GH61), is oxidative enzyme that boosts the overall efficiency of enzyme-mediated hydrolysis of cellulose. It achieves this in concert with canonical cellulases by performing oxidative cleavage of insoluble cellulose using molecular oxygen and an electron donor (Horn *et al,* 2012). Although most characterized LPMOAs oxidize polysaccharides at the C1 position of sugars (type 1 PMOs), yielding lactone (Hemsworth *et al,* 2013; Phillips *et al,* 2011; Vaaje-Kolstad *et al,* 2010), oxidation at C4 (type 2 PMOs) or both C1 and C4 (type 3 PMOs), and at C6 has also been observed. In these cases, the reaction products are ketoaldoses (Bey *et al,* 2013; Isaksen *et al,* 2014; Phillips *et al,* 2011).

Realizing the potential of LPMOAs, enzyme manufacturers have introduced these enzymes into commercial cellulases, forming a new generation of more efficient enzyme cocktails (Cannella *et al,* 2014).

Methods for determining whether an enzyme has a lytic polysaccharide monooxygenase activity are known in the art. By way of example, one can use the method described in Kitt *et al.,* 2012.

Advantageously, the LPMOA is from a fungus or a yeast strain, preferably from a *Trichoderma, Hypocrea, Pochonia, Myceliophthora, Penicillium*, *Rosellinia, Eutypa, Madurella, Thielavia.*

In a preferred embodiment, the LPMOA has a conserved domain in catalytic core domain, defined by the consensus amino acid sequence SEQ ID NO: 107. This consensus amino sequence SEQ ID NO: 107 corresponds to the consensus amino acid sequence obtained by aligning the LPMOA from: *Hypocrea jecorina* of SEQ ID NO: 7 (*Tr*LPMOA), *Trichoderma harzianum* of SEQ ID NO: 108, *Hypocrea atroviridis* of SEQ ID NO: 109, *Hypocrea virens* of SEQ ID NO: 110, *Trichoderma gamsii* of SEQ ID NO: 111, *Pochonia chlamydosporia* of SEQ ID NO: 112, *Myceliophtora thermophile* of SEQ ID NO: 113, *Penicilium oxalicum* of SEQ ID NO: 114, *Rosellinia nexatrix* of SEQ ID NO: 115, *Penicillium expansum* of SEQ ID NO: 116, *Penicillium patulum* of SEQ ID NO: 117, *Eutypa lata* of SEQ ID NO: 118, *Madurella mycetomatis* of SEQ ID NO: 119 and *Thielavia terrestris* of SEQ ID NO: 120.

In a preferred embodiment, the LPMOA is selected from the group consisting of the LPMOA from: *Hypocrea jecorina* of SEQ ID NO: 7 (TrLPMOA), *Trichoderma harzianum* of SEQ ID NO: 108 (which possesses 77.4% identity with the amino acid sequence of *Tr*LPMOA), *Hypocrea atroviridis* of SEQ ID NO: 109 (which possesses 78% identity with the amino acid sequence of *Tr*LPMOA), *Hypocrea virens* of SEQ ID NO: 110 (which possesses 76% identity with the amino acid sequence of *Tr*LPMOA), *Trichoderma gamsii* of SEQ ID NO: 111 (which possesses 74.9% identity with the amino acid sequence of *Tr*LPMOA), *Pochonia chlamydosporia* of SEQ ID NO: 112 (which possesses 60.2% identity with the amino acid sequence of *Tr*LPMOA), *Myceliophtora thermophila* of SEQ ID NO: 113 (which possesses 56.6% identity with the amino acid sequence of *Tr*LPMOA), *Penicilium oxalicum* of SEQ ID NO: 114 (which possesses 54.7% identity with the amino acid sequence of *Tr*LPMOA), *Rosellinia nexatrix* of SEQ ID NO: 115 (which possesses 51.2% identity with the amino acid sequence of *Tr*LPMOA), *Penicillium expansum* of SEQ ID NO: 116 (which possesses 54.4% identity with the amino acid sequence of *Tr*LPMOA), *Penicillium patulum* of SEQ ID NO: 117 (which possesses 55.1% identity with the amino acid sequence of *Tr*LPMOA), *Eutypa lata* of SEQ ID NO: 118 (which possesses 52.3% identity with the amino acid sequence of *Tr*LPMOA), *Madurella mycetomatis* of SEQ ID NO: 119 (which possesses 53.2% identity with the amino acid sequence of *Tr*LPMOA) and *Thielavia terrestris* of SEQ ID NO: 120 (which possesses 57.1 % identity with the amino acid sequence of *Tr*LPMOA), preferably the LPMOA from *Hypocrea jecorina* of SEQ ID NO: 7.

Nucleotide sequence (mature sequence) encoding *Tr*LPMOA is provided in SEQ ID NO: 14.

Nucleotide sequence encoding the native signal peptide of *Tr*LPMOA is provided in SEQ ID NO: 211.

Methods for overexpressing the LPMOA in a yeast strain are described in Kitt *et al*., 2012.

Oleaginous yeast strains, which naturally accumulate lipids to more than 20% of their dry cell weight, are well known in the art (Ratledge, 1994 and 2005). They include the genus *Candida, Cryptoccocus, Lipomyces, Rhodosporidium* (*e.g*., *Rhodosporidium toruloides*), *Rhodotorula* (*e.g*., *Rhodotorula glutinis*), *Trichosporon* and *Yarrowia.* According to the present invention, the term oleaginous yeast also includes an engineered oleaginous *Saccharomyces cerevisiae* transformant able to accumulate lipids. Such oleaginous *S. cerevisiae* strains are known in the art (for a review see Valle-Rodriguez et al., 2014).

In a preferred embodiment, the oleaginous yeast strain is a *Yarrowia* strain, preferably a *Yarrowia lipolytica* strain.

Advantageously, said yeast strain is auxotrophic for leucine (Leu-) and optionally for the decarboxylase orotidine-5'-phosphate (Ura-).

Said yeast can also be a mutant yeast strain wherein the expression or activity of the endogenous isoforms of acyl-coenzymeA oxidases (AOX, EC 6.2.1.3) involved, at least partially, in the β-oxidation of fatty acids, is inhibited. In yeasts, 6 genes (*POX1, POX2*, *POX3, POX4, POX5* and *POX6*) encode these isoforms. Said inhibition of the expression or activity can be total or partial. Total or partial inhibition of the expression or activity of these enzymes leads to accumulation by yeast of dodecanedioic acid without use of accumulated fat. More particularly, the coding sequence of the genes *POX1-6* and the peptide sequence of AOX1-6 from *Y. lipolytica* CLIB122 are available in either at Génolevures database (http://genolevures.org/) or at GRYC database (http://gryc.inra.fr/) or in GenBank database under the following accession numbers or names: *POX1* / AOX1 = YALI0E32835g / YALI0E32835p, *POX2* / AOX2 = YALI0F10857g / YALI0F10857p; *POX3* / AOX3 = YALI0D24750g / YALI0D24750p; *POX4* / AOX4 = YALI0E27654g / YALI0E27654p; *POX5* / AOX5 = YALI0C23859g / YALI0C23859p; *POX6* / AOX6 = YALI0E06567g / YALI0E06567p. The peptide sequences of the acyl-CoA oxidases of *Y. lipolytica* have 45% identity or 50% similarity with those from other yeasts. The degree of identity between the acyl-CoA oxidases varies from 55% to 70% (or from 65 to 76% similarity) (see International Application WO 2006/064131). A method of inhibiting the expression of the 6 endogenous AOX in a *Y. lipolytica* strain is described in Beopoulos *et al.,* 2008 and International Applications WO 2006/064131, WO 2010/004141 and WO 2012/001144.

The yeast strain can further comprise other mutations such as those described in International Applications WO 2006/064131, WO 2010/004141, WO 2012/001144, WO 2014/178014 and WO 2014/136028 which are useful for obtaining a fatty acids producing yeast strain.

In particular, the yeast strain can be genetically modified to improve lipid accumulation. Said yeast strain having improved properties for lipid accumulation can be a mutant yeast strain, preferably a *Y. lipolytica* mutant strain, wherein at least one protein, preferably at least one endogenous protein, selected from the group consisting of an acyl-CoA:diacylglycerol acyltransferase 2 (encoded by *DGAI*), an acyl-CoA:diacylglycerol acyltransferase 1 (encoded by *DGA2*), a glycerol-3-phosphate dehydrogenase NAD+ (encoded by *GPD1*), an acetyl-CoA carboxylase (encoded by *ACC1*) and a hexokinase (encoded by *HXK1*) is overexpressed, and/or the expression or activity of at least one endogenous protein selected from the group consisting of the glycerol 3-phosphate dehydrogenase (encoded by *GUT2*), the triglyceride lipase (encoded by *TGL4*) and the peroxin 10 (encoded by *PEX10*) is inhibited.

Advantageously, the 5 proteins, acyl-CoA:diacylglycerol acyltransferase 2, acyl-CoA:diacylglycerol acyltransferase 1, glycerol-3-phosphate dehydrogenase NAD+, acetyl-CoA carboxylase and hexokinase, are overexpressed, and the expression or activity of the 3 endogenous proteins, glycerol 3-phosphate dehydrogenase, triglyceride lipase and peroxyne, are inhibited in said mutant yeast strain.

In another advantageous embodiment, said yeast strain - preferably a *Yarrowia* strain, more preferably a *Y. lipolytica* strain - having improved properties for lipid accumulation is a mutant yeast strain in which the expression or activity of the endogenous isoforms of acyl-coenzymeA oxidases (AOX, EC 6.2.1.3) involved, at least partially, in the β-oxidation of fatty acids (*e.g., POX1* to *POX6* in *Y. lipolytica*) and the triglyceride lipase (encoded by *TGL4*) are inhibited, and an acyl-CoA:diacylglycerol acyltransferase (encoded by *DGA2*) and a glycerol-3-phosphate dehydrogenase (encoded by *GPD1*) - preferably the endogenous *DGA2* and *GPD1* - are overexpressed.

A method of overexpressing the endogenous genes *DGA1, DGA2, GPD1* and *ACC1* and inhibiting the expression or activity of the endogenous genes *GUT2, TGL4* and *PEX10* in a *Y. lipolytica* strain is described in International Application WO 2014/136028.

A method of overexpressing the endogenous genes *DGA2, GPD1* and *HXK1,* and inhibiting the expression or activity of the endogenous gene *TGL4* in a *Y. lipolytica* strain is described in Lazar *et al.,* 2014.

In a preferred embodiment of the method of the invention, it provides a method for obtaining a *Yarrowia* strain, preferably a *Y. lipolytica* strain, capable of growing on cellulose as carbon source, wherein said method comprises overexpressing in said strain:
- a β-glucosidase 1 selected from the group consisting of SEQ ID NO: 1 and SEQ ID NO: 15, further comprising a N-terminal signal peptide selected from the group consisting of SEQ ID NO: 26 to 31,
- a β-glucosidase 2 selected from the group consisting of SEQ ID NO: 2, SEQ ID NO: 17, SEQ ID NO: 18 and SEQ ID NO: 19, further comprising a N-terminal signal peptide selected from the group consisting of SEQ ID NO: 26 to 31,
- the endoglucanase I of SEQ ID NO: 3 (*Tr*EG I), further comprising a N-terminal signal peptide of lipase 2 of *Y. lipolytica* of sequence SEQ ID NO: 121 (pre-pro sequence of Lipase 2, UniProt accession number: Q9P8F7),
- the endoglucanase II of SEQ ID NO: 4 (*Tr*EG II), further comprising a N-terminal signal peptide of lipase 2 of *Y. lipolytica* of sequence SEQ ID NO: 121 (pre-pro sequence of Lipase 2, UniProt accession number: Q9P8F7),
- the cellobiohydrolase I of SEQ ID NO: 5 (*Nc*CBH I), further comprising a N-terminal signal peptide of lipase 2 of *Y. lipolytica* of sequence SEQ ID NO: 121 (pre-pro sequence of Lipase 2, UniProt accession number: Q9P8F7) and
- the cellobiohydrolase II of SEQ ID NO: 6 (*Tr*CBH II) further comprising a N-terminal signal peptide of lipase 2 of *Y. lipolytica* of sequence SEQ ID NO: 121 (pre-pro sequence of Lipase 2, UniProt accession number: Q9P8F7), and optionally
- the lytic polysaccharide monooxygenase of SEQ ID NO: 7 (*Tr*LPMOA) further comprising a N-terminal signal peptide of lipase 2 of *Y. lipolytica* of sequence SEQ ID NO: 121 (pre-pro sequence of Lipase 2, UniProt accession number: Q9P8F7).

For the genes corresponding to the endoglucanase I of SEQ ID NO: 3 (*Tr*EG I), the endoglucanase II of SEQ ID NO: 4 (*Tr*EG II), the cellobiohydrolase I of SEQ ID NO: 5 (*Nc*CBH I), that were expressed in *Y. lipolytica* strain, the signal peptide of lipase 2 of *Y. lipolytica* of sequence SEQ ID NO: 121 (pre-pro sequence of Lipase 2, UniProt accession number: Q9P8F7) is used to replace the native signal peptides of these genes of sequences, SEQ ID NO: 207 to SEQ ID NO: 211, respectively.

Overexpression of an endogenous, orthologous or heterologous enzyme as defined in the present invention (*i.e.,* BGL 1, BGL 2, EG I, EG I, CBH I, CBH II, LPMOA) in a yeast strain according to the present invention can be obtained in various ways by methods known *per se.*

Overexpression of an enzyme as defined in the present invention may be performed by placing one or more (preferably two or three) copies of the open reading frame (ORF) of the sequence encoding said enzyme under the control of appropriate regulatory sequences. Said regulatory sequences include promoter sequences, located upstream (at 5' position) of the ORF of the sequence encoding said enzyme, and terminator sequences, located downstream (at 3' position) of the ORF of the sequence encoding said β-glucosidase.

Promoter sequences that can be used in yeast are well known to those skilled in the art and may correspond in particular to inducible or constitutive promoters. Examples of promoters which can be used according to the present invention, include the promoter of a *Y. lipolytica* gene which is strongly repressed by glucose and is inducible by the fatty acids or triglycerides such as the promoter of the *POX2* gene encoding the acyl-CoA oxidase 2 (AOX2) of *Y. lipolytica* and the promoter of the *LIP2* gene described in International Application WO 01/83773. One can also use the promoter of the *FBA1* gene encoding the fructose-bisphosphate aldolase (see Application US 2005/0130280), the promoter of the *GPM* gene encoding the phosphoglycerate mutase (see International Application WO 2006/0019297), the promoter of the *YAT1* gene encoding the transporter ammonium (see Application US 2006/0094102), the promoter of the *GPAT* gene encoding the O-acyltransferase glycerol-3-phosphate (see Application US 2006/0057690), the promoter of the *TEF* gene (Muller *et al*., 1998; Application US 2001/6265185), the hybrid promotor HTEF which is comprised or 4 tandem copies of upstream activation sequences (UAS) and the core promoter element of *TEF* (Blazek *et al*., 2011), the hybrid promoter hp4d (described in International Application WO 96/41889), the hybrid promoter XPR2 described in Mazdak *et al.* (2000), the hybrid promoters UAS1-TEF or UAStef-TEF described in Blazeck *et al.* (2011, 2013, 2014), or the hybrid promotor HTEF of sequence SEQ ID NO: 122 which is comprised of 4 tandem copies of upstream activation sequences (UAS) and the core promoter element of *TEF* (Blazek *et al*., 2011).

Advantageously, the promoter is the promoter of the *TEF* gene or the hybrid promotor HTEF which is comprised of 4 tandem copies of upstream activation sequences (UAS) and the core promoter element of *TEF* of SEQ ID NO: 123 (Blazeck *et al.* 2011).

Terminator sequences that can be used in yeast are also well known to those skilled in the art. Example of terminator sequences which can be used according to the present invention include the terminator sequence of the *PGK1* gene and the terminator sequence of the *LIP2* gene described in International Application WO 01/83773.

The nucleotide sequence of the coding sequences of the heterologous genes can be optimized for expression in yeast by methods well known in the art (see for review Hedfalk, 2012).

Overexpression of an endogenous β-glucosidase can be obtained by replacing the sequences controlling the expression of said endogenous β-glucosidase by regulatory sequences allowing a stronger expression, such as those described above. The skilled person can replace the copy of the gene encoding an endogenous β-glucosidase in the genome, as well as its own regulatory sequences, by genetically transforming the yeast strain with a linear polynucleotide comprising the ORF of the sequence coding for said endogenous β-glucosidase under the control of regulatory sequences such as those described above. Advantageously, said polynucleotide is flanked by sequences which are homologous to sequences located on each side of said chromosomal gene encoding said endogenous β-glucosidase. Selection markers can be inserted between the sequences ensuring recombination to allow, after transformation, to isolate the cells in which integration of the fragment occurred by identifying the corresponding markers. Advantageously also, the promoter and terminator sequences belong to a gene different from the gene encoding the endogenous β-glucosidase to be overexpressed in order to minimize the risk of unwanted recombination into the genome of the yeast strain.

Overexpression of an endogenous β-glucosidase can also be obtained by introducing into the yeast strain of extra copies of the gene encoding said endogenous β-glucosidase under the control of regulatory sequences such as those described above. Said additional copies encoding said endogenous β-glucosidase may be carried by an episomal vector, that is to say capable of replicating in yeast. Preferably, these additional copies are carried by an integrative vector, that is to say, integrating into a given location in the yeast genome (Madzak *et al*., 2004). In this case, the polynucleotide comprising the gene encoding said endogenous β-glucosidase under the control of regulatory regions is integrated by targeted integration. Said additional copies can also be carried by PCR fragments whose ends are homologous to a given locus of the yeast, allowing integrating said copies into the yeast genome by homologous recombination. Said additional copies can also be carried by auto-cloning vectors or PCR fragments, wherein the ends have a zeta region absent from the genome of the yeast, allowing the integration of said copies into the yeast genome by random insertion as described in Application US 2012/0034652.

Targeted integration of a gene into the genome of a yeast cell is a molecular biology technique well known to those skilled in the art: a DNA fragment is cloned into an integrating vector, introduced into the cell to be transformed, wherein said DNA fragment integrates by homologous recombination in a targeted region of the recipient genome (Orr-Weaver *et al*., 1981).

Methods for transforming yeast are also well known to those skilled in the art and are described, *inter alia*, by Ito *et al.* (1983), Klebe *et al*., (1983) and Gysler *et al*., (1990).

Any gene transfer method known in the art can be used to introduce a gene encoding enzyme as defined in the present invention. Preferably, one can use the method with lithium acetate and polyethylene glycol described by Gaillardin *et al*., (1987) and Le Dall *et al*., (1994).

The present invention also provides means for carrying out said overexpression.

This includes, in particular, recombinant DNA constructs for expressing one or several enzyme(s) as defined in the present invention in a yeast cell (*e.g*., *Y. lipolytica* strain). These DNA constructs can be obtained and introduced in said yeast strain by the well-known techniques of recombinant DNA and genetic engineering.

Recombinant DNA constructs of the invention include in particular expression cassettes, comprising a polynucleotide encoding one or several enzyme(s) as defined in the present invention, under the control of a promoter functional in yeast cell as defined above.

The expression cassettes generally also include a transcriptional terminator, such as those describes above. They may also include other regulatory sequences, such as transcription enhancer sequences.

Recombinant DNA constructs of the invention also include recombinant vectors containing an expression cassette comprising a polynucleotide encoding one or several enzyme(s) as defined in the present invention, under transcriptional control of a suitable promoter.

Recombinant vectors of the invention may also include other sequences of interest, such as, for instance, one or more marker genes, which allow for selection of transformed yeast cells.

The invention also comprises host cells containing a recombinant DNA construct of the invention. These host cells can be prokaryotic cells (such as bacteria cells) or eukaryotic cells, preferably yeast cells.

The invention also provides a method for obtaining a mutant oleaginous yeast strain, preferably a mutant *Yarrowia* strain (*e.g*., *Y. lipolytica* strain), capable of growing on cellulose as carbon source as defined above, comprising transforming an oleaginous yeast cell with one or several recombinant DNA constructs for expressing a β-glucosidase 1, a β-glucosidase 2, an endoglucanase I, an endoglucanase II, a cellobiohydrolase I, a cellobiohydrolase II and optionally LPMOA as defined in the present invention.

The term "one or several recombinant DNA constructs for expressing a β-glucosidase 1, a β-glucosidase 2, an endoglucanase I, an endoglucanase II, a cellobiohydrolase I, a cellobiohydrolase II and optionally LPMOA as defined in the present invention" refers to:
- a recombinant DNA construct for expressing the 6 or 7 enzymes as defined in the present invention,
- a recombinant DNA construct for expressing 1 of the 6 or 7 enzymes as defined in the present invention and a recombinant DNA construct for expressing the 5 or 6 other enzymes as defined in the present invention,
- a recombinant DNA construct for expressing 2 of the 6 or 7 enzymes as defined in the present invention and a recombinant DNA construct for expressing the 4 or 5 other enzymes as defined in the present invention,

- a recombinant DNA construct for expressing 3 of the 6 or 7 enzymes as defined in the present invention and a recombinant DNA construct for expressing the 3 or 4 other enzymes as defined in the present invention,
- 2 recombinant DNA constructs for expressing respectively 2 of the 6 or 7 enzymes as defined in the present invention and a recombinant DNA construct for expressing the 4 or 5 other enzymes as defined in the present invention,
- 3 recombinant DNA constructs for expressing respectively 3 or 4 of the 6 or 7 enzymes as defined in the present invention and a recombinant DNA construct for expressing the 3 other enzymes as defined in the present invention,
- 4 recombinant DNA constructs for expressing respectively 4 of the 6 or 7 enzymes as defined in the present invention and a recombinant DNA construct for expressing the 2 or 3 other enzymes as defined in the present invention,
- 6 or 7 recombinant DNA constructs for expressing respectively the 6 or 7 enzymes as defined in the present invention,
- 2 recombinant DNA constructs for expressing respectively 2 of the 6 or 7 enzymes as defined in the present invention, a recombinant DNA construct for expressing 2 or 3 other enzymes as defined in the present invention and a recombinant DNA construct for expressing the 2 or 3 remaining enzymes as defined in the present invention,
- a recombinant DNA constructs for expressing 2 of the 6 or 7 enzymes as defined in the present invention, a recombinant DNA construct for expressing 2 or 3 other enzymes as defined in the present invention and a recombinant DNA construct for expressing the 2 or 3 remaining enzymes as defined in the present invention,
- a recombinant DNA construct for expressing 1 of the 6 or 7 enzymes as defined in the present invention, a recombinant DNA construct for expressing 2 other enzymes as defined in the present invention and a recombinant DNA construct for expressing the 3 or 4 remaining enzymes as defined in the present invention.

The invention also comprises an oleaginous yeast strain, preferably a *Yarrowia* strain (*e.g*., *Y. lipolytica* strain), genetically transformed with one or several recombinant DNA constructs for expressing a β-glucosidase 1, a β-glucosidase 2, an endoglucanase I, an endoglucanase II, a cellobiohydrolase I, a cellobiohydrolase II and optionally LPMOA as defined in the present invention, and overexpressing said β-glucosidase 1, β-glucosidase 2, endoglucanase I, endoglucanase II, cellobiohydrolase I, cellobiohydrolase II and optionally LPMOA. In said mutant (transgenic) yeast strain one or several recombinant DNA construct(s) of the invention is/are comprised in a transgene stably integrated in the yeast genome, so that it is passed onto successive yeast generations. Thus the mutant (transgenic) yeast strain of the invention includes not only the yeast cell resulting from the initial transgenesis, but also their descendants, as far as they contain one or several recombinant DNA construct(s) of the invention. The overexpression of said 6 or 7 enzymes as defined above in said yeast strains provides them an ability to grow on cellulose as carbon source, when compared with an oleaginous yeast strain devoid of said transgene(s).

The present invention also comprises a mutant oleaginous yeast strain as defined above, preferably a mutant *Yarrowia* strain (*e.g*., *Y. lipolytica* strain), wherein:
- a β-glucosidase 1 (BGL 1; EC 3.2.1.21) having at least 80% identity, or by order of increasing preference at least 83%, 85%, 90%, 92%, 95%, 96%, 97%, 98%, 99% or 100% identity, with the polypeptide of sequence SEQ ID NO: 1 (mature YALI_BGL1) further comprising a N-terminal signal peptide,
- a β-glucosidase 2 (BGL 2; EC 3.2.1.21) having at least 80% identity, or by order of increasing preference at least 82%, 85%, 90%, 92%, 95%, 96%, 97%, 98%, 99% or 100% identity, with the polypeptide of sequence SEQ ID NO: 2 (mature YALI_BGL2) further comprising a N-terminal signal peptide,
- an endoglucanase I (EG I; EC 3.2.1.4) having at least 55% identity, or by order of increasing preference at least 59%, 60%, 65%, 70%, 75%, 82%, 85%, 90%, 92%, 95%, 96%, 97%, 98%, 99% or 100% identity, with the polypeptide of sequence SEQ ID NO: 3 (*Tr*EG I),
- an endoglucanase II (EG II; EC 3.2.1.4) having at least 55% identity, or by order of increasing preference at least 59%, 60%, 65%, 70%, 75%, 82%, 85%, 90%, 92%, 95%, 96%, 97%, 98%, 99% or 100% identity, with the polypeptide of sequence SEQ ID NO: 4 (*Tr*EG II),
- a cellobiohydrolase I (CBH I; EC 3.2.1.91) having at least 65% identity, or by order of increasing preference at least 70%, 75%, 82%, 85%, 90%, 92%, 95%, 96%, 97%, 98%, 99% or 100% identity, with the polypeptide of sequence SEQ ID NO: 5 (*Nc*CBH I),
- a cellobiohydrolase II (CBH II; EC 3.2.1.91) having at least 60% identity, or by order of increasing preference at least 65%, 70%, 75%, 82%, 85%, 90%, 92%, 95%, 96%, 97%, 98%, 99% identity or 100%, with the polypeptide of sequence SEQ ID NO: 6 (*Tr*CBH II), and optionally, a lytic polysaccharide monooxygenase (LPMOA) having at least 51% identity or by order of increasing preference at least 52%, 53%, 54%, 55%, 56%, 60%, 65%, 70%, 75%, 80%, 85%, 90%, 92%, 95%, 96%, 97%, 98% or 99% identity, with the polypeptide of sequence SEQ ID NO: 7 (*Tr*LPMOA),
as defined above are overexpressed.

This mutant oleaginous yeast strain is obtainable by a method of the invention and contains one or two recombinant expression cassette(s) of the invention.

The present invention further comprises a mutant oleaginous yeast strain as defined above, preferably a mutant *Yarrowia* strain (*e.g*., *Y. lipolytica* strain) comprising, stably integrated in its genome, one or several recombinant DNA constructs for expressing a β-glucosidase 1, a β-glucosidase 2, an endoglucanase I, an endoglucanase II, a cellobiohydrolase I, a cellobiohydrolase II and optionally a LPMOA as defined in the present invention.

The present invention also provides the use of a mutant oleaginous yeast strain, preferably a mutant *Yarrowia* strain (*e.g*., *Y. lipolytica* strain), as defined above for producing lipids from lignocellulosic biomass, in particular from cellulose.

As used herein, the term producing lipids refers to the accumulation and optionally secretion of lipids.

The present invention also provides a method of producing lipids, comprising a step of growing a mutant oleaginous yeast strain, preferably a mutant *Yarrowia* strain (*e.g., Y. lipolytica* strain), of the invention on a lignocellulosic biomass, in particular on cellulose.

Methods for extracting and purifying lipids produced by cultured yeast strains are well known to those skilled in the art (Papanikolaou et al. 2001, 2002 and 2008; André et al., 2009). For example, the total lipids can be extracted according to the method described by Papanikolaou et al., 2001, and fractionated according to the methods described by Guo *et al*., 2000 and Fakas *et al*., 2006.

The present invention also provides a method for enhancing the extracellular expression of a protein in a yeast strain, preferably an oleaginous yeast strain as defined above, comprising the step of placing in said yeast strain the encoding sequence of said protein under the control of the hybrid promotor HTEF, which is comprised of 4 tandem copies of upstream activation sequences (UAS) and the core promoter element of *TEF* gene, of SEQ ID NO: 123 (described in Blazeck *et al.* 2011). Said protein is preferably chosen from the group consisting of an endoglucanase I, an endoglucanase II, a cellobiohydrolase I, a cellobiohydrolase II and optionally a LPMOA as defined in the present invention.

The present invention also provides the use of the hybrid promotor HTEF, which is comprised of 4 tandem copies of upstream activation sequences (UAS) and the core promoter element of *TEF* gene, of SEQ ID NO: 123 (described in Blazeck *et al.* 2011), for enhancing the extracellular expression of a protein in a yeast strain, wherein the encoding sequence of said protein is placed under the control of said hybrid promotor HTEF. Said protein is preferably chosen from the group consisting of an endoglucanase I, an endoglucanase II, a cellobiohydrolase I, a cellobiohydrolase II and optionally a LPMOA as defined in the present invention.

Among non-enzymatic auxiliaries, there are expansins and expansin-like swollenins which are known to mediate disruption of the ordered structure of crystalline cellulose, generating amorphous regions that are amenable to cellulose action (Gilbert *et al*., 2010; Jäger *et al*, 2011). Swollenins are fungal proteins that, like plant expansins which disrupt the crystalline surface of cellulose, making it more amenable enzyme attack (Eibinger *et al*, 2016). Swollenin 1 (SWO1) of sequence SEQ ID NO: 124 is the major expansin-like protein produced by the fungus *T. reesei (*Adav *et al,* 2012).

When the accessory protein swollenin either is expressed in combination with essential cellulases as described above or supplemented as an extra protein, it achieves an efficient degradation of recalcitrant crystalline cellulose.

According to a particular embodiment of the invention, the use of a mutant oleaginous yeast strain as defined above is conducted in presence of swollenin, for producing lipids from lignocellulosic biomass, in particular cellulose.

This swollenin can be selected from the group consisting of the swollenin from: *Hypocrea jecorina* of SEQ ID NO: 124 (*Tr*SWO1), *Trichoderma pseudokoningii* of SEQ ID NO: 126 (which possesses 92.9% identity with the amino acid sequence of *Tr*SWO1), *Hypocrea virens* of SEQ ID NO: 127 (which possesses 90.9% identity with the amino acid sequence of *Tr*SWO1), *Hypocrea atroviridis* of SEQ ID NO: 128 (which possesses 85.1% identity with the amino acid sequence of *Tr*SWO1), *Trichoderma asperellum* of SEQ ID NO: 129 (which possesses 84.1 % identity with the amino acid sequence of *Tr*SWO1), *Penicillium oxalicum* strain 114-2 / CGMCC 5302) of SEQ ID NO: 130 (which possesses 65.9% identity with the amino acid sequence of *Tr*SWO1), *Neosartorya fumigata* strain CEA10/ CBS 144.89/FGSC A1163 of SEQ ID NO: 131 (which possesses 66.2% identity with the amino acid sequence of *Tr*SWO1), *Neosartorya fumigata* strain AF210 of SEQ ID NO: 132 (which possesses 66.4% identity with the amino acid sequence of *Tr*SWO1), *Penicillium decumbens* of SEQ ID NO: 133 (which possesses 65.9% identity with the amino acid sequence of *Tr*SWO1), *Neosartorya fumigata* strain ATCC MYA-4609 / Af293 / CBS 101355 / FGSC A1100 of SEQ ID NO: 134 (which possesses 63.5% identity with the amino acid sequence of *Tr*SWO1), *Talaromyces stipitatus* strain ATCC 10500 / CBS 375.48 / QM 6759 / NRRL 1006 of SEQ ID NO: 135 (which possesses 65.4% identity with the amino acid sequence of *Tr*SWO1), *Talaromyces marneffei* strain PM1 of SEQ ID NO: 136 (which possesses 64.3% identity with the amino acid sequence of *Tr*SWO1), *Aspergillus fumigatus* of SEQ ID NO: 137 (which possesses 72.2% identity with the amino acid sequence of *Tr*SWO1), *Aspergillus udawae* of SEQ ID NO: 138 (which possesses 77.7% identity with the amino acid sequence of *Tr*SWO1), *Talaromyces marneffei* strain ATCC 18224 / CBS 334.59 / QM 7333 of SEQ ID NO: 139 (which possesses 73.1% identity with the amino acid sequence of *Tr*SWO1) and *Talaromyces cellulolyticus* of SEQ ID NO: 140 (which possesses 51.5% identity with the amino acid sequence of *Tr*SWO1), preferably the swollenin from *Hypocrea jecorina* of SEQ ID NO: 159 (*Tr*SWO1).

The nucleotide sequence (mature sequence) of *Tr*SWO1 is provided in SEQ ID NO: 125.

Nucleotide sequence encoding the native signal peptide of *Tr*SWO1 is provided in SEQ ID NO: 212.

For the gene corresponding to the Swollenin 1 of SEQ ID NO: 125 (*Tr*SWO1), that is expressed in *Y. lipolytica* strain, the signal peptide of lipase 2 of *Y. lipolytica* of sequence SEQ ID NO: 121 (pre-pro sequence of Lipase 2, UniProt accession number: Q9P8F7) is used to replace the native signal peptide of this gene of sequences SEQ ID NO: 212.

Methods for overexpressing the swollenin in a yeast strain are described in Jäger et al., 2011.

According to another particular embodiment of the invention, the method of producing lipids comprises a step of growing a mutant oleaginous yeast strain as defined above and a step of pre-treatment in presence of swollenin, on a lignocellulosic biomass, in particular on cellulose.

Preferably, the swollenin is previously incubated in the culture medium containing a lignocellulosic biomass, in particular on cellulose, during between 5 to 50 hours, preferably during 24 hours.

According to another particular embodiment of the invention, the method for enhancing the extracellular expression of a protein in a yeast strain as defined above in presence of swollenin comprises the step of placing in said yeast the encoding sequence of said protein under control of the hybrid promoter HTEF of SEQ ID NO: 122.

The present invention also provides the use of the hybrid promoter HTEF of SEQ ID NO: 122 for enhancing the extracellular expression of a protein in a yeast strain as defined above in presence of swollenin, wherein the encoding sequence of said protein is placed under the control of said hybrid promoter HTEF.

The present invention will be understood more clearly from the further description which follows, which refers to non-limitative examples illustrating the overexpression of a β-glucosidase 1, a β-glucosidase 2, an endoglucanase I, an endoglucanase II, a cellobiohydrolase I, a cellobiohydrolase II and optionally a LPMOA in *Y. lipolytica.*
**Figure 1****:** Production of rb*Tr*EGI and rh*Tr*EGII in *Y. lipolytica* (a) enzyme production on YTD versus time, (b) SDS-PAGE analysis of the culture supernatant of *Y. lipolytica* transformants compared with the control and (c) western blot analysis, lane 1 and 3, culture supernatant of transformant ylEGI and ylEGII, respectively; lane 2 and 4, culture supernatant of transformant ylEGI and ylEGII treated by endo-H, respectively.
**Figure 2****:** SDS-PAGE analysis of the purified rh*Tr*EGI and rh*Tr*EGII produced in *Y. lipolytica* JMY1212 transformants; lane 1 and 3, purified rh*Tr*EGI and rh*Tr*EGII; lane 2 and 4, endo-H treated rh*Tr*EGI and rh*Tr*EGII.
**Figure 3****:** Western blot analysis of the heterologous CBH proteins produced by *Y. lipolytica* (a) lane 1, 3 and 5, rh*Nc*CBHI, rh*Pf*CBHI and rh*Tr*CBHI, respectively; lane 2, 4 and 6, corresponding rhCBHIs treated by endo-H, (b) lane 1 and 2, rh*Tr*CBHII and endo-H treated rh*Tr*CBHII, respectively.
**Figure 4****:** SDS-PAGE analysis of the purified rh*Nc*CBHI (lane 1) and rh*Tr*CBHII (lane 2) produced by *Y. lipolytica* JMY1212.
**Figure 5****:** Production of rh*Nc*CBHI and rh*Tr*CBHII under the control of *TEF* or *4UASTEF* promoter in *Y. lipolytica* (a and c) enzyme production on YTD versus time, (b and d) SDS-PAGE analysis of the culture supernatant of *Y. lipolytica* transformants.
**Figure 6****:** PCR verification of *Y. lipolytica* transformants expressing multiple cellulases (A) YLC1, Lane 1, 2, 3, 4, 5: *BGL1, BGL2, TrEGI, NcCBHI, TrCBHII*; (B) YLC2, Lane 1, 2, 3, 4, 5: *BGL1*, *BGL2, TrEGII, NcCBHI, TrCBHII*; (C) YLC3, Lane 1, 2, 3, 4, 5, 6: *BGL1, BGL2, TrEGI, TrEGII, NcCBHI, TrCBHII*; (D) YLC4, Lane 1, 2, 3, 4, 5, 6: *BGL1, BGL2, TrEG1, TrEGII, NcCBHI, 4UASTrCBHII*; (E) YLC5, Lane 1, 2, 3, 4, 5, 6: *BGL1, BGL2, TrEGI*, *TrEGII,4UASNcCBHI, TrCBHII*; (F) YLC6, Lane 1, 2, 3, 4, 5, 6: *BGL1, BGL2, TrEGI, TrEGII, 4UASNcCBHI, 4UASTrCBHII.*
**Figure 7****:** Comparison of the hydrolytic activity of the total secreted cellulases produced by different cellulolytic *Y. lipolytica* strains on various cellulosic substrates.
**Figure 8****:** Expression of *Tr*LPMOA in *Y. lipolytica.* (a) Western blot analysis of the heterologous LPMOA protein produced by *Y. lipolytica:* lane 1, rh*Tr*LPMOA; lane 2, rh*Tr*LPMOA treated by endo-H; (b) enzyme production on YTD versus time; (c) SDS-PAGE analysis of the culture supernatant of *Y. lipolytica* transformants.
**Figure 9****:** Characterization of *Tr*LPMOA expressed in *Y. lipolytica.* (a) SDS-PAGE analysis of the purified rh*Tr*LPMOA (lane 1) and rh*Tr*LPMOA treated by endo-H (lane 2); (b) H₂O₂ generation by rh*Tr*LPMOA in the presence and absence of various oligosaccharides substrates; (c) gluconic acid produced from cello-oligosaccharides (DP4-DP6) by action of rh*Tr*LPMOA in the presence of ascorbic acid.
**Figure 10****:** Comparison of the hydrolytic activity of the total secreted cellulases produced by different cellulolytic *Y. lipolytica* strains on various cellulosic substrates.
**Figure 11****:** Hydrolysis of Avicel in the presence of rhLPMOA at different concentrations.
**Figure 12****:** PCR verification of *Y. lipolytica* transformants expressing multiple cellulases (A) YLC7, Lane 1, 2, 3, 4, 5, 6, 7: *BGL1, BGL2, TrEGI, TrEGII, 4UASNcCBHI, 4UASTrCBHII*, *TrLPMOA;* (B) YLC8, Lane 1, 2, 3, 4, 5, 6, 7: *BGL1, BGL2, TrEGI, TrEGII, 4UASNcCBHI, 4UASTrCBHII, TrSWO1.*
**Figure 13****:** The growth of *Y. lipolytica* in defined medium containing 10 g/L gluconic acid or 10 g/L glucose.
**Figure 14****:** Expression of *Tr*SWO1 in *Y. lipolytica.* (a) SDS-PAGE analysis of the culture supernatant of *Y. lipolytica* transformants; (b) Western blot analysis of the heterologous SWO1 protein produced by *Y. lipolytica*: lane 1, rh*Tr*SWO1; lane 2, rh*Tr*SWO1 treated by endo-H; (c) SDS-PAGE analysis of the purified rh*Tr*SWO1.
**Figure 15****:** Hydrolysis of Avicel after pretreatment with swollenin at different concentrations.
**Figure 16****:** Comparison of the hydrolytic activity of the total secreted cellulases produced by different cellulolytic *Y. lipolytica* strains on various cellulosic substrates.

### EXAMPLE 1: Conferring cellulose-degrading ability to Yarrowia lipolytica

### Materials and Methods

### Strains and media

The genotypes of the microbial strains used in the present study are summarized in Table 1 below.

**Table 1: Microbial strains used**

| Strains | Relevant genotype | Source of reference |
|---|---|---|
| *T. reesei* QM9414 | Wildtype | DSMZ |
| *Phanerochaete chrysosporium* | Wildtype | DSMZ |
| *E. coli* DH5 | Φ80dlacZΔm15, *recA1, endA1, gyrA96, thi-1, hsdR17* (rk⁻, mk⁺), *supE44, relA1, deoR,* Δ(*lacZY*A-argF) U169 | Invitrogen |
| *Y. lipolytica* JMY1212 (Zeta) | *MATA, ura3-302, leu2-270-LEU2-zeta, xpr2-322* Δ*lip2,* Δ*lip7,* Δ*lip8* | Bordes *et al*., 2007 |
| *Y. lipolytica* Δ*pox*B12 | *pTEF-YlBGL1, pTEF-YlBGL2* | Guo Z, *et al.* 2015 |
| ylTrEGI | Zeta, *pTEF-TrEG I*-*His6* | Present study |
| ylTrEGII | Zeta, *pTEF-TrEG II-His6* | Present study |
| yl*Nc*CBHI | Zeta, *pTEF-NcCBH I-His6* | Present study |
| yl*Tr*CBHI | Zeta, *pTEF-TrCBH I-His6* | Present study |
| yl*Pf*CBHI | Zeta, *pTEF-PfCBH I-His6* | Present study |
| yl4UAS*Nc*CBHI | Zeta, *pHTEF-NcCBH I-His6* | Present study |
| yl*Tr*CBHII | Zeta, *pTEF-TrCBH II-His6* | Present study |
| yl*Pc*CBHII | Zeta, *pTEF-PcCBH II-His6* | Present study |
| yl4UAS*Tr*CBHII | Zeta, *pHTEF-TrCBH II-His6* | Present study |
| YLC1 | Δ*pox*B12, *pTEF-TrEG I, pTEF-NcCBH I, pTEF-TrCBH II* | Present study |
| YLC2 | Δ*pox*B12, *pTEF-TrEG II, p.TEF-NcCBH I, pTEF-TrCBH II* | Present study |
| YLC3 | ΔpoxB12, *pTEF-TrEG I, pTEF-TrEG II*, *pTEF-NcCBH I, pTEF-TrCBH II* | Present study |
| YLC4 | ΔpoxB12, *pTEF-TrEG I, pTEF-TrEG II, pTEF-NcCBH I, pHTEF-TrCBH II* | Present study |
| YLC5 | Δ*pox*B12, *pTEF-TrEG I, pTEF-TrEG II, pHTEF-NcCBH I, pTEF-TrCBH II* | Present study |
| YLC6 | Δ*pox*B12*, pTEF-TrEG I, pTEF-TrEG II; pHTEF*-*NcCBH I, pHTEF-TrCBH II* | Present study |
| YLC7 | Δ*pox*B12*, pTEF-TrEG I, pTEF-TrEG II; pHTEF*-*NcCBH I, pHTEF-TrCBH II, pTEF-TrLPMOA* | Present study |

*E. coli* DH5 were purchased from Invitrogen (Paisley, UK) and used for plasmid construction. The *Y. lipolytica* strains were routinely cultivated in a medium composed of 1% w/v yeast extract, 1% w/v Bacto peptone, and 1% w/v glucose (YPD). Transformants were selected on solid YNB medium (0.17% w/v YNB, 1% glucose or cellobiose w/v, 0.5% w/v ammonium chloride, with (for Ura⁺) or without (for Leu⁺) 0.2% w/v casamino acids and 50 mM sodium-potassium phosphate buffer, pH 6.8), supplemented with uracil (440 mg/L) or leucine (440 mg/L) depending on the auxotrophic requirements. Solid media contained 1.5% agar. The detection of endoglucanase activity in solid YNBcasa medium was achieved by incorporating 0.2% w/w Azo-CM-Cellulose (Megazyme). For cellulases characterization, enzymes were produced in YT2D5 medium (1% w/v yeast extract, 2% w/v tryptone, 5% w/v glucose and 100 mM phosphate buffer, pH 6.8). To evaluate the growth of engineered cellulolytic *Y. lipolytica* on cellulose, transformants were aerobically cultivated in defined medium containing vitamins, trace elements (Verduyn *et al*., 1992) and salts, including 3.5 g/L (NH₄)₂SO₄, 3.0 g/L K₂HPO₄, 3.0 g/L NaH₂PO₄ and 1.0 g/L MgSO₄•7H₂O with 27 g/L CIMV-cellulose (cellulose content≈93%, provided by CIMV S.A.) or 25 g/L Avicel PH-101 (Sigma).

### Plasmid constructions

The plasmids constructed in the present study are summarized in Table 2 below and all primers are listed in Table 3 below.

**Table 2: Plasmids used or created**

| Plasmids | Description | Source of reference |
|---|---|---|
| JMP62UraTEF | *URA3, pTEF* | Taleb and Radford, 1995 |
| JMP62LeuTEF | *LEU2, pTEF* | Nicaud *et al*. 2002 |
| JMP62UraTB1his | *URA3, pTEF-YlBGL1-His6* | Guo *et al.* 2015 |
| PUB4-CRE | *hph, hp4d-CRE* | Fickers *et al*., 2003 |
| JMP62UraHTEF | *URA3, pHTEF* | Present study |
| JMP62LeuHTEF | *LEU2, pHTEF* | Present study |
| JMP62UraEG1 | *URA3, pTEF-TrEG I* | Present study |
| JMP62LeuEG2 | *LEU2, pTEF-TrEG II* | Present study |
| JMP62UraNcCBH1 | *URA3, pTEF-NcCBH I* | Present study |
| JMP62UraNcCBH1His | *URA3, pTEF-NcCBH I-His6* | Present study |
| JMP62UraTrCBH1His | *URA3, pTEF-TrCBH I-His6* | Present study |
| JMP62UraPfCBH1His | *URA3, pTEF-PfCBH I-His6* | Present study |
| JMP62UraTrCBH2 | *URA3, pTEF-TrCBH II* | Present study |
| JMP62LeuTrCBH2 | *LEU2, pTEF-TrCBH II* | Present study |
| JMP62UraPcCBH2 | *URA3, pTEF-PcCBH II* | Present study |
| JMP62UraTrCBH2 | *URA3, pTEF-TrCBH II-His 6* | Present study |
| JMP62UraPcCBH2 | *URA3, pTEF-PcCBH II-His6* | Present study |
| JMP62UraHCBH1 | *URA3, pHTEF -NcCBH I* | Present study |
| JMP62LeuHCBH2 | *LEU2, pHTEF-TrCBH II* | Present study |
| JMP62UraLPMOA | *URA3, pTEF-LPMOA* | Present study |
| JMP62UraLPMOA | *URA3, pTEF-LPMOA-His6* | Present study |

**Table 3: Sequences of the oligonucleotide primers**

| Primer names | Sequence (5'-3'), 15-bp homologous sequence for infusion is underlined. | SEQ ID NO: |
|---|---|---|
| F1 | GTTCTCCAGAAGCGACAGCAACCGGGTACCAGCAC | 141 |
| R1 | CACAGACACCCTAGGCTAAAGGCATTGCGAGTAGTAGTC GT | 142 |
| F2 | GTTCTCCAGAAGCGAGCACAGCAGACTGTCTGGGGCC | 143 |
| R2 | CACAGACACCCTAGGCTACTTTCTTGCGAGACACGAGCT GAC | 144 |
| F3 | GTTCTCCAGAAGCGAGCCCAGGCTTGCTCAAGCGTC | 145 |
| R3 | CACAGACACCCTAGGTTACAGGAACGATGGGTTTGCGT | 146 |
| F4 | GTTCTCCAGAAGCGACATGGACATATTAATGACATTGTCA TCAACG | 147 |
| R4 | CACAGACACCCTAGGCTAGTTAAGGCACTGGGCGTAGTA GGG | 148 |
| F5 | GTTCTCCAGAAGCGACAGGCGTCGGAGTGGGGACA | 149 |
| R5 | CACAGACACCCTAGGCTACAGCGGCGGGTTGGCAG | 150 |
| JMP1F | CCTAGGGTGTCTGTGGTATCTAAGCTATT | 151 |
| JMP1R | TCGCTTCTGGAGAACTGCGG | 152 |
| F6 | ACACCCGAAGGATCCCACAATGAAGCTTTCCACCATCC | 153 |
| R6 | ATGGTGATGATGGTGAAGGCATTGCGAGTAGTAGTCGT | 154 |
| R7 | ATGGTGATGATGGTGCTTTCTTGCGAGACACGAGCTGAC | 155 |
| R8 | ATGGTGATGATGGTGCAGGAACGATGGGTTTGCGT | 156 |
| R9 | ATGGTGATGATGGTGCAGCGGCGGGTTGGCAGC | 157 |
| R10 | ATGGTGATGATGGTGGTTAAGGCACTGGGCGTAGTAGGG | 158 |
| JMP2F | CACCATCATCACCATCATTAAAACT | 159 |
| JMP2R | GGATCCTTCGGGTGTGAGTTG | 160 |
| HTF | ATCCCTAGAATCGATGCCGCCGCAAGGAATGG | 161 |
| HTR | GCCAACCCGGTCTCTGCACTTTTGCCCGTGATCAGTG | 162 |
| JMP3F | AGAGACCGGGTTGGCGG | 163 |
| JMP3R | ATCGATTCTAGGGATAACAGGGTAATT | 164 |

Briefly, the total RNA from 5-day cultured *Trichoderma reesei* QM9414 was isolated using RNeasy Plus Mini Kit (QIAGEN) and reverse transcription was performed with iScript™ cDNA Synthesis Kit (BIO-RAD) according to the manufacturer's instructions. For the expression of wild-type proteins, EG I (GenBank accession code: XM_006965612.1), EG II (GenBank accession code: XM_006965612.1), CBH II (GenBank accession code: XM_006962518.1) and *LPMOA* (formerly known as GH61A, GenBank accession code: Y11113.1), respectively, were amplified from the cDNA of *T*. *reesei* by PCR using F (1-4) as forward primers and R (1-4) as reverse primers. As a comparison, CBH II from *Phanerochaete chrysosporium* (GenBank accession code: AB699568.1) was cloned from the cDNA of *P. chrysosporium* by PCR using primers F5 and R5. A 15-base pair homologous sequence of the target plasmid was introduced into the end of each gene during PCR amplification. After that, the genes encoding *T. reesei* EG I, EG II, CBH II, *LPMOA* and *P. chrysosporium* CBH II, were fused with the PCR fragment (primers JMP1F/JMP1R) of secretion vector JMP62UraTEF or JMP62LeuTEF under the control of TEF promoter and the pre-pro sequence of lipase 2 (33 N-terminal amino acids of Lipase 2, Genbank accession number: Q9P8F7) of *Y. lipolytica* by In-Fusion Cloning Kits (Clontech). For the expression of His-tagged proteins, EG I, EG II and CBH II were cloned by PCR with F6 as forward primer and R (6-10) as reverse primers using the expression vectors constructed in last step as template, and fused with PCR fragment (primers JMP2F/JMP2R) of the vector JMP62UraTB1his (Guo *et al.* 2015).

To express CBH I in yeast, the gene coding sequences of CBH I from *T. reesei*, *Penicillium funiculosum* and *Neurospora crassa* were codon-optimized based on the codon bias of *Y. lipolytica,* and were synthesized by GenScript (USA) and cloned into the plasmid JMP62UraTEF, JMP62LeuTEF, or JMP62UraTB1his under the control of TEF promoter and the pre-pro sequence of lipase 2. The codon-optimized nucleotide sequences of TrCBH1, *Pf*CBH1 and *Nc*CBH1 are respectively referred to as SEQ ID NO: 165, 166 and 167.

These nucleotide sequences of SEQ ID NO: 165, 166 and 167 are codon-optimized based on the codon bias of *Y. lipolytica* and start with the signal peptide of lipase 2 of *Y. lipolytica* of nucleotide sequence SEQ ID NO: 213 (pre-pro sequence of Lipase 2).

In addition, an enhancer comprised of 4 tandem copies of upstream activation sequences (4UASs) was cloned by PCR with primers HTF and HTR using vector PUB4-CRE as template. The fusion of this DNA fragment with the PCR product (primers JMP3F/JMP3R) of vector JMP62UraTEF or JMP62LeuTEF yielded vectors JMP62UraHTEF and JMP62LeuHTEF, respectively. To enhance the expression level of CBH proteins, the plasmids JMP62UraCBH1 and JMP62LeuCBH2 were digested using *Bam*HI/*Avr*II*,* and the *Nc*CBHI and *Tr*CBHII genes were respectively recovered and then inserted at the corresponding sites into the plasmid JMP62UraHTEF or JMP62LeuHTEF.

After construction, all expression vectors were verified by DNA sequencing (GATC Biotech, Konstanz, Germany). For *Y. lipolytica* transformation, vectors were digested using *Not*I, thus generating a linear DNA with Zeta sequences at both extremities, and purified liberating the expression cassettes. Then the gel purified expression cassettes were introduced into the Zeta docking platform of *Y. lipolytica* JMY1212 Zeta for the expression of single cellulase, or randomly into the genome of Δ*pox*B12 strain, for co-expression of multiple cellulases, using the lithium acetate method (Duquesne *et al*., 2012). For the latter case, the *LoxP-*Cre recombination system was used for marker rescue and ensure the multistep insertion of the target genes (Fickers P, *et al.* 2003). The successful multiple integration of the heterologous genes into the genome of *Y. lipolytica* was verified by PCR using gene specific primers shown in Table 4 below.

**Table 4: Sequences of the oligonucleotide primers used for PCR verification of Y. lipolytica transformants**

| Primer names | Sequence (5'-3') | SEQ ID NO: |
|---|---|---|
| VBGL1A | TTGACCCAGTAGCGGACCCAA | 168 |
| VBGL1B | GCCGACATTAGCCCTAACAGCAT | 169 |
| VBGL2A | GGTTGGCGGCGCATTTGT | 170 |
| VBGL2B | TGTCGTCCACTCGGCTTTCATC | 171 |
| VEGIA | CACTTGCCGTTAAGGGCGTAGGGT | 172 |
| VEGIB | CTGGCTGTTGTCGTTCCAAATGCTG | 173 |
| VEGIIA | CTTGCCGTTAAGGGCGTAGGG | 174 |
| VEGIIB | CCATCGAGCATAATTGTGGATGTCG | 175 |
| VCBHIA | TTTGCTTTGTGGTTGGGACTTTAGC | 176 |
| VCBHIB | GGTTCGAGATCCGATGTTAGTGGAGTA | 177 |
| VCBHIIA | TTTGCTTTGTGGTTGGGACTTTAGCC | 178 |
| VCBHIIB | AGGCGTTGGACCAGCCGTGA | 179 |
| V4UASCBHIA | CGCCGCAAGGAATGGTGCA | 180 |
| V4UASCBHIB | GGTTCGAGATCCGATGTTAGTGGAGTA | 181 |
| V4UASCBHIIA | CGCCGCAAGGAATGGTGCA | 182 |
| V4UASCBHIIB | CCGATCCGACTGGAGGTACTCTGGTAG | 183 |
| VLPMOA | TTGACCCAGTAGCGGACCCAACCC | 184 |
| VLPMOB | TGATCTCGTGGCGGAGCACGTAATT | 185 |

In addition, transformants expressing multiple enzymes were tested for growth on cellobiose, and for degradation of Azo-CM-Cellulose. Clones displaying both activities were retained for further analysis. Table 4 summarizes the expressed cellulase genes and their corresponding *Y. lipolytica* transformants.

### Enzyme production and activity assay

Recombinant protein production by *Y. lipolytica* was carried in YTD medium in shake flask at 28°C and 120 rpm for 5 days. PASC (phosphoric acid swollen cellulose) was prepared from Avicel PH-101 as previously described in Zhang *et al.* (2006). The activities of endoglucanases (EGs) were measured on carboxymethyl cellulose (CMC), PASC, β-1, 4-glucan, Avicel PH-101 and cellotriose using the method previously described in Ghose (1987) with slight modifications. Briefly, the reaction mixture contained 1 % (w/v) insoluble cellulosic substrate, or 5mM cellotriose, 50 mM citrate buffer (pH 4.8) and proper volume of diluted enzyme solution. The reaction was conducted at 50°C for 30 min and then reducing sugars were quantified using the dinitrosalicylic acid (DNS) reagent. A similar method was used to evaluate cellobiohydrolase (CBH) activity using PASC, β-1, 4-glucan or Avicel as substrates. One unit of activity (U) was defined as the amount of enzyme required to release 1 µmol of reducing sugars per min. All protein concentrations were measured using the Bradford method and bovine serum albumin as a standard (Bradford, 1976). All enzymatic activity measurements were performed in triplicate unless otherwise stated.

### SDS-PAGE and Western blot analysis

SDS-PAGE was conducted using Mini-PROTEAN TGX Stain-Free precast gels (Biorad) according to the manufacturer's instructions. 15 µL of culture supernatant or enzyme solution was loaded into each well. Western blotting of proteins was performed as described previously in Duquesne *et al.* (2014). Crude supernatant of *Y. lipolytica* JMY1212 expressing EGs and CBHs fused with the His6 tag were concentrated 10-fold using an ultra-centrifugation filter unit (Amicon® Ultra-4 10 kDa cut-off, Merk Millipore, Bedford, MA, USA). Blots were sequentially treated with mouse non position-specific His-Tag antibody 1:2500 (THE™ from Genscript, Piscataway, NJ, US) and the alkaline phosphatase-conjugated goat anti-mouse IgG (1:10000). For the detection, the membrane was incubated with a mixture of nitro blue tetrazolium chloride and 5-Bromo-4-chloro-3-indolyl phosphate (NBT/BCIP) (Sigma).

### Purification of recombinant cellulases and deglycosylation

*Y. lipolytica* JMY1212 expressing His6-tagged EG I, EG II, CBH I and CBH II, respectively, was grown in 200 mL YTD medium at 120 rpm, 28°C for 48 h. After centrifugation (8,000×g for 5 min at 4°C), the supernatant was concentrated 10-fold by ultrafiltration with an Omega™ membrane disc filter at 10KDa cut off (Pall, France), and applied to 2 mL of TALON Metal Affinity Resin (Clontech, Takara-Bio, Kyoto, Japan). Subsequently, protein was eluted using imidazole buffer according to the manufacturer's instructions. Deglycosylation was carried out by treating the purified proteins with endoglycosidase H according to the manufacturer's instructions (New England Biolabs, Beverly, MA, USA) to remove N-linked carbohydrates. Protein samples were analyzed by SDS-PAGE and visualized with colloidal coomassie blue staining.

### Growth of yeast expressing multiple cellulases on CIMV and Avicel cellulose

Yeast growth on CIMV-cellulose and Avicel was performed in 50 mL defined medium containing 27 g/L CIMV or 25 g/L Avicel cellulose in 250 mL Erlenmeyer flasks. A fresh yeast culture in exponential phase was used to inoculate the defined medium to yield an initial biomass concentration of 1.0 g-DCW/L. The cultivations were conducted at 28°C and samples were taken at the end of 5 days to determine concentrations of biomass and residual cellulose. In parallel, two control experiments were conducted under the same conditions, with the addition of Cellic® CTec2 (120 IU/mL, gift from Novozyme, Denmark) at 20 IU/g cellulose as recommended.

### Analysis of residual cellulose and determination of dry cell weight

The quantification of cellulose residues and dry cell matter was conducted as previously described in Wei *et al.* (2014) with slight modifications. Briefly, the cell pellets mixed with cellulose residues from the above cultures were harvested by centrifugation at 8,000×g for 10 min at 4°C. After 2-time wash with distilled water, the collected cellulose-cell pellet was freeze-dried and weighed. The amount of cellulose remained was calculated from the total glucose released from enzymatic hydrolysis of the cellulose residues using Cellic® CTec2, and verified by diluted acid hydrolysis of the residues with 2.5% sulfuric acid at 121°C for 1 h. Dry cell weight was deduced by subtracting the amount of cellulose from the weight of cellulose-cell pellet. Glucose was measured by HPLC as descried in Guo *et al.* (2015). The biomass yield was calculated as the ratio of the amount of biomass obtained divided by the amount of carbon source consumed.

### Amplex Red/horseradish peroxidase assay

The oxygen reactivity of *Tr*LPMOA was measured in a quantitative time resolved assay of H₂O₂ using Amplex Red and horseradish peroxidase as described previously (Isaksen *et al,* 2014; Kittl *et al*, 2015). The reaction mixture (100 µL) containing 100 mM sodium acetate buffer (pH 6.0), 50 µM Amplex Red reagent (Invitrogen, France), 7.14 U/mL horseradish peroxidase, 0.1 to 0.5 µM LPMOA and 30 µM ascorbate as reductant was assembled in the well of a 96-well microtiter plate and incubated at 30°C, measuring fluorescence (excitation and emission wavelengths of 560 and 595 nm respectively) using a Tecan Infinite M200 plate reader (Tecan, Männedorf, Switzerland). The activity of *Tr*LPMOA was calculated was derived from the data using a standard H₂O₂ calibration curve. The inhibition of H₂O₂ production was used to test the activity of *Tr*LPMOA on various β-glucan and xylan substrates as described previously (Bennati-Granier *et al*, 2015). A final concentration of 1.0 % (w/v) PASC, CMC, Avicel PH-101, barley glucan (β-1, 3; 1,4) and xylan was added into the above reaction mixture. All measurements were performed in triplicates.

### Cello-oligosaccharide cleavage assay

The cleavage of cello-oligosaccharides by LPMOA was assayed by incubating 16 mg of *Tr*LPMOA, 1 mM of ascorbate, and 5 mM cello-oligosaccharides (DP4-DP6) in 50 mM sodium acetate buffer (pH 4.8) at 50 °C for 24 h under shaking (1000 rpm). To stop the reaction, samples were boiled (100 °C for 10 min) and then cooled before adding an aliquot of Novozyme 188 β-glucosidase (12.0 IU/g cello-oligosaccharides, 810 IU/mL). After, the reaction was incubated for a further 12 h and then the oxidized mono-saccharide in the supernatant was measured using a D-gluconic acid/D-glucono-δ-Lactone assay kit (Megazyme) according to manufacturer's instructions. All assays were carried out in triplicate.

### Study the synergy of LPMOA on enzymatic hydrolysis of Avicel

Enzymatic hydrolysis of cellulose was carried out in 50 mM citrate buffer (pH 4.8) containing 2.0 % (w/v) microcrystalline cellulose (Avicel PH-101, Sigma). Celluclast 1.5L (60 FPU/mL) and β-glucosidase (810 IU/mL, Novozyme 188, gift from Novozyme, Denmark) was added at 5.0 U/g-cellulose and 12.0 IU/g-cellulose, respectively. The reaction (1 mL) was conducted in Eppendorf tubes (2 mL) using a thermomixer at 50 °C and 1000 rpm. To study the synergistic effect of *Tr*LPMOA on the enzymatic hydrolysis of cellulose, different amount of this protein (5, 10 and 15 mg-protein/g-cellulose respectively) was added to the reaction mixtures containing Avicel (2.0 % w/v). In addition, ascorbate (1 mM) was added into the reactions as the reducing agent to reactivate LPMOA.

### Results

### Expression of T. reesei endoglucanases in Y. lipolytica

The first step towards the construction of a fully cellulolytic *Y. lipolytica* strain was the introduction of sequences encoding *T. reesei* EG I (Cel7B) and EG II (Cel5A) into the *Y. lipolytica* zeta strain (Bordes *et al*., 2007). Subsequent screening of transformants producing either EG I or EG II with or without His6 revealed the presence of clones possessing the ability to hydrolyze Azo-CMC in solid agar medium (data not shown).

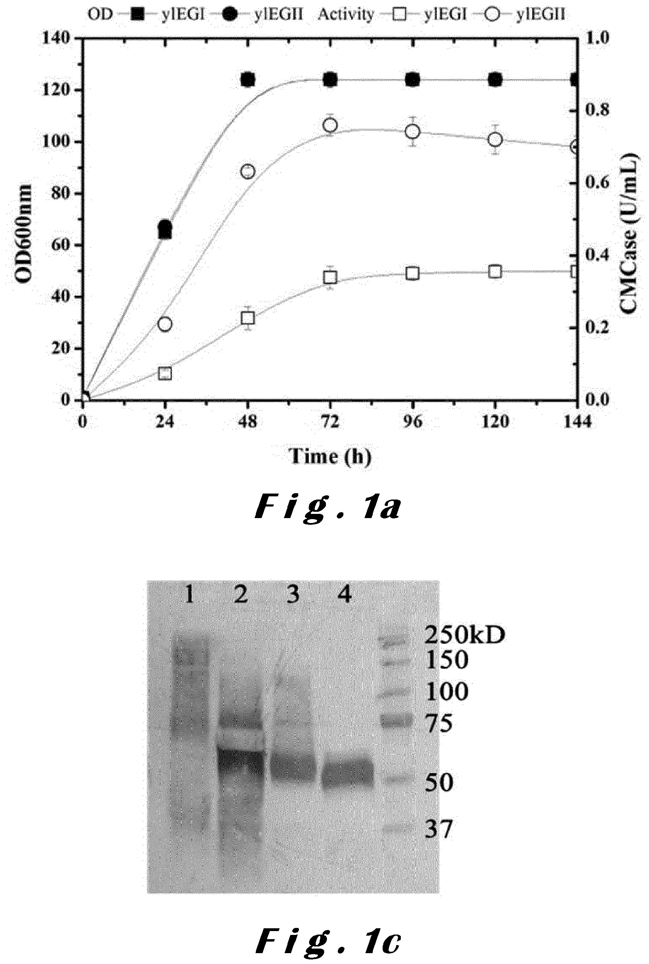

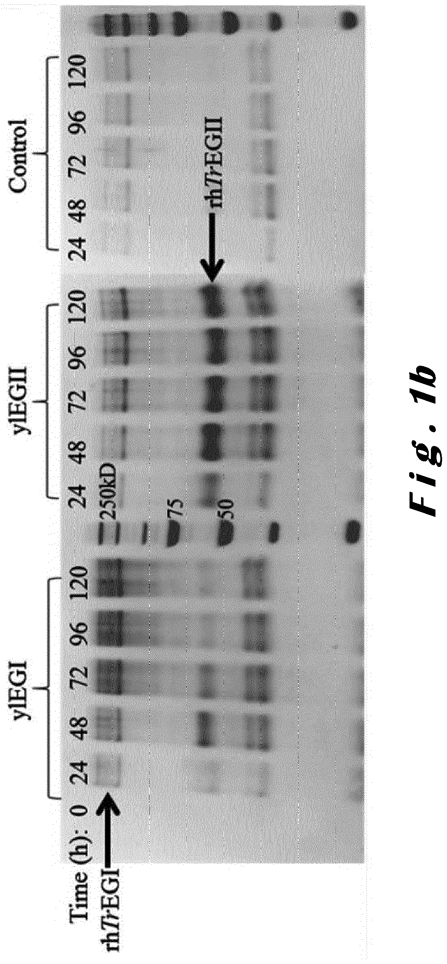

To further confirm the successful expression of EG I and II, positive clones were grown and His-tagged recombinant proteins (rh) rh*Tr*EGI and rh*Tr*EGII were purified from culture supernatant. Accordingly, it was possible to demonstrate that these secreted proteins possessed CMCase activity and that their expression steadily increased over a 48h-period until the glucose in the culture medium was depleted, with rh*Tr*EGII activity (0.78 U/mL) at the end of the period being twice that of rb*Tr*EGI (0.39 U/mL) (Fig. 1a). Furthermore, SDS-PAGE analysis of culture supernatants containing either rh*Tr*EGI or rh*Tr*EGII (Fig. 1b), and comparison to that of a control culture, revealed in the first case the presence of a smear (70-200k Da) and in the latter case a discrete species migrating to a position corresponding to an approximate Mw (molecular weight) of 55 kDa, which is a little high when compared to the actual expected Mw of EG II (47 kDa). In the case of both putative rh*Tr*EGI and II, the anti-His antibody confirmed that these protein species were His-tagged (Fig. 1c). Taken together, these observations suggest that the recombinant proteins are glycosylated forms of EG I and II, in agreement with previous results (Boonvitthya *et al*., 2013; Park *et al*., 2000) and with the findings of a bioinformatics study (http://www.imtech.res.in/raghava/glycoep/) (Chauhan *et al*., 2013) that predicted that EG I bears 6 potential N-glycosylation sites, while EG II possesses only one. To confirm the presence of N-glycosylation, EndoH treatment and then Western blot analysis were performed (Fig. 1c). This revealed that the EndoH-treated protein samples were still detected using anti-His antibodies, but for rh*Tr*EGI a discrete protein species of approximately 56 kDa appeared in place of the smear, which is a little high when compared to the actual expected Mw (49 kDa). Likewise, the migration of EndoH-treated rh*Tr*EGII was slightly modified, consistent with that of a lowered Mw.

### Characterization of the recombinant endoglucanases expressed in Y. lipolytica

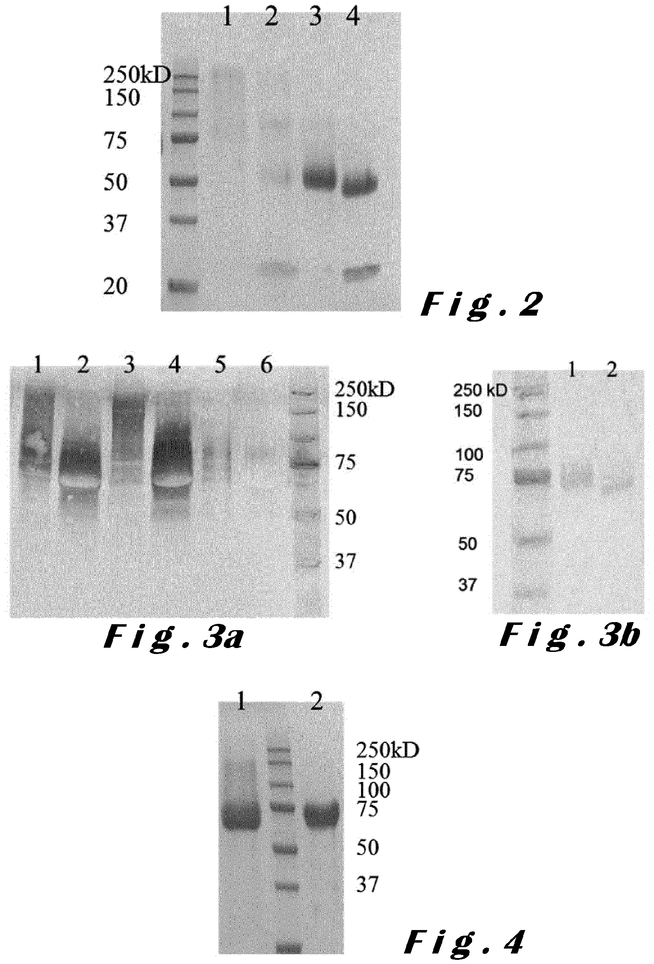

Rh*Tr*EGI and rh*Tr*EGII expressed in *Y. lipolytica* were purified and characterized. For the purification of rh*Tr*EGII, a one-step affinity method procured good overall yield (>60%), whereas the yield of rh*Tr*EGI was lower (18%), probably due to the hyper-glycosylated state of this protein (Fig. 2).

Subsequently, the hydrolytic activities of purified rh*Tr*EGs were measured on various cellulosic substrates (Table 5).

**Table 5: Comparison of the hydrolytic activity of purified rhTrEGI and rhTrEGII expressed in Y. lipolytica on various cellulosic substrates (The mean value of three independent experiments is shown and the standard deviation is below 10%.)**

| | Specific activity µmol/min/mg) | | | | | |
|---|---|---|---|---|---|---|
| | Avicel | β-1, 4 glucan | β-1, 3 glucan | CMC | RAC | Cellotriose |
| EG I | 0.04 | 18.0 | 0.5 | 12.8 | 9.0 | 1.1 |
| EG II | 0.02 | 11.3 | 0.2 | 11.6 | 7.1 | 0.1 |

Significantly, compared to rh*Tr*EGII (0.1 µmol/min/mg), rh*Tr*EGI not only displayed 10 times higher specific activity on soluble cellotriose (1.1 µmol/min/mg), but also exhibited higher activity on Avicel, β-1, 3 and β-1, 4 glucans and phosphoric acid swollen cellulose (PASC). On the other hand, the specific activities of the two proteins on soluble carboxymethyl cellulose (CMC) were similar. Moreover, it is noteworthy that rh*Tr*EGI exhibited highest hydrolytic activity on β-1, 4 glucan while CMC was the preferred substrate for rh*Tr*EGII.

The amount of rh*Tr*EGI and rh*Tr*EGII secreted by *Y. lipolytica* in YTD medium during flask batch culture, as calculated from the total CMCase activity of the culture supernatant and specific activity of the enzyme, were approximately 29 and 67 mg/L, respectively. The secretion yield of rh*Tr*EGII obtained in this study was comparable to that reported in the literature (Boonvitthya *et al.,* 2013; Park *et al.* 2000).

### Expression of cellobiohydrolases in Y. lipolytica

*Tr*CBHI, *Pf*CBHI and *Nc*CBHI were produced in *Y. lipolytica* as His-tagged proteins (rh*Tr*CBH1, rh*Pf*CBHI and rh*Nc*CBHI) using the TEF promoter and the *Y. lipolytica* lipase 2 pre-pro region. Subsequent SDS-PAGE and anti-His Western blot analysis of the culture supernatants indicated that rh*Tr*CBHI could only be detected as a faint band. Contrastingly, the successful expression of rh*Nc*CBHI and rh*Pf*CBHI was confirmed by the clear presence of new protein species. In the case of rh*Nc*CBHI a Mw of approximately 75 kDa was determined, while expression of rh*Pf*CBHI produced a smear in the Mw range 70-200 kDa. Since the theoretical Mw of *Pf*CBHI and *Nc*CBHI is 52 kDa each, it was possible to deduce that rh*Pf*CBHI and rh*Nc*CBHI are glycosylated (Fig. 3a). Therefore, along with rh*Tr*CBHI, rh*Pf*CBHI and rh*Nc*CBHI were submitted to EndoH-mediated deglycosylation. This yielded protein products with Mw of approximately 75 kDa, suggesting that the enzymes might bear other post-translational modifications other than N-glycosylation (Fig.3a). Further analysis of culture supernatants using an activity assay revealed that the Avicelase activity in the culture supernatant of the transformant yl*Nc*CBHI was 10 times higher than that in the supernatant of y1*Pf*CBHI (0.01 U/mL). No detectable Avicelase activity was found in the case of yl*Tr*CBHI. Based upon this simple screening approach, *Nc*CBHI was retained as the CBH I component for future work.

Western blot analysis indicated that rh*Tr*CBHII bears a modest amount of post-translational modifications, since its apparent Mw is 75 kDa, which is higher than the theoretical value of 62 kDa even after EndoH-mediated deglycosylation (Fig. 3b). However, no protein product was detected by anti-His Western blot analysis of the culture supernatant of *Y. lipolytica* expressing rh*Pc*CBHII (data not shown). Finally, it is noteworthy that the expression of the different rCBHs without the His-tag yielded similar activities on Avicel and PASC when culture supernatants were tested (*i.e.* comparison ofunpurified protein preparations).

### Characterization of the recombinant cellobiohydrolases expressed in Y. lipolytica

In order to investigate the cellulose-degrading abilities of rh*Nc*CBHI and rh*Tr*CBHII, the recombinant CBHs were purified and characterized. Purification was achieved in a single step using IMAC with both proteins being obtained in good yields (>60% of the expressed protein) (Fig. 4). Afterwards, each enzyme was tested for its ability to hydrolyze various substrates (Table 6).

**Table 6: Comparison of the hydrolytic activity of purified His-tagged NcCBHI and TrCBHII expressed in Y. lipolytica with native T. reesei CBHs on various cellulosic substrates. The mean value of three independent experiments is shown and the standard deviation is below 10%.**

| | Specific activity (µmol/min/mg) | |
|---|---|---|
| | Avicel | PASC |
| *Nc*CBHI | 0.11 | 2.5 |
| Native *Tr*CBHI (Baker *et al*., 1998) | 0.065 | 0.6 |
| *Tr*CBHII | 0.056 | 0.6 |
| Native *Tr*CBHII (Baker *et al*., 1998) | 0.065 | 0.6 |

The specific activity of rh*Nc*CBHI on Avicel and PASC was 2 and 4 times, respectively, of the reported value for native *Tr*CBHI (Baker *et al*., 1998), that is to say two times the amount of the only cellobiohydrolase of family I expressed in *Y. lipolytica* (Wei *et al*., 2014). In contrast, the comparison of native and rh*Tr*CBHII revealed similar specific activities on these substrates, consistent with the previously reported values (Boonvitthya *et al.* 2013; Baker *et al*., 1998). The high Avicelase activity of rh*Nc*CBHI is noteworthy, because this substrate is known to be crystalline and quite recalcitrant to enzyme hydrolysis. Likewise, the specific activity of rh*Nc*CBHI on amorphous PASC is four times that of native *Tr*CBHI and CBH II (Table 6).

Finally, based on the measurement of total PASCase activities in the culture supernatant and specific activities of the rhCBH enzymes, it was possible to determine that the amount of rh*Nc*CBHI and rh*Tr*CBHII secreted by *Y. lipolytica* in YTD medium was approximately 24 mg/L and 75 mg/L, respectively.

### Enhancement of cellobiohydrolase production in Y. lipolytica using hybrid promoter

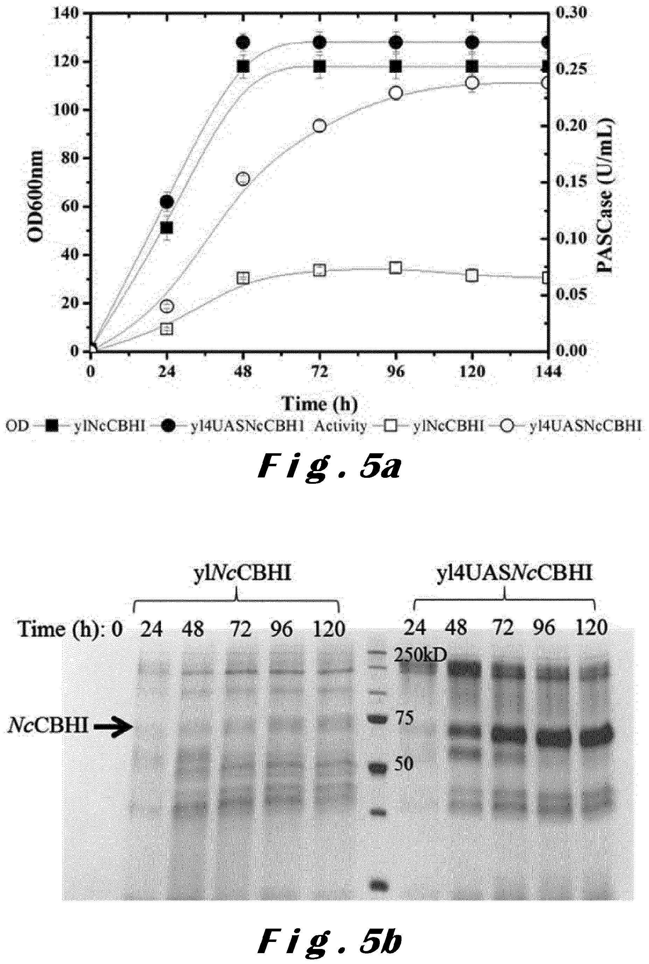

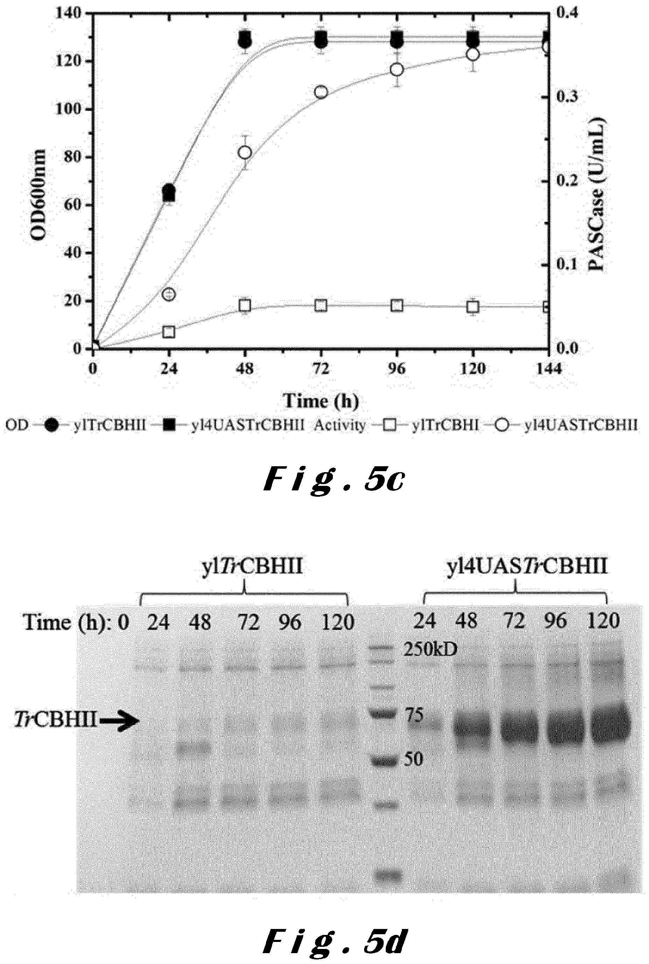

To enhance the expression of r*Tr*CBHII and r*Nc*CBHI, their encoding sequences were placed under the control of the hybrid promoter (HTEF), which is comprised of 4 tandem copies of upstream activation sequences (UAS) and the core promoter element of TEF (Blazeck *et al*., 2011). Monitoring PASCase activity and using SDS-PAGE revealed that while the TEF-controlled production of r*Nc*CBHI was highly cell growth-dependent, reaching its highest level at the beginning of stationary phase (Fig. 5a and b), HTEF-controlled production was much higher and continued to increase over the 5-day growth period. Importantly, when using the HTEF promoter, the final yield of rh*Nc*CBHI (95 mg/L) was 4 times higher than that obtained using TEF and was much higher than previously reported yields (Wei *et al*., 2014). Similarly, when rh*Tr*CBHII was produced under the control of HTEF, the yield of this protein was 8-fold higher (600 mg/L) than that obtained when using the TEF promoter (Fig. 5c). This significant increase was also evidenced upon SDS-PAGE (Fig. 5d). In this regard, although previous studies have already demonstrated the benefits of using the hybrid promoter to enhance protein production in *Y. lipolytica,* studies so far have only been focused on intracellular proteins (Blazeck *et al*., 2011). Here it is supplied two examples of enhanced extracellular production and further shown that in the case of *Tr*CBHII the production level surpasses expectations as according to Blazeck *et al.* (2011), 4 tandem copies of UAS yield 4 folds of increase in protein production.

### Construction of recombinant Y. lipolytica strains expressing different combination and ratio of cellulases

To confer optimal cellulose-degrading ability to *Y. lipolytica*, several strains were constructed, using the previously developed BGL-producing strain as a platform (Guo Z, *et al.* 2015). Moreover, to identify the best configuration, strains were built using different enzyme combinations and protein production ratios (Table 1 *supra*).

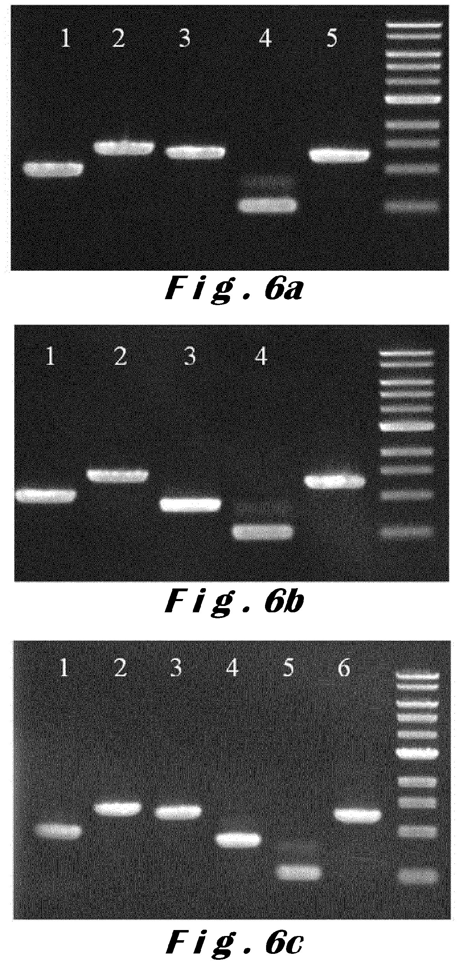

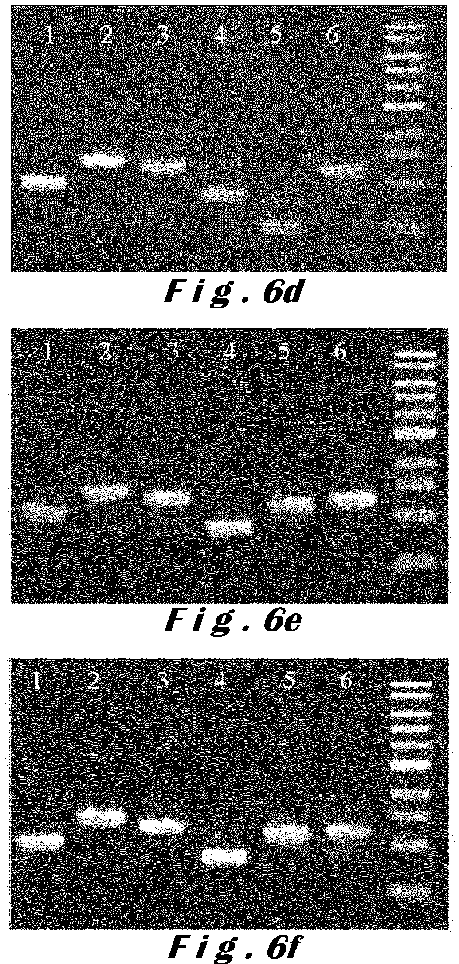

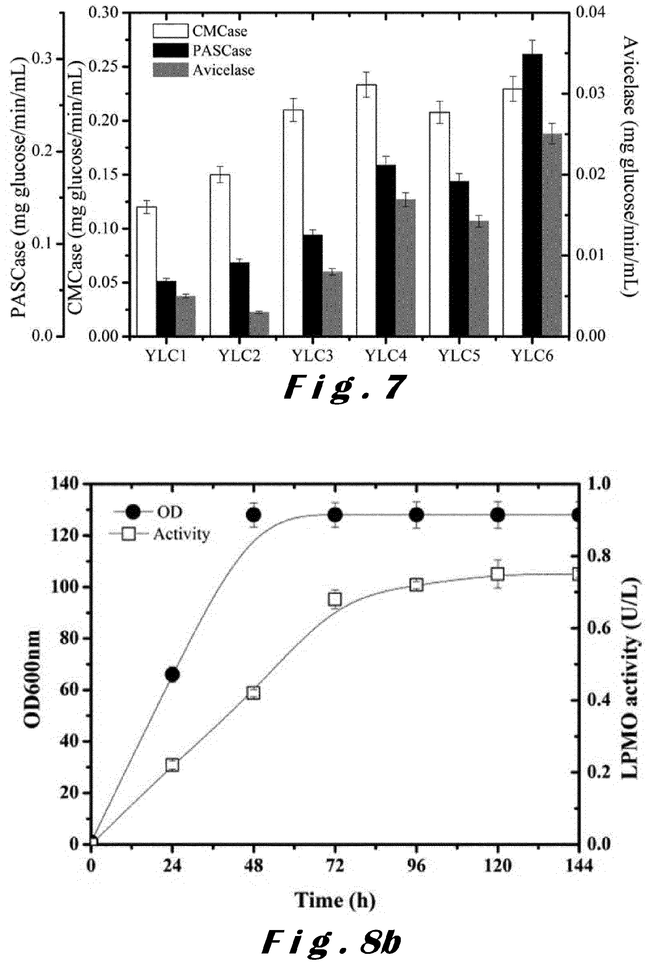

More than 10 transformants of each of the constructions were verified by PCR (Fig. 6) and best individual clones were chosen based on the results of enzymatic activity assays (data not shown). Subsequently, these selected strains were cultivated in YT2D5 medium and the hydrolytic activities of the total secretory secreted proteins were analyzed on cellulosic substrates CMC, PASC and Avicel (Fig. 7).

The strain YLC3, expressing two r*Tr*EGs, displayed higher CMCase activity than YLC1 and YLC2, which express either r*Tr*EGI or r*Tr*EGII, alone (Fig. 7). Moreover, strain YLC1 expressing only r*Tr*EGI and two CBHs, was more active on Avicel than YLC2, which is a homolog that expresses r*Tr*EGII instead of r*Tr*EGI. Consistently, YLC2 displayed better activity on PASC. When both r*Tr*EGI and II are present (strains YLC4-6), the hydrolysis of Avicel and PASC was enhanced, demonstrating that the presence of both EGs is necessary to achieve optimal cellulolytic activity. In contrast, the ability of strain YLC3 to hydrolyze CMC was similar to that of strains YLC4-6, implying that the basic combination of the six enzymes is sufficient to achieve optimal results with this substrate. On the other hand, increasing the expression level of either r*Tr*CBHII (YLC4) or r*Nc*CBHI (YLC5) using the HTEF promoter resulted in improved Avicel and PASC hydrolysis. In this respect, it is significant that the relative proportions of each cellulase component in YLC5 was predicted to be similar to that of the native cellulase system of *T. reesei* (*i.e.* EG I/EG II/CBH I/CBH II≈10/10/60/20 % w/w) as estimated from single cellulase production experiment and verified by in *vitro* activity assay on the mixture of purified enzymes. A further increase of Avicel and PASC hydrolysis was achieved by enhancing the expression levels of both *Nc*CBHI and *Tr*CBHII (YLC6). The presence and the enrichment of each expressed enzyme in YLC6 were confirmed by proteomics analysis (Plateforme Protéomique de la Génopole Toulouse Midi-Pyrénées, IPBS, France).

Overall, the performance of YLC6 was rather encouraging.

### CIMV-cellulose and Avicel fermentation with recombinant Y. lipolytica strains

In order to explore the potentiality of the *Y. lipolytica* strains developed in this study for use as CBP microorganisms, these were grown in defined minimum media containing either industrial cellulose pulp (CIMV-cellulose content≈93%) or Avicel as the carbon source. Gratifyingly, all of the strains consumed CIMV-cellulose, although Avicel was less amenable to hydrolysis. The strain YLC3 expressing all the essential cellulase components used 40% of the CIMV-cellulose provided and produced 0.38 g DW cells (g substrate)⁻¹. These values increased to approximately 50% and 0.40 g DW cells (g substrate)⁻¹ in the case of YLC4 and 5, and to 59% and 0.41 g DW cells (g substrate)⁻¹ in the case of YCL6. Overall, these results demonstrate that cellulolytic capacity had been successfully conferred to *Y. lipolytica* and support findings that CIMV-cellulose is mainly amorphous and highly amenable to enzyme-mediated cellulolysis.

### Expression of lytic polysaccharide monooxygenase in Y. lipolytica

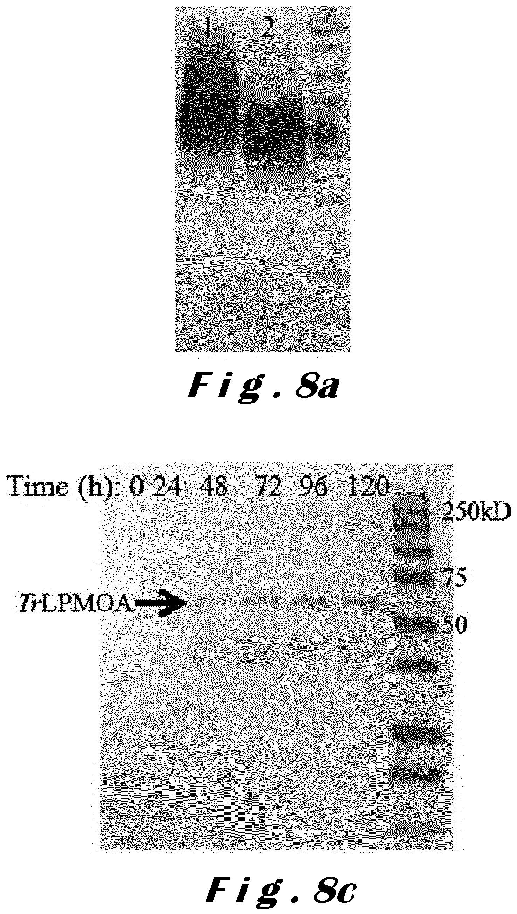

In the extensively studied cellulolytic secretome of the soft-rot fungus *T. reesei,* LPMOs form a very minor component (Adav *et al,* 2012). However, recent *in vitro* studies have shown that the inclusion of these enymes in cellulose cocktails can reduce overall enzyme loadings while maintaining the efficiency of cellulose conversion (Cannella *et al,* 2015; Hu *et al,* 2015). Therefore, in the context of our recent studies on cellulase expression in *Y. lipolytica* we considered that it would be useful to include LMPOs in the panoply of enzymes. To achieve this, *Tr*LPMOA was cloned and expressed under TEF promoter control in *Y. lipolytica* as His-tagged proteins (rh*Tr*LPMOA) using the lipase 2 pre-pro region. In the case of rh*Tr*LPMOA, compared to the control experiment, SDS-PAGE and anti-His Western blot analysis of the culture supernatants revealed a smear in the Mw range 60-200 kDa, instead of the expected single 34.6 kDa species (Figure 8a). Considering that *Y. lipolytica* is known to glycosylate recombinant proteins and that rh*Tr*LPMOA contains a serine/threonine-rich linker region it was assumed that the smear represented a heterogeneous population of glycosylated rh*Tr*LPMOA. Therefore, rh*Tr*LPMOA was submitted to EndoH-mediated deglycosylation and further SDS-PAGE/Western blot analysis, which revealed a better defined protein population that migrated to a position approximately correlated to a Mw of 55 kDa. This result suggests that rh*Tr*LPMOA was N-glycosylated, but that it is might bear additional post-translational modifications.

To confirm that the expressed recombinant protein was active, the ability of rh*Tr*LPMOA to mediate the reduction of O₂ to H₂O₂ in the presence of ascorbate was established. Moreover, using this test, it was shown that the expression level of rh*Tr*LPMOA steadily increased over a 72h-period to the final activity of 0.75 U/L (Figure 8b).

### Characterization of the rhTrLPMOA expressed in Y. lipolytica

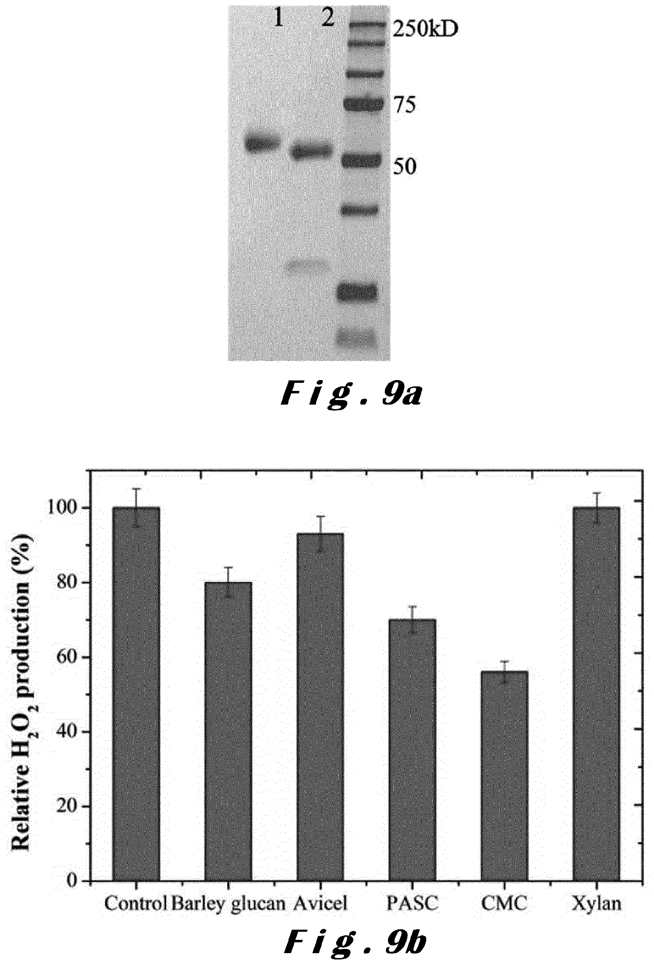

To further assess the functionality of the expressed protein, His-tagged *Tr*LPMOA was purified from culture supernatant and characterized. Purification of rh*Tr*LPMOA was achieved by a one-step affinity method procured good overall yield (>60%) (Figure 9a). To further assess the functionality of the expressed protein, His-tagged *Tr*LPMOA was purified from culture supernatant and characterized. Purification of rh*Tr*LPMOA was achieved by a one-step affinity method procured good overall yield (>60%) (Figure 9a). The purified rh*Tr*LPMOA exhibited a specific activity of 14.4 U/g. This value is similar to that of the *Neurospora crassa* LPMO9F (15 U/g) expressed in *P. pastoris,* which is the most active LPMO reported so far using the same activity assay *(*Isaksen *et al,* 2014). The amount of rh*Tr*LPMOA secreted by *Y. lipolytica* in YTD medium during batch cultivation in flasks was approximately 52 mg/L, a yield that is moderate compared to other examples of PMO heterologous expression (Bennati-Granier *et al,* 2015; Isaksen *et al,* 2014).

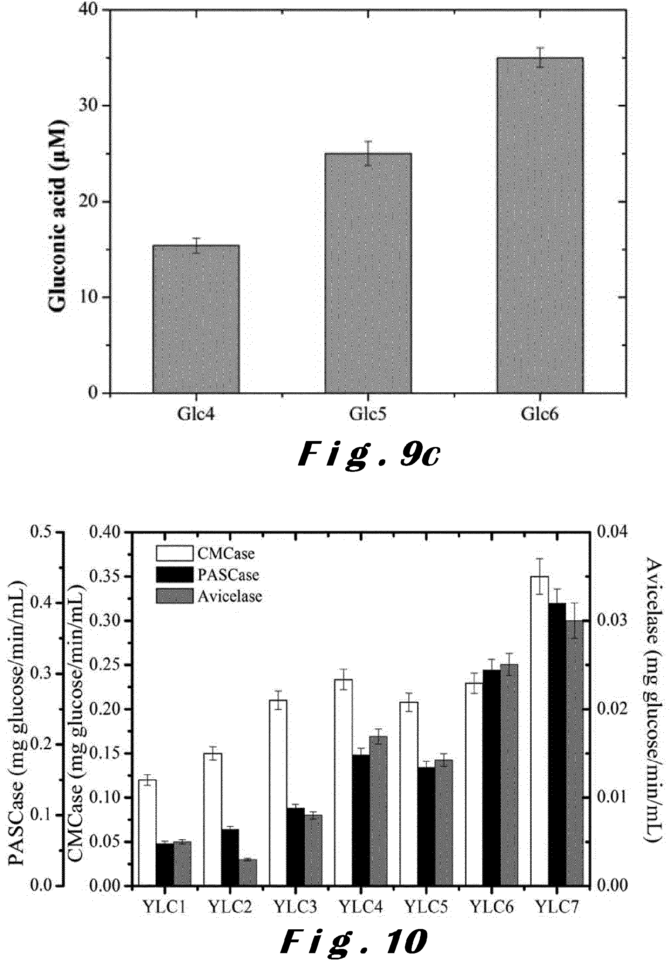

To further investigate the substrate specificities of rh*Tr*LPMOA, we measured the production of H₂O₂ in the presence of 1.0 % (w/v) PASC, CMC, Avicel PH-101, barley glucan (β-1, 3; 1, 4) and xylan. The repression in H₂O₂ production was observed for rh*Tr*LPMOA in the presence of PASC, CMC, Avicel PH-101, barley glucan (β-1, 3; 1, 4) (Figure 9b) while xylan had no effect on H₂O₂ production. These results suggest that rh*Tr*LPMOA displays broad specificity against β-(1, 4)-linked glucans, but is inactive on xylan. The oxidative cleavage of cello-oligosaccharides including cellotetraose (DP4), cellopentaose (DP5) and cellohexaose (DP6) was further investigated using D-gluconic acid/D-glucono-δ-Lactone assay kit (Megazyme). Novozyme 188 β-Glucosidase (810 IU/mL) was added into the reaction mixture to hydrolyze the oxidized species to their monomeric forms before the assay. Three regioselective groups of AA9 LPMOs (types 1, 2 and 3) have been established based on sequence alignments and phylogenetic analyses (Vu *et al,* 2014). The oxidative cleavage of cello-oligosaccharides by rh*Tr*LPMOA in the presence of ascorbic acid to yield the release of C1-oxidized oligosaccharides was confirmed by the successful detection of D-gluconic acid (Figure 9c). However, no C4-oxidized species were detected by HPAEC (data not shown) evidenced that *Tr*LPMOA behaved as a type 1 LPMO (oxidation at the C1 position) although from the sequence it was predicted as a type 3 LPMO (oxidation at both the C1 and C4 position). Therefore, *Tr*LPMOA might be another exception for this classification which based on alignments since a subgroup of type 3 LPMOs (PMO3*) was recently created because there are LPMOs such as the *Myceliophtora thermophile* AA9 LPMO (MYCTH_92668) was shown to oxidize at C1 position only (Vu et al, 2014).

### The synergy of LPMOA on enzymatic hydrolysis of Avicel

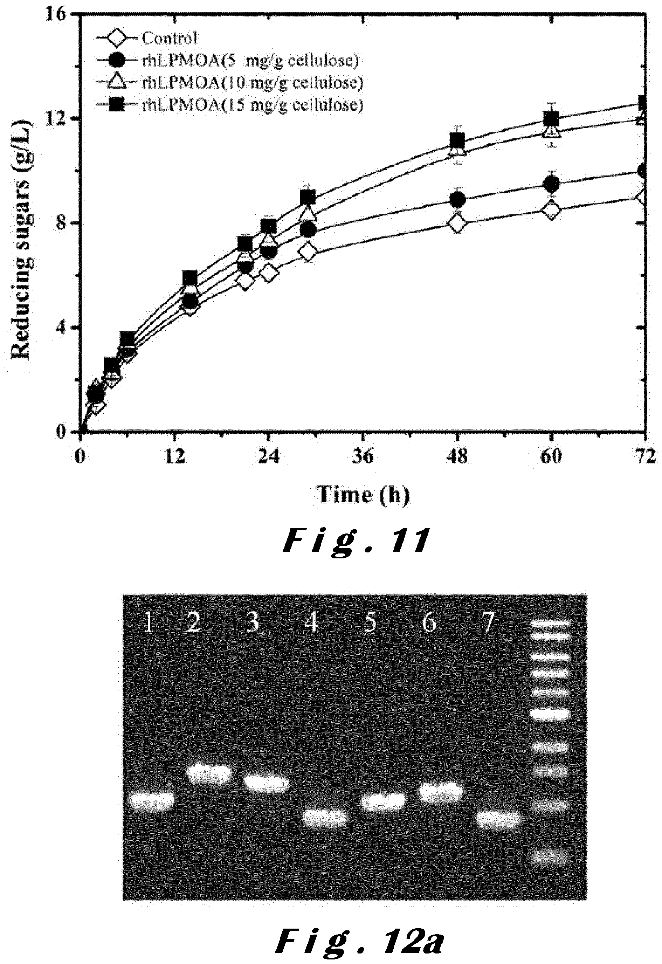

To investigate synergy between helper protein and cellulases, enzymatic hydrolysis of Avicel was performed using a commercial cellulase cocktail (Cellulcast) supplemented with the rh*Tr*LPMOA at different concentrations. Celluclast was used because, unlike Cellic™ CTEC2 (Cannella *et al,* 2012), it is devoid of LPMOs. For the control experiment, an equivalent amount of BSA was used instead of rh*Tr*LPMOA. The addition of rh*Tr*LPMOA significantly accelerated cellulose hydrolysis (Figure 10), in a rh*Tr*LPMOA-concentration dependent manner (higher LPMO-mg/cellulose-g ratio, faster hydrolysis rate). In contrast, cellulose hydrolysis in the presence of BSA was similar to that in the control reaction containing buffer (data not shown). In addition, a conversion of 60% Avicel to soluble reducing sugars in 72 h was achieved by supplementation of 15 mg rh*Tr*LPMOA per g cellulose, a 30% increase compared with the control (Figure 11).

### Construction of recombinant Y. lipolytica strains expressing different combination and ratio of cellulases

To confer optimal cellulose-degrading ability to *Y. lipolytica,* several strains were constructed, using the previously developed BGL-producing strain as a platform. Moreover, to identify the best configuration, strains were built using different enzyme combinations and protein production ratios (Table 7).

**Table 7: Comparison of cellulose utilization and biomass yield of cellulolytic Y. lipolytica grown for 120h in aerobic cultivation on CIMV-cellulose and Avicel^{a}**

| Strains | CIMV-cellulose consumed% | Biomass yield (g-DCW/g-CI MV consumed) | Avicel consumed% | Biomass yield (g-DCW/g-Avic el consumed) |
|---|---|---|---|---|
| YLC1 | 30.5 | 0.36 | 19.6 | 0.29 |
| YLC2 | 36.8 | 0.36 | 17.2 | 0.26 |
| YLC3 | 40.2 | 0.37 | 22.0 | 0.30 |
| YLC4 | 50.4 | 0.40 | 27.1 | 0.31 |
| YLC5 | 52.0 | 0.40 | 26.3 | 0.30 |
| YLC6 | 58.6 | 0.41 | 30.2 | 0.32 |
| YLC7 | 64.0 | 0.39 | 36.3 | 0.29 |

| | | | | |
|---|---|---|---|---|
| ^{a}The mean value of three independent experiments is shown and the standard deviation is below 10%. | | | | |

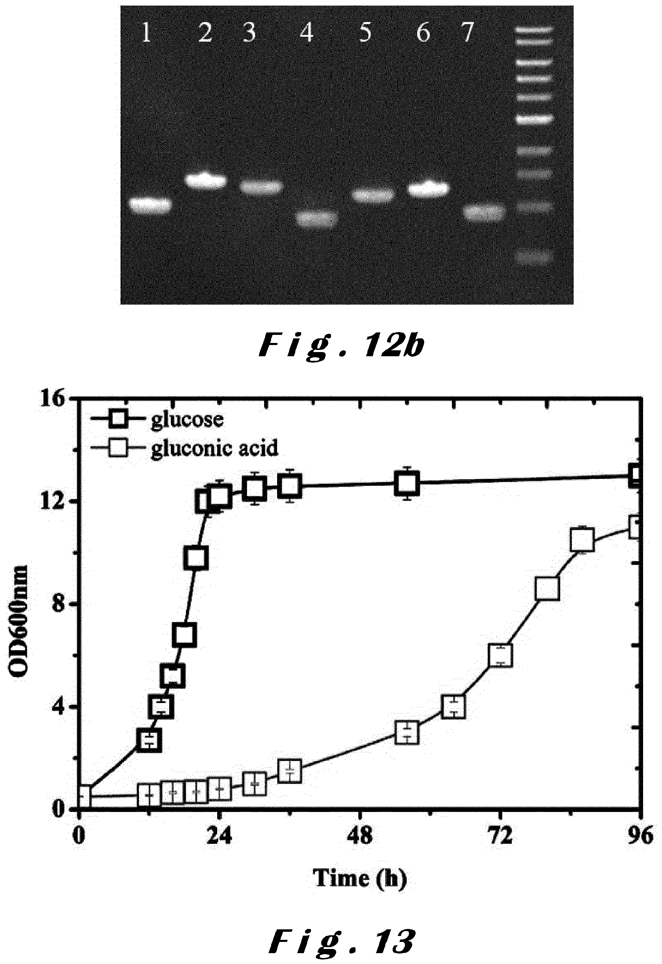

More than 10 transformants of each of the constructions were verified by PCR (Figure 12) and best individual clones were chosen based on the results of enzymatic activity assays (data not shown). Subsequently, these selected strains were cultivated in YTD medium and the hydrolytic activities of the total secretory secreted proteins were analyzed on cellulosic substrates CMC, PASC and Avicel (Figure 10). The strain YLC3, expressing two rTrEGs, displayed higher CMCase activity than YLC1 and YLC2, which express either rTrEGI or rTrEGII, alone (Figure 10). Moreover, quite predictably, strain YLC1 expressing only rTrEGI and two CBHs, was more active on Avicel than YLC2, which is a homolog that expresses rTrEGII instead of rTrEGI. Consistently, YLC2 displayed better activity on PASC. When both rTrEGI and II are present (strains YLC4-6), the hydrolysis of Avicel and PASC was enhanced, demonstrating that the presence of both EGs is necessary to achieve optimal cellulolytic activity. In contrast, the ability of strain YLC3 to hydrolyze CMC was similar to that of strains YLC4-6, implying that the basic combination of the six enzymes is sufficient to achieve optimal results with this substrate. On the other hand, increasing the expression level of either rNcCBHI (YLC4) or rTrCBHII (YLC5) using the HTEF promoter resulted in improved Avicel and PASC hydrolysis. In this respect, it is significant that the relative proportions of each cellulase component in YLC5 was predicted to be similar to that of the native cellulase system of *T. reesei* (i.e. EG I/EG II/CBH I/CBH II≈10/10/60/20 % w/w) as estimated from single cellulase production experiment and verified by in vitro activity assay on the mixture of purified enzymes. A further increase of Avicel and PASC hydrolysis was achieved by enhancing the expression levels of both NcCBHI and TrCBHII (YLC6). To further enhance the potency of cellulose hydrolysis by engineered *Y. lipolytica,* the genes encoding *Tr*LPMOA was expressed in the cellulase-producing strain YLC6 (Table 7). Testing the new daughter strain YCL7 (*Tr*LPMOA) using CMC, PASC and Avicel as substrates, revealed that the presence of *Tr*LPMOA enhanced hydrolysis of all three substrates (Figure 10). Overall, the performance of YLC7 was rather encouraging. However, accounting for the fact that Avicel and PASC are model substrates, our conclusions cannot be further extrapolated to predict the aptitude of YLC7 for use on industrial cellulose pulps, since these are generally of a much more complex nature.

### CIMV-cellulose and Avicel fermentation with recombinant Y. lipolytica strains

When TrLPMOA was expressed in strain YLC6, a further increase in CIMV-cellulose consumption up to 63% was achieved (YLC7).

In the case of YLC7, an increase in Avicel consumption to 36% was achieved. Overall, the results emphasize the important roles of helper proteins in boosting cellulose degradation with hydrolytic enzymes, especially for recalcitrant highly crystalline substrates. The proper function of LPMO requires election donor and oxygen. The addition of an external electron donor is needed (e.g. ascorbic acid in this work) when using pure cellulose as substrate, whereas this is not required in complex biomass degradation in the presence of lignin. In addition, it has been shown that when using conventional cellulase cocktails supplemented with LPMOs, enhancement of cellulose saccharification can only occur under aerobic conditions (Müller *et al,* 2015). Therefore, incorporating LPMO activity in cellulolytic *Y. lipolytica* is advantageous, because *Y. lipolytica* is a strict aerobic production host for a variety of biotechnological applications. Moreover, *Y. lipolytica* is able to assimilate gluconic acid, the C1-oxidized product by LPMO, as confirmed in this work (Figure 13).

Further improvements of cellulose degradation, especially for the recalcitrant crystalline cellulose, were achieved by introducing lytic polysaccharide monooxygenase into the cellulolytic strains described here.

### EXAMPLE 2: Expression of swollenin to improve the conversion yield of cellulose with cellulotic Yarrowia lipolytica

### Materials and Methods

### Strains and media

### Plasmid constructions

The codon-optimized gene encoding *T. reesei* swollenin of nucleotide sequence SEQ ID NO: 186 (GenBank accession code: AJ245918.1) was synthesized by GenScript (USA) and cloned into the plasmid JMP62UraTEF under the control of TEF promoter directly. This nucleotide sequence of SEQ ID NO: 186 is codon-optimized based on the codon bias of *Y. lipolytica* and start with the signal peptide of lipase 2 of *Y. lipolytica* of nucleotide sequence SEQ ID NO: 213 (pre-pro sequence of Lipase 2).

For *Y. lipolytica* transformation, vector was digested using *Not*I, thus generating a linear DNA with Zeta sequences at both extremities, and purified liberating the expression cassettes. Then the gel purified expression cassettes were introduced into *Y. lipolytica* JMY1212 Zeta for the expression of single protein, or randomly into the genome of recombinant cellulolytic strain YLC6 using the lithium acetate method (Table 8).

**Table 8: Microbial strains used in the present study**

| Strains | Relevant genotype | Source of reference |
|---|---|---|
| YLC8 | *Δpox*B12, *pTEF-TrEG I, pTEF-TrEG II; pHTEF-NcCBH I, pHTEF-TrCBH II, pTEF-TrSWO1* | This investigation |

The successful multiple integration of the heterologous genes into the genome of *Y. lipolytica* was verified by PCR using gene specific primers (Table 9, Figure 12).

**Table 9: The sequences of the oligonucleotide primers used for PCR verification of yl-transformants**

| Primer names | Sequence (5'-3') | SEQ ID NO: |
|---|---|---|
| VBGL1A | TTGACCCAGTAGCGGACCCAA | 187 |
| VBGL1B | GCCGACATTAGCCCTAACAGCAT | 188 |
| VBGL2A | GGTTGGCGGCGCATTTGT | 189 |
| VBGL2B | TGTCGTCCACTCGGCTTTCATC | 190 |
| VEGIA | CACTTGCCGTTAAGGGCGTAGGGT | 191 |
| VEGIB | CTGGCTGTTGTCGTTCCAAATGCTG | 192 |
| VEGIIA | CTTGCCGTTAAGGGCGTAGGG | 193 |
| VEGIIB | CCATCGAGCATAATTGTGGATGTCG | 194 |
| VCBHIA | TTTGCTTTGTGGTTGGGACTTTAGC | 195 |
| VCBHIB | GGTTCGAGATCCGATGTTAGTGGAGTA | 196 |
| VCBHIIA | TTTGCTTTGTGGTTGGGACTTTAGCC | 197 |
| VCBHIIB | AGGCGTTGGACCAGCCGTGA | 198 |
| V4UASCBHIA | CGCCGCAAGGAATGGTGCA | 199 |
| V4UASCBHIB | GGTTCGAGATCCGATGTTAGTGGAGTA | 200 |
| V4UASCBHIIA | CGCCGCAAGGAATGGTGCA | 201 |
| V4UASCBHIIB | CCGATCCGACTGGAGGTACTCTGGTAG | 202 |
| VLPMOA | TTGACCCAGTAGCGGACCCAACCC | 203 |
| VLPMOB | TGATCTCGTGGCGGAGCACGTAATT | 204 |
| VSWOA | TTTTGCTTTGTGGTTGGGACTTTAGCC | 205 |
| VSWOB | GGGTGTAGTAGTCGCCGTTGGGAGC | 206 |

### Study the synergy of SWO1 on enzymatic hydrolysis of Avicel

Enzymatic hydrolysis of cellulose was carried out in 50 mM citrate buffer (pH 4.8) containing 2.0 % (w/v) microcrystalline cellulose (Avicel PH-101, Sigma). Celluclast 1.5L (60 FPU/mL) and β-glucosidase (810 IU/mL, Novozyme 188, gift from Novozyme, Denmark) was added at 5.0 U/g-cellulose and 12.0 IU/g-cellulose, respectively. The reaction (1 mL) was conducted in Eppendorf tubes (2 mL) using a thermomixer at 50 °C and 1000 rpm. To study the synergistic effect of swollenin on the enzymatic hydrolysis of cellulose, this protein was either added into reaction mixture together with cellulases at different concentrations (5, 10 and 15 mg-swollenin/g-cellulose respectively) or, incubated with the Avicel at 50 °C under shaking for 24 h prior to the addition of cellulases. Control experiments were conducted under the same conditions, with or without, BSA (at 5, 10 and 15 mg-protein/g-cellulose). Samples were taken at regular intervals to determine the concentration of reducing sugars.

### Growth of yeast expressing multiple cellulases on CIMV and Avicel cellulose

Yeast growth on CIMV-cellulose and Avicel was performed in 50 mL defined medium containing 27 g/L CIMV or 25 g/L Avice cellulose in 250 mL Erlenmeyer flasks. Yeasts were pre-cultivated in defined media until mid exponential phase and the cells were collected by centrifugation. After washing with deionized water, the cells wereused to inoculate the defined medium to yield an initial biomass concentration at 1.0 g-DCW/L. In addition, CIMV and Avicel cellulose were incubated with swollenin at 50 °C under shaking for 24 h prior to the inoculation of strain YLC7. All the cultivations were conducted at 28°C and samples were taken at the end of 5 days to determine concentrations of biomass and residual cellulose.

### Results and discussion

### Expression of Swollenin in Y. lipolytica

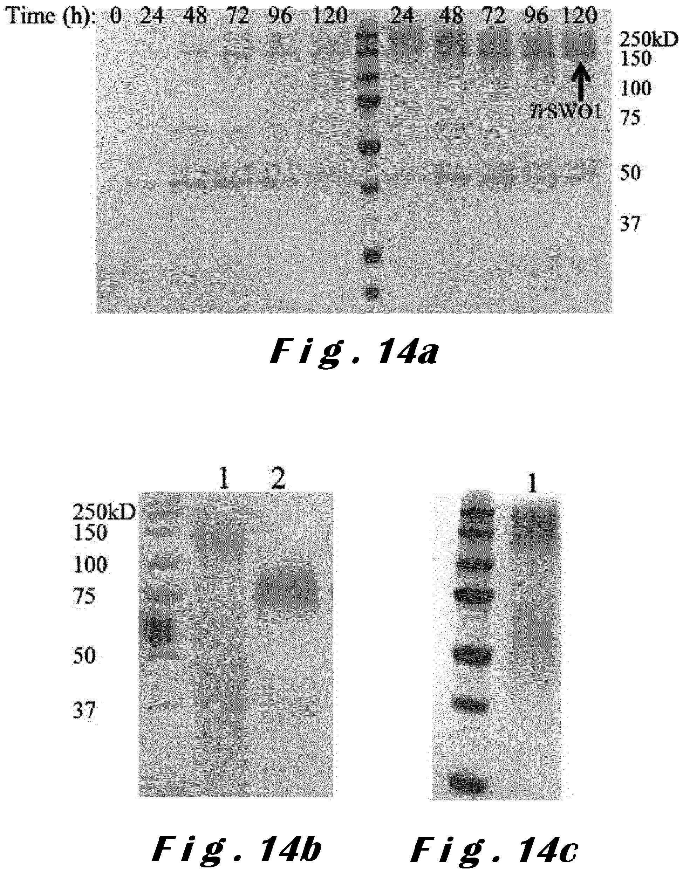

SWO1 was first expressed alone in *Y. lipolytica* and then investigated how it could be co-expressed in *Y. lipolytica* with cellulases. To facilitate protein detection and purification, *Tr*SWO1 was produced in *Y. lipolytica* as His-tagged protein (rh*Tr*SWO1) using the TEF promoter and the *Y. lipolytica* lipase 2 pre-pro region. SDS-PAGE analysis of culture supernatants of *Y. lipolytica* transformants containing rh*Tr*SWO1 revealed the presence of a discrete species migrating to a position corresponding to an approximate Mw of 200 kDa compared to that of a control culture, which is much higher than the actual expected Mw of rh*Tr*SWO1 (50 kDa) (Figure 14a). Further Western blot analysis using anti-His antibody confirmed that these protein species were His-tagged (Figure 14b). Taken together, these observations suggest that the recombinant proteins are glycosylated forms of rh*Tr*SWO1, in agreement with previous results (Eibinger *et al,* 2016; Jäger *et al,* 2011). Further analysis of the sequence of SWO1 using NetNGlyc 1.0 predicted that this protein bears 3 potential N-glycosylation sites. To confirm the presence of N-glycosylation, Endo-H treatment followed by Western blot analysis were performed. This revealed that the EndoH-treated protein samples were still detected using anti-His antibodies, and a discrete protein species of approximately 75 kDa appeared for rh*Tr*SWO1 (Figure 14b), which is consistent with the Mw of native SWO1 expressed by *T. reesei (*Eibinger *et al,* 2016). Therefore, the difference between the theoretical Mw of SWO1 and the observed Mw of rh*Tr*SWO1 may be due to the presence of O-glycosylation and other post-translational modifications (Jäger *et al,* 2011). In order to further investigate the functionality of rh*Tr*SWO1, the recombinant SWO1 was purified using immobilized metal ion affinity chromatography (IMAC) (Figure 14c).

### The synergy of SWO1 on enzymatic hydrolysis of Avicel

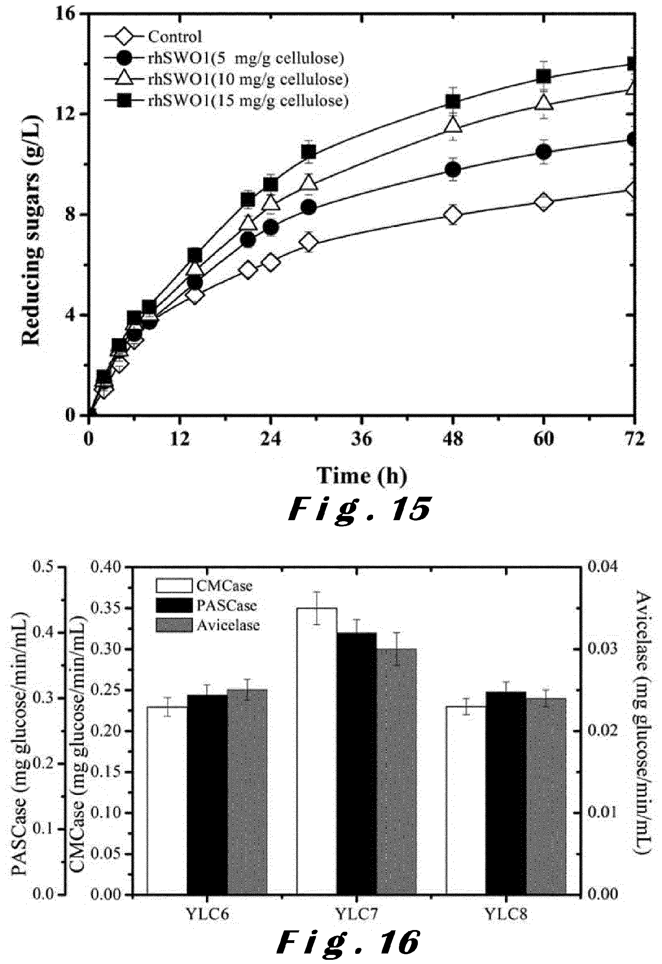

To investigate synergy between helper protein and cellulases, enzymatic hydrolysis of Avicel was performed using a commercial cellulase cocktail (Cellulcast) supplemented with the *Tr*SWO1 at different concentrations. For the control experiment, an equivalent amount of BSA was used instead of rh*Tr*SWO1. The investigation is conducted by first incubating Avicel with swollenin (5 to 15 mg protein/g cellulose) for 24 h. Consistent with the non-enzymatic nature of swollenin, no reducing sugars were detectable at the end of this period, however upon addition of the cellulose cocktail hydrolysis was enhanced, with 70% of Avicel being converted to soluble reducing sugars after 72 h in the reaction containing 15 mg swollenin/g cellulose (Figure 15). Significantly, this enhancement of hydrolysis (1.5-fold compared to the control) by swollenin was more potent than the increase in hydrolysis procured by a similar amount (g protein) of rh*Tr*LPMOA. Importantly, further increases in swollenin concentration (e.g. use of 20 mg/g cellulose) did not procure more hydrolysis, suggesting that 15 mg *Tr*SWO1/g cellulose is a near optimal loading (data not shown). Taken together, these results indicate that swollenin adsorption is a prerequisite for amorphogenesis and that this governed by a time-dependant equilibrium. For the optimal amorphogenesis of Avicel, it is no doubt necessary to leave sufficient time for several cycles of adsorption/desorption (Jäger *et al,* 2011; Jalak et al., and Väljamäe et al., 2010).

### CIMV-cellulose and Avicel fermentation with recombinant Y. lipolytica strains

Strain YLC8 expressing all the essential cellulase components and TrSWO1 consumed similar amount of CIMV-cellulose and Avicel to that of YLC6 (Table 10). Even though swollenin pretreatment of CIMV-cellulose did not yield obvious change on its fermentation with strain YLC7, incubation of Avicel with swollenin prior to fermentation greatly enhanced the degradation of this substrate and a consumption of 45% of Avicel by strain YLC7 was achieved in 5 days (Table 10). Overall, the results emphasize the important roles of helper proteins in boosting cellulose degradation with hydrolytic enzymes, especially for recalcitrant highly crystalline substrates. The advent of swollenin has a great potential, but requires rethinking of industrial bioprocessing procedures. The contribution of swollenin to overall efficiency in cellulose conversion may be large if the process conditions are further adapted to the key determinants of swollenin function, namely pretreatment time, temperature, protein dosages, according to the type of biomass.

**Table 10: Comparison of cellulose utilization and biomass yield of cellulolytic Y. lipolytica grown for 120h in aerobic cultivation on CIMV-cellulose and Avicel^{a}**

| Strains | CIMV-cellulose consumed% | Biomass yield (g-DCW/g-CI MV consumed) | Avicel consumed% | Biomass yield (g-DCW/g-Avic el consumed) |
|---|---|---|---|---|
| YLC6 | 58.6 | 0.41 | 30.2 | 0.32 |
| YLC8 | 59.0 | 0.40 | 31.0 | 0.31 |
| ^{b}YLC7+ S | 64.2 | 0.38 | 45.0 | 0.30 |

| | | | | |
|---|---|---|---|---|
| ^{a}The mean value of three independent experiments is shown and the standard deviation is below 10%. ^{b}The cellulose was treated by SWO1 at the dosage of 15 mg/g cellulose for 24 h before enzymatic hydrolysis. | | | | |

Further improvements of cellulose degradation, especially for the recalcitrant crystalline cellulose, was achieved by incubating the cellulose with recombinant swollenin.

### References:

Adav SS, et al. 2012; Mol Cell Proteomics. 11 (7).
Baker JO, et al. 1998; Appl Biochem Biotechnol. 70-72:395-403.
Bennati-Granier C, et al. 2015 ; Biotechnol Biofuels. 8:90.
Beopoulos A, et al. 2008; Appl. Environ. Microbiol. 74:7779-7789.
Bey M, et al. 2013 ; Appl Environ Microbiol. 79,488-496
Blazeck J, et al. 2011; Appl. Environ Microbiol. 77: 7905-7914.
Blazeck J, et al. 2013; Appl. Microbiol Biotechnol. 97:3037-3052.
Blazeck J, et al. 2014; Nat. Commun. 5:3131.
Boonvitthya N, et al. 2013; Mol Biotechnol. 54:158-169.
Bordes F, et al. 2007; J Microbiol Methods. 70:493-502.
Bradford MM, 1976; Anal Biochem. 72:248-254.
Brethauer S, et al. 2014; Energy Environ Sci. 7:1446-1453.
Cannella D, et al. 2014; Biotechnol Bioeng. 111(1):59-68.
Cannella D, et al. 2012; Biotechnol Biofuels. 5(1):26.
Chauhan JS, et al. 2013; PLoS ONE. 8:e67008.
Delmas M, et al. 2008; Chem Eng Technol [Internet]. WILEY-VCH Verlag; 31:792-7.den
Duquesne S, et al. 2012; Methods Mol Biol. 861: 301-312.
Duquesne S, et al. 2014; PLoS ONE. 9:e95128.
Eibinger M, et al. 2016; Biotechnol Biofuels. 9(1):178.
Fabre E, et al., 1991. J Biol Chem., 266:3782-3790.
Fakas S, et al., 2006; Appl Microbiol Biotechnol., 73:676-683.
Fickers P, et al. 2003; J Microbiol Methods. 55:727-737.
Frank K and Sippl MJ, 2008; Bioinformatics. 24:2172-2176.
Gaillardin C, et al., 1987; Curr Genet., 11:369-375.
Gasmi N, et al., 2011; Appl Microbiol Biotechnol. 89:109-119.
Gilbert HJ, et al, 2010; Plant Physiol. 153(2):444-55.
Ghose TK, et al., 1987; Pure Appl Chem. 59:257-268.
Gourlay K, et al., 2013; Bioresour Technol. 142:498-503.
Groenewald M, et al. 2014; Crit Rev Microbiol. 40:187-206.
Guo X, et al., 2000; J. Appl. Microbiol., 89:107-115.
Guo ZP, et al., 2011; Enzyme Microb. Technol. 49:105-112.
Guo Z, et al. 2015; Biotechnol Biofuels. 8:109.
Gysler C, et al., 1990; Biotechn Techn., 4:285-290.
Haan R, et al. 2015; Curr Opin Biotechnol. 33:32-38.
Hashimoto Y, et al., 1998; Protein Engineering. 11:75-77.
Hedfalk K. et al., 2012; Optimisation for Heterologous Gene Expression in Yeast'. In Springer Protocols :Methods in Molecular Biology. Recombinant protein production in yeast :methods and protocols. Volume 866 pp. 47-55. Springer Eds.
Hemsworth GR, et al., 2013; J Am Chem Soc. 135,6069-6077.
Horn SJ, et al., 2012; Biotechnol Biofuels. 5(1):45.
Hu J, et al., 2015; Bioresour Technol. 186:149-53.
Ito H, et al., 1983; J Bacteriol., 153:163-168.
Isaksen T, et al., 2014; J Biol Chem., 289(5):2632-42.
Jäger G, et al., 2011; Biotechnol Biofuels. 4(1):33.
Kittl R, et al., 2012; Biotechnol Biofuels. 5:79.
Klebe RJ, et al., 1983; Gene, 25:333-341.
Koganesawa N, et al., 2001 ; Protein Eng. 14:705-710.
La Grange DC, et al. 2010; Appl Microbiol Biotechnol. 87:1195-1208.
Lazar Z, et al., 2014, Metab. Eng. 2689-99.
Le Dal MT, et al, 1994; Curr Genet., 26:38-44.
Madzak C, et al., 2004; J Biotechnol. 109:63-81.
Madzak C and Beckerich JM, 2013; Heterologous protein expression and secretion in Yarrowia lipolytica. In : Yarrowia lipolytica: Biotechnological Applications, Microbiology Monographs Vol. 25. Ed : Barth G, Springer, Heidelberg, Germany. pp. 1-76.
Menon V, et al., 2012; Progr Energy Combust Sci. 38, 522-550.
Muller S, et al., 1998; Yeast, 14:1267-1283.
Müller G, et al., 2015; Biotechnol Biofuels 8:187.Nicaud J-M, et al., 2002; FEMS Yeast Res. 2:371-379.
Olson DG, et al. 2012; Curr Opin Biotechnol. 23:396-405.
Orr-Weaver TL, et al., 1981; Proc. Natl. Acad. Sci. USA, 78:6354-6358.
Papanikolaou S, et al., , 2001; Antonie van Leeuwenhoek 80:215-224.
Papanikolaou S, et al., 2002; J. Appl. Microbiol., 92:737-744.
Papanikolaou S, et al., 2008; Bioresour. Technol., 99:2419-2428.
Park CS, et al. 2000; Appl Biochem Biotechnol. 87:1-15.
Petersen TN, et al., 2011; N. Methods. 8:785-786.
Pignède G, et al., 2000; J. Bacteriol. 182:2802-2810.
Phillips CM, et al., 2011; ACS Chem Biol. 2011.6,1399-1406.
Ratledge C, et al., 1994. Yeasts, moulds, algae and bacteria as sources of lipids. Technological advances in improved and alternative sources of lipids. B. S. Kamel, Kakuda, Y, et al., London, Blackie academic and professional, 235-291.
Ratledge C, et al., 2005; Single cell oils for the 21th century, in:Cohen, R. (Eds.), Single cell oils. AOCS Press, Champaign, pp. 1-20.
Ryu S, , et al., 2011; Appl Microbiol Biotechnol. 91:529-542.
Sreekrishna K, , et al., 1997; Gene. 190:55-62.
Taleb F, et al., 1995; Gene. 161:137-138.
Tatusova TA, , et al., 1999; FEMS Microbiol. Lett. 174:247-250.
Thevenieau F, et al., 2013; OCL. 20:1-8.
Valle-Rodriguez JO, et al., 2014; Appl. Energy. 115, 226-232.
Vaaje-Kolstad G, et al., 2010; Science. 330:219-222.
Verduyn C, et al. 1992; Yeast. 8:501-517.
Von Heijne G, et al., 1985; J Mol Biol. 184: 99-105.
Vu VV, et al., 2014 ; J Am Chem Soc. 136(2):562-5.
Wei H, et al. 2014; Biotechnol Biofuels. 7:148.
Yanase S, et al. 2010; Appl Microbiol Biotechnol. 88:381-388.
Zhang YHP, et al. 2006; Biomacromolecules. 7:644-648.
Zhang Y-HP, et al. 2006; Biotechnol. Bioeng. 94:888-898.

## Claims

1. A method for obtaining an oleaginous yeast strain capable of growing on cellulose as carbon source, wherein said method comprises overexpressing in said strain
- a β-glucosidase 1 (BGL 1; EC 3.2.1.21) having at least 80% identity with the polypeptide of sequence SEQ ID NO: 1 further comprising a N-terminal signal peptide,
- a β-glucosidase 2 (BGL 2; EC 3.2.1.21) having at least 80% identity with the polypeptide of sequence SEQ ID NO: 2 further comprising a N-terminal signal peptide,
- an endoglucanase I (EG I; EC 3.2.1.4) having at least 55% identity with the polypeptide of sequence SEQ ID NO: 3 (*Tr*EG I), further comprising a N-terminal signal peptide,
- an endoglucanase II (EG II; EC 3.2.1.4) having at least 55% identity with the polypeptide of sequence SEQ ID NO: 4 (*Tr*EG II), further comprising a N-terminal signal peptide,
- a cellobiohydrolase I (CBH I; EC 3.2.1.91) having at least 65% identity with the polypeptide of sequence SEQ ID NO: 5 (*Nc*CBH I), further comprising a N-terminal signal peptide and
- a cellobiohydrolase II (CBH II; EC 3.2.1.91) having at least 60% identity with the polypeptide of sequence SEQ ID NO: 6 (*Tr*CBH II) further comprising a N-terminal signal peptide.

2. The method according to claim 1, wherein said method further comprises overexpressing in said strain, a lytic polysaccharide monooxygenase having at least 51% identity with the polypeptide of sequence SEQ ID NO: 7.

3. The method according to claim 1 or claim 2, wherein:
- the β-glucosidase 1 is selected from the group consisting of SEQ ID NO: 1 and SEQ ID NO: 15,
- the β-glucosidase 2 has the consensus amino acid sequence SEQ ID NO: 16 or is selected from the group consisting of SEQ ID NO: 2, SEQ ID NO: 17, SEQ ID NO: 18 and SEQ ID NO: 19,
- the N-terminal signal peptide of the BGL 1 and the N-terminal signal peptide of the BGL 2 are identical or different, and are independently selected from the group consisting of SEQ ID NO: 26 to 31,
- the endoglucanase I has three conserved domains in catalytic core domain of consensus amino acid sequences SEQ ID NO: 32, SEQ ID NO: 33 and amino acid sequence SEQ ID NO: 34 or is selected from the group consisting of SEQ ID NO: 3 and SEQ ID NO: 35 to SEQ ID NO: 52,
- the endoglucanase II has two conserved domains in catalytic core domain of consensus amino acid sequences SEQ ID NO: 53 and SEQ ID NO: 54, or is selected from the group consisting of SEQ ID NO: 4 and SEQ ID NO: 55 to SEQ ID NO: 69,
- the cellobiohydrolase I has two conserved domains in catalytic core domain of consensus amino acid sequences SEQ ID NO: 70 and SEQ ID NO: 71, or is selected from the group consisting of SEQ ID NO: 5 and SEQ ID NO: 72 to SEQ ID NO: 86,
- the cellobiohydrolase II has two conserved domains in catalytic core domain of consensus amino acid sequences SEQ ID NO: 87 and SEQ ID NO: 88, or is selected from the group consisting of SEQ ID NO: 6 and SEQ ID NO: 89 to SEQ ID NO: 106, and optionally,
- the lytic polysaccharide monooxygenase has a conserved domain in catalytic core domain of the consensus amino sequence SEQ ID NO: 107, or is selected from the group consisting of SEQ ID NO: 7, SEQ ID NO: 108 to SEQ ID NO: 120,
- the N-terminal signal peptide of the EG I, the N-terminal signal peptide of the EG II, the N-terminal signal peptide of the CBH I, the N-terminal signal peptide of the CBH II, the N-terminal signal peptide of the LPMOA, of sequence SEQ ID NO: 121.

4. The method according to any one of claims 1 to 3, wherein BGL 1 and/or BGL 2 are from an oleaginous yeast strain and/or EG I and/or EG II and/or CBH I and/or CBH II and/or LPMOA is from a fungus or yeast strain, .

5. The method of any one of claims 1 to 4, wherein the oleaginous yeast strain is selected from the group consisting of the genus *Candida, Cryptoccocus, Lipomyces, Rhodosporidium, Rhodotorula, Trichosporon, Yarrowia,* the fungus is selected from the group consisting of the genus *Trichoderma, Hypocrea, Thielavia, Dactylellina, Myceliophthora, Aspergillus, Neosartorya, Penicillium, Aspergillus, Rosellinia, Phialophora, Botryotinia, Jaapia, Cylindrobasidium, Polyporus, Ceriporiopsis, Phlehiopsis, Neurospora, Sordaria, Acremonium, Myceliophthora, Humicola, Chaetomium, Podospora, Scedosporium, Togninia, Gibberella, Gaeumannomyces, Fusarium, Bionectria, Colletotrchum, Stachybotrys, Nectria, Neonectria, Pestalotiopsis, Verticillium, Pochonia, Eutypa* or *Madurella.*

6. The method of claim 5, wherein the oleaginous yeast strain is *Yarrowia lipolytica.*

7. The method of any one of claims 1 to 6, wherein the expression or activity of the endogenous isoforms of acyl-coenzymeA oxidases in said oleaginous yeast strain is inhibited.

8. The method of claim 7, wherein said oleaginous yeast strain is a *Yarrowia* strain and wherein in said strain at least one protein selected from the group consisting of an acyl-CoA:diacylglycerol acyltransferase 2, an acyl-CoA:diacylglycerol acyltransferase 1, a glycerol-3-phosphate dehydrogenase NAD+, an acetyl-CoA carboxylase and a hexokinase is overexpressed, and/or the expression or activity of at least one endogenous protein selected from the group consisting of the glycerol 3-phosphate dehydrogenase, the triglyceride lipase and the peroxin 10 is inhibited.

9. The method of any one of claims 1 to 8, wherein it comprises transforming an oleaginous yeast cell with one or several recombinant DNA constructs for expressing a β-glucosidase 1, a β-glucosidase 2, an endoglucanase I, an endoglucanase II, a cellobiohydrolase I, a cellobiohydrolase II and optionally LPMOA as defined in any one of claims 1 to 4.

10. An oleaginous yeast strain genetically transformed with one or several recombinant DNA constructs for expressing a β-glucosidase 1, a β-glucosidase 2, an endoglucanase I, an endoglucanase II, a cellobiohydrolase I, a cellobiohydrolase II and optionally LPMOA as defined in any one of claims 1 to 4, and overexpressing said β-glucosidase 1, β-glucosidase 2, endoglucanase I, endoglucanase II, cellobiohydrolase I, cellobiohydrolase II and optionally LPMOA.

11. A mutant oleaginous yeast strain comprising, stably integrated in its genome, one or several recombinant DNA constructs for expressing a β-glucosidase 1, a β-glucosidase 2, an endoglucanase I, an endoglucanase II, a cellobiohydrolase I, a cellobiohydrolase II and optionally LPMOA as defined in in any one of claims 1 to 4.

12. Use of a mutant oleaginous yeast strain as defined in claims 10 or 11 for producing lipids from lignocellulosic biomass, in particular from cellulose.

13. A method of producing lipids, comprising a step of growing a mutant oleaginous yeast strain as defined in claim 10 or claim 1, and optionally a step of pre-treatment in presence of swollenin, on a lignocellulosic biomass, in particular on cellulose.

14. A method for enhancing the extracellular expression of a protein in a yeast strain comprising the step of placing in said yeast strain the encoding sequence of said protein under the control of the hybrid promotor HTEF of SEQ ID NO: 122.

15. Use of the hybrid promotor HTEF of SEQ ID NO: 122 for enhancing the extracellular expression of a protein in a yeast strain, wherein the encoding sequence of said protein is placed under the control of said hybrid promotor HTEF.
